# EUROPEAN PATENT APPLICATION

(11) **EP 4 792 811 A1**
(43) Date of publication of application: **19.08.2026**
(21) Application number: 24876520.8
(22) Date of filing: 08.10.2024
(51) Int. Cl.: A61K 31/40, A61K 31/397, A61K 31/401, A61K 31/675, A61P 21/00

(54) **S1PR4 REGULATOR COMPOUND FOR MUSCLE DISEASE TREATMENT AND APPLICATION THEREOF**

(30) Priority: 08.10.2023 CN 202311292806; 14.09.2024 CN 202411294698
(71) Applicant: Shengyuan Zetong (Shanghai) Pharmaceutical Co., Ltd, Shanghai 201100 (CN)
(72) Inventor: LI, Honglin, Shanghai 200241 (CN); QIAN, Xuhong, Shanghai 200241 (CN); WANG, Rui, Shanghai 200237 (CN); ZHAO, Zhenjiang, Shanghai 200237 (CN); XU, Yufang, Shanghai 200237 (CN); HE, Huan, Shanghai 200241 (CN); GAO, Wei, Shanghai 200241 (CN); XIAO, Yunping, Shanghai 200237 (CN); YE, Tianyu, Shanghai 200237 (CN)
(74) Representative: Mewburn Ellis LLP
(86) International application number: PCT/CN2024/123498
(87) International publication number: WO 2025/077706

(57) **Abstract**

Disclosed are a compound as shown in formula I and an application thereof as an S1PR regulator, especially a selective S1PR4 agonist in the treatment of a muscle disease. The compound can treat related diseases mediated by S1PR4 and mutants thereof. < img file = "PCT/CN2024123498-ISRE-I0000001 JPG" he = "15.08" img-content = "drawing" img-format = "jpg" in line = "YES" orientation = "portraitit" wi = "37.57"/>

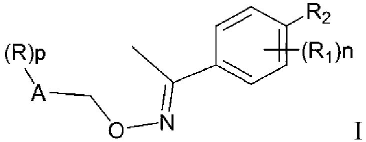

## Description

### Technical field

The present invention relates to the field of pharmacy. Specifically, the present invention relates to novel compounds of S1PR4 regulators for the prevention and treatment of myopathy and uses thereof.

### Background

Muscular dystrophy is a progressive muscle disease caused by genetic mutations, characterized by chronic oxidative stress and inflammation. Clinically, it manifests as progressive muscle weakness, affecting limb, pharyngeal, and facial muscles to varying degrees. Based on the onset time, it can be divided into congenital muscular dystrophy and late-onset muscular dystrophy. Generally, congenital muscular dystrophy shows obvious muscle weakness symptoms at birth or in the first few months after birth. While late-onset muscular dystrophy presents with muscle weakness symptoms only after the patient has achieved independent walking. Currently, various protein mutations involved in the development and maintenance of skeletal muscles have been identified in patients with muscular dystrophy, including Laminin α2, Fukutin, Selenoprotein N1, etc., which play important roles in prenatal skeletal muscle development and function. Mutations in these proteins can lead to congenital muscular dystrophy. Proteins, such as Dystrophin, Emerin, and Lamin A/C are involved in the maintenance of skeletal muscles, and mutations in them can cause late-onset muscular dystrophy.

Currently, the main treatment strategies for muscular dystrophy are gene therapy and antiinflammatory therapy. Gene therapy uses small molecules or antisense oligonucleotides to inhibit nonsense mutations that cause abnormal termination of transcription or induce skipping of specified exons, thereby restoring the reading frame and producing dystrophin. Alternatively, adeno-associated viruses are used as vectors to introduce a normal wild-type gene for the expression of dystrophin. However, the limitations of gene therapy include immune reactions to the vector and the limited packaging size of the vector. In addition, gene therapy is only effective for patients with specific pathogenic gene variants and cannot be applied to all patients with muscular dystrophy. Inflammation is a secondary result caused by gene variants. The advantage of inhibiting inflammation is that it can be applied to all subtypes of muscular dystrophy, regardless of the variant gene. Glucocorticoids have been used to reduce inflammation levels in the treatment of muscular dystrophy, but their efficacy is limited and they have significant side effects, so we need to find new inflammatory targets.

Sphingosine-1-phosphate receptors (S1PRs) belong to the GPCR family and have 5 subtypes, S1PR1-5, which mediate various physiological processes regulated by their endogenous ligand sphingosine 1-phosphate (S1P). Among them, S1PR1 regulates the transport of lymphocytes from secondary lymphoid organs to the blood and lymph flow, and is an important and emerging drug target for the treatment of various autoimmune diseases. S1PR4 is related to the migration and differentiation of immune cells, the migration of skeletal muscle precursor cells, platelet regeneration, and TGFβ1-mediated inhibition of skeletal muscle cell apoptosis. Targeting S1PR4 can effectively treat autoimmune diseases, cancer, atherosclerosis, and diseases caused by accelerated platelet aggregation, showing good application prospects. In addition, targeted regulation of S1PR4 also has potential application value for muscle diseases caused by S1PR4 and its mutants.

Currently, research on myopathies is insufficient. The underlying causes of different types of myopathies are related to relevant gene mutations. Therefore, to find drugs for treating myopathies, it is first necessary to understand and confirm the targets that cause such myopathies, and then discover therapeutic drugs based on the mutant proteins.

Therefore, researching targets for myopathy and further developing therapeutic drugs thereof have significant clinical significance and application prospects in the treatment of muscle diseases and autoimmune diseases.

### Summary of the invention

The purpose of the present invention is to provide related compounds as S1PR regulators, particularly S1PR4 agonists.

Another purpose of the present invention is to provide pharmaceutical compositions containing the above-mentioned compounds.

Yet another purpose of the present invention is to provide the use of the above-mentioned compounds or pharmaceutical compositions in the preparation of drugs for treating S1PR4-related diseases or regulating S1P receptors.

In the first aspect, the present invention provides a compound of formula I, or an optical isomer or a pharmaceutically acceptable salt thereof: 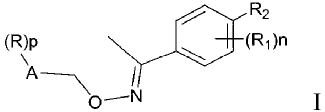
A is selected from: a substituted or unsubstituted C₅-C₈ aromatic ring (such as phenyl) or C₈-C₁₄ fused aromatic ring (such as naphthyl), substituted or unsubstituted five-membered or six-membered heterocyclic group containing 1-3 heteroatoms selected from N or O;
R is independently selected from the group consisting of a hydrogen, halogen, cyano, substituted or unsubstituted C₁-C₁₀ alkyl (for example, a methyl, trifluoromethyl, trifluoroethyl), substituted or unsubstituted C₃-C₈ cycloalkyl or cycloalkenyl, substituted or unsubstituted C₁-C₅ alkoxy, substituted or unsubstituted C₅-C₈ aryl (for example, phenyl), substituted or unsubstituted five-membered or six-membered heterocyclic group or heteroaryl containing 1-2 heteroatoms selected from N, O or S, substituted or unsubstituted C₁-C₁₀ alkyl formyl, substituted or unsubstituted C₅-C₈ aryl formyl; p is an integer from 1 to 3;
R¹ is selected from the group consisting of a hydrogen, halogen, cyano, substituted or unsubstituted C₁-C₅ alkoxy, substituted or unsubstituted C₁-C₁₀ alkyl, substituted or unsubstituted C₃-C₈ cycloalkyl, substituted or unsubstituted C₂-C₆ alkenyl, substituted or unsubstituted C₃-C₆ cycloalkenyl, substituted or unsubstituted C₃-C₈ lactone group, substituted or unsubstituted C₁-C₁₀ amide group, substituted or unsubstituted C₅-C₈ aryl, substituted or unsubstituted five-membered or six-membered heterocyclic group containing 1-2 heteroatoms selected from N, O or S, substituted or unsubstituted C₅-C₈ aromatic heterocyclic group;
n is an integer selected from 1-3;
R² is selected from the following group: 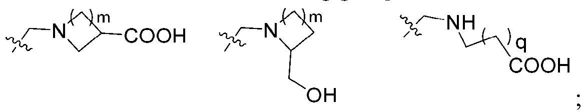
m is an integer selected from 1-4;
q is an integer selected from 1-2.

In a preferred embodiment, the compound is not the following compound:

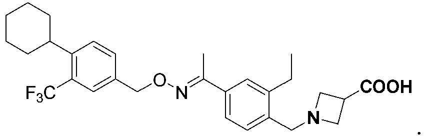

In a specific embodiment, the compound is represented by the following formula II: 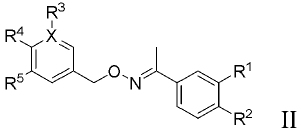 wherein,
R¹ is selected from the group consisting of a hydrogen, halogen (preferably F, Cl or Br), cyano, substituted or unsubstituted C₁-C₅ alkoxy (preferably methoxy), substituted or unsubstituted C₁-C₁₀ alkyl (preferably a substituted or unsubstituted C₁-C₆ alkyl, such as a methyl, ethyl, trifluoromethyl), substituted or unsubstituted C₃-C₈ cycloalkyl, substituted or unsubstituted C₂-C₆ alkenyl, substituted or unsubstituted C₃-C₆ cycloalkenyl, substituted or unsubstituted C₅-C₈ aryl, substituted or unsubstituted five-membered or six-membered heterocyclic group containing 1-2 heteroatoms selected from N, O or S, and substituted or unsubstituted C₅-C₈ aromatic heterocyclic group;
R² is selected from the following group: 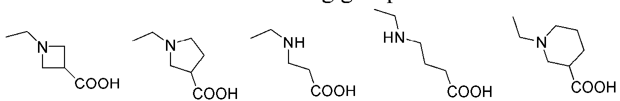
X is selected from C or N;
R³ is not present or is a substituent selected from the following group: a hydrogen, cyano, halogen, substituted or unsubstituted C₁-C₁₀ alkyl (for example, a methyl, trifluoromethyl, trifluoroethyl), substituted or unsubstituted C₃-C₈ cycloalkyl, cyano, substituted or unsubstituted C₁-C₅ alkoxy, substituted or unsubstituted C₁-C₁₀ alkyl formyl, substituted or unsubstituted C₅-C₈ aryl formyl, substituted or unsubstituted C₅-C₈ aryl, substituted or unsubstituted five-membered or six-membered heterocyclic group containing 1-2 heteroatoms selected from N, O or S;
R⁵ is selected from the group consisting of a hydrogen, cyano, halogen, substituted or unsubstituted C₁-C₁₀ alkyl (for example, a methyl, trifluoromethyl, trifluoroethyl), substituted or unsubstituted C₃-C₈ cycloalkyl, cyano, substituted or unsubstituted C₁-C₅ alkoxy, substituted or unsubstituted C₁-C₁₀ alkyl formyl, substituted or unsubstituted C₅-C₈ aryl formyl, substituted or unsubstituted C₅-C₈ aryl, substituted or unsubstituted five-membered or six-membered heterocyclic group containing 1-2 heteroatoms selected from N, O or S;
R⁴ is selected from the group consisting of a hydrogen, halogen, substituted or unsubstituted C₁-C₁₀ alkyl, substituted or unsubstituted C₃-C₈ cycloalkyl or cycloalkenyl, substituted or unsubstituted C₅-C₈ aryl, substituted or unsubstituted five-membered or six-membered heterocyclic group or heteroaryl groups containing 1-2 heteroatoms selected from N, O or S, substituted or unsubstituted C₁-C₁₀ alkyl formyl, and substituted or unsubstituted aryl formyl.

In a specific embodiment, the compound is represented by the following formula III: 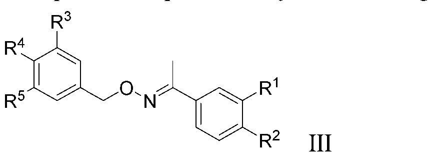 wherein,
R¹ is selected from the following group: a hydrogen, halogen (preferably F, Cl, or Br), cyano, substituted or unsubstituted C₁-C₅ alkoxy (preferably a methoxy), substituted or unsubstituted C₁-C₁₀ alkyl (preferably a substituted or unsubstituted C₁-C₆ alkyl, such as a methyl, ethyl, trifluoromethyl), substituted or unsubstituted C₃-C₈ cycloalkyl, substituted or unsubstituted C₂-C₆ alkenyl, substituted or unsubstituted C₃-C₆ cycloalkenyl, substituted or unsubstituted C₅-C₈ aryl, substituted or unsubstituted five-membered or six-membered heterocyclic group containing 1-2 heteroatoms selected from N, O, or S, substituted or unsubstituted C₅-C₈ aromatic heterocyclic group;
R² is selected from the following group: 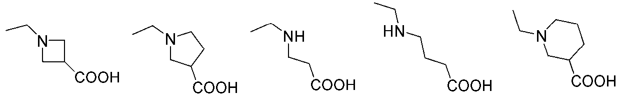
R³ is selected from the group consisting of a hydrogen, cyano, halogen, substituted or unsubstituted C₁-C₁₀ alkyl (for example, a methyl, trifluoromethyl, trifluoroethyl), substituted or unsubstituted C₃-C₈ cycloalkyl, cyano, substituted or unsubstituted C₁-C₅ alkoxy, substituted or unsubstituted C₁-C₁₀ alkyl formyl, substituted or unsubstituted C₅-C₈ aryl formyl, substituted or unsubstituted C₅-C₈ aryl, substituted or unsubstituted five-membered or six-membered heterocyclic group containing 1-2 heteroatoms selected from N, O or S;
R⁵ is selected from the following group: a hydrogen, cyano, halogen, substituted or unsubstituted C₁-C₁₀ alkyl (for example, a methyl, trifluoromethyl, trifluoroethyl), substituted or unsubstituted C₃-C₈ cycloalkyl, cyano, substituted or unsubstituted C₁-C₅ alkoxy, substituted or unsubstituted C₁-C₁₀ alkyl formyl, substituted or unsubstituted C₅-C₈ aryl formyl, substituted or unsubstituted C₅-C₈ aryl, substituted or unsubstituted five-membered or six-membered heterocyclic group containing 1-2 heteroatoms selected from N, O or S;
R⁴ is selected from the group consisting of a hydrogen, halogen, substituted or unsubstituted C₁-C₁₀ alkyl, substituted or unsubstituted C₃-C₈ cycloalkyl or cycloalkenyl, substituted or unsubstituted C₅-C₈ aryl, substituted or unsubstituted five-membered or six-membered heterocyclic group or heteroaryl containing 1-2 heteroatoms selected from N, O or S, substituted or unsubstituted C₁-C₁₀ alkyl formyl, and substituted or unsubstituted aryl formyl.

In a specific embodiment, the present invention provides a compound selected from the following group, or an optical isomer or a pharmaceutically acceptable salt thereof:

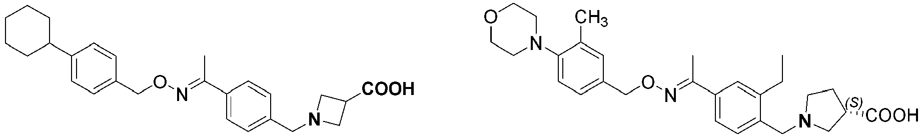

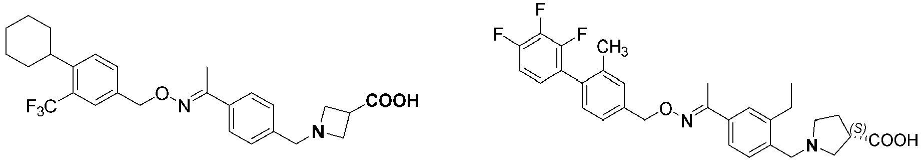

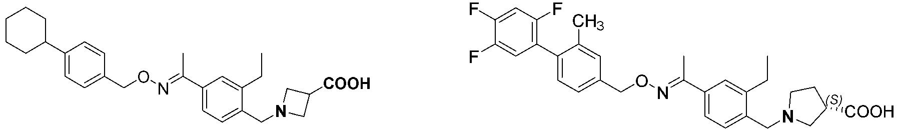

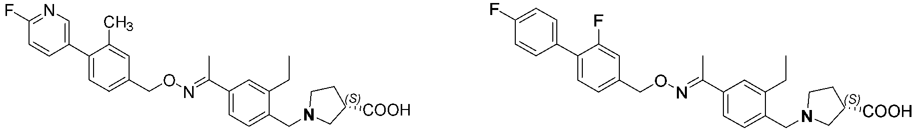

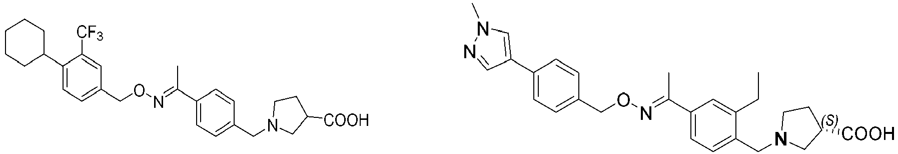

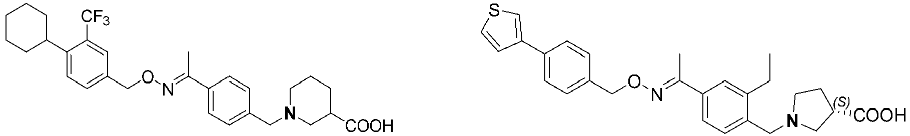

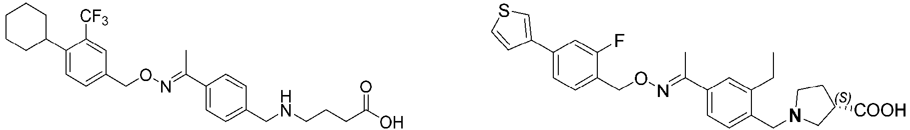

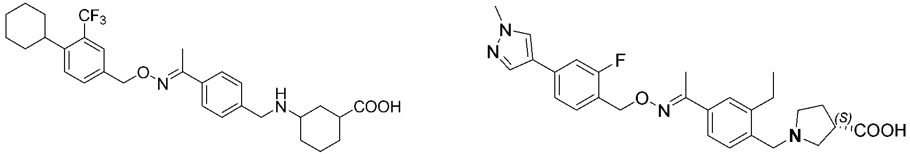

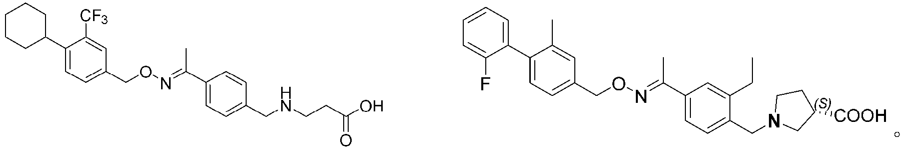

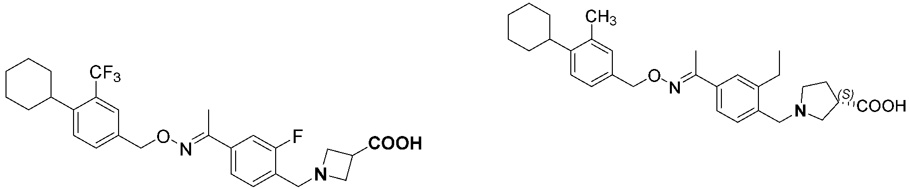

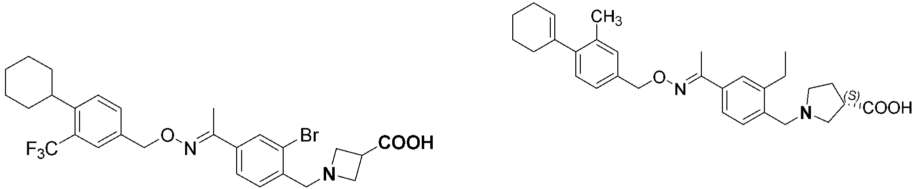

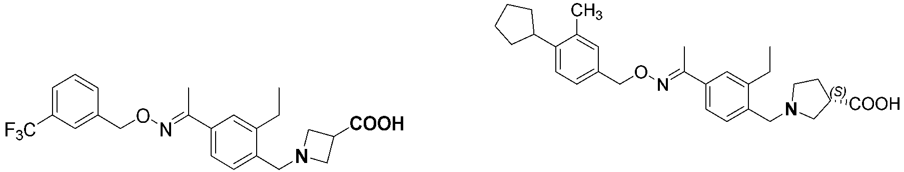

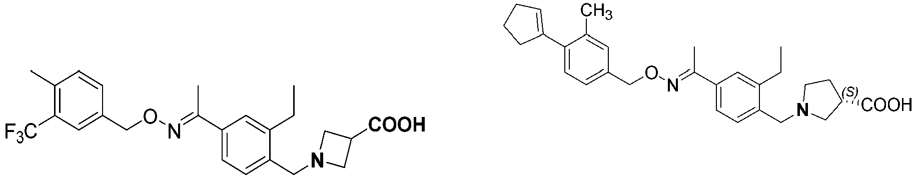

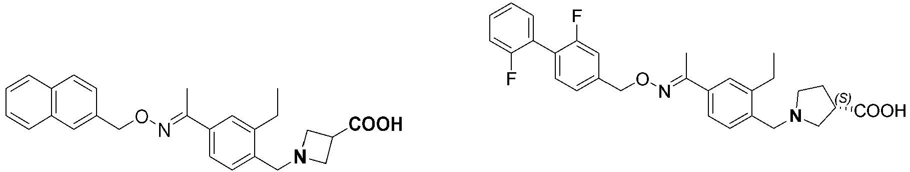

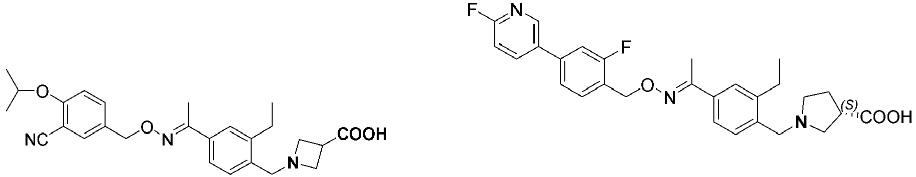

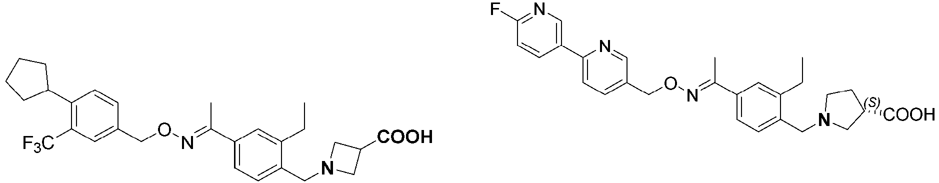

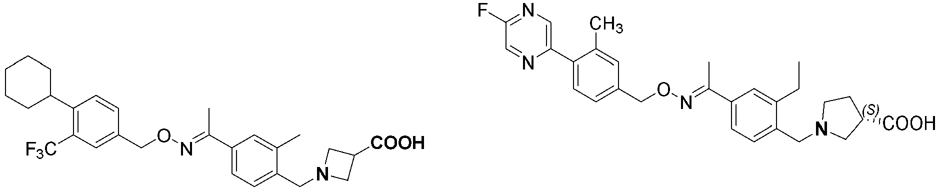

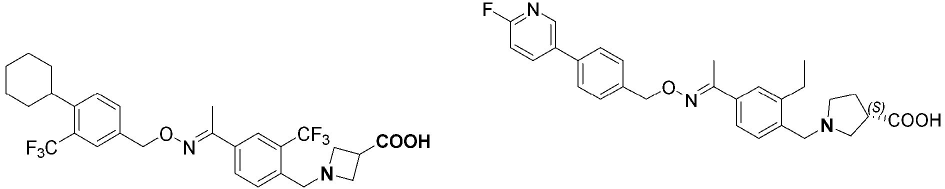

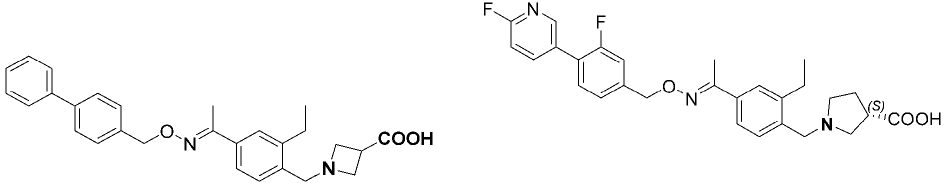

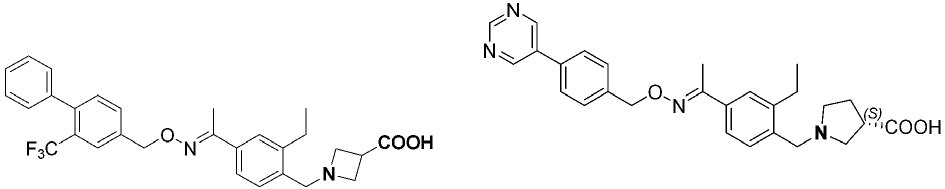

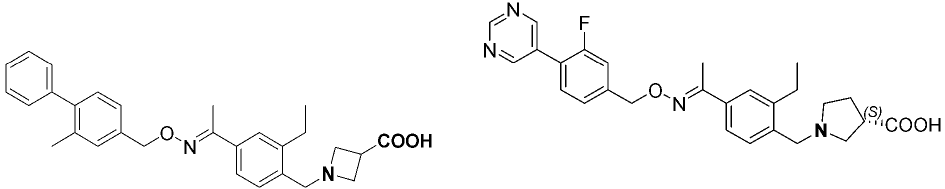

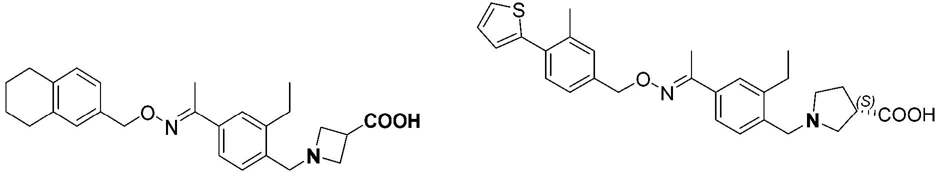

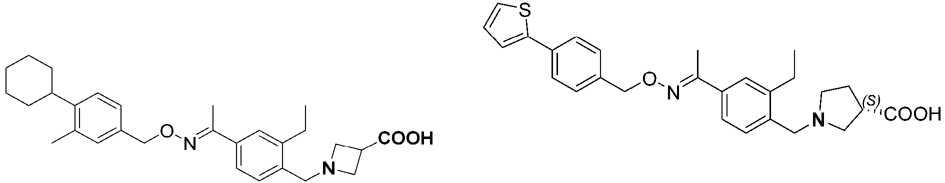

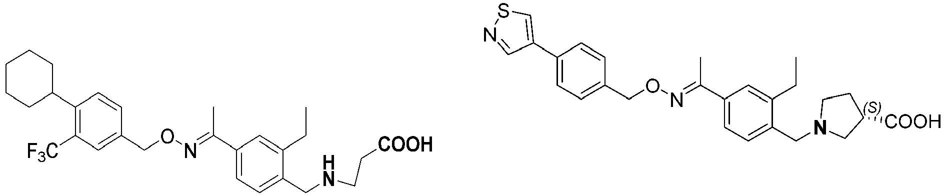

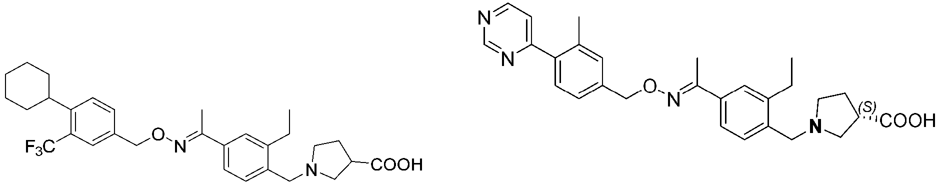

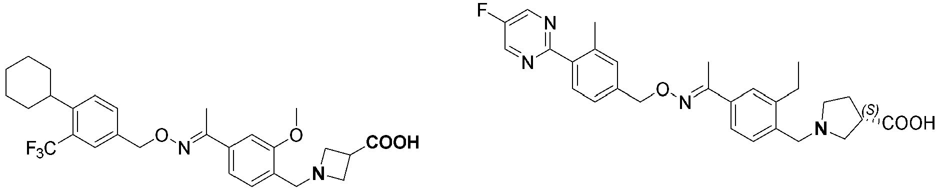

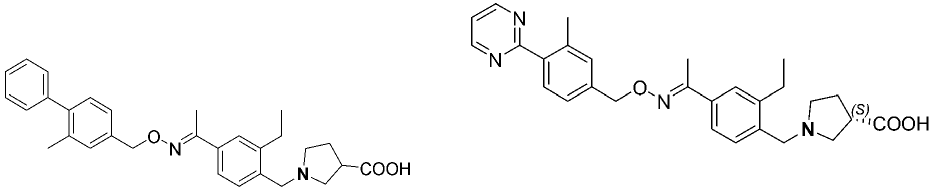

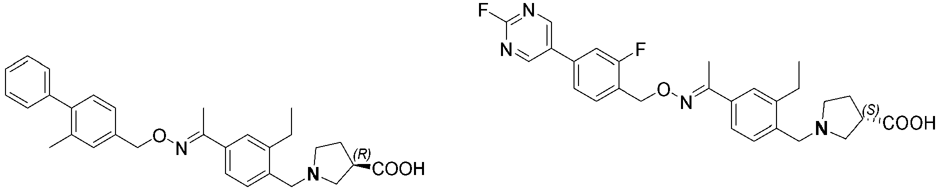

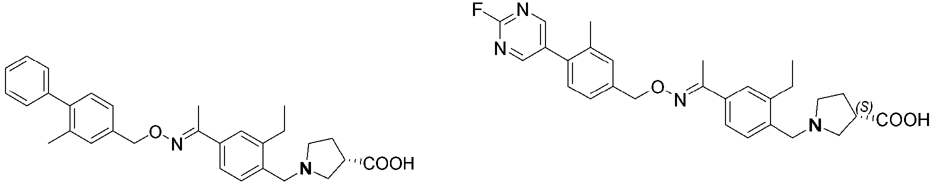

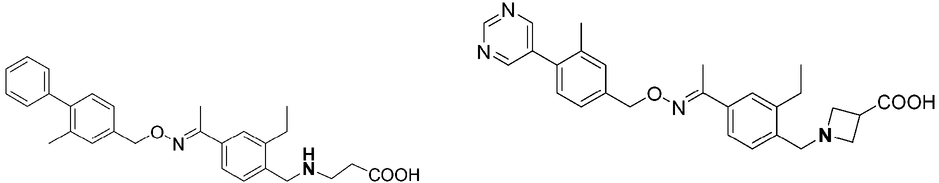

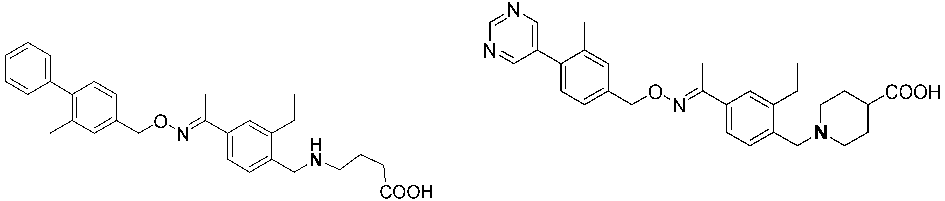

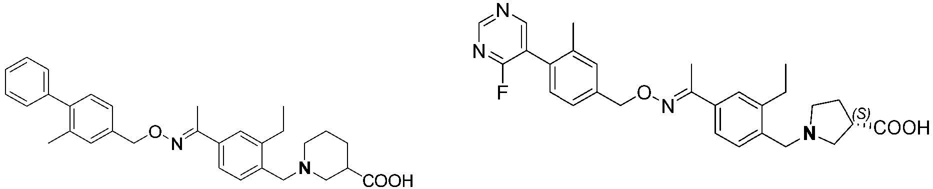

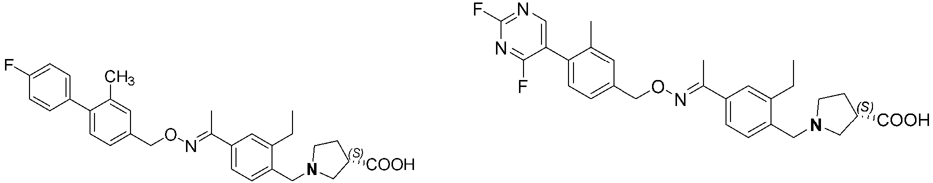

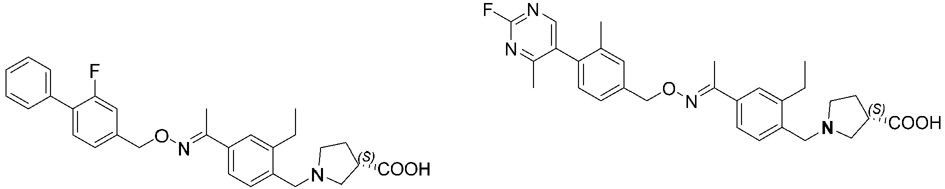

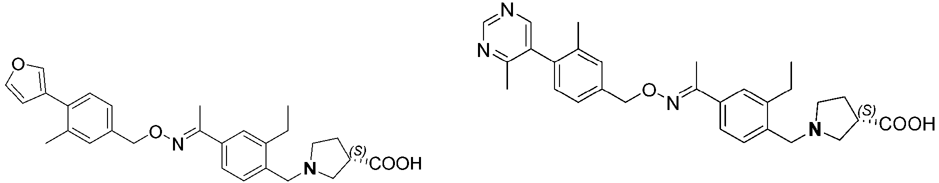

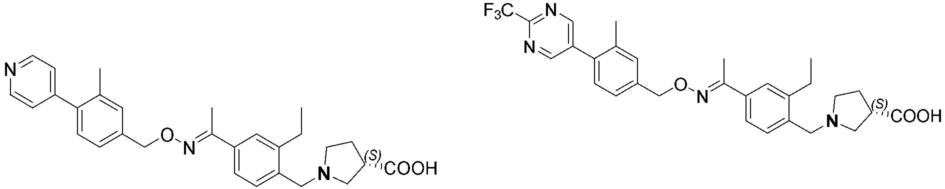

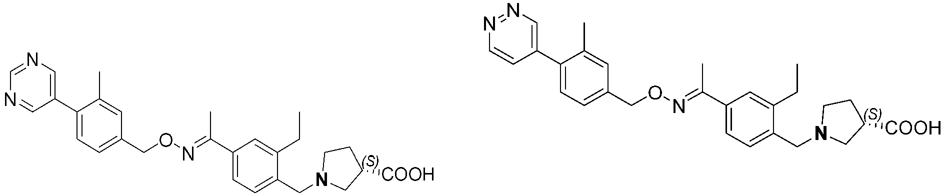

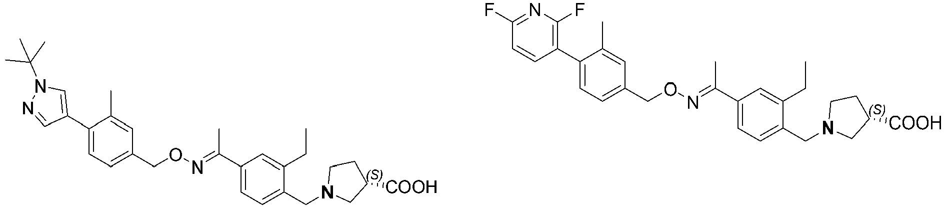

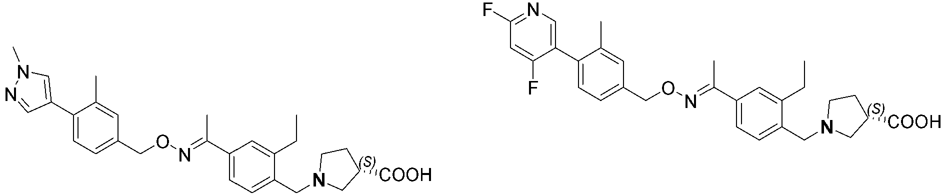

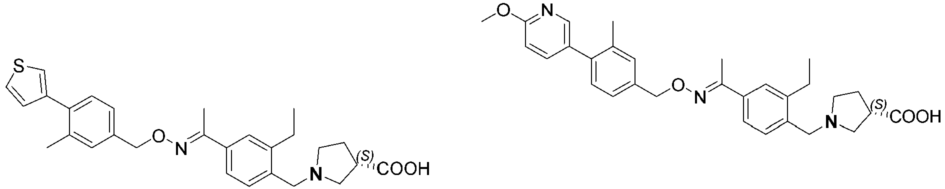

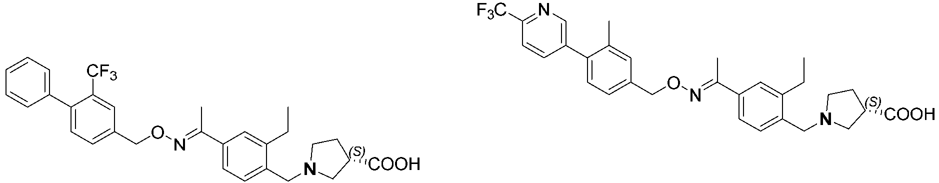

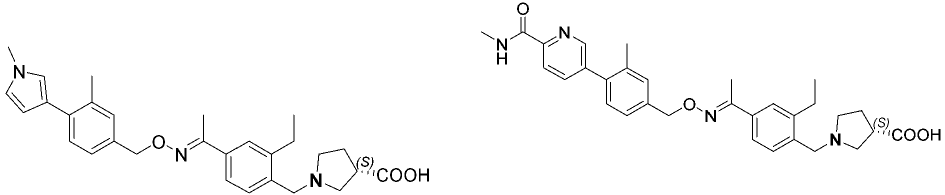

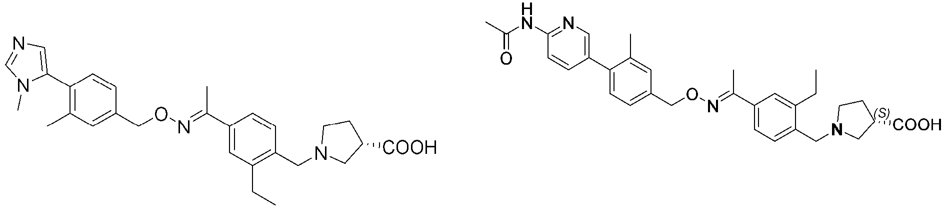

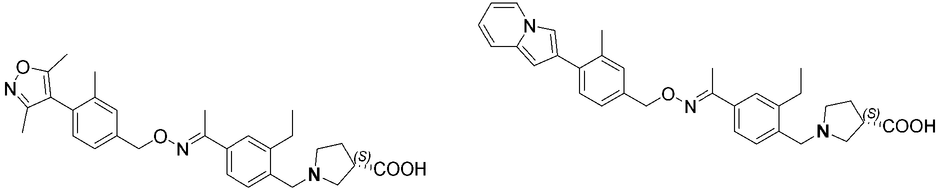

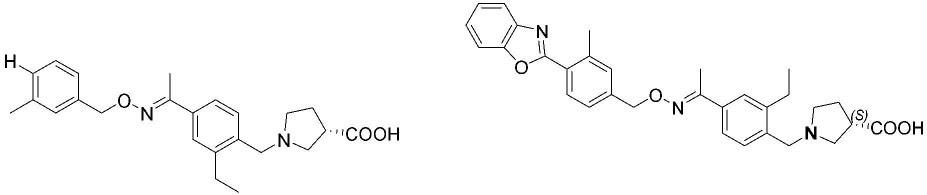

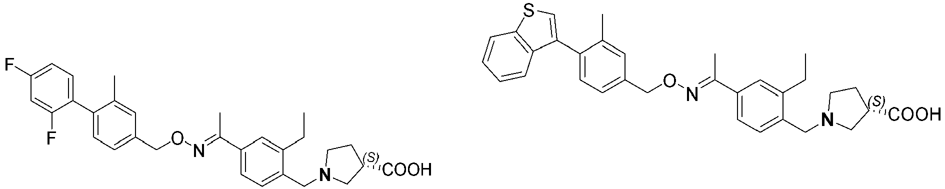

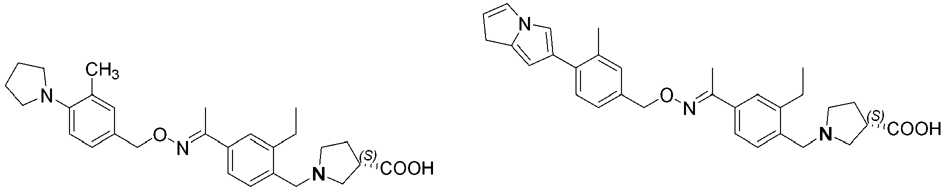

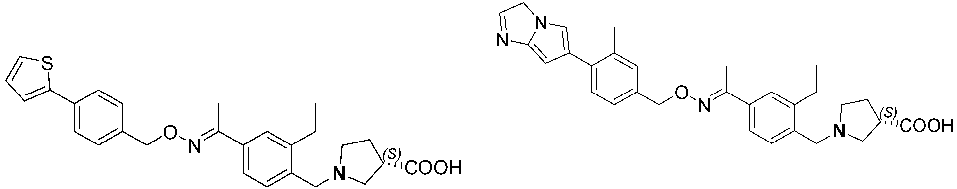

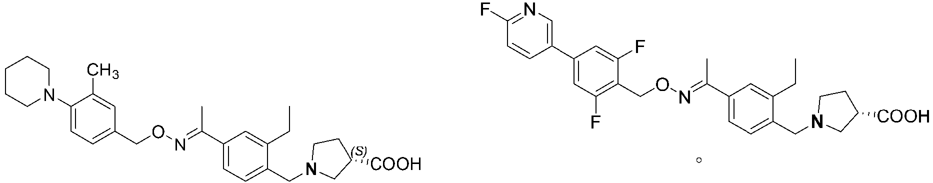

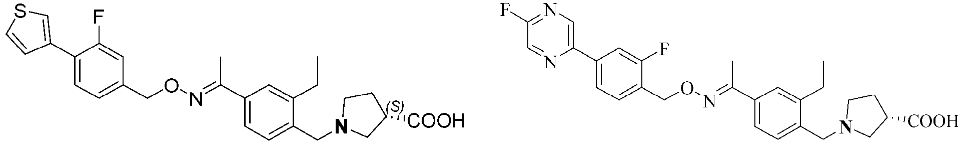

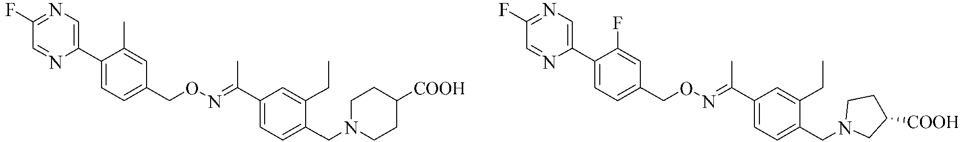

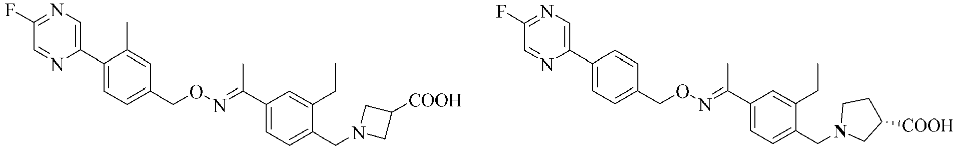

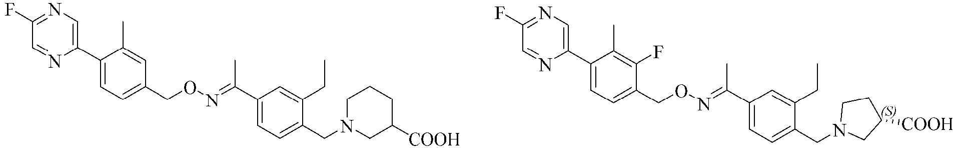

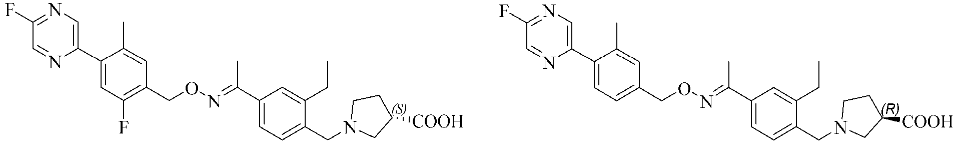

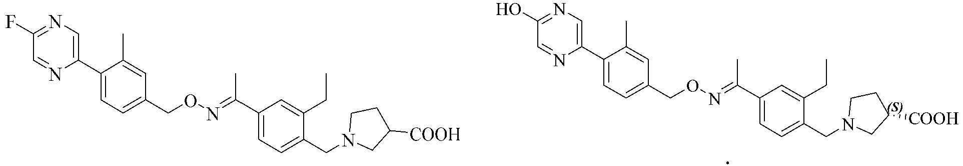

In a specific embodiment, in Formula III,
R¹ is selected from the group consisting of: a hydrogen, halogen (preferably F, Cl, or Br), cyano, substituted or unsubstituted C₁-C₅ alkoxy (preferably a methoxy), substituted or unsubstituted C₁-C₆ alkyl (preferably a methyl, ethyl, propyl);
R² is: 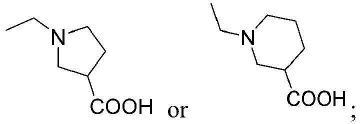
R³ and R⁵ are independently selected from the group consisting of a hydrogen, substituted or unsubstituted C₁-C₆ alkyl (preferably a methyl, ethyl or propyl), and halogen (preferably F);
R⁴ is selected from the group consisting of a substituted or unsubstituted C₅-C₈ aryl (preferably a phenyl or F-substituted phenyl), substituted or unsubstituted five-membered or six-membered heterocyclic group or heteroaryl containing 1-2 heteroatoms selected from N, O or S.

In a specific embodiment, the present invention provides a compound selected from the following group, or an optical isomer or a pharmaceutically acceptable salt thereof:

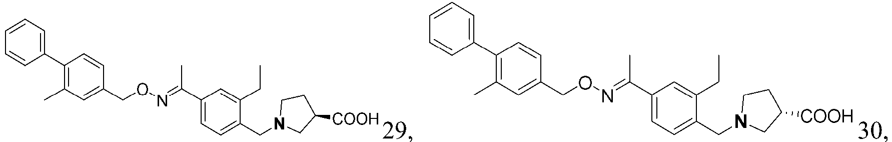

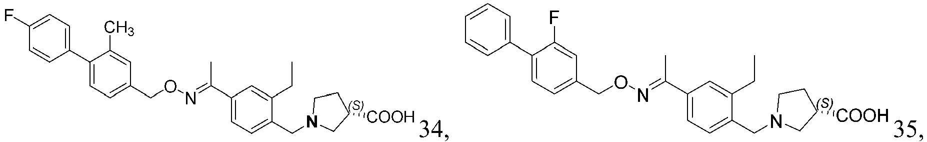

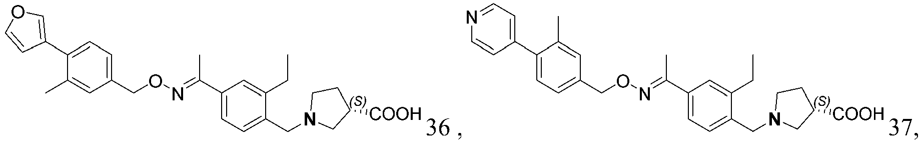

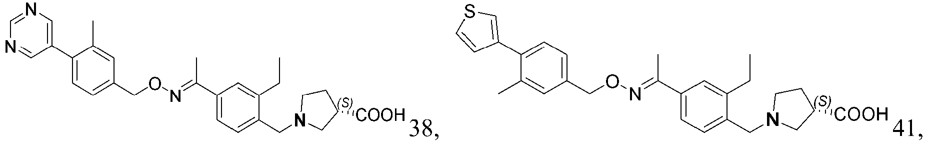

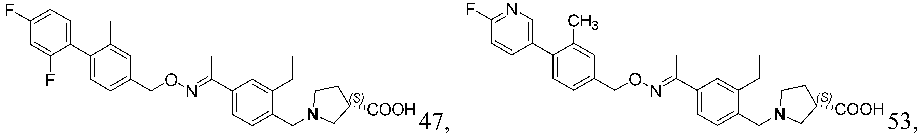

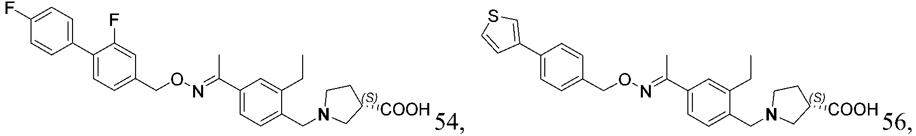

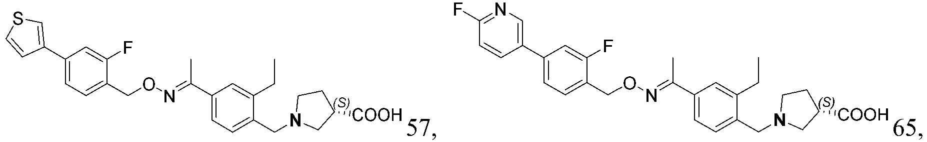

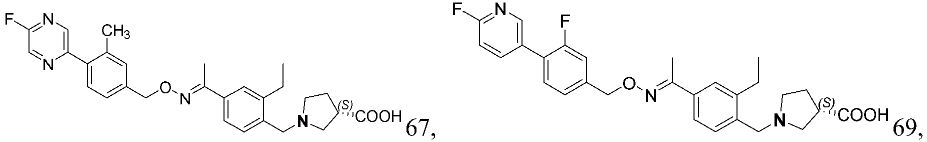

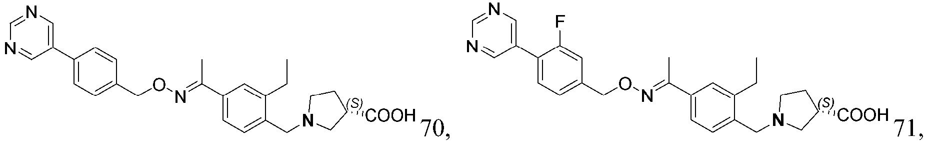

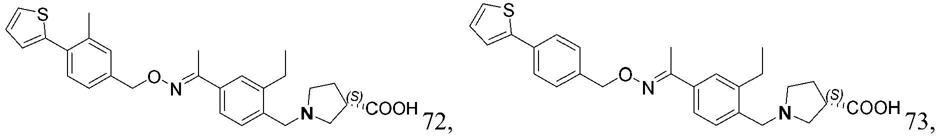

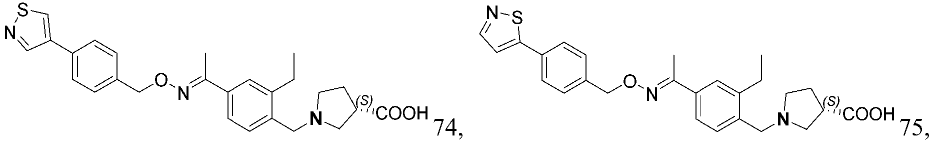

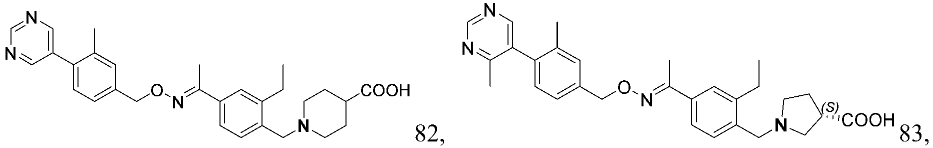

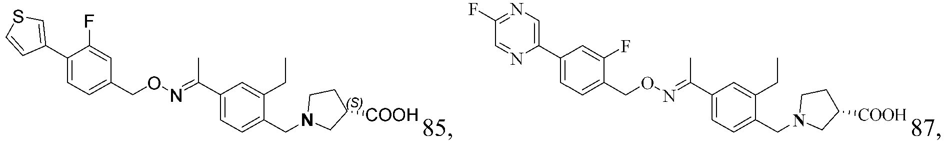

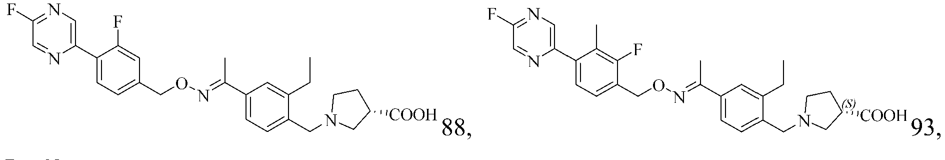

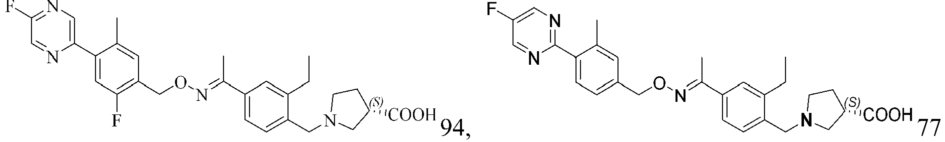

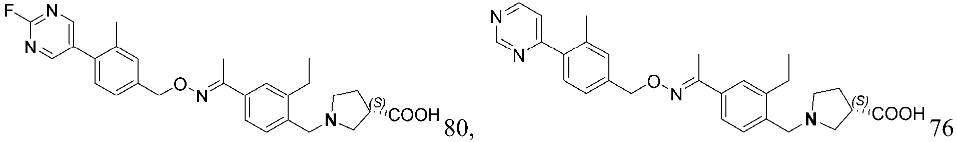

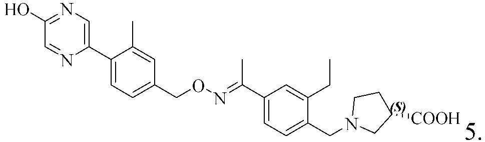

In a specific embodiment, in Formula III,
R¹ is selected from the group consisting of: a cyano, substituted or unsubstituted C₁-C₅ alkoxy (preferably a methoxy), substituted or unsubstituted C₁-C₆ alkyl (preferably a methyl, ethyl, propyl);
R² is: 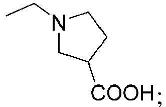
R³ and R⁵ are independently selected from the group consisting of a hydrogen, substituted or unsubstituted C₁-C₃ alkyl (preferably a methyl, ethyl or propyl), and halogen (preferably F);
R⁴ is selected from the group consisting of a substituted or unsubstituted C₅-C₈ aryl (preferably a phenyl or F-substituted phenyl), and substituted or unsubstituted five-membered or six-membered heteroaryl containing 1-2 heteroatoms selected from N, O or S.

In a specific embodiment, the present invention provides a compound selected from the following group, or an optical isomer or a pharmaceutically acceptable salt thereof:

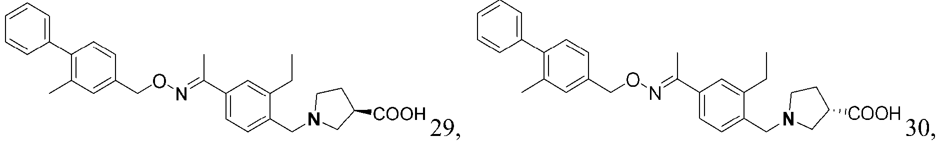

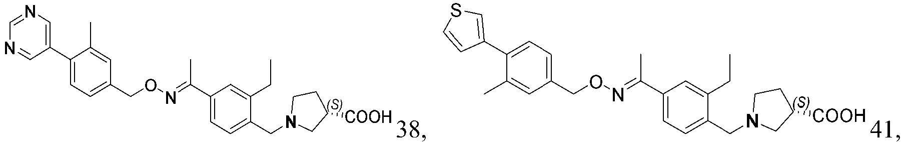

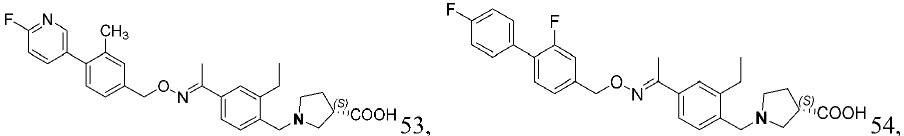

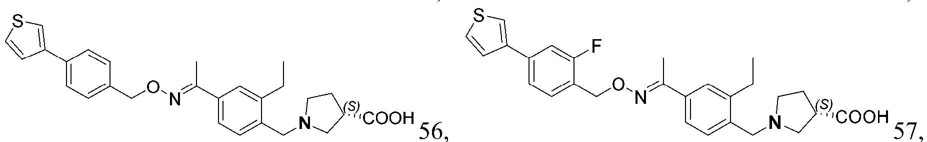

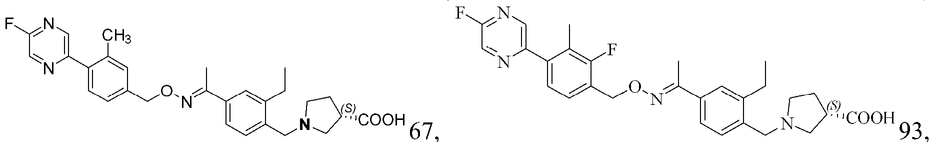

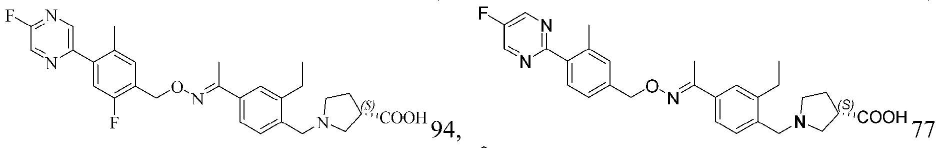

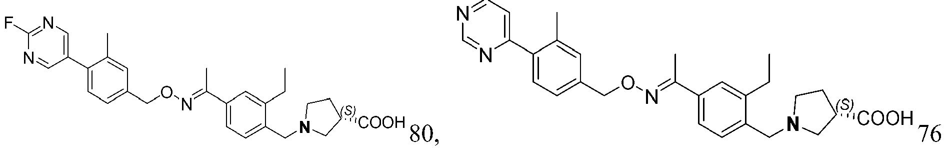

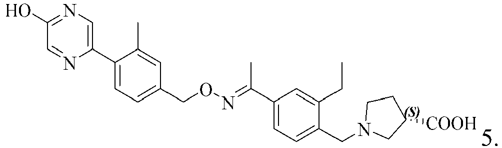

In a preferred embodiment, the compound is an S1PR regulator; preferably, the compound is an S1PR4 agonist; and more preferably, the compound has agonistic effects on S1PR4 but no agonistic effects on S1PR1.

In the second aspect, the present invention provides a pharmaceutical composition, comprising the compound described in the first aspect, or an optical isomer or a pharmaceutically acceptable salt thereof, as well as a pharmaceutically acceptable carrier or excipient.

In a preferred embodiment, the pharmaceutical composition is in a dosage form suitable for oral administration, including but not limited to tablets, solutions, suspensions, capsules, granules, and powders.

In a preferred embodiment, the pharmaceutical composition is used as an S1PR4 agonist or an S1PR4 mutant agonist.

In a preferred embodiment, the pharmaceutical composition is used for treating or preventing diseases mediated by S1PR4.

In a preferred embodiment, the diseases mediated by S1PR4 include myopathy.

In a preferred embodiment, the myopathy includes but is not limited to: idiopathic inflammatory myopathy, infectious myopathy, drug-induced myopathy, toxic myopathy, hereditary myopathy, etc.

In a preferred embodiment, the myopathy includes but is not limited to: Duchenne muscular dystrophy, pseudohypertrophic muscular dystrophy, limb-girdle muscular dystrophy, facioscapulohumeral muscular dystrophy, distal muscular dystrophy, myotonic dystrophy, congenital muscular dystrophy, Emery-Dreifuss muscular dystrophy, oculopharyngeal muscular dystrophy, amyotrophic lateral sclerosis, etc.

In a preferred embodiment, the myopathy includes but is not limited to: sepsis-induced myopathy, or sepsis-acquired muscle weakness, especially symptoms such as atrophy of the gastrocnemius and tibialis anterior muscles.

In the third aspect, the present invention provides the use of the compound described in the first aspect, or an optical isomer thereof or a pharmaceutically acceptable salt thereof, in the preparation of an S1PR regulator. In a preferred embodiment, the S1PR regulator is a selective agonist of S1PR4 or a selective agonist of S1PR4 mutant.

In a preferred embodiment, the S1PR regulator is an S1PR4 agonist or an S1PR4 mutant agonist.

In a preferred embodiment, the S1PR4 agonist or an S1PR4 mutant agonist is a medicamenmt for treating or preventing diseases mediated by S1PR4.

In a preferred embodiment, the diseases mediated by S1PR4 include myopathy.

In a preferred embodiment, the myopathy includes but is not limited to: idiopathic inflammatory myopathy, infectious myopathy, drug-induced myopathy, toxic myopathy, hereditary myopathy, etc.

In a preferred embodiment, the myopathy includes but is not limited to: Duchenne muscular dystrophy, pseudohypertrophic muscular dystrophy, limb-girdle muscular dystrophy, facioscapulohumeral muscular dystrophy, distal muscular dystrophy, myotonic dystrophy, congenital muscular dystrophy, Emery-Dreifuss muscular dystrophy, oculopharyngeal muscular dystrophy, amyotrophic lateral sclerosis, etc.

In the fourth aspect, the present invention provides a method for regulating S1PR in a subject, comprising the step of administering an effective amount of the compound according to the first aspect, or an optical isomer thereof, or a pharmaceutically acceptable salt thereof, or the pharmaceutical composition according to the second aspect to a subject in need thereof.

In a preferred embodiment, the method is a method for activating S1PR4 in a subject; and more preferably, the method is a method for activating S1PR4 in a subject without activating S1PR1.

In a preferred embodiment, the method is used for treating or preventing diseases mediated by S1PR4.

In a preferred embodiment, the diseases mediated by S1PR4 include myopathy.

In a preferred embodiment, the myopathy includes but is not limited to: idiopathic inflammatory myopathy, infectious myopathy, drug-induced myopathy, toxic myopathy, hereditary myopathy, etc.

In a preferred embodiment, the myopathy includes but is not limited to: Duchenne muscular dystrophy, pseudohypertrophic muscular dystrophy, limb-girdle muscular dystrophy, facioscapulohumeral muscular dystrophy, distal muscular dystrophy, myotonic dystrophy, congenital muscular dystrophy, Emery-Dreifuss muscular dystrophy, oculopharyngeal muscular dystrophy, amyotrophic lateral sclerosis, etc.

In the fifth aspect, the present invention provides the compound described in the first aspect, or an optical isomer, pharmaceutically acceptable salt thereof, or the pharmaceutical composition described in the second aspect, for treating or preventing diseases mediated by S1PR4.

In a preferred embodiment, the diseases mediated by S1PR4 include myopathy.

In a preferred embodiment, the myopathy includes but is not limited to: idiopathic inflammatory myopathy, infectious myopathy, drug-induced myopathy, toxic myopathy, hereditary myopathy, etc.

In a preferred embodiment, the myopathy includes but is not limited to: Duchenne muscular dystrophy, pseudohypertrophic muscular dystrophy, limb-girdle muscular dystrophy, facioscapulohumeral muscular dystrophy, distal muscular dystrophy, myotonic dystrophy, congenital muscular dystrophy, Emery-Dreifuss muscular dystrophy, oculopharyngeal muscular dystrophy, amyotrophic lateral sclerosis, etc.

In the sixth aspect, the present invention provides a medicament comprising the compound described in the first aspect, or an optical isomer thereof, a pharmaceutically acceptable salt thereof, or the pharmaceutical composition described in the second aspect, for treating or preventing diseases mediated by S1PR4.

In a preferred embodiment, the diseases mediated by S1PR4 include myopathy.

In a preferred embodiment, the myopathy includes but is not limited to: idiopathic inflammatory myopathy, infectious myopathy, drug-induced myopathy, toxic myopathy, hereditary myopathy, etc.

In a preferred embodiment, the myopathy includes but is not limited to: Duchenne muscular dystrophy, pseudohypertrophic muscular dystrophy, limb-girdle muscular dystrophy, facioscapulohumeral muscular dystrophy, distal muscular dystrophy, myotonic dystrophy, congenital muscular dystrophy, Emery-Dreifuss muscular dystrophy, oculopharyngeal muscular dystrophy, amyotrophic lateral sclerosis, etc.

It should be understood that within the scope of the present invention, the above-mentioned technical features of the present invention and the technical features specifically described below (such as in the examples) can be combined with each other to form new or preferred technical solutions. Due to the limited contents, they will not be described one by one here.

### Description of drawings

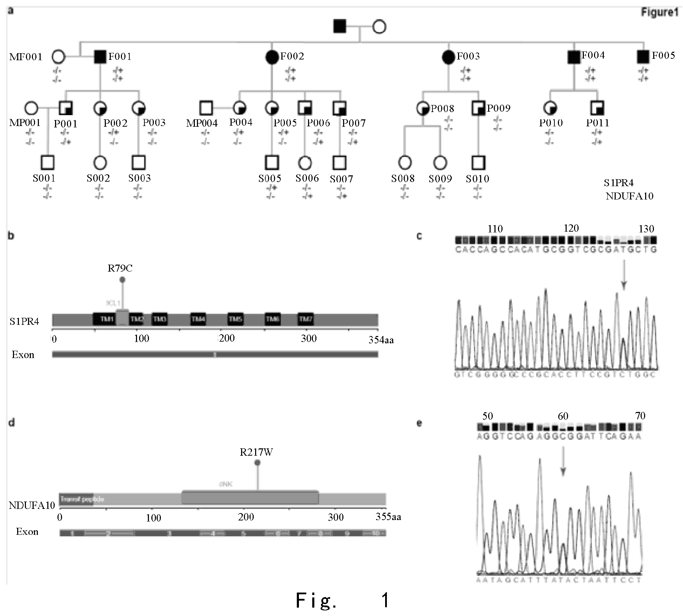

Figure 1 shows the identification of mutations of S1PR4 R79C and NDUFA10 R217W in the late-onset muscular dystrophy spectrum; among them, (a) a Chinese pedigree chart containing four generations. Patients are marked with black filling, individuals with mild symptoms are marked with black squares, males are represented by squares and females by circles, with the first and second generations already affected. The symbols below each member indicate the mutation status of S1PR4 R79C (green) and NDUFA10 R217W (purple) respectively; (b) S1PR4 R79C is located within ICL1 (intracellular loop 1); (c) Sanger sequencing verification of S1PR4 R79C; (d) NDUFA10 R217W is located within the deoxyribonucleoside kinase domain (dNK); and (e) Sanger sequencing verification of NDUFA10 R217W;
Figure 2 shows the detection results of paw grip strength, serum creatine kinase levels, and muscle fibrosis in S1PR4 mutant mice. Among them, there was no difference in paw grip strength between S1PR4 mutant mice and normal mice (Figure a), no difference in serum creatine kinase levels between S1PR4 mutant mice and WT mice (Figure b), and no muscle fibrosis in the muscles of S1PR4 mutant mice (HE staining and Sirius red staining). These results indicate that S1PR4 mutation does not cause muscular dystrophy.
Figure 3 shows the detection results of paw grip strength, serum creatine kinase levels, and muscle fibrosis in NDUFA10 mutant mice; among them, there is no difference in paw grip strength between NDUFA10 mutant mice and normal mice (Figure a), no difference in serum creatine kinase levels between NDUFA10 mutant mice and WT mice (Figure b), and no muscle fibrosis in the muscles of NDUFA10 mutant mice (HE staining and Sirius red staining). These results indicate that NDUFA10 mutation does not cause muscular dystrophy.

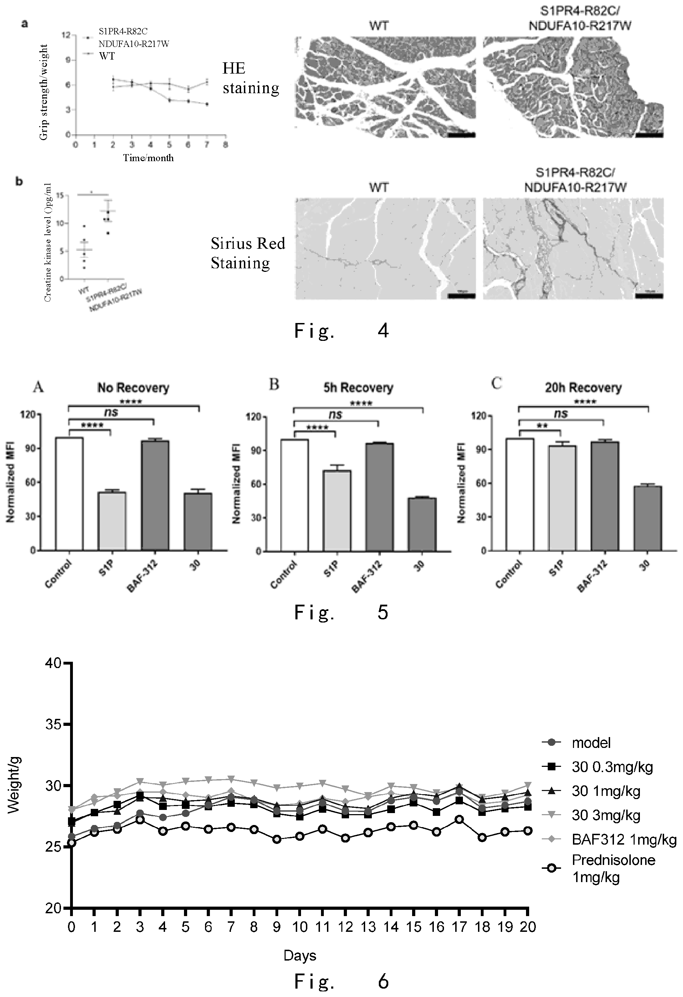

Figure 4 shows the detection results of paw grip strength, serum creatine kinase levels, and nucleated fibers and collagen deposition in muscles of S1PR4/NDUFA10 mutant mice; among them, the paw grip strength of S1PR4/NDUFA10 mutant mice was significantly lower than that of normal mice starting from 5 months of age (Figure a), the serum creatine kinase level in S1PR4/NDUFA10 mutant mice was significantly increased (Figure b), and the muscles of S1PR4/NDUFA10 mutant mice showed nucleated fibers and collagen deposition, indicating muscle fibrosis (HE staining and Sirius red staining), which can be diagnosed as muscular dystrophy.
Figure 5 shows the recovery effect after the compound 30 of the present invention induces S1PR4 internalization;
Figure 6 shows the body weight changes of double-mutant mice after administration;
Figure 7 shows the grip strength changes of double-mutant mice after administration;
Figure 8 shows that the serum creatine kinase level in double mutant mice is significantly higher compared with healthy mice; after administration of the drug, the creatine kinase level decreases significantly, while there is no reduction in the prednisolone-administered group.
Figure 9 is a representative image of centrally nucleated fibers in muscle after administration.
Figure 10 shows the detection results of the number of centrally nucleated fibers in muscle after administration.
Figure 11 is a representative diagram of collagen area after administration;
Figure 12 is a statistical chart of collagen area after administration;
Figure 13 is a representative immunofluorescence diagram of macrophages after administration;
Figure 14 is a statistical chart of immunofluorescence of macrophages after administration;
Figure 15 shows the changes in body weight of Duchenne muscular dystrophy model mice after administration;
Figure 16 shows the changes in grip strength of Duchenne muscular dystrophy model mice after administration;
Figure 17 shows the changes in creatine kinase in Duchenne muscular dystrophy model mice;
Figure 18 is a representative diagram of centrally nucleated fibers in muscles after administration;
Figure 19 shows the detection results of the number of centrally nucleated fibers in the muscle after administration;
Figure 20 is a representative diagram of the collagen area after administration;
Figure 21 is a statistical chart of the collagen area after administration;
Figure 22 is a representative immunofluorescence diagram of macrophages after administration;
Figure 23 is a statistical chart of macrophage immunofluorescence after administration;
Figure 24 shows the effect of compound 30 of the present invention on the tibialis anterior muscle (tibialis) and gastrocnemius muscle (gastrocnemius) in the LPS-induced sepsis model;
Figure 25 shows that, compared with the model group, there was no significant difference in body weight changes among the various administration groups in Duchenne muscular dystrophy model mice after administration;
Figure 26 shows that, after 42 days of treatment with compound 67 at 3 mg/kg and Vamorolone at 20 mg/kg, the grip strength of mice in each administration group increased significantly compared with the model group, showing a good therapeutic effect;
Figure 27 shows that on day 42 after administration of Compound 67 at 3 mg/kg and Vamorolone at 20 mg/kg, the rotarod time in each administration group increased compared with the model group, indicating a good therapeutic effect, and the increase in grip strength of Compound 67 at 3 mg/kg was more significant than that of Vamorolone at 20 mg/kg;
Figure 28 is a representative diagram of centrally nucleated fibers after administration;
Figure 29 shows the statistics of nucleated fibers after administration;
Figure 30 is a representative diagram of collagen area after administration;
Figure 31 shows the statistics of collagen area after administration;
Figure 32 is a representative diagram of the number of F4/80 macrophage markers (muscle inflammation) after administration;
Figure 33 shows the statistics of the number of F4/80 macrophage markers (muscle inflammation) after administration;
Figure 34 shows the statistics of serum creatine kinase after administration.

### Modes for carrying out the invention

After extensive and in-depth research, the inventors found that S1PR4 agonists have therapeutic effects on certain myopathies at the animal level. Furthermore, the inventors synthesized candidate compounds with S1PR4 receptor regulatory activity. The inventors optimized the structure of the obtained candidate compounds, designed and synthesized a series of selective S1PR4 compounds that have not been reported in the literature, and conducted structural characterization. The inventors performed activity tests on this series of compounds at the cellular level and obtained a batch of compounds with S1PR4 agonistic activity. These compounds can regulate the activity of S1P receptors, among which the EC₅₀ value for S1PR4 agonistic activity reaches the nM level, showing a good targeting effect on S1PR4. The present invention was completed on this basis.

### Definition on terms

The definitions on some groups involved herein are as follows:
As used herein, "alkyl" refers to a saturated branched, straight-chain, or cycloalkyl group with a carbon chain length of 1-10 carbon atoms. Preferred alkyls include those with 1-5, 1-2, 1-6, 1-4, or 3-8 carbon atoms, and the like. Examples of alkyls include, but are not limited to: methyl, ethyl, n-propyl, isopropyl, n-butyl, isobutyl, heptyl, etc. Alkyls can be substituted by one or more substituents, such as halogens or haloalkyls. For example, an alkyl can be an alkyl substituted by 1-4 fluorine atoms, or it can be an alkyl substituted by a fluoroalkyl.

As used herein, "alkenyl" generally refers to a monovalent hydrocarbon group with at least one double bond, usually containing 2-8 carbon atoms, preferably 2-6 carbon atoms, and can be straight-chain or branched-chain. Examples of alkenyls include, but are not limited to, vinyl, propenyl, isopropenyl, butenyl, isobutenyl, hexenyl, and the like.

As used herein, "ester group" usually refers to carboxylic acid derivatives with at least one ester group, typically containing 3-8 carbon atoms, preferably 3-6 carbon atoms, and can be straight-chain or branched-chain. Examples of ester groups include, but are not limited to, methyl formate, ethyl formate, methyl acetate, ethyl acetate, propyl acetate, and the like.

As used herein, "hydroxyl" refers to branched or straight-chain alcohols with a carbon chain length of 1-10 carbon atoms, usually containing 1-10 carbon atoms, preferably 1-6 carbon atoms, and can be straight-chain or branched. Examples of ester hydroxyl include, but are not limited to, 1-hydroxy-n-butyl, 1-hydroxy-isobutyl, and so on.

As used herein, "amido group" refers to a group with the structural formula "-R'-NH-C(O)-R", where R' can be selected from a hydrogen or alkyl, and R can be selected from an alkyl, alkenyl, alkynyl, alkyl substituted by NR_{c}R_{d}, alkenyl substituted by NR_{c}R_{d}, alkynyl substituted by NR_{c}R_{d}, alkyl substituted by a halogen, and alkenyl substituted by cyano. Among them, R_{c} and R_{d} can be selected from an alkyl or alkenyl.

As used herein, "aryl" refers to monocyclic, bicyclic, or tricyclic aromatic groups containing 6 to 14 carbon atoms, including phenyl, naphthyl, phenanthryl, anthryl, indenyl, fluorenyl, tetrahydronaphthyl, dihydroindenyl, etc. The aryl group may optionally be substituted with 1-5 (for example, 1, 2, 3, 4, or 5) substituents selected from: a halogen, C₁₋₄ aldehyde group, C₁₋₆ alkyl, cyano, nitro, amino, amide group, hydroxyl, hydroxymethyl, halogen-substituted alkyl (such as trifluoromethyl), halogen-substituted alkoxy (such as trifluoromethoxy), carboxyl, C₁₋₄ alkoxy, ethoxy formyl, N(CH₃), C₁₋₄ acyl, and the like, heterocyclic group, or heteroaryl, and the like.

As used herein, "heterocyclic group" includes, but is not limited to, 5-membered or 6-membered heterocyclic groups containing 1-3 heteroatoms selected from O, S or N, including but not limited to furyl, thienyl, pyrrolyl, pyrrolidinyl, pyrazolyl, imidazolyl, triazolyl, oxazolyl, pyranyl, pyridyl, pyrimidinyl, pyrazinyl, piperidinyl, morpholinyl, etc.

As used herein, "aromatic heterocyclic group" refers to a group containing 5-14 ring atoms, with 6, 10, or 14 electrons shared in the ring system. Moreover, the contained ring atoms are carbon atoms and 1-3 heteroatoms selected from oxygen, nitrogen, or sulfur. Useful aromatic heterocyclic groups include piperazinyl, morpholinyl, piperidinyl, pyrrolidinyl, thienyl, furyl, pyranyl, pyrrolyl, imidazolyl, pyrazolyl, pyridyl, including but not limited to pyrimidinyl, etc. The aromatic heterocyclic group may optionally be substituted with 1-5 (for example, 1, 2, 3, 4, or 5) substituents selected from: a halogen, C₁₋₄ aldehyde group, C₁₋₆ straight-chain or branched alkyl, cyano, nitro, amino, hydroxyl, hydroxymethyl, halogen-substituted alkyl (such as trifluoromethyl), halogen-substituted alkoxy (such as trifluoromethoxy), carboxyl, C₁₋₄ alkoxy, ethoxy formyl, N(CH₃), and C₁₋₄ acyl.

As used herein, "alkoxy" refers to an oxygen group substituted by an alkyl. Preferred alkoxys are those with 1-6 carbon atoms in length, and more preferred are those with 1-3 carbon atoms in length. Examples of alkoxy includes, but is not limited to: a methoxy, ethoxy, propoxy, etc. Alkoxys can be substituted by one or more substituents, such as a halogen or haloalkyl. For example, an alkoxy can be an alkyl group substituted by 1-4 fluorine atoms, or the alkyl can be an alkyl substituted by a fluoroalkyl.

As used herein, "halogen" refers to a fluorine, chlorine, bromine, or iodine.

As used herein, "optionally substituted" means that the substituent it modifies can be optionally substituted with 1-5 (for example, 1, 2, 3, 4, or 5) substituents selected from the following: a halogen, C₁₋₄ aldehyde group, C₁₋₆ straight-chain or branched alkyl, cyano, nitro, amino, hydroxyl, hydroxymethyl, halogen-substituted alkyl (such as a trifluoromethyl), halogen-substituted alkoxy (such as a trifluoromethoxy), carboxyl, C₁₋₄ alkoxy, ethoxy formyl, N(CH₃), and C₁₋₄ acyl.

### Compounds of the present invention

The inventors discovered that both males and females in a family began to exhibit muscular dystrophy phenotypes from the age of 50, and these phenotypes worsened with increasing age, showing strong characteristics of a familial hereditary disease. Through studies such as whole-genome sequencing of this family, it was found that mutations in NDUFA10 and S1PR4 are potential pathogenic gene mutations for muscular dystrophy.

Given that there are currently no medicaments targeting S1PR4 in clinical practice, the S1P receptor regulators that are on the market or under research mainly focus on targets related to S1PR1 and S1PR5. The inventors have designed and synthesized a series of compounds with entirely new structures that have selectivity among different subtypes of S1PR. Therefore, the compounds of the present invention have different regulatory effects on different subtypes of S1PR; in other words, the compounds of the present invention are regulators of S1PR. In a specific embodiment, the compounds of the present invention are S1PR4 agonists. In a preferred embodiment, the compounds of the present invention have selectivity between S1PR4 and S1PR1; that is, the compounds of the present invention have an agonistic effect on S1PR4 but no agonistic effect on S1PR1.

In a specific embodiment, the compound of the present invention is a compound represented by the following formula I, or an optical isomer or a pharmaceutically acceptable salt thereof, 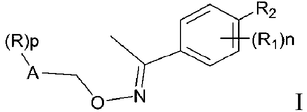 wherein A, R, R¹, n, R², m, and q are described as above.

In a preferred embodiment, the following compound is not included in the compound of the present invention:

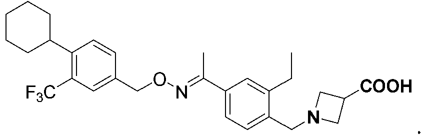

In a preferred embodiment, the compound of the present invention is represented by the following formula II: 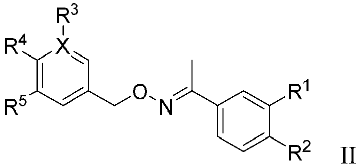 wherein R¹, R², X, R³, R⁴, and R⁵ are described as above.

In a preferred embodiment, the compound of the present invention is represented by the following formula III: 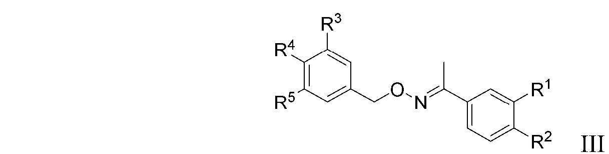 wherein R¹, R², R³, R⁴, and R⁵ are described as above.

Specifically, the present invention provides compounds selected from the following group, or an optical isomer or pharmaceutically acceptable salt thereof:

| No of compound | Structural formula |
|---|---|
| 1 | 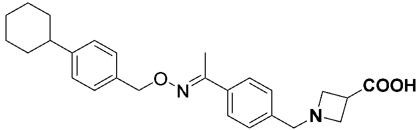 |
| 2 | 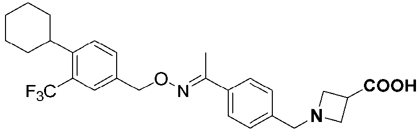 |
| 3 | 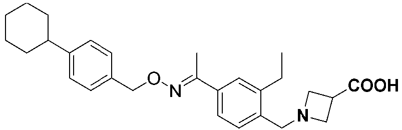 |
| 4 | 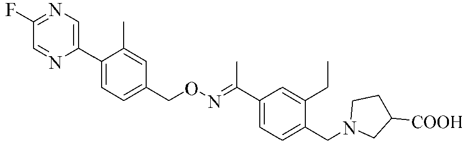 |
| 5 | 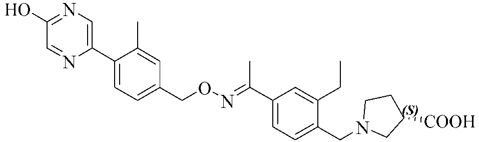 |
| 6 | 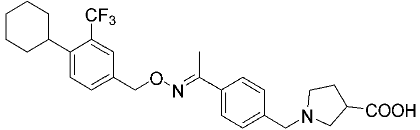 |
| 7 | 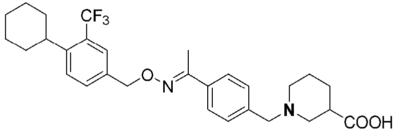 |
| 8 | 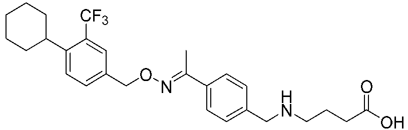 |
| 9 | 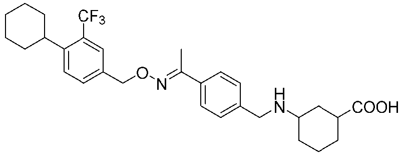 |
| 10 | 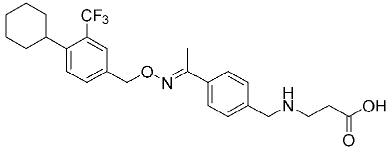 |
| 11 | 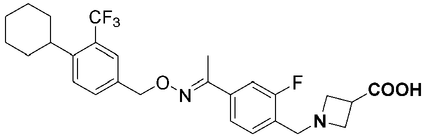 |
| 12 | 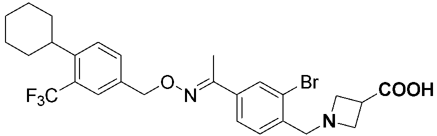 |
| 13 | 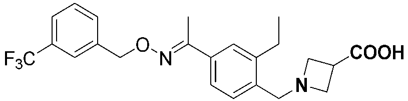 |
| 14 | 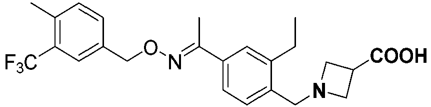 |
| 15 | 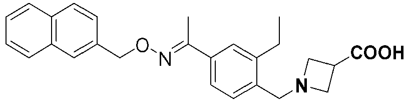 |
| 16 | 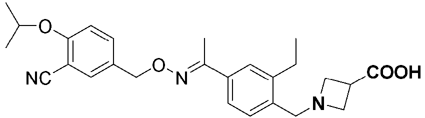 |
| 17 | 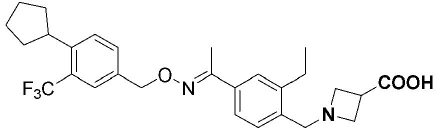 |
| 18 | 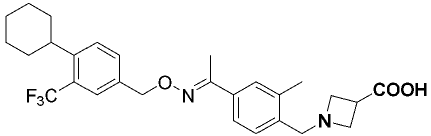 |
| 19 | 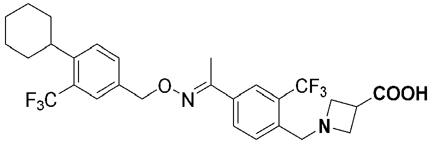 |
| 20 | 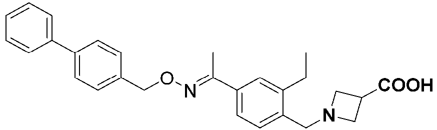 |
| 21 | 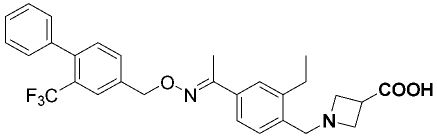 |
| 22 | 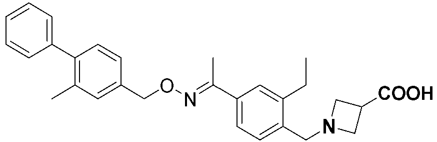 |
| 23 | 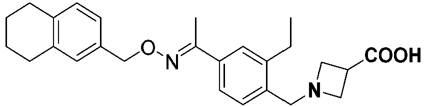 |
| 24 | 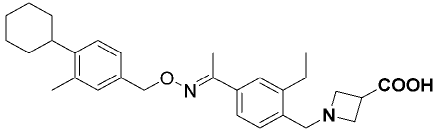 |
| 25 | 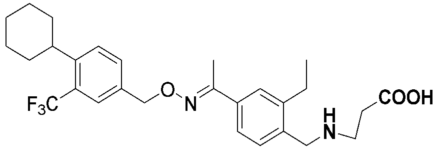 |
| 26 | 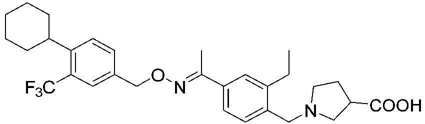 |
| 27 | 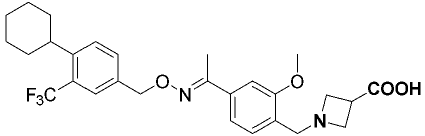 |
| 28 | 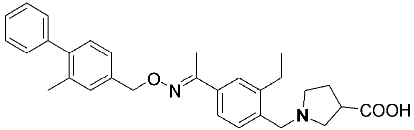 |
| 29 | 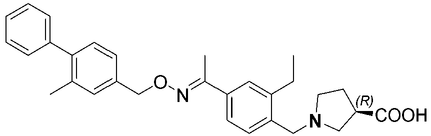 |
| 30 | 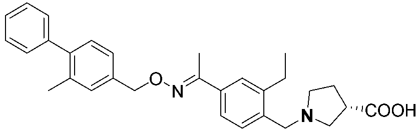 |
| 31 | 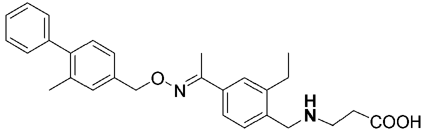 |
| 32 | 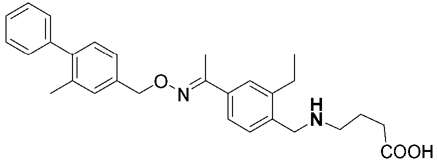 |
| 33 | 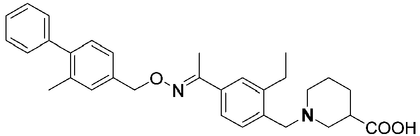 |
| 34 | 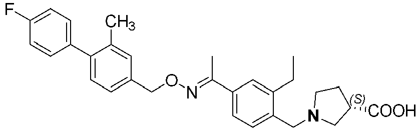 |
| 35 | 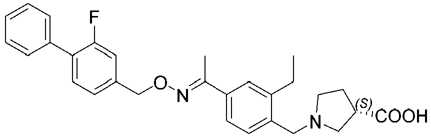 |
| 36 | 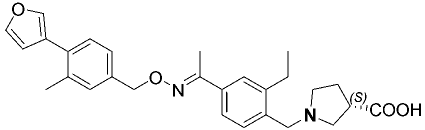 |
| 37 | 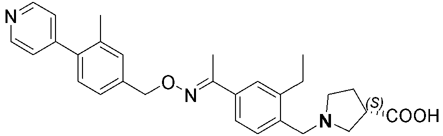 |
| 38 | 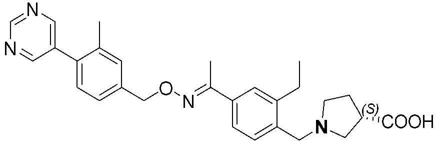 |
| 39 | 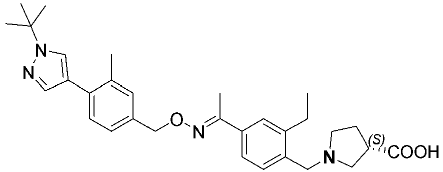 |
| 40 | 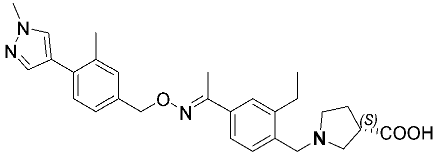 |
| 41 | 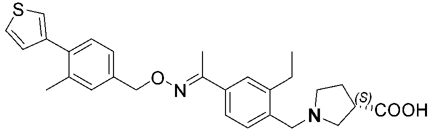 |
| 42 | 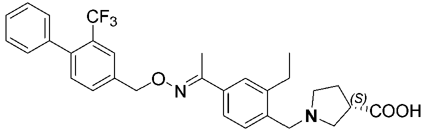 |
| 43 | 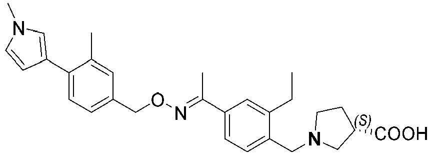 |
| 44 | 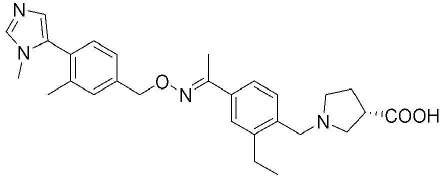 |
| 45 | 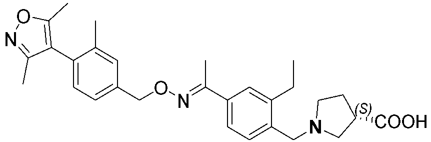 |
| 46 | 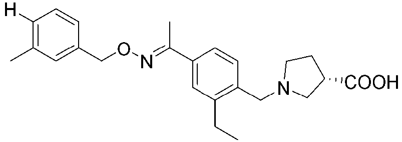 |
| 47 | 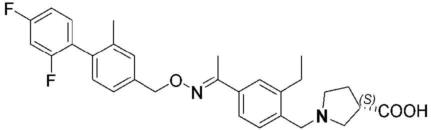 |
| 48 | 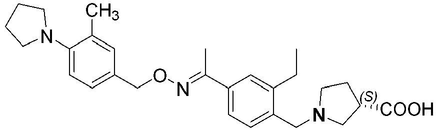 |
| 49 | 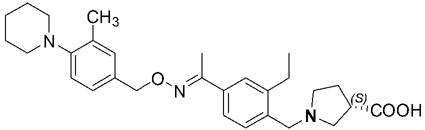 |
| 50 | 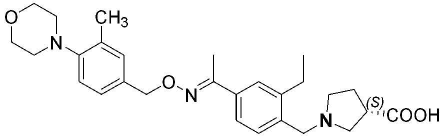 |
| 51 | 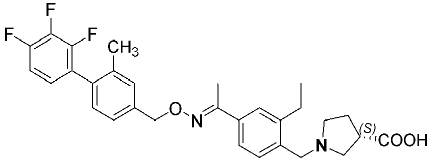 |
| 52 | 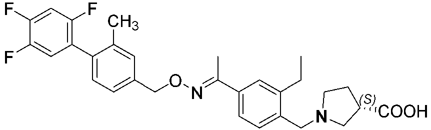 |
| 53 | 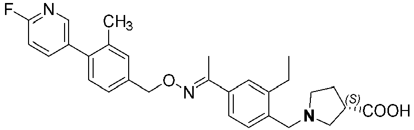 |
| 54 | 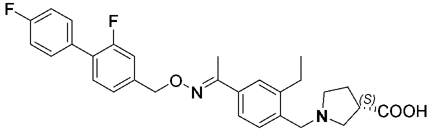 |
| 55 | 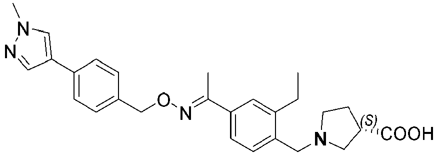 |
| 56 | 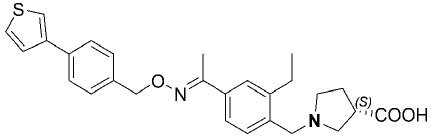 |
| 57 | 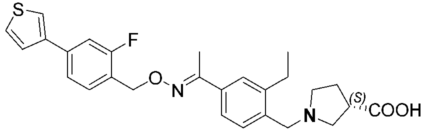 |
| 58 | 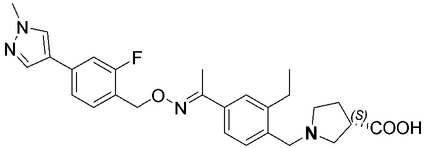 |
| 59 | 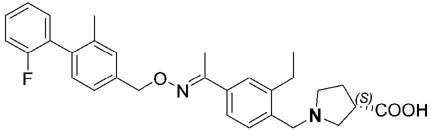 |
| 60 | 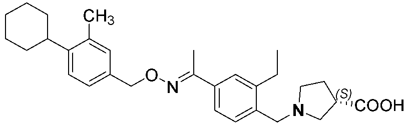 |
| 61 | 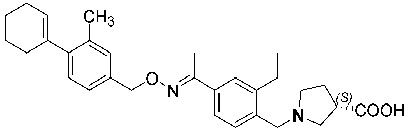 |
| 62 | 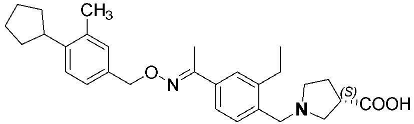 |
| 63 | 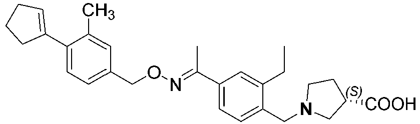 |
| 64 | 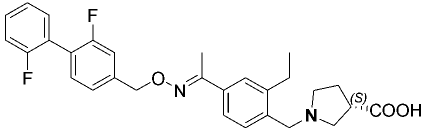 |
| 65 | 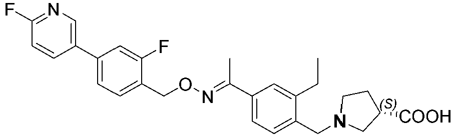 |
| 66 | 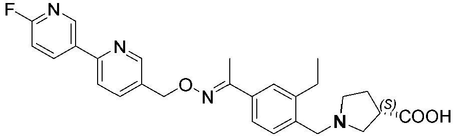 |
| 67 | 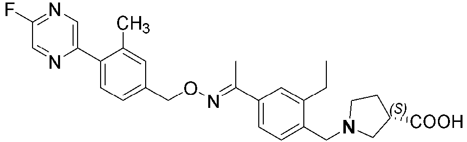 |
| 68 | 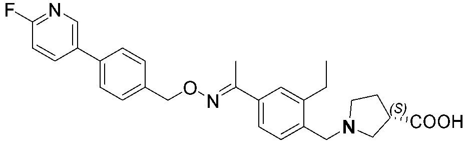 |
| 69 | 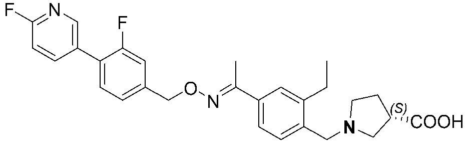 |
| 70 | 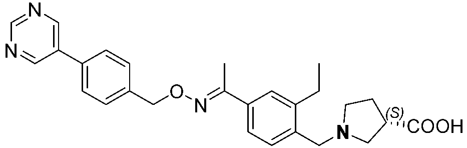 |
| 71 | 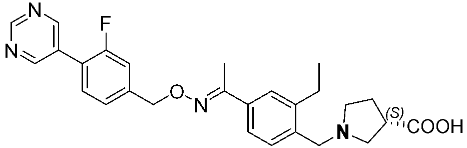 |
| 72 | 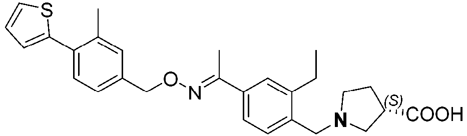 |
| 73 | 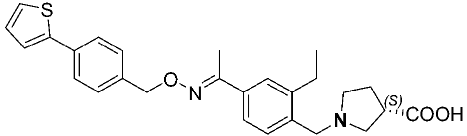 |
| 74 | 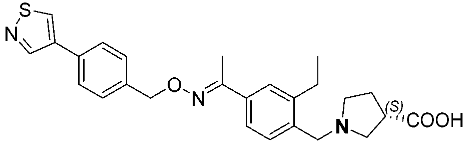 |
| 75 | 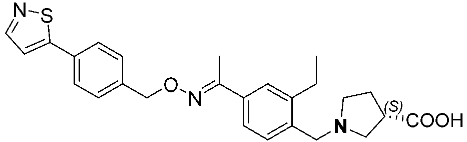 |
| 76 | 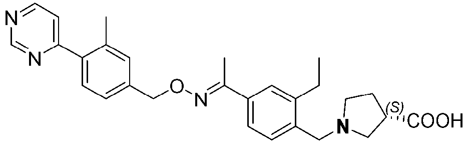 |
| 77 | 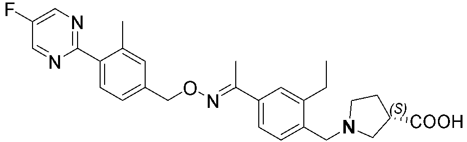 |
| 78 | 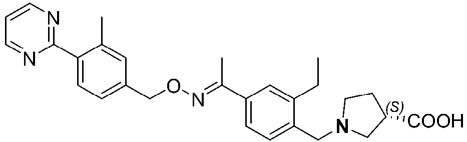 |
| 79 | 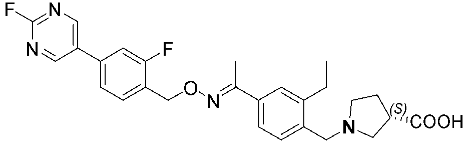 |
| 80 | 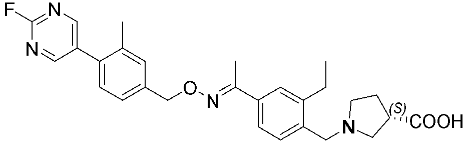 |
| 81 | 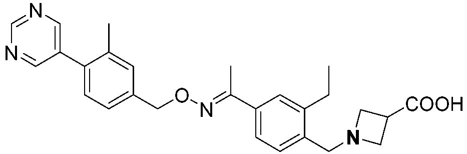 |
| 82 | 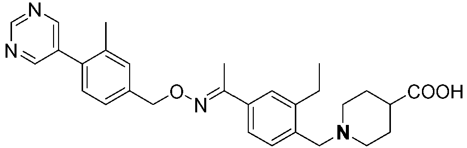 |
| 83 | 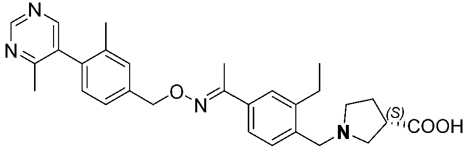 |
| 84 | 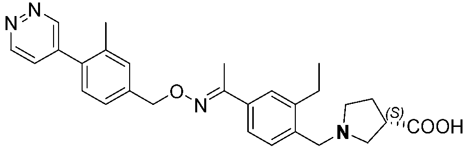 |
| 85 | 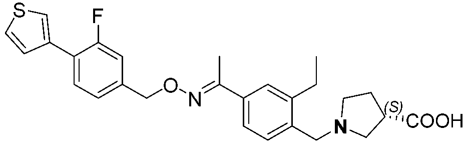 |
| 86 | 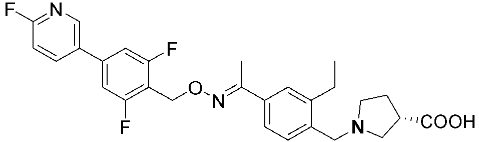 |
| 87 | 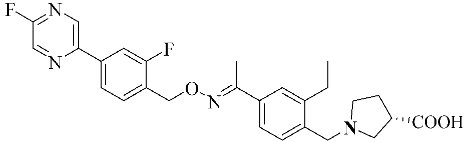 |
| 88 | 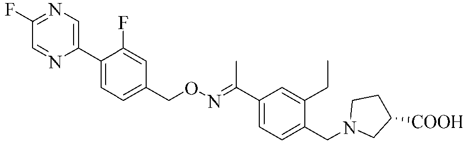 |
| 89 | 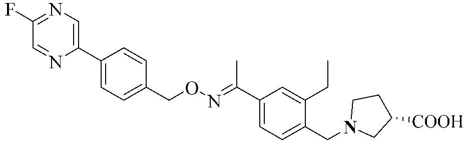 |
| 90 | 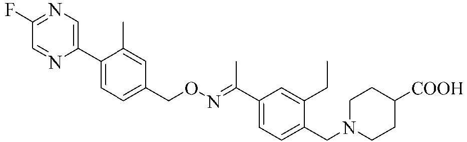 |
| 91 | 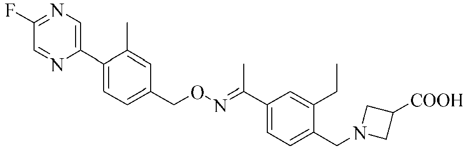 |
| 92 | 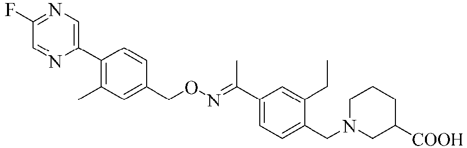 |
| 93 | 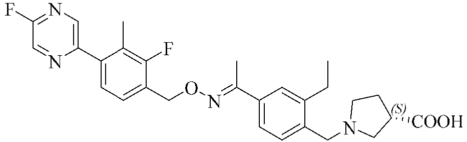 |
| 94 | 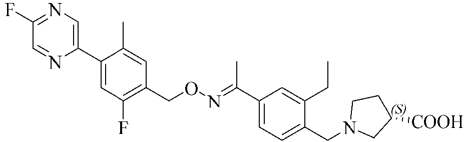 |
| 95 | 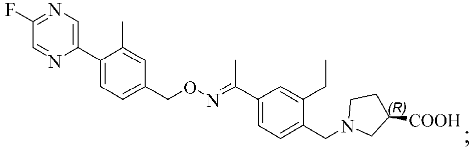 |

preferably, following compounds:

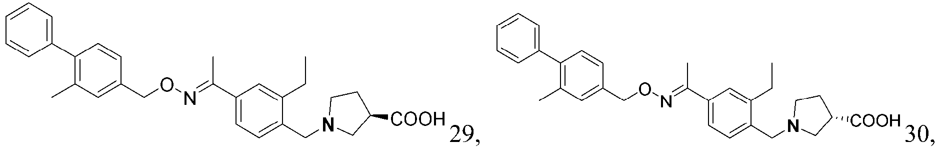

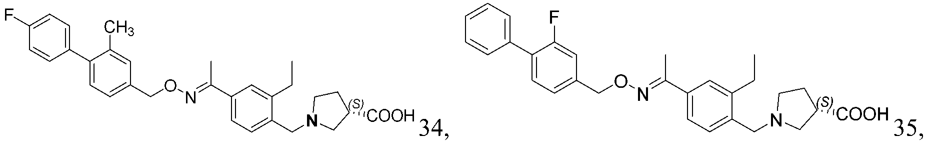

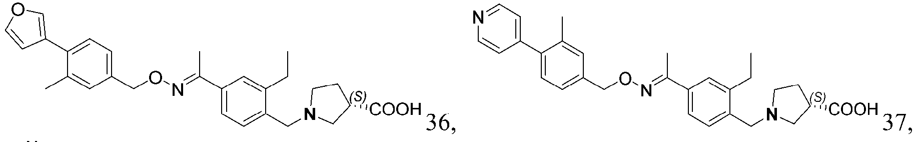

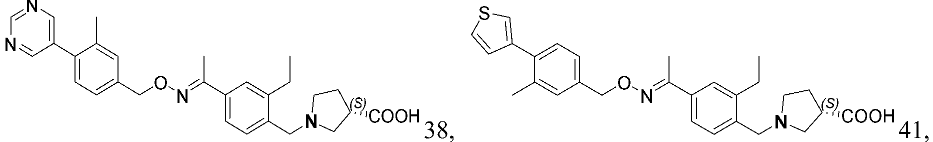

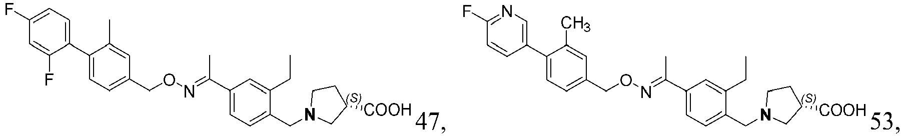

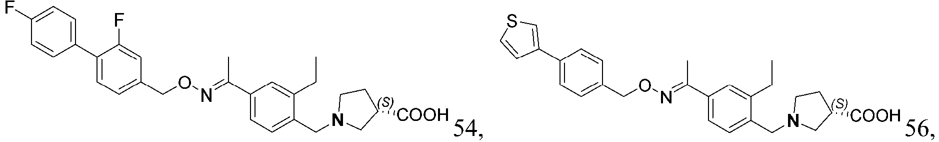

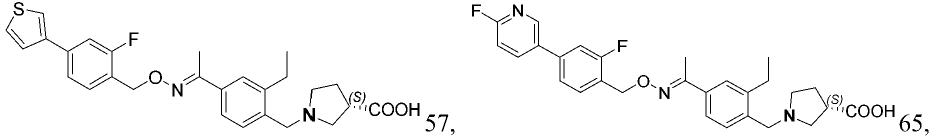

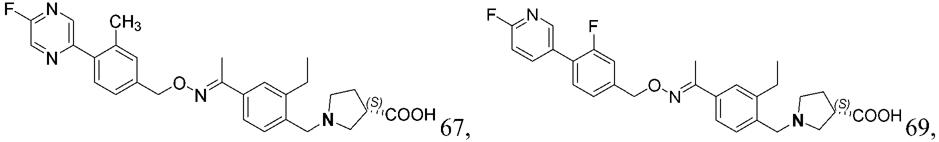

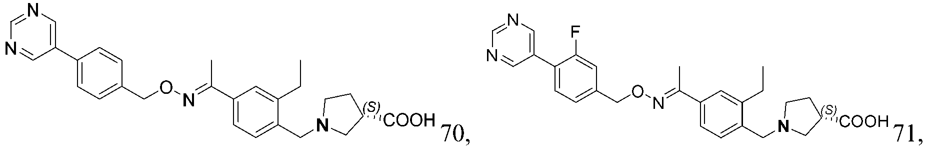

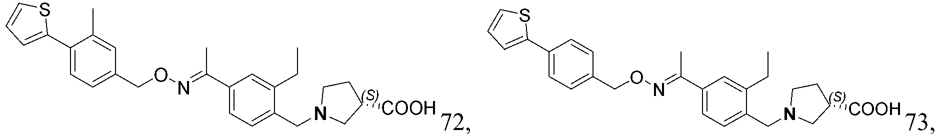

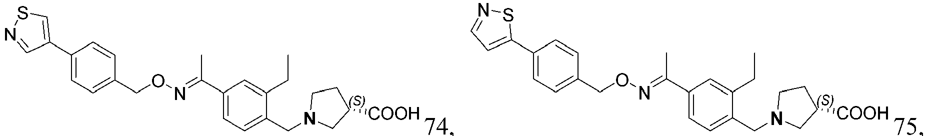

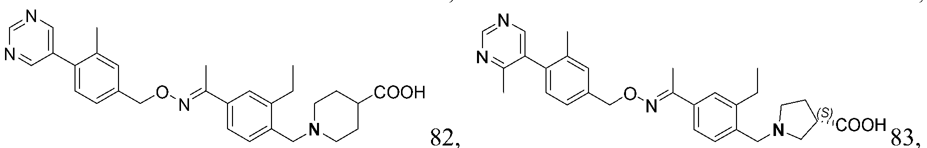

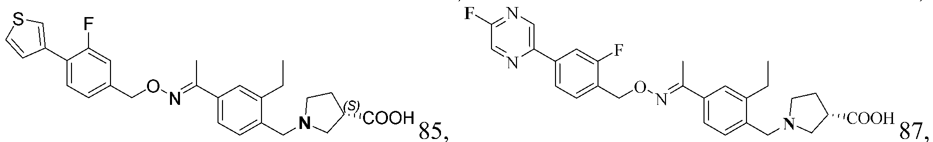

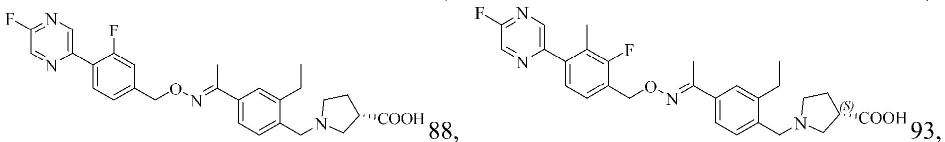

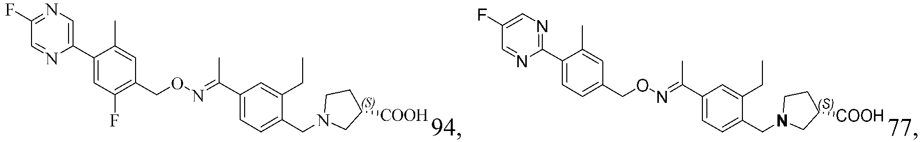

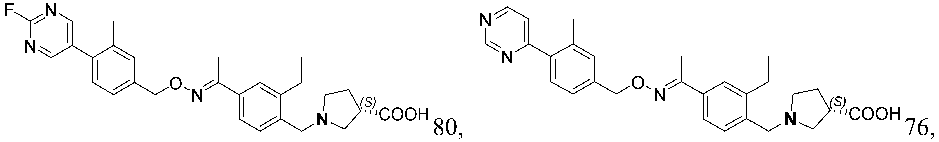

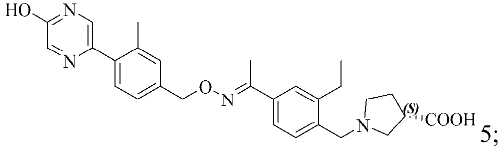

More preferably, following compounds:

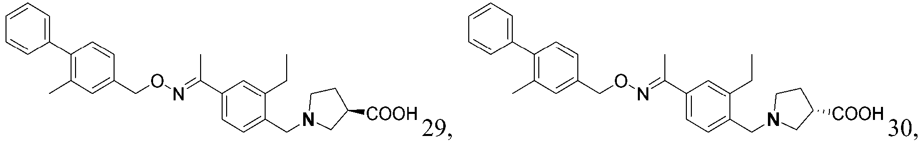

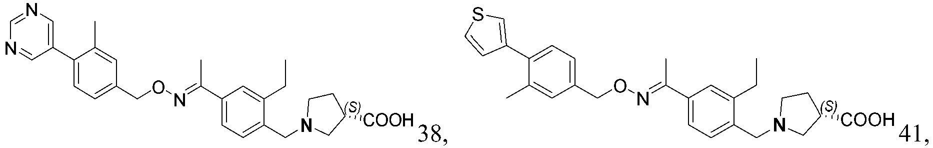

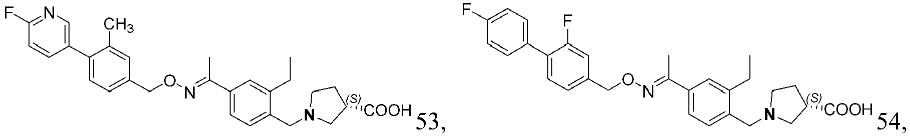

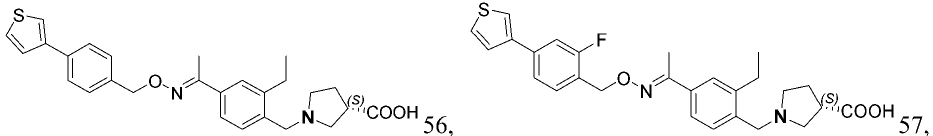

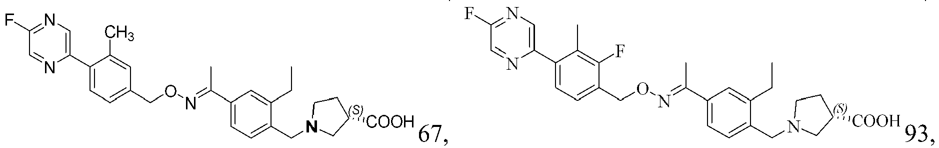

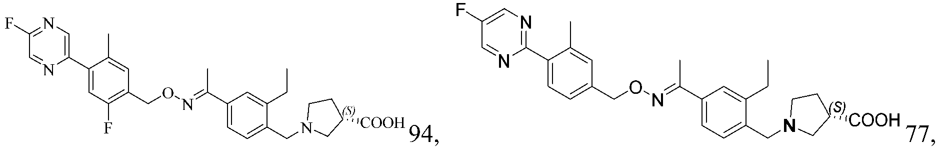

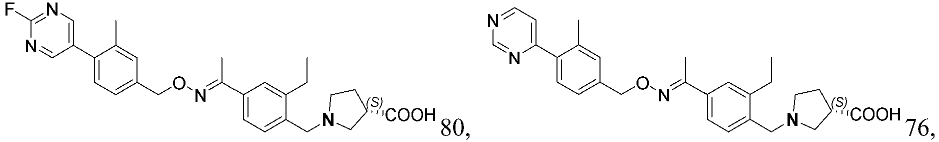

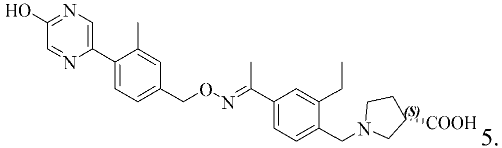

Based on the compounds of the present invention, the present invention provides a pharmaceutical composition, which contains a therapeutically effective amount of the compound of the present invention or a pharmaceutically acceptable salt thereof, as well as a pharmaceutically acceptable carrier or excipient.

Examples of pharmaceutically acceptable salts of the compounds of the present invention include, but are not limited to, inorganic and organic acid salts, such as hydrochloride, hydrobromide, sulfate, citrate, lactate, tartrate, maleate, fumarate, mandelate, and oxalate; as well as inorganic and organic base salts formed with bases, such as sodium hydroxide, tris(hydroxymethyl)aminomethane (TRIS, tromethamine), and N-methylglucamine.

The pharmaceutical composition of the present invention can be formulated into preparations suitable for various administration routes, including but not limited to those formulated for parenteral, subcutaneous, intravenous, intramuscular, intraperitoneal, transdermal, oral, intrathecal, intracranial, nasal, or topical administration, for the treatment of tumors and other diseases. The administered dosage is an amount effective to ameliorate or eliminate one or more conditions. For the treatment of a specific disease, an effective amount is a dosage sufficient to ameliorate or alleviate in some way the symptoms associated with the disease. Such an amount may be administered as a single dose or according to an effective treatment regimen. The administered dosage may cure the disease, but administration is usually intended to ameliorate the symptoms of the disease. Repeated administration is generally required to achieve the desired improvement in symptoms. The dosage will be determined according to the patient's age, health and weight, the type of concurrent treatment, the frequency of treatment, and the desired therapeutic effect.

The pharmaceutical preparation of the present invention can be administered to any mammal, as long as they can obtain the therapeutic effect of the compound of the present invention. Among these mammals, humans are the most important.

The compounds of the present invention or a pharmaceutical composition thereof can be used for the preparation of S1PR regulators. In a specific embodiment, the S1PR regulators are S1PR4 agonists and S1PR4 mutant agonists. Therefore, the compounds of the present invention or a pharmaceutical composition thereof can be used for treating various diseases mediated by S1PR4 (including its mutants), including myopathies or autoimmune diseases. In a specific embodiment, the myopathies mediated by S1PR4 (including its mutants) are idiopathic inflammatory myopathies, infectious myopathies, drug-induced myopathies, toxic myopathies, hereditary myopathies, etc., specifically including (but not limited to) Duchenne muscular dystrophy, pseudohypertrophic muscular dystrophy, limb-girdle muscular dystrophy, facioscapulohumeral muscular dystrophy, distal muscular dystrophy, myotonic dystrophy, congenital muscular dystrophy, Emery-Dreifuss muscular dystrophy, and oculopharyngeal muscular dystrophy.

The pharmaceutical preparations of the present invention can be manufactured in a known way. For example, they can be produced by traditional mixing, granulating, tabletting, dissolving, or freeze-drying processes. When manufacturing oral preparations, solid excipients can be combined with the active compound, and the mixture can be optionally ground. If needed or necessary, after adding an appropriate amount of auxiliaries, the granular mixture is processed to obtain tablets or dragee cores.

Suitable excipients are, especially fillers, such as sugars like lactose or sucrose, mannitol or sorbitol; cellulose preparations or calcium phosphates, such as tricalcium phosphate or dicalcium phosphate; and binders, such as starch pastes including corn starch, wheat starch, rice starch, potato starch, gelatin, tragacanth, methyl cellulose, hydroxypropyl methyl cellulose, sodium carboxymethyl cellulose, or polyvinylpyrrolidone. If necessary, disintegrants can be added, such as the starches mentioned above, as well as carboxymethyl starch, crospovidone, agar, or alginic acid or its salts, such as sodium alginate. Auxiliaries, especially flow regulators and lubricants, for example, silica, talc, stearates such as calcium magnesium stearate, stearic acid or polyethylene glycol. If needed, a suitable coating that can resist gastric juice can be provided for the tablet core. For this purpose, concentrated sugar solutions can be applied. This solution can contain gum arabic, talc, polyvinylpyrrolidone, polyethylene glycol and/or titanium dioxide, lacquer solutions and suitable organic solvents or solvent mixtures. To prepare gastric juice-resistant coatings, appropriate cellulose solutions can be used, such as cellulose acetate phthalate or hydroxypropyl methyl cellulose phthalate. Dyes or pigments can be added to the coating of tablets or tablet cores, for example, to identify or characterize combinations of active ingredient dosages.

Based on the above compounds and pharmaceutical compositions, the present invention further provides a method for treating diseases mediated by S1PR4, comprising administering the compound or pharmaceutical composition of the present invention to a subject in need thereof.

The administration methods include, but are not limited to, various administration methods well known in the art, which can be determined according to the actual situation of a patient. These methods include, but are not limited to, parenteral, subcutaneous, intravenous, intramuscular, intraperitoneal, transdermal, oral, intrathecal, intracranial, nasal or topical administration routes.

In view of the fact that the compound of the present invention or a pharmaceutical composition thereof can be used to prepare S1PR regulators, the present invention also provides a method for regulating S1PR in a subject, comprising administering an effective amount of the compound of the present invention, or an optical isomer, pharmaceutically acceptable salt or pharmaceutical composition thereof to a subject in need thereof. In a specific embodiment, the method is a method for activating S1PR4 in a subject; more preferably, the method activates S1PR4 in a subject without activating S1PR1.

**Advantages of the present invention:**
1. The compound provided in the present invention is a novel S1PR regulator, especially a selective S1PR4 receptor regulator;
2. The compound provided in the present invention has excellent biological activity on S1PR4 and S1PR4 mutant proteins;
3. The compound provided in the present invention lays a foundation for the development of medicaments that can target the S1PR4 target, has great prospects for industrialization and commercialization as well as market value, and has significant economic benefits.

The following further describes the technical solution of the present invention in conjunction with specific implementation cases, however the following examples do not constitute a limitation to the present invention. All application methods adopted based on the principles and technical means of the present invention shall fall within the scope of the present invention. For the experimental methods in the following examples where specific conditions are not indicated, they are usually carried out in accordance with conventional conditions or the conditions suggested by the manufacturer. Unless otherwise specified, percentages and parts are calculated by weight.

### Material and method

### Compounds of the present invention are synthesized as follows:

### Example 1

### Synthesis of compounds

### Synthesis of 4-(bromomethyl)-2-methyl-1,1'-biphenyl

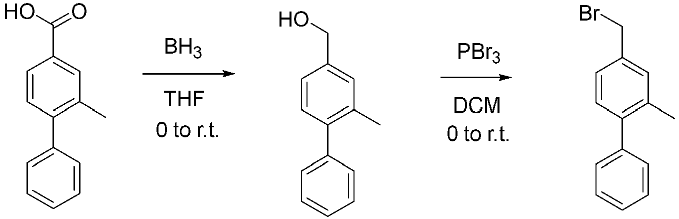

3-methyl-4-phenylbenzoic acid (2.00 g, 9.40 mmol) was dissolved in 10 mL of anhydrous tetrahydrofuran, and stirred in an ice bath. 1.0 M borane-tetrahydrofuran solution (14 mL, 14.20 mmol) was added dropwise. Upon the dropwise addition, the mixture was thawed to room temperature and stirred. After 2 hours, the reaction was complete, and quenched by adding water dropwise, then extracted with dichloromethane, washed with saturated brine. the organic phases were combined, dried over anhydrous sodium sulfate, and concentrated under a reduced pressure to obtain 1.50 g of a colorless oil with a yield of 80.6%. The product was directly used in the next step without further purification. LC-MS (ESI): m/z: 199.1 (M+H)+.

(2-methyl-[1,1'-biphenyl]-4-yl)methanol (1.50 g, 7.60 mmol) was dissolved in 10 mL of dichloromethane, and phosphorus tribromide (820 mg, 3.10 mmol) was added dropwise under ice bath. Upon the dropwise addition, the mixture was thawed to room temperature and stirred. After 0.5 hour, the reaction was complete, quenched with water, extracted with dichloromethane, and washed with saturated brine. The organic phases were combined, dried over anhydrous sodium sulfate, and concentrated under a reduced pressure to obtain 1.80 g of a colorless oil with a yield of 91.0%.

¹H NMR (400 MHz, DMSO-*d*₆) δ 7.45 (dd, *J* = 14.9, 7.9 Hz, 2H), 7.39-7.30 (m, 5H), 7.18 (d, *J* = 7.8 Hz, 1H), 4.72 (s, 2H), 2.21 (s, 3H). LC-MS (ESI): m/z: 261.3 (M+H)⁺.

### Synthesis of (E)-Ethyl N-((2-methyl-[1,1'-biphenyl]-4-yl)methoxy)acetamidocarbamate

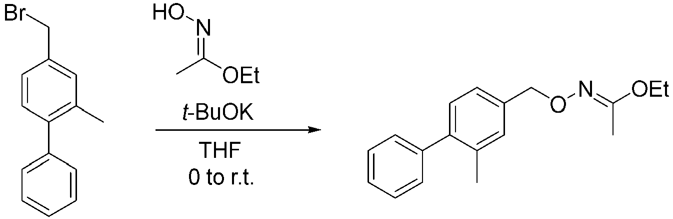

Ethyl acetohydroxamate (400 mg, 3.90 mmol) was dissolved in 10 mL of tetrahydrofuran solution and then placed in an ice bath. A 1.0 M solution of potassium tert-butoxide in tetrahydrofuran (15.2 mL, 15.20 mmol) was added dropwise to the reaction system. Upon the dropwise addition, the reaction system was stirred in the ice bath for another 0.5 hours. 4-(bromomethyl)-2-methyl-1,1'-biphenyl (1.01 g, 3.90 mmol) was dissolved in 5 mL of tetrahydrofuran and then added dropwise to the reaction system. Upon the dropwise addition, the mixture was thawed to room temperature and stirred, with the reaction progress monitored by TLC. After the reaction was completed, water was added to quench the reaction, followed by extraction with dichloromethane and washing with saturated brine. The combined organic phases were dried over anhydrous sodium sulfate, concentrated, and purified by silica gel column chromatography to obtain 821 mg of a colorless oily liquid with a yield of 74.4%.

¹H NMR (400 MHz, DMSO-*d*₆) δ 7.43 (t, *J* = 7.3 Hz, 2H), 7.38-7.30 (m, 3H), 7.27-7.16 (m, 3H), 4.89 (s, 2H), 3.95 (q, *J* = 7.5 Hz, 2H), 2.22 (s, 3H), 1.90 (s, 3H), 1.20 (t, *J* = 7.5 Hz, 3H).

### Synthesis of (E)-1-(3-ethyl-4-(hydroxymethyl)phenyl)ethan-1-one O-((2-methyl-[1,1'-biphenyl]-4-yl)methyl)oxime

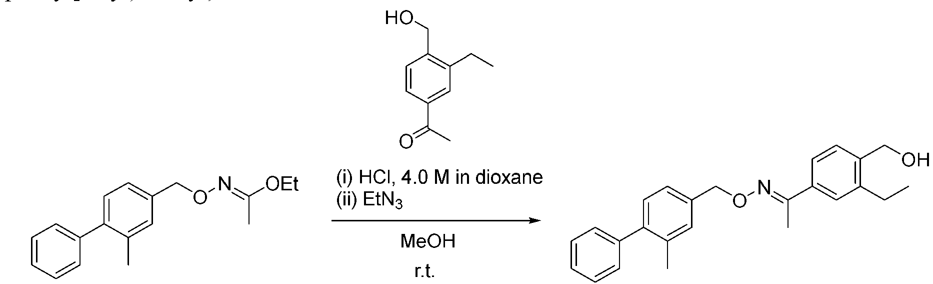

Ethyl (E)-*N*-((2-methyl-[1,1'-biphenyl]-4-yl)methoxy)acetimidate (200 mg, 0.70 mmol) was dissolved in 5 mL of methanol. A 4.0 M solution of hydrogen chloride in dioxane (0.4 mL, 0.30 mmol) was added dropwise to the reaction mixture. After stirring for 0.5 hours, triethylamine was added dropwise to adjust the pH to between 4 and 6. Then, 1-(3-ethyl-4-(hydroxymethyl)phenyl)ethanone (125 mg, 0.70 mmol) was dissolved in 2 mL of methanol and added dropwise to the reaction system. The progress of the reaction was monitored by TLC. After the reaction was completed, water was added to quench the reaction, followed by extraction with dichloromethane and washing with saturated brine. The combined organic phases were dried over anhydrous sodium sulfate, concentrated, and purified by silica gel column chromatography to obtain a colorless oily liquid (140 mg) with a yield of 53.6%.

¹H NMR (400 MHz, CDCl₃) δ 7.46 (s, 1H), 7.42 (dd, *J* = 8.0, 1.6 Hz, 1H), 7.36-7.27 (m, 4H), 7.29-7.18 (m, 3H), 7.18-7.06 (m, 2H), 5.18 (s, 2H), 4.66 (s, 2H), 2.65 (q, *J* = 7.5 Hz, 2H), 2.21 (s, 3H), 1.50 (s, 3H), 1.18 (t, *J* = 7.5 Hz, 3H).

### Synthesis of (E)-2-ethyl-4-(1-((2-methyl-[1,1'-biphenyl]-4-yl)methoxy)iminoethyl)benzaldehyde

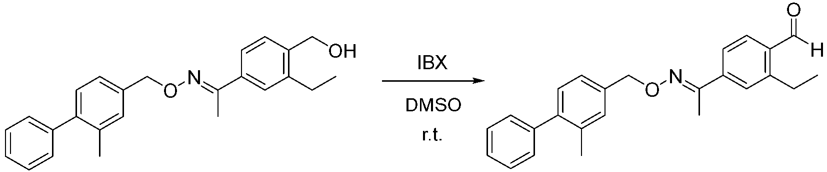

(*E*)-1-(3-ethyl-4-(hydroxymethyl)phenyl)ethan-1-one *O*-((2-methyl-[1,1'-biphenyl]-4-yl)methyl)oxime (112 mg, 0.30 mmol) was dissolved in 3 mL of DMSO solvent. 2-iodoxybenzoic acid (IBX) oxidant (84 mg, 0.30 mmol) was added. Then the reaction system was stirred at room temperature for approximately 2 hours. The reaction progress was monitored by TLC. After the reaction is completed, the reaction was quenched by adding water, extracted with dichloromethane, and washed with saturated brine. The organic phases were combined, dried over anhydrous sodium sulfate, and concentrated to obtain a colorless oily liquid (99 mg) with a yield of 89.1%.

¹H NMR (400 MHz, CDCl₃) δ 10.29 (s, 1H), 7.82 (d, *J* = 6.4 Hz, 1H), 7.64 (dd, *J* = 12.9, 5.5 Hz, 1H), 7.60 (d, *J* = 9.7 Hz, 1H), 7.40 (dd, *J* = 18.7, 11.4 Hz, 2H), 7.36-7.30 (m, 5H), 7.25-7.19 (m, 1H), 5.29 (s, 2H), 3.09 (q, *J* = 7.5 Hz, 2H), 2.31 (s, 3H), 2.30 (s, 3H), 1.33 (t, *J* = 7.5 Hz, 3H).

### Synthesis of (S, E)-1-(2-ethyl-4-(1-((2-methyl-[1,1'-biphenyl]-4-yl)methoxy)imino)ethyl)benzyl)pyrrolidine-3-carboxylic acid (Compound 30)

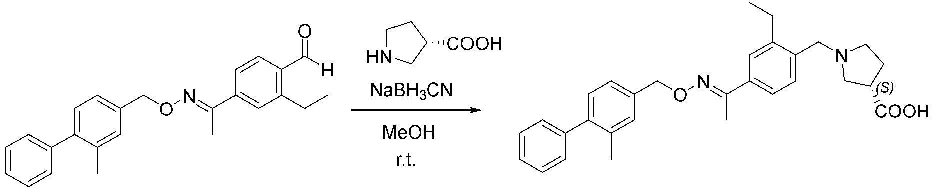

(*E*)-2-ethyl-4-(1-((2-methyl-[1,1'-biphenyl]-4-yl)methoxy)iminoethyl)benzaldehyde (74 mg, 0.20 mmol) was dissolved in 5 mL of methanol, 1 drop of acetic acid was added, and then *S-*pyrrolidine-3-carboxylic acid (35 mg, 0.30 mmol) was added. The reaction system was stirred at room temperature for 3 minutes, and then sodium cyanoborohydride (19 mg, 0.30 mmol) was added. The reaction progress was monitored by TLC. After the reaction is complete, the reaction system was distilled under a reduced pressure, extracted by adding water and dichloromethane, and washed with saturated brine. The organic phases were combined, dried over anhydrous sodium sulfate, concentrated, and pass through a silica gel column to obtain 29 mg of a white solid with a yield of 30.8%.

¹H NMR (400 MHz, CD₃OD) δ 7.57 (s, 1H), 7.51 (d, *J* = 8.0 Hz, 1H), 7.44-7.35 (m, 3H), 7.33-7.26 (m, 5H), 7.17 (d, *J* = 7.7 Hz, 1H), 5.20 (s, 2H), 4.02 (s, 2H), 3.19 (t, *J* = 8.6 Hz, 1H), 3.11-3.00 (m, 3H), 2.90 (dd, *J* = 17.2, 7.9 Hz, 1H), 2.79 (q, *J* = 7.5 Hz, 2H), 2.26 (s, 3H), 2.24 (s, 3H), 2.15 (dd, *J* = 14.6, 7.3 Hz, 2H), 1.23 (t, *J* = 7.5 Hz, 3H). ¹³C NMR (151 MHz, CD₃OD) δ 179.95, 154.70, 143.25, 141.79, 141.48, 137.08, 136.62, 134.91, 130.01, 129.84, 129.31, 128.79, 127.77, 126.49, 126.28, 125.37, 123.57, 75.60, 57.40, 55.54, 53.76, 44.56, 28.06, 25.20, 19.22, 14.48, 11.50. HRMS (ESI) (m/z): [M+H]⁺ calcd for C₃₀H₃₄N₂O₃, 471.2648; found: 471.2650. HPLC Purity: 98.16%, retention time: 1.52 min.

The synthetic route of representative compound 13 is as follows:

### Synthesis of (E)-Ethyl N-((3-(trifluoromethyl)benzyl)oxy)acetimidate

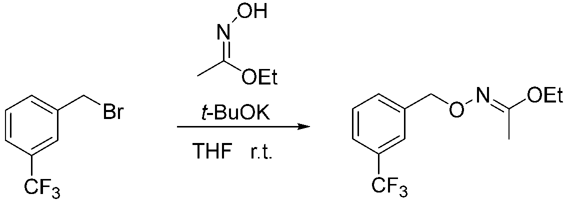

Ethyl acetohydroxamate (865 mg, 8.40 mmol) was dissolved in 10 mL of tetrahydrofuran solution and then placed in an ice bath. A 1.0 M solution of potassium tert-butoxide in tetrahydrofuran (17 mL, 16.70 mmol) was added dropwise to the reaction system. Upon the dropwise addition, the reaction system was stirred in the ice bath for another 0.5 hours. 3-(trifluoromethyl)benzyl bromide (1.99 g, 8.40 mmol) was dissolved in 5 mL of tetrahydrofuran and then added dropwise to the reaction system. Upon the dropwise addition, the mixture was stirred at room temperature, and the progress of the reaction was monitored by TLC. After the reaction was completed, water was added to quench the reaction, followed by extraction with dichloromethane and washing with saturated brine. The combined organic phases were dried over anhydrous sodium sulfate, concentrated, and passed through a silica gel column to obtain 1.81 g of a colorless oily liquid with a yield of 82.6%.

¹H NMR (400 MHz, CDCl₃) *δ* 7.63 (s, 1H), 7.54 (d, *J* = 7.1 Hz, 2H), 7.45 (t, *J* = 7.7 Hz, 1H), 4.97 (s, 2H), 3.98 (q, *J* = 7.1 Hz, 2H), 1.95 (s, 3H), 1.24 (t, *J* = 7.1 Hz, 3H).

### Synthesis of (E)-1-(3-ethyl-4-(hydroxymethyl)phenyl)ethan-1-one O-(3-(trifluoromethyl)benzyl)oxime

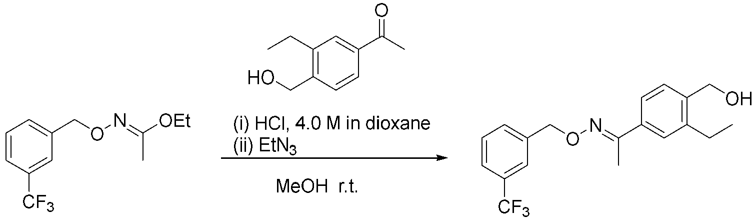

ethyl (*E*)-N-((3-(trifluoromethyl)benzyl)oxy)acetimidate (157 mg, 0.60 mmol) was dissolved in 5 mL of methanol. 0.4 mL of 4.0 M hydrogen chloride was added in dioxane solution dropwise to the reaction mixture. The reaction system was stirred for 0.5 hours, and then triethylamine was added dropwise to adjust the pH to between 4-6. Then, 1-(3-ethyl-4-(hydroxymethyl)phenyl)ethanone (107 mg, 0.60 mmol) was dissolved in 2 mL of methanol and added dropwise to the reaction system. The reaction progress was monitored by TLC. After the reaction is completed, the reaction was quenched by adding water, extracted with dichloromethane, and washed with saturated brine. The organic phases were combined, dried over anhydrous sodium sulfate, concentrated, and passed through a silica gel column to obtain 150 mg of a colorless oily liquid with a yield of 71.1%.

¹H NMR (400 MHz, CDCl₃) *δ* 7.69 (s, 1H), 7.59 (dd, *J* = 12.7, 7.9 Hz, 3H), 7.51-7.46 (m, 2H), 7.37 (d, *J* = 8.0 Hz, 1H), 5.28 (s, 2H), 4.74 (s, 1H), 4.72 (s, 2H), 2.71 (q, *J* = 7.6 Hz, 2H), 2.28 (s, 3H), 1.24 (t, *J* = 7.5 Hz, 3H).

### Synthesis of (E)-2-ethyl-4-(1-(((3-(trifluoromethyl)phenyl)oxy)imino)ethyl)benzaldehyde

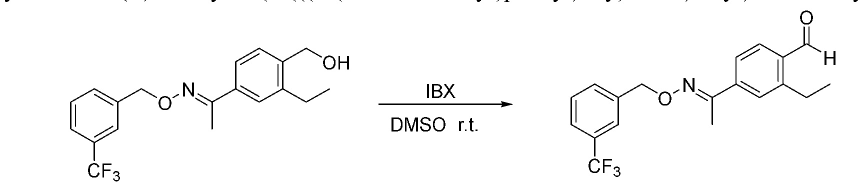

(*E*)-1-(3-ethyl-4-(hydroxymethyl)phenyl)ethan-1-one *O*-(3-(trifluoromethyl)benzyl)oxime (211 mg, 0.60 mmol) was dissolved in 3 mL of DMSO solvent. IBX oxidant (308 mg, 1.10 mmol) was added. Then, the mixture was stirred at room temperature for approximately 2 hours. The progress of the reaction was monitored by TLC. After the reaction was completed, water was added to quench the reaction, followed by extraction with dichloromethane and washing with saturated brine. The combined organic phases were dried over anhydrous sodium sulfate, concentrated, and then purified by silica gel column chromatography to obtain a colorless oily liquid (126 mg) with a yield of 60.0%.

¹H NMR (400 MHz, CDCl₃) *δ* 10.29 (s, 1H), 7.82 (d, *J* = 8.1 Hz, 1H), 7.69 (s, 1H), 7.64-7.53 (m, 4H), 7.50 (d, *J* = 7.7 Hz, 1H), 5.31 (s, 2H), 3.08 (d, *J* = 7.5 Hz, 2H), 2.30 (s, 3H), 1.28 (t, *J* = 7.5 Hz, 3H).

### Synthesis of (E)-1-(2-ethyl-4-(1-((3-(trifluoromethyl)phenyl)oxy)imino)ethyl)benzyl)azetidine-3-carboxylic acid (Compound 13)

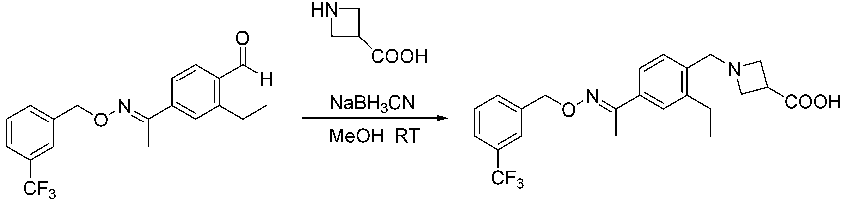

(*E*)-2-ethyl-4-(1-(((3-(trifluoromethyl)phenyl)oxy)imino)ethyl)benzaldehyde (105 mg, 0.30 mmol) was dissolved in 5 mL of methanol, 1 drop of acetic acid was added, and then 3-azetidinecarboxylic acid (61 mg, 0.60 mmol) added. After the reaction system was stirred at room temperature for 3 minutes, sodium cyanoborohydride (38 mg, 0.60 mmol) was added. The reaction progress was monitored by TLC. After the reaction is complete, the reaction system was distilled under a reduced pressure, an appropriate amount of water was added, extracted with dichloromethane, washed with saturated brine. The organic phases were combined, dried over anhydrous sodium sulfate, concentrated, and passed through a silica gel column to obtain 78 mg of an oily substance with a yield of 60.0%.

¹H NMR (400 MHz, CD₃OD) δ 7.71 (s, 1H), 7.67 (d, *J* = 7.4 Hz, 1H), 7.62-7.51 (m, 3H), 7.47 (dd, *J* = 8.0, 1.6 Hz, 1H), 7.31 (d, *J* = 8.0 Hz, 1H), 5.28 (s, 2H), 3.98 (s, 2H), 3.83 (t, *J* = 8.8 Hz, 2H), 3.67 (t, *J* = 8.4 Hz, 2H), 3.28 (dd, J = 15.2, 6.9 Hz, 1H), 2.73 (q, *J* = 7.5 Hz, 2H), 2.26 (s, 3H), 1.21 (t, *J* = 7.5 Hz, 3H). ¹³C NMR (151 MHz, CD₃OD) δ 178.20, 155.32, 142.88, 139.73, 136.16, 133.52, 131.41, 130.23 (q, *J* = 32.0 Hz), 128.87, 128.82, 126.17, 124.32, 124.31 (q, *J* = 272.0 Hz), 124.06, 123.61, 74.70, 57.68, 35.94, 25.11, 14.28, 11.47. HRMS (ESI) (m/z): [M+H]⁺ calcd for C₂₃H₂₅F₃N₂O₃, 435.1896; found: 435.1897. HPLC Purity: 99.79%, retention time: 1.17 min.

The synthetic route of the representative compound 16 is shown as follows:

### Synthesis of 5-(bromomethyl)-2-isopropoxybenzonitrile

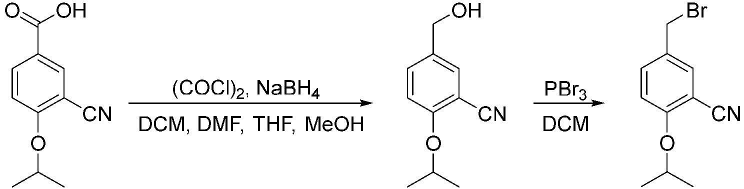

A 2.0 M solution of oxalyl chloride in dichloromethane (14.6 mL, 29.20 mmol) was added to 3-cyano-4-(isopropoxy)benzoic acid (2.01 g, 9.80 mmol) along with one drop of DMF. The reaction mixture was stirred at room temperature for 0.5 hours and then concentrated under a reduced pressure. 3-cyano-4-(isopropoxy)benzoyl chloride (923 mg, 4.50 mmol) was dissolved in 10 mL of tetrahydrofuran and cooled to 0°C. Sodium borohydride (414 mg, 11.20 mmol) was added, followed by 2 mL of methanol. The reaction mixture was stirred at 0°C for 20 minutes and then thawed to room temperature. After 2 hours, the reaction mixture was acidified to pH = 3 with 1.0 M hydrochloric acid. The aqueous layer was extracted with ethyl acetate, and the combined organic layers were dried over anhydrous sodium sulfate and concentrated to obtain a colorless oil (1.12 g) with a yield of 59.9%.

¹H NMR (400 MHz, CDCl₃) *δ* 7.48 (s, 1H), 7.47 (d, *J* = 12 Hz, 1H), 7.45 (s, 1H), 6.92 (d, *J* = 8.6 Hz, 1H), 4.61 (dt, *J* = 12.2, 6.2 Hz, 1H), 4.57 (s, 2H), 1.36 (d, *J* = 6.1 Hz, 6H).

5-(Hydroxymethyl)-2-isopropoxybenzonitrile (993 mg, 5.20 mmol) was dissolved in 10 mL of dichloromethane. Phosphorus tribromide (0.2 mL, 2.10 mmol) was added dropwise under an ice bath. Upon the dropwise addition, the mixture was stirred at room temperature, and the reaction was complete after 0.5 hours. The reaction was quenched by adding water, then extracted with dichloromethane and washed with saturated brine. The combined organic phases were dried over anhydrous sodium sulfate and concentrated to obtain 1.19 g of a colorless oil with a yield of 90.5%.

¹H NMR (400 MHz, CDCl₃) *δ* 7.56 (d, *J* = 2.1 Hz, 1H), 7.51 (dd, *J* = 8.7, 2.2 Hz, 1H), 6.92 (d, *J* = 8.7 Hz, 1H), 4.65 (dt, *J* = 12.0, 6.0 Hz, 1H), 4.42 (s, 2H), 1.39 (d, *J* = 6.1 Hz, 6H).

### Synthesis of Ethyl N-((3-cyano-4-(isopropoxy)benzyl)oxy)acetylcarbamate

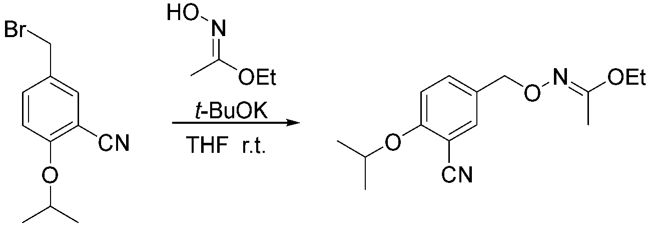

Ethyl acetohydroxamate (402 mg, 3.90 mmol) was dissolved in 10 mL of tetrahydrofuran solution and then placed in an ice bath. A 1.0 M solution of potassium tert-butoxide in tetrahydrofuran (8.0 mL, 8.00 mmol) was added dropwise to the reaction system. Upon the dropwise addition, the reaction system was stirred in the ice bath for another 0.5 hour. 3-cyano-4-isopropoxybenzyl bromide (987 mg, 3.90 mmol) was dissolved in 5 mL of tetrahydrofuran and then added dropwise to the reaction system. Upon the dropwise addition, the mixture was thawed to room temperature and stirred, with the reaction progress monitored by TLC. After the reaction was completed, water was added to quench the reaction, followed by extraction with dichloromethane and washing with saturated brine. The combined organic phases were dried over anhydrous sodium sulfate, concentrated, and purified by silica gel column chromatography to obtain 619 mg of a colorless oily liquid with a yield of 57.5%.

¹H NMR (400 MHz, CDCl₃) *δ* 7.54 (d, *J* = 2.0 Hz, 1H), 7.48 (dd, *J* = 8.7, 2.1 Hz, 1H), 6.92 (d, *J* = 8.7 Hz, 1H), 4.82 (s, 2H), 4.64 (dt, *J* = 12.1, 6.1 Hz, 1H), 3.97 (q, *J* = 7.1 Hz, 2H), 1.92 (s, 3H), 1.39 (d, *J* = 6.1 Hz, 6H), 1.24 (t, *J* = 7.1 Hz, 3H).

### Synthesis of (E)-1-(3-ethyl-4-(hydroxymethyl)phenyl)ethan-1-one-O-(3-cyano-4-(isopropoxy)benzyl)oxime

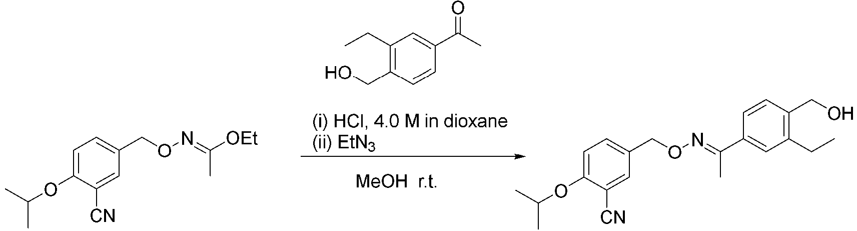

Ethyl *N*-((3-cyano-4-(isopropoxy)benzyl)oxy)acetylcarbamate (138 mg, 0.50 mmol) was dissolved in 5 mL of methanol. A 4.0 M solution of hydrogen chloride in dioxane (0.4 mL, 0.30 mmol) was added dropwise to the reaction mixture. After the reaction system was stirred for 0.5 hours, triethylamine was added dropwise to adjust the pH to between 4 and 6. Then, 1-(3-ethyl-4-(hydroxymethyl)phenyl)ethanone (90 mg, 0.50 mmol) was dissolved in 2 mL of methanol and added dropwise to the reaction system. The progress of the reaction was monitored by TLC. After the reaction was completed, water was added to quench the reaction, followed by extraction with dichloromethane and washing with saturated brine. The combined organic phases were dried over anhydrous sodium sulfate, concentrated, and passed through a silica gel column to obtain a colorless oily liquid (127 mg) with a yield of 69.3%.

¹H NMR (400 MHz, CDCl₃) *δ* 7.61 (d, *J* = 2.0 Hz, 1H), 7.54 (dd, *J* = 8.7, 2.1 Hz, 1H), 7.45 (dd, *J* = 12.1, 4.1 Hz, 2H), 7.38 (d, *J* = 7.9 Hz, 1H), 6.94 (d, *J* = 8.7 Hz, 1H), 5.13 (s, 2H), 4.73 (s, 2H), 4.64 (dt, *J* = 12.1, 6.0 Hz, 1H), 2.72 (q, *J* = 7.6 Hz, 2H), 2.24 (s, 3H), 1.40 (d, *J* = 6.1 Hz, 6H), 1.24 (t, *J* = 7.6 Hz, 3H).

### Synthesis of (E)-5-((((1-(3-ethyl-4-formylphenyl)ethylidene)amino)oxy)methyl)-2-isopropoxybenzonitrile

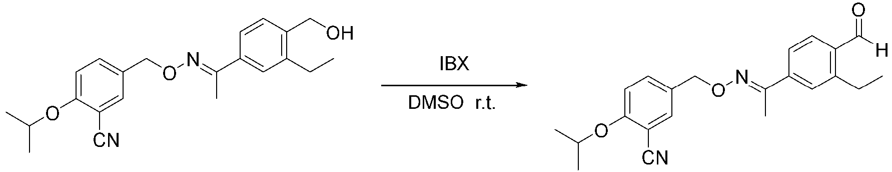

(*E*)-1-(3-ethyl-4-(hydroxymethyl)phenyl)ethan-1-one-*O*-(3-cyano-4-(isopropoxy)benzyl)oxime (147 mg, 0.40 mmol) was dissolved in 4 mL of DMSO solvent, and IBX oxidant (225 mg, 0.80 mmol) was added and stirred at room temperature for approximately 2 hours. The reaction progress was monitored by TLC. After the reaction is completed, the reaction was quenched by adding water, extracted with dichloromethane, and washed with saturated brine. The organic phases were combined, dried over anhydrous sodium sulfate, concentrated, and passed through a silica gel column to obtain 119 mg of a colorless oily liquid with a yield of 81.2%.

¹H NMR (400 MHz, CDCl₃) *δ* 10.28 (s, 1H), 7.82 (d, *J* = 8.1 Hz, 1H), 7.65 - 7.58 (m, 2H), 7.58 - 7.50 (m, 2H), 6.95 (d, *J* = 8.7 Hz, 1H), 5.16 (s, 2H), 4.65 (dt, *J* = 12.1, 6.1 Hz, 1H), 3.08 (q, *J* = 7.5 Hz, 2H), 2.26 (s, 3H), 1.40 (d, *J* = 6.1 Hz, 6H), 1.28 (t, *J* = 7.5 Hz, 3H).

### Synthesis of (E)-1-(2-ethyl-4-(1-((3-cyano-4-(isopropoxy)benzyl)oxy)imino)ethyl)benzyl)azetidine-3-carboxylic acid (Compound 16)

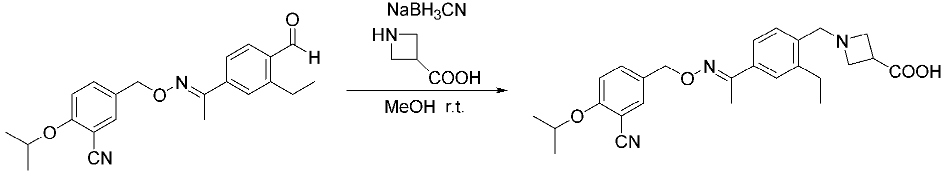

(*E*)-5-((((1-(3-ethyl-4-formylphenyl)ethylidene)amino)oxy)methyl)-2-isopropoxybenzonitrile (109 mg, 0.30 mmol) was dissolved in 5 mL of methanol, 1 drop of acetic acid was added, then 3-azetidinecarboxylic acid (61 mg, 0.60 mmol) was added. The reaction mixture was stirred at room temperature for 3 minutes, afterwards, sodium cyanoborohydride (19 mg, 0.30 mmol) was added. The reaction progress was monitored by TLC. After the reaction is complete, the reaction system was distilled under a reduced pressure, extracted by adding water and dichloromethane, and washed with saturated brine. The organic phases were combined, dried over anhydrous sodium sulfate, concentrated, and passed through a silica gel column to obtain 70 mg of a light-colored solid with a yield of 52.3%.

¹H NMR (400 MHz, CD₃OD) δ 7.64 (d, *J* = 6.7 Hz, 2H), 7.56 (s, 1H), 7.50 (dd, *J* = 8.0, 1.6 Hz, 1H), 7.33 (d, *J* = 8.1 Hz, 1H), 7.16 (d, *J* = 9.4 Hz, 1H), 5.14 (s, 2H), 4.78-4.73 (m, 1H), 4.18 (s, 2H), 3.99 (t, *J* = 9.3 Hz, 2H), 3.90 (t, *J* = 8.6 Hz, 2H), 3.40-3.33 (m, 1H), 2.76 (q, *J* = 7.5 Hz, 2H), 2.23 (s, 3H), 1.38 (s, 3H), 1.37 (s, 3H), 1.23 (t, *J* = 7.5 Hz, 3H). ¹³C NMR (151 MHz, CD₃OD) δ 177.23, 159.53, 154.92, 143.23, 136.96, 134.67, 133.44, 131.00, 129.23, 126.42, 123.80, 116.06, 113.66, 101.88, 74.22, 71.68, 57.49, 56.20, 35.40, 25.12, 20.71, 14.38, 11.39. HRMS (ESI) (m/z): [M+H]⁺ calcd for C₂₆H₃₁N₃O₄, 450.2396; found: 450.2395. HPLC Purity: 96.46%, retention time: 1.02 min.

The synthetic route of Compound 17 is shown as follows:

### Synthesis of (E)-Ethyl N-((4-cyclopentyl-3-(trifluoromethyl)benzyl)oxy)acetimidate

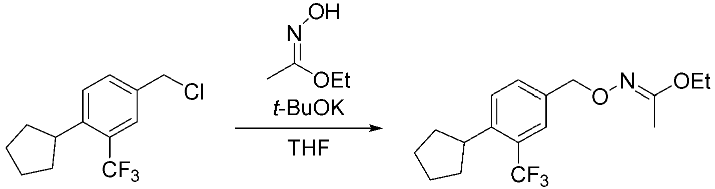

Ethyl acetohydroxamate (196 mg, 1.90 mmol) was dissolved in 5 mL of tetrahydrofuran solution and then placed in an ice bath. A 1.0 M solution of potassium tert-butoxide in tetrahydrofuran (4 mL, 3.90 mmol) was added dropwise to the reaction system. Upon the dropwise addition, the reaction system was stirred in the ice bath for another 0.5 hours. 4-chloromethyl-1-cyclopentyl-2-trifluoromethylbenzene (498 mg, 1.90 mmol) was dissolved in 3 mL of tetrahydrofuran and then added dropwise to the reaction system. Upon the dropwise addition, the mixture was stirred at room temperature, and the progress of the reaction was monitored by TLC. After the reaction was completed, water was added to quench the reaction, followed by extraction with dichloromethane and washing with saturated brine. The organic phases were combined, dried over anhydrous sodium sulfate, concentrated, and then passed through a silica gel column to obtain 532 mg of a colorless oily liquid with a yield of 85.1%.

¹H NMR (400 MHz, CDCl₃) *δ* 7.59 (s, 1H), 7.48 (d, *J* = 8.4 Hz, 1H), 7.43 (d, *J* = 8.1 Hz, 1H), 4.91 (s, 2H), 3.99 (q, *J* = 7.1 Hz, 2H), 3.41-3.28 (m, 1H), 2.07 (m, 2H), 1.94 (s, 3H), 1.89-1.80 (m, 2H), 1.73-1.68 (m, 2H), 1.63-1.56 (m, 2H), 1.25 (t, *J* = 7.1 Hz, 3H).

### Synthesis of (E)-1-(3-ethyl-4-(hydroxymethyl)phenyl)ethan-1-one-O-(4-cyclopentyl-3-(trifluoromethyl)benzyl)oxime

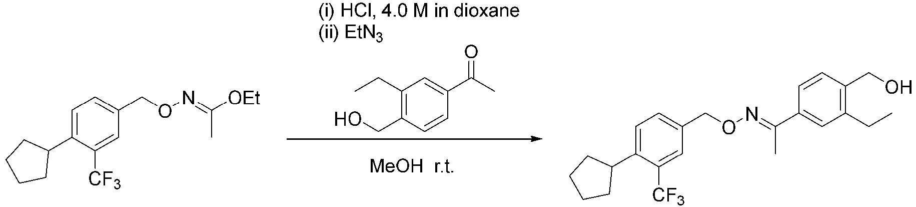

Ethyl (*E*)-N-((4-cyclopentyl-3-(trifluoromethyl)benzyl)oxy)acetimidate (461 mg, 1.40 mmol) was dissolved in 5 mL of methanol. A solution of 4.0 M hydrogen chloride in dioxane (1.0 mL, 0.70 mmol) was added dropwise to the reaction liquid. The reaction liquid was stirred for 0.5 hours, and then triethylamine was added dropwise to adjust the pH to between 4-6. Afterwards, 1-(3-ethyl-4-(hydroxymethyl)phenyl)ethanone (267 mg, 1.50 mmol) was dissolved in 2 mL of methanol and added dropwise to the reaction system. The reaction progress was monitored by TLC. After the reaction is completed, the reaction was quenched by adding water, extracted with dichloromethane, and washed with saturated brine. The organic phases were combined, dried over anhydrous sodium sulfate, concentrated, and passed through a silica gel column to obtain 489 mg of a colorless oily liquid with a yield of 83.4%.

¹H NMR (400 MHz, CDCl₃) *δ* 7.66 (s, 1H), 7.54 (d, *J* = 8.0 Hz, 1H), 7.49 (d, *J* = 6.2 Hz, 1H), 7.45 (d, *J* = 8.6 Hz, 2H), 7.38 (t, *J* = 8.6 Hz, 1H), 5.22 (s, 2H), 4.72 (s, 2H), 3.36 (p, *J* = 8.5 Hz, 1H), 2.71 (q, *J* = 7.6 Hz, 2H), 2.22 (s, 3H), 2.14-2.04 (m, 2H), 1.92-1.81 (m, 2H), 1.77-1.69 (m, 2H), 1.61-1.56 (m, 2H), 1.25 (t, *J* = 7.1 Hz, 3H).

### Synthesis of (E)-4-(1-((4-cyclopentyl-3-(trifluoromethyl)benzyl)oxy)imino)ethyl)-2-ethylbenzaldehyde

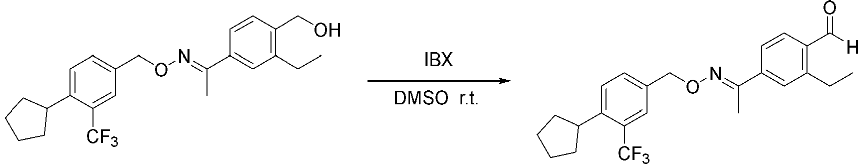

(*E*)-1-(3-ethyl-4-(hydroxymethyl)phenyl)ethan-1-one-*O*-(4-cyclopentyl-3-(trifluoromethyl)benzyl)oxime (503 mg, 1.20 mmol) was dissolved in 3 mL of DMSO solvent. IBX oxidant (952 mg, 2.40 mmol) was added, then stirred at room temperature for approximately 2 hours. The reaction progress was monitored by TLC. After the reaction is completed, the reaction was quenched by adding water, extracted with dichloromethane, and washed with saturated brine. The organic phases were combined, dried over anhydrous sodium sulfate, and passed through a silica gel column to obtain 302 mg of a colorless oily liquid with a yield of 60.3%.

¹H NMR (400 MHz, CDCl₃) *δ* 10.29 (s, 1H), 7.82 (d, *J* = 8.1 Hz, 1H), 7.69-7.59 (m, 2H), 7.59-7.52 (m, 2H), 7.47 (d, *J* = 8.1 Hz, 1H), 5.25 (s, 2H), 3.45-3.30 (m, 1H), 3.08 (q, *J* = 7.5 Hz, 2H), 2.28 (s, 3H), 2.07 (d, *J* = 12.3 Hz, 2H), 1.88-1.80 (m, 2H), 1.73-1.70 (m, 2H), 1.64-1.54 (m, 2H), 1.28 (t, *J* = 7.1 Hz, 3H).

### Synthesis of (E)-1-(4-(1-((4-cyclopentyl-3-(trifluoromethyl)benzyl)oxy)imino)ethyl)-2-ethylbenzyl)azetidine-3-carboxylic acid (Compound 17)

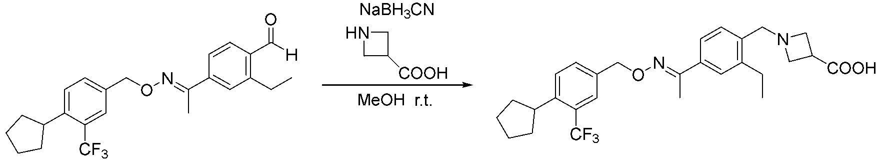

(*E*)-4-(1-((4-cyclopentyl-3-(trifluoromethyl)benzyl)oxy)imino)ethyl)-2-ethylbenzaldehyde (500 mg, 1.20 mmol) was dissolved in 5 mL of methanol, 1 drop of acetic acid was added, and then 3-azetidinecarboxylic acid (242 mg, 2.40 mmol) was added. After the reaction system was stirred at room temperature for 3 minutes, sodium cyanoborohydride (113 mg, 1.80 mmol) was added. The reaction progress was monitored by TLC. After the reaction is complete, the reaction system was distilled under a reduced pressure, and an appropriate amount of water was added, extracted with dichloromethane, and washed with saturated brine. The organic phases were combined, dried over anhydrous sodium sulfate, concentrated, and passed through a silica gel column to obtain 182 mg of a light-colored solid with a yield of 30.2%.

¹H NMR (400 MHz, CD₃OD) δ 7.64 (s, 1H), 7.53 (dt, *J* = 9.8, 5.1 Hz, 4H), 7.38 (t, *J* = 11.5 Hz, 1H), 5.22 (s, 2H), 4.25 (s, 2H), 4.02 (d, *J* = 8.3 Hz, 4H), 3.43-3.33 (m, 1H), 2.75 (dd, *J* = 15.0, 7.5 Hz, 2H), 2.29-2.14 (s, 3H), 2.03 (m, 2H), 1.93-1.81 (m, 2H), 1.78-1.57 (m, 4H), 1.20 (t, *J* = 7.1 Hz, 3H). ¹³C NMR (151 MHz, CD₃OD) δ 177.79, 154.79, 145.36, 143.28, 137.08, 136.13, 131.80, 130.92, 129.27, 127.96, 127.80 (q, *J* = 28.7 Hz), 126.46, 124.78 (q, *J* = 273.5 Hz), 124.76, 123.84, 74.79, 57.14, 40.91, 35.57, 25.62, 25.20, 14.36, 11.42. HRMS (ESI) (m/z): [M+H]⁺ calcd for C₂₈H₃₃F₃N₂O₃, 503.2522; found: 503.2523. HPLC Purity: 96.46%, retention time: 2.28 min.

Reaction conditions for other compounds are similar to those of the above compounds.

### (E)-1-(4-(1-((4-cyclohexylbenzyl)oxy)imino)ethyl)benzyl)azetidine-3-carboxylic acid (Compound 1)

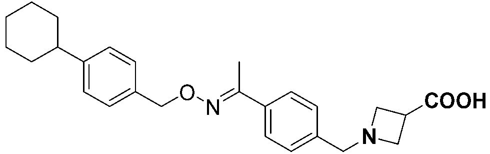

¹H NMR (400 MHz, Methanol-*d*₄) δ 7.60 (d, *J* = 8.3 Hz, 2H), 7.30 (dd, *J* = 8.2, 2.7 Hz, 4H), 7.19 (d, *J* = 8.1 Hz, 2H), 5.14 (s, 2H), 3.64 (d, *J* = 2.4 Hz, 2H), 3.53 (t, *J* = 8.1 Hz, 2H), 3.38 - 3.32 (m, 2H), 3.20 (dd, *J* = 16.6, 8.2 Hz, 1H), 2.57 - 2.45 (m, 1H), 2.22 (s, 3H), 1.84 (d, *J* = 9.2 Hz, 4H), 1.75 (d, *J* = 12.2 Hz, 1H), 1.43 (d, *J* = 4.4 Hz, 6H). ¹³C NMR (151 MHz, Methanol-*d*₄) δ 154.60, 147.52, 138.04, 135.64, 135.45, 128.59, 128.01, 126.36, 125.82, 75.68, 62.35, 57.58, 44.40, 36.48, 34.30, 29.46, 26.62, 25.86, 11.45. LC-MS (ESI): m/z: 421.1 (M+H)⁺.

### (E)-1-(4-(1-((4-cyclohexyl-3-(trifluoromethyl)benzyl)oxy)imino)ethyl)benzyl)azetidine-3-carboxylic acid (Compound 2)

¹H NMR (400 MHz, Methanol-*d*₄) δ 7.56 (d, *J* = 17.6 Hz, 1H), 7.53 (d, *J* = 7.4 Hz, 1H), 7.39 (t, *J* = 12.5 Hz, 1H), 7.30 (d, *J* = 7.9 Hz, 2H), 7.19 (d, *J* = 7.9 Hz, 2H), 5.11 (s, 2H), 4.31 (s, 2H), 4.10 (dd, *J* = 21.9, 12.7 Hz, 2H), 4.02 (t, *J* = 8.4 Hz, 2H), 3.35 (s, 3H), 2.78-2.70 (m, 2H), 1.49-1.36 (m, 9H). LC-MS (ESI): m/z: 431.1 (M+H)⁺.

### (E)-1-(4-(1-((4-cyclohexylbenzyl)oxy)imino)ethyl)-2-ethylbenzyl)azetidine-3-carboxylic acid (Compound 3)

¹H NMR (400 MHz, DMSO) δ 8.36 (s, 1H), 8.04 (dt, *J* = 28.8, 6.4 Hz, 4H), 7.71 - 7.46 (m, 4H), 4.38-4.33 (m, 2H), 4.10-4.01 (m, 2H), 3.59-3.55 (m, 2H), 2.85 - 2.76 (m, 2H), 2.79 (q, *J* = 7.5 Hz, 2H), 2.57 - 2.45 (m, 1H), 1.24-1.21 (m, 3H), 1.20 (t, *J* = 7.5 Hz, 3H). LC-MS (ESI): m/z: 449.1 (M+H)⁺.

### Structure of (E)-1-(2-ethyl-4-(1-(((4-(5-fluoropyrazin-2-yl)-3-methylbenzyl)oxy)imino)ethyl)benzyl)pyrrolidine-3-carboxylic acid (Compound 4)

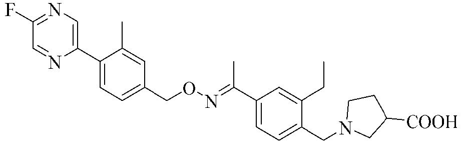

Pale yellow solid, yield 18.4%. ¹H NMR (500 MHz, Methanol-*d*₄) δ 8.58 (dd, *J* = 8.3, 1.3 Hz, 1H), 8.37 (d, *J* = 1.5 Hz, 1H), 7.64 (d, *J* = 1.9 Hz, 1H), 7.58 (dd, *J* = 8.1, 1.9 Hz, 1H), 7.49 (d, *J* = 8.1 Hz, 1H), 7.42 (d, *J* = 7.8 Hz, 1H), 7.40 - 7.35 (m, 2H), 5.26 (s, 2H), 4.40 (s, 2H), 3.53 (dd, *J* = 11.2, 5.8 Hz, 1H), 3.39 - 3.35 (m, 2H), 3.28 (dd, *J* = 11.0, 7.4 Hz, 1H), 3.15 - 3.06 (m, 1H), 2.82 (q, *J* = 7.5 Hz, 2H), 2.37 (s, 3H), 2.34 - 2.22 (m, 5H), 1.26 (t, *J* = 7.5 Hz, 3H). ¹³C NMR (126 MHz, Methanol-*d*₄) δ 177.85, 160.27, 158.28, 154.49, 152.90, 143.73, 141.29, 141.22, 139.46, 137.74, 136.37, 135.08, 131.94, 131.64, 130.48, 130.33, 130.21, 129.58, 126.66, 125.50, 123.97, 75.34, 56.55, 54.38, 53.52, 43.60, 27.72, 25.23, 19.04, 14.55, 11.40. MS (ESI) (m/z): [M+H]⁺ 491.25.

### Structure of (S, E)-1-(2-ethyl-4-(1-(((3-methyl-4-(5-hydroxypyrazin-2-yl)-benzyl)oxy)imino)ethyl)benzyl)pyrrolidine-3-carboxylic acid (Compound 5)

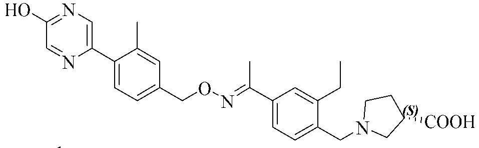

Yellow solid, yield 72.5%. ¹H NMR (500 MHz, Methanol-d4) δ 8.17 (d, *J* = 1.4 Hz, 1H), 7.67 (d, *J* = 1.8 Hz, 1H), 7.61 (dd, *J* = 8.1, 1.9 Hz, 1H), 7.57 (d, *J* = 8.1 Hz, 1H), 7.51 (d, *J* = 1.4 Hz, 1H), 7.35 (d, *J* = 8.1 Hz, 2H), 7.33 - 7.29 (m, 1H), 5.24 (s, 2H), 4.53 (s, 2H), 3.70 - 3.57 (m, 2H), 3.51 - 3.43 (m, 2H), 3.38 (q, *J* = 8.6, 8.2 Hz, 1H), 2.85 (q, *J* = 7.5 Hz, 2H), 2.49 - 2.41 (m, 1H), 2.38 (s, 3H), 2.36 - 2.30 (m, 1H), 2.29 (s, 3H), 1.27 (t, *J* = 7.5 Hz, 3H).¹³C NMR (126 MHz, Methanol-d4) δ 154.34, 143.95, 138.49, 138.17, 136.24, 135.20, 130.78, 130.24, 129.10, 129.01, 126.79, 125.48, 124.11, 75.49, 55.17, 54.38, 53.64, 27.07, 25.36, 19.14, 14.60, 11.39. MS(m/z): [M+H]⁺489.20

### (E)-1-(4-(1-((4-cyclohexyl-3-(trifluoromethyl)benzyloxy)imino)ethyl)benzyl)pyrrolidine-3-carboxylic acid (Compound 6)

¹H NMR (400 MHz, Methanol-d4) δ 7.70 - 7.63 (m, 3H), 7.57 (d, *J* = 16.3 Hz, 2 H), 7.44 (d, *J* = 8.2 Hz, 2H), 5.22 (s, 2H), 3.96 (d, *J* = 5.7 Hz, 2H), 3.35 (s, 2H), 3.16-3.12 (m, 1H), 3.05-2.98 (m, 2H), 2.95 - 2.82 (m, 2H), 2.26 (s, 3H), 2.16 (d, *J* = 8.0 Hz, 2H), 1.82-1.80 (m, , 2H), 1.78-1.75 (m, 3H), 1.58-1.45 (m, 6H). LC-MS (ESI): m/z: 503. 1 (M+H)⁺.

### (E)-1-(4-(1-((4-cyclohexyl-3-(trifluoromethyl)benzyl)oxy)imino)ethyl)benzyl)piperidine-3-carboxylic acid (Compound 7)

¹H NMR (400 MHz, Methanol-d4) δ 7.70 - 7.63 (m, 3H), 7.57 (d, *J* = 16.3 Hz, 2H), 7.44 (d, *J* = 8.2 Hz, 2H), 5.22 (s, 2H), 3.96 (d, *J* = 5.7 Hz, 2H), 3.35 (s, 2H), 3.16-3.12 (m, 1H), 3.05-2.98 (m, 2H), 2.95 - 2.82 (m, 2H), 2.26 (s, 3H), 2.16 (d, *J* = 8.0 Hz, 2H), 1.82-1.80 (m, , 2H), 1.78-1.75 (m, 3H), 1.58-1.45 (m, 8H). LC-MS (ESI): m/z: 517.2 (M+H)⁺.

### (E)-4-((4-((1-((4-cyclohexyl-3-(trifluoromethyl)benzyl)oxy)imino)ethyl)benzyl)amino)butanoic acid (Compound 8)

¹H NMR (400 MHz, Methanol-d4) δ 7.65 (d, *J* = 8.1 Hz, 3H), 7.58 (t, *J* = 10.4 Hz, 1H), 7.53 (d, *J* = 8.1 Hz, 1H), 7.41 (t, *J* = 10.5 Hz, 2H), 5.21 (s, 2H), 3.88 (s, 2H), 2.91 (t, *J* = 11.2 Hz, 1H), 2.76 (t, *J* = 7.1 Hz, 2H), 2.30 - 2.17 (m, 5H), 1.85-1.75 (m, 6H), 1.61 - 1.29 (m, 6H). LC-MS (ESI): m/z: 491.2 (M+H)⁺.

### (E)-3-((4-((1-((4-cyclohexyl-3-(trifluoromethyl)benzyl)oxy)imino)ethyl)benzyl)amino)cyclohexane-1-carboxylic acid (Compound 9)

¹H NMR (400 MHz, Methanol-d4) δ 7.77 - 7.32 (m, 7H), 5.21 (s, 2H), 3.91 (s, 2H), 2.91 (s, 1H), 2.68 (s, 1H), 2.25 (s, 3H), 2.18-2.15 (m, 2H), 1.88-1.70 (m, 9H), 1.65 - 1.33 (m, 8H). ¹³C NMR (151 MHz, MeOD) δ 182.91, 155.03, 146.14, 136.31, 135.59, 131.60, 128.55, 128.47, 128.10, 125.97, 124.84, 124.79, 74.73, 55.54, 49.13, 45.59, 40.06, 34.19, 31.24, 29.43, 26.60, 25.70, 11.40. LC-MS (ESI): m/z: 531.2 (M+H)⁺.

### (E)-3-((4-((1-((4-cyclohexyl-3-(trifluoromethyl)benzyl)oxy)imino)ethyl)benzyl)amino)propanoic acid (Compound 10)

¹H NMR (400 MHz, Methanol-d4) δ 7.65 (d, *J* = 7.4 Hz, 3H), 7.56 (dd, *J* = 24.3, 8.1 Hz, 2H), 7.39 (d, *J* = 8.0 Hz, 2H), 5.21 (s, 2H), 3.90 (s, 2H), 2.95-2.90 (m, 3H), 2.44 (t, *J* = 6.6 Hz, 2H), 2.25 (s, 3H), 1.87 (d, *J* = 16.6 Hz, 2H), 1.78 (d, *J* = 11.1 Hz, 3H), 1.48 (ddd, *J* = 33.8, 22.3, 10.1 Hz, 5H). ¹³C NMR (151 MHz, Methanol-d4) δ 178.44, 154.94, 146.14, 137.98, 136.30, 135.86, 131.60, 128.53, 128.10, 126.03, 124.84, 124.79, 74.74, 51.72, 45.19, 40.06, 35.04, 34.18, 26.59, 25.70, 11.38. LC-MS (ESI): m/z: 478.2 (M+H)⁺.

### Structure of (E)-1-(4-(1-((4-cyclohexyl-3-(trifluoromethyl)benzyl)oxy)imino)ethyl)-2-fluorobenzyl)azetidine-3-carboxylic acid (Compound 11)

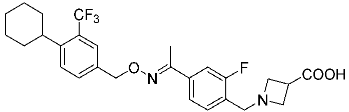

White solid, yield 40.6%, melting point: 144.6-144.9 °C. ¹H NMR (400 MHz, CDCl₃) δ 7.74-7.27 (m, 6H), 5.26 (s, 2H), 3.97 (s, 2H), 3.80 (d, *J* = 8.2 Hz, 2H), 3.72-3.67 (m, 3H), 2.94-2.90 (m, 1H), 2.27 (s, 3H), 1.94-1.82 (m, 2H), 1.80-1.75 (m, 2H), 1.56-1.50 (m, 2H), 1.45-1.40 (m, 4H). HRMS (ESI) (m/z): [M+H]⁺ calcd for C₂₇H₃₀F₄N₂O₃, 507.2271; found: 507.2270. HPLC Purity: 91.98%, retention time: 2.26 min.

### Structure of (E)-1-(2-bromo-4-(1-((4-cyclohexyl-3-(trifluoromethyl)benzyl)oxy)imino)ethyl)azetidine-3-carboxylic acid (Compound 12)

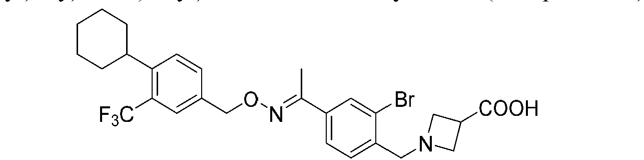

White solid, yield 40.6%, melting point: 131.6-131.9 °C. ¹H NMR (400 MHz, CD₃OD) δ 7.91 (d, *J* = 1.6 Hz, 1H), 7.69-7.63 (m, 2H), 7.63-7.49 (m, 2H), 7.45 (d, *J* = 8.1 Hz, 1H), 5.23 (s, 2H), 4.08 (s, 2H), 3.87 (t, *J* = 8.6 Hz, 2H), 3.75 (t, *J* = 8.2 Hz, 2H), 2.93-2.86 (m, 2H), 2.30 (s, 3H), 1.84-1.80 (m, 2H), 1.77-1.70 (m, 3H), 1.60-1.34 (m, 5H). ¹³C NMR (151 MHz, CD₃OD) δ 178.85, 154.09, 147.89, 146.25, 138.90, 131.73, 129.72, 129.46, 128.84, 128.53, 128.16, 127.33, 124.97, 121.07, 112.36, 90.94, 74.97, 61.53, 58.01, 40.07, 34.17, 26.69, 25.70, 12.97. HRMS (ESI) (m/z): [M+H]⁺ calcd for C₂₇H₃₀BrF₃N₂O₃, 567.1470; found: 567.1473. HPLC Purity: 90.25%, retention time: 2.87 min.

### Structure of (E)-1-(4-(1-((4-cyclohexyl-3-(trifluoromethyl)benzyl)oxy)imino)ethyl)-2-methylbenzyl)azetidine-3-carboxylic acid (Compound 18)

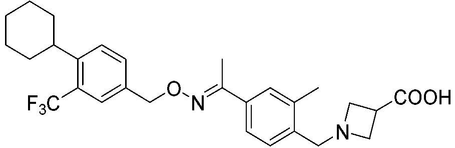

White solid, yield 40.6%, melting point: 155.1-155.6 °C. ¹H NMR (400 MHz, CD₃OD) δ 7.64 (d, *J* = 5.9 Hz, 1H), 7.59-7.42 (m, 4H), 7.37-7.29 (m, 1H), 5.20 (s, 2H), 4.05 (s, 2H), 3.91 (t, *J* = 8.9 Hz, 2H), 3.77 (dd, *J* = 17.0, 8.5 Hz, 2H), 3.60 (dd, *J* = 14.1, 7.1 Hz, 1H), 2.91 (t, *J* = 11.3 Hz, 1H), 2.39 (s, 3H), 2.23 (s, 3H), 1.88-1.80 (m, 2H), 1.79-1.70 (m, 3H), 1.46-1.39 (m, 5H). ¹³C NMR (151 MHz, CD₃OD) δ 178.49, 154.91, 146.15, 138.90, 136.99, 136.37, 131.61, 128.89, 128.52, 128.11, 127.91, 127.31, 124.88, 124.80 (q, *J* = 273.5 Hz), 123.68, 74.75, 57.59, 40.05, 35.70, 34.18, 34.13, 26.59, 25.70, 17.99, 16.97, 11.38. HRMS (ESI) (m/z): [M+H]+ calcd for C₂₈H₃₃F₃N₂O₃, 503.2522; found: 503.2523. HPLC Purity: 94.29%, retention time: 2.57 min.

### Structure of (E)-1-(4-(1-((4-cyclohexyl-3-(trifluoromethyl)benzyl)oxy)imino)ethyl)-2-methoxybenzyl)azetidine-3-carboxylic acid (Compound 27)

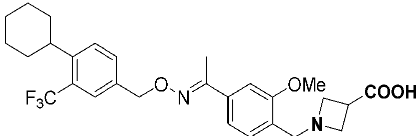

White solid, yield 40.6%, melting point: 145.2-145.6 °C. ¹H NMR (400 MHz, CD₃OD) δ 7.66 (s, 1H), 7.64-7.49 (m, 3H), 7.25 (t, *J* = 8.7 Hz, 2H), 5.21 (s, 2H), 3.84 (s, 3H), 3.70 (s, 2H), 3.59 (d, *J* = 8.2 Hz, 2H), 3.52 (t, *J* = 8.0 Hz, 1H), 3.42 (t, *J* = 8.2 Hz, 2H), 2.91 (d, *J* = 10.6 Hz, 1H), 2.23 (s, 3H), 1.92-1.83 (m, 2H), 1.79-1.87 (m, 3H), 1.63-1.48 (m, 5H). ¹³C NMR (151 MHz, CD₃OD) δ 175.87, 157.76, 154.59, 146.26, 139.82, 138.93, 136.16, 131.75, 128.55, 127.30, 127.01, 124.98, 124.80 (q, *J* = 273.5 Hz), 119.36, 118.66, 107.91, 74.92, 60.20, 57.19, 54.77, 40.06, 34.72, 34.18, 26.58, 25.69, 11.39. HRMS (ESI) (m/z): [M+H]⁺ calcd for C₂₈H₃₃F₃N₂O₄, 519.2471; found: 519.2473. HPLC Purity: 96.28%, retention time: 1.27 min.

### Structure of (E)-1-(4-(1-((4-cyclohexyl-3-(trifluoromethyl)benzyl)oxy)imino)ethyl)-2-(trifluoromethyl)benzyl)azetidine-3-carboxylic acid (Compound 19)

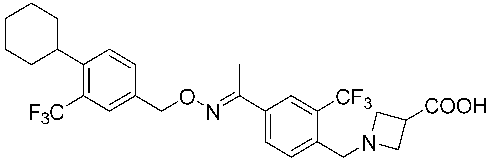

White solid, yield 40.6%, melting point: 135.6-138.9 °C. ¹H NMR (400 MHz, CD₃OD) δ 7.98 (s, 1H), 7.88 (d, *J* = 8.1 Hz, 1H), 7.69-7.51 (m, 4H), 5.24 (s, 2H), 3.96 (s, 2H), 3.73 (t, *J* = 8.3 Hz, 2H), 3.53 (t, *J* = 8.0 Hz, 2H), 3.29-3.19 (m, 1H), 2.92 (t, *J* = 11.3 Hz, 1H), 1.86-1.80 (m, 2H), 1.78-1.70 (m, 3H), 1.62-1.35 (m, 5H). ¹³C NMR (151 MHz, CD₃OD) δ 178.45, 153.61, 146.30, 138.92, 135.99, 135.83, 131.79, 129.85, 129.41, 128.52, 128.15, 127.29, 125.06, 124.77 (q, *J* = 273.5 Hz), 124.22 (q, *J* = 273.6 Hz), 123.02, 75.08, 58.10, 57.75, 40.06, 36.07, 34.17, 26.59, 25.70, 11.05. LC-MS (ESI): m/z: 557.2 (M+H)⁺. HRMS (ESI) (m/z): [M+H]⁺ calcd for C₂₈H₃₀F₆N₂O₃, 557.2239; found: 557.2237. HPLC Purity: 97.16%, retention time: 2.56 min.

### Structure of (E)-1-(4-(1-(([1,1'-biphenyl]-4-ylmethoxy)imino)ethyl)-2-ethylbenzyl)azetidine-3-carboxylic acid (Compound 20)

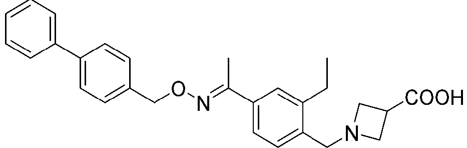

White solid, yield 40.6%, melting point: 120.3-121.9 °C. ¹H NMR (400 MHz, CD₃OD) δ 7.64-7.53 (m, 4H), 7.49-7.39 (m, 6H), 7.36-7.23 (m, 2H), 5.24 (s, 2H), 3.76 (s, 2H), 3.67-3.58 (m, 2H), 3.42 (t, *J* = 8.1 Hz, 2H), 3.29-3.15 (m, 1H), 2.72 (q, *J* = 7.5 Hz, 2H), 2.23 (s, 3H), 1.22 (t, *J* = 7.5 Hz, 3H). ¹³C NMR (151 MHz, CD₃OD) δ 179.26, 155.07, 142.50, 140.80, 140.59, 137.27, 135.67, 135.39, 129.45, 128.52, 128.44, 128.33, 126.93, 126.58, 126.55, 126.52, 126.50, 125.94, 123.39, 75.38, 58.84, 57.94, 36.49, 25.11, 14.23, 11.55. LC-MS (ESI): m/z: 443.2 (M+H)⁺. HRMS (ESI) (m/z): [M+H]⁺ calcd for C₂₈H₃₀N₂O₃, 443.2335; found: 443.2337. HPLC Purity: 98.03%, retention time: 1.30 min.

### Structure of (E)-1-(4-(1-((4-cyclopentyl-3-(trifluoromethyl)benzyl)oxy)imino)ethyl)-2-ethylbenzyl)azetidine-3-carboxylic acid (Compound 17)

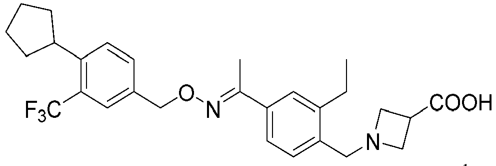

Light-colored solid, yield 30.2%, melting point: 131.7-133.2 °C. ¹H NMR (400 MHz, CD₃OD) δ 7.64 (s, 1H), 7.53 (dt, *J* = 9.8, 5.1 Hz, 4H), 7.38 (t, *J* = 11.5 Hz, 1H), 5.22 (s, 2H), 4.25 (s, 2H), 4.02 (d, *J* = 8.3 Hz, 4H), 3.43-3.33 (m, 1H), 2.75 (dd, *J* = 15.0, 7.5 Hz, 2H), 2.29-2.14 (s, 3H), 2.03 (m, 2H), 1.93-1.81 (m, 2H), 1.78-1.57 (m, 4H), 1.20 (t, *J* = 7.1 Hz, 3H). ¹³C NMR (151 MHz, CD₃OD) δ 177.79, 154.79, 145.36, 143.28, 137.08, 136.13, 131.80, 130.92, 129.27, 127.96, 127.80 (q, *J* = 28.7 Hz), 126.46, 124.78 (q, *J* = 273.5 Hz), 124.76, 123.84, 74.79, 57.14, 40.91, 35.57, 25.62, 25.20, 14.36, 11.42. HRMS (ESI) (m/z): [M+H]⁺ calcd for C₂₈H₃₃F₃N₂O₃, 503.2522; found: 503.2523. HPLC Purity: 96.46%, retention time: 2.28 min.

### Structure of (E)-1-(4-(1-((4-cyclohexyl-3-methylbenzyl)oxy)imino)ethyl)-2-ethylbenzyl)azetidine-3-carboxylic acid (Compound 24)

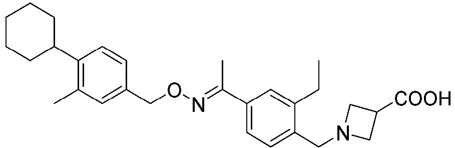

Yellow solid, yield 33.3%, melting point: 135.8-136.3 °C. ¹H NMR (400 MHz, CD₃OD) δ 7.53 (s, 1H), 7.47 (dd, *J* = 8.0, 1.5 Hz, 1H), 7.30 (d, *J* = 8.1 Hz, 1H), 7.15 (d, *J* = 8.5 Hz, 3H), 5.11 (s, 2H), 4.00 (s, 2H), 3.84 (t, *J* = 8.8 Hz, 2H), 3.69 (t, *J* = 8.4 Hz, 2H), 3.30-3.22 (m, 1H), 2.82-2.60 (m, 3H), 2.29 (s, 3H), 2.20 (s, 3H), 1.83-1.80 (m, 2H), 1.76-1.72 (m, 3H), 1.48-1.29 (m, 5H), 1.21 (t, *J* = 7.8 Hz, 3H). ¹³C NMR (151 MHz, CD₃OD) δ 178.12, 154.47, 145.22, 142.85, 136.54, 134.88, 134.61, 133.03, 129.89, 128.89, 126.20, 125.88, 124.96, 123.61, 75.80, 57.62, 57.34, 39.83, 35.88, 33.49, 26.85, 26.03, 25.14, 18.10, 14.33, 11.49. HRMS (ESI) (m/z): [M+H]⁺ calcd for C₂₉H₃₈N₂O₃, 463.2961; found: 463.2962. HPLC Purity: 98.19%, retention time: 2.39 min.

### (E)-3-((4-((1-((4-cyclohexyl-3-(trifluoromethyl)benzyl)oxy)imino)ethyl)-2-ethylbenzyl)amino)propanoic acid (Compound 25)

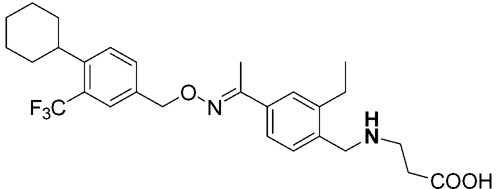

¹H NMR (400 MHz, CD₃OD) δ 7.65 (s, 1H), 7.58 (dd, *J* = 11.7, 7.9 Hz, 2H), 7.51 (t, *J* = 9.5 Hz, 2H), 7.46 - 7.35 (m, 1H), 5.21 (s, 2H), 4.19 (s, 2H), 3.18 (t, *J* = 6.3 H z, 2H), 3.07 (t, *J* = 6.4 Hz, 1H), 2.91 (t, *J* = 11.3 Hz, 1H), 2.78 (q, *J* = 7.5 Hz, 2H), 2. 51 (t, *J* = 6.3 Hz, 2H), 2.24 (s, 3H), 1.83-1.80 (m, 2H), 1.77-1.70 (m, 3H), 1.59 - 1.33 (m, 5H). ¹³C NMR (151 MHz, CD₃OD) δ 177.24, 154.83, 146.18, 143.23, 137.30, 136.22, 131.68, 131.45, 129.89, 128.13, 127.19, 127.00, 126.43, 124.96, 124.91, 123.96, 74.81, 44.8 4, 40.06, 34.19, 33.03, 32.12, 26.59, 25.70, 25.07, 14.37, 11.42. LC-MS (ESI): m/z: 505.2 (M+H)⁺.

### (E)-1-(4-(1-((4-cyclohexyl-3-(trifluoromethyl)benzyl)oxy)imino)ethyl)-2-ethylbenzyl)pyrroli dine-3-carboxylic acid (Compound 26)

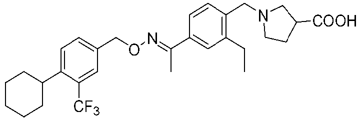

¹H NMR (400 MHz, CD₃OD) δ 7.65 (s, 1H), 7.59 (d, *J* = 7.2 Hz, 2H), 7.53 (d, *J* = 7.5 Hz, 2H), 7.45 (d, *J* = 8.1 Hz, 1H), 5.22 (s, 2H), 4.33 (s, 2H), 3.40-3.42 (m, 1H), 3.38 - 3.32 (m, 1H), 3.25 (t, *J* = 6.9 Hz, 2H), 3.11 - 3.00 (m, 1H), 2.91 (t, *J* = 11.3 Hz, 1H), 2.80 (q, *J* = 7.5 Hz, 2H), 2.24 (s, 3H), 1.85 (d, *J* = 11.7 Hz, 2H), 1.77 (d, *J* = 11.9 Hz, 3H), 1.59 - 1.50 (m, 2H), 1.46 - 1.31 (m, 3H), 1.27 (t, *J* = 7.1 Hz, 3H). ¹³C NMR (151 MHz, CD₃OD) δ 178.42, 154.79, 146.19, 143.65, 137.44, 136.23, 131.68, 130.98, 130.40, 128.13, 127.20, 127.01, 126.59, 125.71, 124.95, 124.91, 123.91, 74.82, 56.80, 54.66, 53.60, 43.92, 40.06, 34.18, 26.59, 25.69, 25.21, 14.51, 11.39. LC-MS (ESI): m/z: 531.3 (M+H)⁺.

### Structure of (E)-1-(2-ethyl-4-(1-((2-methyl-[1,1'-biphenyl]-4-yl)methoxy)imino)ethyl)azetidine-3-carboxylic acid (Compound 22)

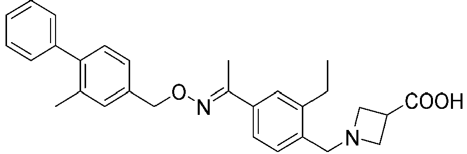

White solid, yield 40.6%, melting point: 154.2-154.8 °C. ¹H NMR (400 MHz, CD₃OD) δ 7.55 (s, 1H), 7.49 (d, *J* = 8.0 Hz, 1H), 7.38 (t, *J* = 7.3 Hz, 2H), 7.34-7.23 (m, 6H), 7.15 (d, *J* = 7.7 Hz, 1H), 5.17 (s, 2H), 4.00 (s, 2H), 3.84 (t, *J* = 8.7 Hz, 2H), 3.69 (t, *J* = 8.3 Hz, 2H), 3.27-3.20 (m, 1H), 2.74 (q, *J* = 7.5 Hz, 2H), 2.24 (s, 3H), 2.23 (s, 3H), 1.21 (q, *J* = 7.5 Hz, 3H). ¹³C NMR (151 MHz, CD₃OD) δ 178.15, 154.69, 142.88, 141.76, 141.46, 137.05, 136.48, 134.91, 133.09, 129.85, 129.32, 128.92, 128.79, 127.78, 126.51, 126.21, 125.39, 123.63, 75.61, 57.61, 57.33, 35.89, 25.15, 19.26, 14.34, 11.52. HRMS (ESI) (m/z): [M+H]⁺ calcd for C₂₉H₃₂N₂O₃, 457.2492; found: 457.2493. HPLC Purity: 91.98%, retention time: 2.30 min.

### Structure of (E)-1-(2-ethyl-4-(1-((2-(trifluoromethyl)-[1,1'-biphenyl]-4-yl)methoxy)imino)ethyl)azetidine-3-carboxylic acid (Compound 21)

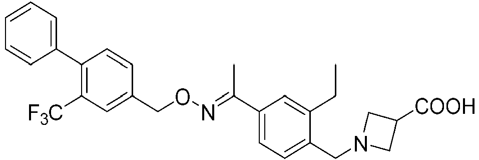

White solid, yield 40.6%, melting point: 155.5-155.9 °C. ¹H NMR (400 MHz, CD₃OD) δ 7.69 (d, *J* = 12.3 Hz, 1H), 7.56 (t, *J* = 8.1 Hz, 1H), 7.49 (s, 1H), 7.42 (d, *J* = 8.0 Hz, 1H), 7.26 (dd, *J* = 5.3, 2.9 Hz, 4H), 7.22 (d, *J* = 7.8 Hz, 1H), 7.19-7.13 (m, 2H), 5.21 (s, 2H), 4.11 (s, 2H), 3.93 (t, *J* = 9.1 Hz, 2H), 3.83 (t, *J* = 8.4 Hz, 2H), 3.29-3.22 (m, 1H), 2.66 (q, *J* = 7.5 Hz, 2H), 2.16 (s, 3H), 1.12 (t, *J* = 7.5 Hz, 3H). ¹³C NMR (151 MHz, CD₃OD) δ 177.22, 155.11, 143.30, 140.77, 139.59, 138.22, 136.91, 132.05, 131.31, 130.97, 129.29, 128.64, 127.87 (q, *J* = 29.8 Hz), 127.46, 127.37, 126.45, 125.32, 124.23 (q, *J* = 273.8 Hz), 123.86, 74.64, 57.34, 55.98, 35.39, 25.18, 14.40, 11.50. HRMS (ESI) (m/z): [M+H]⁺ calcd for C₂₉H₂₉F₃N₂O₃, 511.2209; found: 511.2211. HPLC Purity: 97.71%, retention time: 1.56 min.

### Structure of (E)-1-(2-ethyl-4-(1-((3-(trifluoromethyl)phenyl)oxy)imino)ethyl)benzyl)azetidine-3-carboxylic acid (Compound 13)

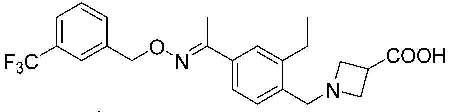

Pale yellow oily, yield 60.0%. ¹H NMR (400 MHz, CD₃OD) δ 7.71 (s, 1H), 7.67 (d, *J* = 7.4 Hz, 1H), 7.62-7.51 (m, 3H), 7.47 (dd, *J* = 8.0, 1.6 Hz, 1H), 7.31 (d, *J* = 8.0 Hz, 1H), 5.28 (s, 2H), 3.98 (s, 2H), 3.83 (t, *J* = 8.8 Hz, 2H), 3.67 (t, *J* = 8.4 Hz, 2H), 3.28 (dd, J = 15.2, 6.9 Hz, 1H), 2.73 (q, *J* = 7.5 Hz, 2H), 2.26 (s, 3H), 1.21 (t, *J* = 7.5 Hz, 3H). ¹³C NMR (151 MHz, CD₃OD) δ 178.20, 155.32, 142.88, 139.73, 136.16, 133.52, 131.41, 130.23 (q, *J* = 32.0 Hz), 128.87, 128.82, 126.17, 124.32, 124.31 (q, *J* = 272.0 Hz), 124.06, 123.61, 74.70, 57.68, 35.94, 25.11, 14.28, 11.47. HRMS (ESI) (m/z): [M+H]⁺ calcd for C₂₃H₂₅F₃N₂O₃, 435.1896; found: 435.1897. HPLC Purity: 99.79%, retention time: 1.17 min.

### Structure of (E)-1-(2-ethyl-4-(1-((4-methyl-3-(trifluoromethyl)benzyl)oxy)imino)ethyl)azetidine-3-carboxylic acid (Compound 14)

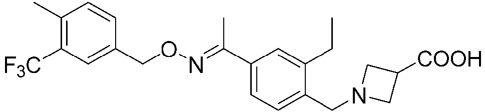

Pale yellow oily, yield 21.3%. ¹H NMR (400 MHz, CD₃OD) δ 7.67 (s, 1H), 7.57 (s, 1H), 7.52 (t, *J* = 7.9 Hz, 2H), 7.35 (dd, *J* = 12.5, 8.1 Hz, 2H), 5.22 (s, 2H), 4.22 (s, 2H), 3.97 (dd, J = 22.5, 8.5 Hz, 4H), 3.40-3.33 (m, 1H), 2.76 (q, *J* = 7.5 Hz, 2H), 2.47 (s, 3H), 2.25 (s, 3H), 1.22 (t, *J* = 7.5 Hz, 3H). ¹³C NMR (151 MHz, CD₃OD) δ 177.72, 155.00, 143.07, 136.62, 136.50, 135.81, 132.36, 131.84, 131.45, 129.07, 128.29 (q, *J* = 30.2 Hz), 126.30, 125.10, 124.70 (q, *J* = 273.3 Hz), 123.70, 74.70, 57.55, 56.78, 35.67, 25.12, 17.72, 14.32, 11.40. HRMS (ESI) (m/z): [M+H]⁺ calcd for C₂₄H₂₇F₃N₂O₃, 449.2052; found: 449.2050. HPLC Purity: 99.32%, retention time: 1.35 min.

### Structure of (E)-1-(2-ethyl-4-(1-((naphthalen-2-ylmethoxy)imino)ethyl)azetidine-3-carboxylic acid (Compound 15)

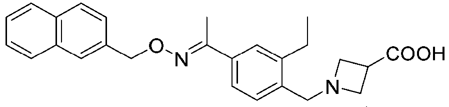

White solid, yield 25.9%, melting point: 131.5-131.7 °C. ¹H NMR (400 MHz, CD₃OD) δ 7.89-7.79 (m, 4H), 7.61-7.51 (m, 2H), 7.52-7.41 (m, 3H), 7.35-7.18 (m, 1H), 5.40 (s, 2H), 3.95 (s, 2H), 3.80 (t, *J* = 8.6 Hz, 2H), 3.61 (dt, *J* = 10.7, 5.9 Hz, 2H), 3.29-3.22 (m, 1H), 2.77-2.59 (m, 2H), 2.27 (s, 3H), 1.25-1.14 (m, 3H). ¹³C NMR (151 MHz, CD₃OD) δ 177.73, 154.96, 142.81, 136.31, 135.65, 133.40, 133.14, 128.83, 127.62, 127.55, 127.29, 126.58, 126.18, 125.78, 125.74, 125.59, 123.60, 75.84, 57.73, 36.00, 25.11, 14.28, 11.53. HRMS (ESI) (m/z): [M+H]⁺ calcd for C₂₆H₂₈N₂O₃, 417.2179; found: 417.2179. HPLC Purity: 98.77%, retention time: 1.24 min.

### Structure of (E)-1-(2-ethyl-4-(1-(((5,6,7,8-tetrahydronaphthalen-2-yl)methoxy)imino)ethyl)benzyl)azetidine-3-carboxylic acid (Compound 23)

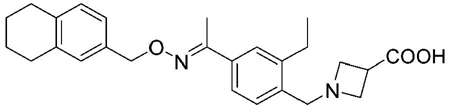

White solid, yield 10.2%, melting point: 135.3-138.9 °C. ¹H NMR (400 MHz, CD₃OD) δ 7.50 (s, 1H), 7.44 (dd, *J* = 8.0, 1.5 Hz, 1H), 7.28 (d, *J* = 8.0 Hz, 1H), 7.13-7.05 (m, 2H), 7.00 (d, *J* = 7.7 Hz, 1H), 5.09 (s, 2H), 3.83 (s, 2H), 3.70 (t, *J* = 8.3 Hz, 2H), 3.51 (t, *J* = 8.2 Hz, 2H), 3.24 (dd, *J* = 16.6, 8.3 Hz, 1H), 2.78-2.71 (m, 6H), 2.20 (s, 3H), 1.80-1.70 (m, 4H), 1.23 (t, J = 7.5 Hz, 3H). ¹³C NMR (151 MHz, CD₃OD) δ 178.90, 154.68, 142.58, 136.56, 136.32, 136.01, 134.99, 134.56, 128.69, 128.62, 128.61, 126.00, 125.26, 123.45, 75.81, 58.35, 57.83, 36.29, 29.01, 28.78, 25.11, 23.06, 14.25, 11.51. HRMS (ESI) (m/z): [M+H]⁺ calcd for C₂₆H₃₂N₂O₃, 421.2492; found: 421.2492. HPLC Purity: 98.54%, retention time: 1.48 min.

### Structure of (E)-1-(4-(1-(((3-cyano-4-isopropoxybenzyl)oxy)imino)ethyl)-2-ethylbenzyl)azetidine-3-carboxylic acid (Compound 16)

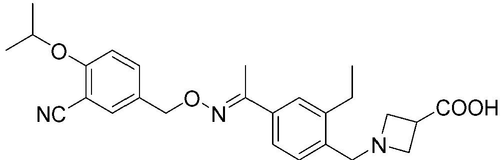

Pale yellow solid, yield 52.3%, melting point: 112.6-112.9 °C. ¹H NMR (400 MHz, CD₃OD) δ 7.64 (d, *J* = 6.7 Hz, 2H), 7.56 (s, 1H), 7.50 (dd, *J* = 8.0, 1.6 Hz, 1H), 7.33 (d, *J* = 8.1 Hz, 1H), 7.16 (d, *J* = 9.4 Hz, 1H), 5.14 (s, 2H), 4.78-4.73 (m, 1H), 4.18 (s, 2H), 3.99 (t, *J* = 9.3 Hz, 2H), 3.90 (t, *J* = 8.6 Hz, 2H), 3.40-3.33 (m, 1H), 2.76 (q, *J* = 7.5 Hz, 2H), 2.23 (s, 3H), 1.38 (s, 3H), 1.37 (s, 3H), 1.23 (t, *J* = 7.5 Hz, 3H). ¹³C NMR (151 MHz, CD₃OD) δ 177.23, 159.53, 154.92, 143.23, 136.96, 134.67, 133.44, 131.00, 129.23, 126.42, 123.80, 116.06, 113.66, 101.88, 74.22, 71.68, 57.49, 56.20, 35.40, 25.12, 20.71, 14.38, 11.39. HRMS (ESI) (m/z): [M+H]⁺ calcd for C₂₆H₃₁N₃O₄, 450.2396; found: 450.2395. HPLC Purity: 96.46%, retention time: 1.02 min.

### Structure of (E)-1-(2-ethyl-4-(1-((2-methyl-[1,1'-biphenyl]-4-yl)methoxy)imino)ethyl)benzyl)pyrrolidine-3-carboxylic acid (Compound 28)

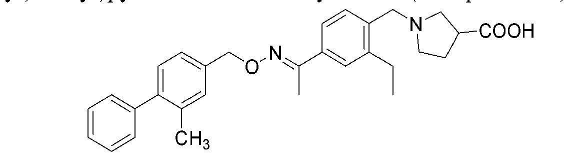

Greige solid, yield 30.1%, melting point: 144.3-146.4 °C. ¹H NMR (600 MHz, CD₃OD) δ 7.62 (s, 1H), 7.55 (dd, *J* = 8.0, 1.6 Hz, 1H), 7.47 (dd, *J* = 14.4, 6.3 Hz, 1H), 7.41-7.35 (m, 2H), 7.33-7.28 (m, 2H), 7.26 (dd, *J* = 5.0, 3.2 Hz, 3H), 7.14 (d, *J* = 7.7 Hz, 1H), 5.20 (s, 2H), 4.29 (s, 2H), 3.47-3.35 (m, 1H), 3.34-3.30 (m, 2H), 3.24 (t, *J* = 7.1 Hz, 2H), 3.03-3.09 (m, 1H), 2.80 (q, *J* = 7.5 Hz, 2H), 2.31-2.27 (m, 1H), 2.25 (s, 3H), 2.22 (s, 3H), 1.20 (t, *J* = 7.1 Hz, 3H). ¹³C NMR (151 MHz, CD₃OD) δ 178.58, 154.38, 143.61, 141.73, 141.47, 137.53, 137.03, 134.92, 130.99, 130.41, 129.86, 129.34, 128.79, 127.79, 126.57, 126.52, 125.40, 123.90, 75.69, 56.76, 54.66, 53.58, 43.99, 27.89, 25.26, 19.27, 14.57, 11.48. HRMS (ESI) (m/z): [M+H]⁺ calcd for C₃₀H₃₄N₂O₃, 471.2648; found: 471.2647. HPLC Purity: 96.16%, retention time: 1.66 min.

### Structure of (R, E)-1-(2-ethyl-4-(1-((2-methyl-[1,1'-biphenyl]-4-yl)methoxy)imino)ethyl)benzyl)pyrrolidine-3-carboxylic acid (Compound 29)

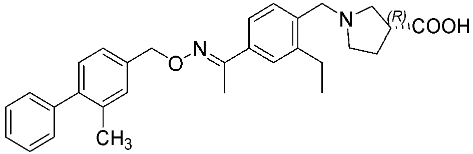

White solid, yield 14.9%, melting point: 149.6-150.6 °C. ¹H NMR (400 MHz, CD₃OD) δ 7.60 (s, 1H), 7.54 (d, *J* = 8.0 Hz, 1H), 7.45 (d, *J* = 8.0 Hz, 1H), 7.38 (t, *J* = 7.3 Hz, 2H), 7.34-7.18 (m, 5H), 7.14 (d, *J* = 7.7 Hz, 1H), 5.20 (s, 2H), 4.26 (s, 2H), 3.42-3.29 (m, 3H), 3.20 (t, *J* = 7.1 Hz, 2H), 3.09-2.97 (m, 1H), 2.79 (q, *J* = 7.5 Hz, 2H), 2.30-2.27 (m, 1H), 2.24 (s, 3H), 2.22 (s, 3H), 1.22 (t, *J* = 7.5 Hz, 3H). ¹³C NMR (151 MHz, CD₃OD) δ 178.63, 154.39, 143.55, 141.74, 141.47, 137.42, 137.04, 134.92, 131.32, 130.35, 129.85, 129.33, 128.79, 127.78, 126.53, 126.51, 125.39, 123.85, 75.68, 56.79, 54.73, 53.59, 44.01, 27.89, 25.24, 19.26, 14.55, 11.47. HRMS (ESI) (m/z): [M+H]⁺ calcd for C₃₀H₃₄N₂O₃, 471.2648; found: 471.2650. HPLC Purity: 97.51%, retention time: 1.45 min.

### Structure of (S, E)-1-(2-ethyl-4-(1-((2-methyl-[1,1'-biphenyl]-4-yl)methoxy)imino)ethyl)benzyl)pyrrolidine-3-carboxylic acid (Compound 30)

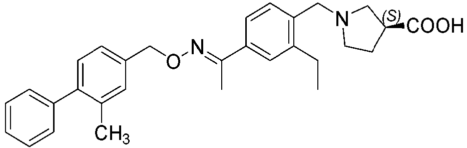

White solid, yield 31.8%, melting point: 145.6-148.9 °C. ¹H NMR (400 MHz, CD₃OD) δ 7.57 (s, 1H), 7.51 (d, *J* = 8.0 Hz, 1H), 7.44-7.35 (m, 3H), 7.33-7.26 (m, 5H), 7.17 (d, *J* = 7.7 Hz, 1H), 5.20 (s, 2H), 4.02 (s, 2H), 3.19 (t, *J* = 8.6 Hz, 1H), 3.11-3.00 (m, 3H), 2.90 (dd, *J* = 17.2, 7.9 Hz, 1H), 2.79 (q, *J* = 7.5 Hz, 2H), 2.26 (s, 3H), 2.24 (s, 3H), 2.15 (dd, *J* = 14.6, 7.3 Hz, 2H), 1.23 (t, *J* = 7.5 Hz, 3H). ¹³C NMR (151 MHz, CD₃OD) δ 179.95, 154.70, 143.25, 141.79, 141.48, 137.08, 136.62, 134.91, 130.01, 129.84, 129.31, 128.79, 127.77, 126.49, 126.28, 125.37, 123.57, 75.60, 57.40, 55.54, 53.76, 44.56, 28.06, 25.20, 19.22, 14.48, 11.50. HRMS (ESI) (m/z): [M+H]⁺ calcd for C₃₀H₃₄N₂O₃, 471.2648; found: 471.2650. HPLC Purity: 98.16%, retention time: 1.52 min.

### Structure of (E)-3-((2-ethyl-4-(1-((2-methyl-[1,1'-biphenyl]-4-yl)methoxy)imino)ethyl)benzyl)amine)propanoic acid (Compound 31)

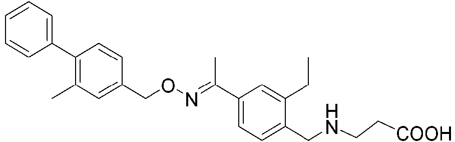

White solid, yield 22.4%, melting point: 149.6-149.8 °C. ¹H NMR (400 MHz, CD₃OD) δ 7.66 (s, 1H), 7.60 (d, *J* = 8.1 Hz, 1H), 7.43 (dd, *J* = 17.6, 7.9 Hz, 3H), 7.36-7.28 (m, 5H), 7.19 (d, *J* = 7.7 Hz, 1H), 5.24 (s, 2H), 4.28 (s, 2H), 3.25 (t, *J* = 6.2 Hz, 2H), 2.83 (q, *J* = 7.5 Hz, 2H), 2.60-2.47 (m, 2H), 2.28 (s, 3H), 2.25 (s, 3H), 1.29 (t, *J* = 7.5 Hz, 3H). ¹³C NMR (151 MHz, CD₃OD) δ 176.80, 154.30, 143.38, 141.76, 141.51, 137.84, 137.02, 134.93, 130.29, 130.06, 129.85, 129.31, 128.78, 127.78, 126.55, 126.52, 125.39, 124.05, 75.71, 44.64, 31.36, 25.08, 21.94, 19.22, 14.38, 11.40. HRMS (ESI) (m/z): [M+H]⁺ calcd for C₂₈H₃₂N₂O₃, 445.2491; found: 445.2490. HPLC Purity: 99.29%, retention time: 1.24 min.

### Structure of (E)-4-((2-ethyl-4-(1-((2-methyl-[1,1'-biphenyl]-4-yl)methoxy)imino)ethyl)benzyl)aminobutyric acid (Compound 32)

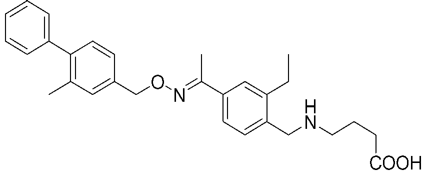

White solid, yield 13.2%, melting point: 133.3-136.3 °C. ¹H NMR (400 MHz, CD₃OD) δ 7.63 (s, 1H), 7.58 (d, *J* = 8.1 Hz, 1H), 7.49-7.36 (m, 3H), 7.36-7.28 (m, 5H), 7.17 (d, *J* = 7.7 Hz, 1H), 5.21 (s, 2H), 4.21 (s, 2H), 3.15 (t, *J* = 6.2 Hz, 2H), 2.79 (q, *J* = 7.5 Hz, 2H), 2.45-2.37 (m, 2H), 2.26 (s, 3H), 2.24 (s, 3H), 1.90-1.85 (m, 2H), 1.26 (t, *J* = 7.5 Hz, 3H). ¹³C NMR (151 MHz, CD₃OD) δ 176.86, 154.32, 143.24, 141.77, 141.51, 137.67, 137.04, 134.93, 130.46, 129.84, 129.80, 129.31, 128.78, 127.78, 126.52, 126.48, 125.38, 124.04, 75.70, 35.68, 25.21, 21.93, 21.44, 19.22, 14.36, 11.40. HRMS (ESI) (m/z): [M+H]⁺ calcd for C₂₉H₃₄N₂O₃, 459.2648; found: 459.2647. HPLC Purity: 98.69%, retention time: 1.34 min.

### Structure of (E)-1-(2-ethyl-4-(1-((2-methyl-[1,1'-biphenyl]-4-yl)methoxy)imino)ethyl)benzyl)piperidine-3-carboxylic acid (Compound 33)

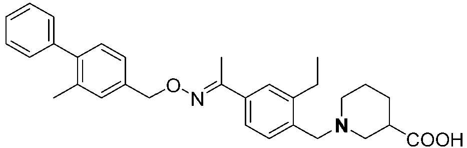

Pale yellow oily, yield 15.3%. ¹H NMR (400 MHz, CD₃OD) δ 7.61 (s, 1H), 7.56 (d, *J* = 8.0 Hz, 1H), 7.50-7.37 (m, 3H), 7.36-7.28 (m, 5H), 7.22-7.09 (m, 1H), 5.23 (s, 2H), 4.12 (s, 2H), 3.22-2.88 (m, 4H), 2.83 (q, *J* = 7.5 Hz, 2H), 2.61 (s, 1H), 2.27 (s, 3H), 2.25 (s, 3H), 1.79-1.70 (m, 4H), 1.31-1.19 (m, 3H). ¹³C NMR (151 MHz, CD₃OD) δ 178.19, 154.48, 143.99, 141.75, 141.48, 137.42, 137.05, 134.92, 130.86, 129.85, 129.32, 128.81, 128.79, 128.77, 127.79, 126.51, 125.39, 123.77, 75.67, 57.43, 55.07, 52.96, 25.22, 22.28, 19.25, 14.48, 11.48. HRMS (ESI) (m/z): [M+H]⁺ calcd for C₃₁H₃₆N₂O₃, 485.2804; found: 485.2802. HPLC Purity: 97.56%, retention time: 1.42 min.

### Structure of (S, E)-1-(2-ethyl-4-(1-(((4'-fluoro-2-methyl-[1,1'-biphenyl]-4-yl)methoxy)imino)ethyl)benzyl)pyrrolidine-3-carboxylic acid (Compound 34)

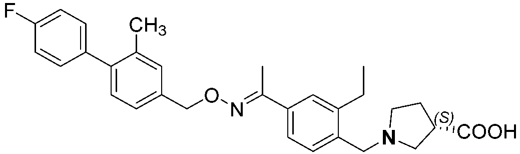

Pale yellow solid, yield 11.3%. ¹H NMR (600 MHz, CD₃OD) δ 7.64 (d, *J* = 1.9 Hz, 1H), 7.58 (dd, *J* = 8.1, 1.9 Hz, 1H), 7.47 (d, *J* = 8.1 Hz, 1H), 7.33-7.25 (m, 4H), 7.19-7.10 (m, 3H), 5.22 (s, 2H), 4.42-4.34 (m, 2H), 3.53- 3.47 (m, 1H), 3.38-3.32 (m, 2H), 3.29-3.24 (m, 1H), 3.12-3.04 (m, 1H), 2.82 (q, *J* = 7.5 Hz, 2H), 2.32 (dt, *J* = 14.2, 7.9 Hz, 1H), 2.27 (s, 3H), 2.24 (s, 3H), 2.23-2.18 (m, 1H), 1.26 (t, *J* = 7.5 Hz, 3H). ¹³C NMR (151 MHz, CD₃OD) δ 177.91, 162.81, 161.19, 154.29, 143.70, 140.39, 137.84, 137.27, 135.04, 130.62, 130.56, 130.44, 129.88, 129.37, 114.54, 114.40, 75.64, 56.64, 54.50, 53.58, 43.83, 27.74, 25.22, 19.19, 14.54, 11.38. MS (ESI) (m/z): [M+H]⁺ 489.25.

### Structure of (S, E)-1-(2-ethyl-4-(1-(((2-fluoro-[1,1'-biphenyl]-4-yl)methoxy)imino)ethyl)benzyl)pyrrolidine-3-carboxylic acid (Compound 35)

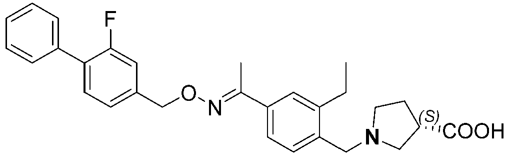

Pale yellow solid, yield 18.7%. ¹H NMR (600 MHz, CD₃OD) δ 7.63 (s, 1H), 7.57 (d, *J* = 6.1 Hz, 1H), 7.52 (d, *J* = 8.0 Hz, 2H), 7.48 - 7.40 (m, 4H), 7.35 (t, *J* = 7.4 Hz, 1H), 7.28 (d, *J* = 9.4 Hz, 1H), 7.22 (d, *J* = 11.5 Hz, 1H), 5.26 (s, 2H), 4.33 (d, *J* = 4.1 Hz, 2H), 3.46 (dd, *J* = 11.1, 5.8 Hz, 1H), 3.34 (d, *J* = 11.2 Hz, 2H), 3.25 (t, *J* = 9.0 Hz, 1H), 3.07 (ddd, *J* = 14.5, 8.6, 5.8 Hz, 1H), 2.81 (q, *J* = 7.5 Hz, 2H), 2.29 (s, 4H), 2.22 (dq, *J* = 13.0, 6.4 Hz, 1H), 1.24 (t, *J* = 7.5 Hz, 3H). ¹³C NMR (151 MHz, CD₃OD) δ 178.47, 159.53, 154.78, 143.69, 135.54, 130.42, 130.38, 128.61, 128.58, 128.09, 127.35, 126.66, 123.95, 123.69, 123.66, 115.10, 114.94, 74.70, 56.65, 54.60, 53.63, 43.70, 27.76, 25.22, 14.52, 11.42. MS (ESI) (m/z): [M+H]⁺ 475.30.

### Structure of (S, E)-1-(2-ethyl-4-(1-(((4-(furan-3-yl)-3-methylbenzyl)oxy)imino)ethyl)benzyl)pyrrolidine-3-carboxylic acid (Compound 36)

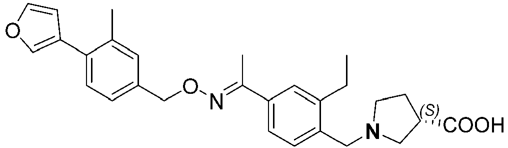

Pale yellow solid, yield 21.2%. ¹H NMR (600 MHz, CD₃OD) δ 7.61 (dd, *J* = 13.5, 1.8 Hz, 2H), 7.56 - 7.53 (m, 2H), 7.47 (d, *J* = 8.1 Hz, 1H), 7.30 (d, *J* = 7.8 Hz, 1H), 7.27 (s, 1H), 7.23 (dd, *J* = 7.8, 1.8 Hz, 1H), 6.62 (d, *J* = 1.9 Hz, 1H), 5.18 (s, 2H), 4.44 - 4.28 (m, 2H), 3.52 (dd, *J* = 11.3, 5.7 Hz, 1H), 3.40 - 3.36 (m, 1H), 3.35 - 3.32 (m, 1H), 3.27 (dt, *J* = 11.1, 7.4 Hz, 1H), 3.08 (ddd, *J* = 14.4, 8.8, 5.7 Hz, 1H), 2.80 (q, *J* = 7.5 Hz, 2H), 2.36 (s, 3H), 2.30 (dt, *J* = 15.6, 7.0 Hz, 1H), 2.24 (s, 3H), 2.22 (dd, *J* = 13.5, 6.2 Hz, 1H), 1.23 (t, *J* = 7.5 Hz, 3H). ¹³C NMR (151 MHz, CD₃OD) δ 177.76, 154.25, 143.72, 142.57, 139.97, 137.86, 136.85, 135.34, 131.69, 130.51, 130.15, 128.74, 126.66, 125.53, 123.98, 110.90, 75.65, 56.45, 54.32, 53.53, 43.55, 27.70, 25.24, 20.03, 14.56, 11.41.

### Structure of (S, E)-1-(2-ethyl-4-(1-(((3-methyl-4-(pyridin-4-yl)benzyl)oxy)imino)ethyl)benzyl)pyrrolidine-3-carboxylic acid (Compound 37)

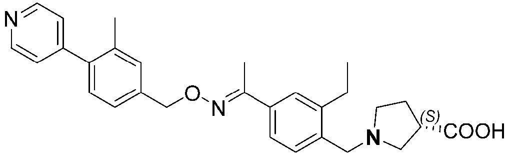

Pale yellow solid, yield 19.2%. ¹H NMR (600 MHz, CD₃OD) δ8.56 (d, *J* = 6.2 Hz, 2H), 7.63 (s, 1H), 7.56 (d, *J* = 8.1 Hz, 1H), 7.47 (d, *J* = 8.1 Hz, 1H), 7.40 (d, *J* = 6.2 Hz, 2H), 7.37 (s, 1H), 7.34 (d, *J* = 7.8 Hz, 1H), 7.22 (d, *J* = 7.8 Hz, 1H), 5.24 (s, 2H), 4.42 - 4.33 (m, 2H), 3.50 (dd, *J* = 11.2, 5.7 Hz, 1H), 3.38 (d, *J* = 7.8 Hz, 1H), 3.34 (s, 1H), 3.30 - 3.24 (m, 1H), 3.11 - 3.04 (m, 1H), 2.81 (q, *J* = 7.5 Hz, 2H), 2.32 (dd, *J* = 15.2, 7.1 Hz, 1H), 2.28 (s, 3H), 2.27 (s, 3H), 2.22 (dt, *J* = 13.6, 6.7 Hz, 1H), 1.24 (t, *J* = 7.5 Hz, 3H). ¹³C NMR (151 MHz, CD₃OD) δ 177.89, 154.44, 150.81, 148.56, 143.72, 138.78, 138.11, 137.73, 134.81, 130.48, 130.13, 128.98, 126.66, 125.69, 124.62, 123.97, 75.40, 56.56, 54.41, 53.57, 43.62, 27.73, 25.20, 19.02, 14.56, 11.38.

### Structure of (S, E)-1-(2-ethyl-4-(1-(((3-methyl-4-(pyrimidin-5-yl)benzyl)oxy)imino)ethyl)benzyl)pyrrolidine-3-carboxylic acid (Compound 38)

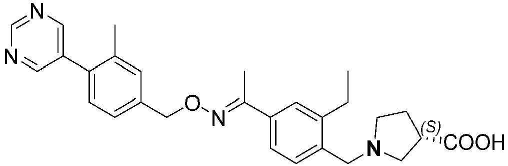

Pale yellow solid, yield 19.7%. ¹H NMR (600 MHz, CD₃OD) δ 9.14 (s, 1H), 8.78 (s, 2H), 7.62 (d, *J* = 1.9 Hz, 1H), 7.56 (dd, *J* = 8.1, 1.9 Hz, 1H), 7.47 (d, *J* = 8.1 Hz, 1H), 7.40 (d, *J* = 1.7 Hz, 1H), 7.37 (dd, *J* = 7.8, 1.8 Hz, 1H), 7.26 (d, *J* = 7.8 Hz, 1H), 5.24 (s, 2H), 4.42 - 4.32 (m, 2H), 3.49 (dd, *J* = 11.2, 5.8 Hz, 1H), 3.38 - 3.32 (m, 2H), 3.27 (dt, *J* = 10.8, 7.3 Hz, 1H), 3.08 (ddd, *J* = 14.4, 8.7, 5.8 Hz, 1H), 2.81 (q, *J* = 7.5 Hz, 2H), 2.35 - 2.31 (m, 1H), 2.29 (s, 3H), 2.27 (s, 3H), 2.25 - 2.19 (m, 1H), 1.24 (t, *J* = 7.5 Hz, 3H). ¹³C NMR (151 MHz, CD₃OD) δ 178.06, 156.57, 156.36, 154.49, 143.72, 139.33, 137.69, 135.64, 135.61, 133.41, 130.46, 130.18, 129.63, 126.65, 125.90, 123.96, 75.31, 56.55, 54.46, 53.59, 43.61, 27.73, 25.24, 18.95, 14.55, 11.39.

### Structure of (S, E)-1-(2-ethyl-4-(1-((4-(-1-(tert-butyl)-1H-pyrazol-4-yl)-3-methylbenzyl)oxy)imino)ethyl)benzyl)pyrrolidine-3-carboxylic acid (Compound 39)

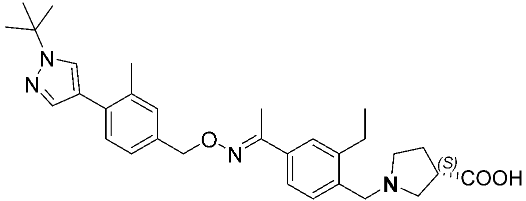

Pale yellow solid, yield 31.1%. ¹H NMR (600 MHz, CD₃OD) δ 7.85 (d, *J* = 0.8 Hz, 1H), 7.65 - 7.61 (m, 2H), 7.56 (dd, *J* = 8.1, 1.9 Hz, 1H), 7.45 (d, *J* = 8.1 Hz, 1H), 7.33 (d, *J* = 7.8 Hz, 1H), 7.29 - 7.26 (m, 1H), 7.23 (dd, *J* = 7.8, 1.8 Hz, 1H), 5.18 (s, 2H), 4.39 - 4.31 (m, 2H), 3.47 (dd, *J* = 11.1, 5.8 Hz, 1H), 3.33 (dd, *J* = 10.8, 6.1 Hz, 2H), 3.25 (d, *J* = 10.3 Hz, 1H), 3.07 (ddd, *J* = 14.7, 8.8, 5.9 Hz, 1H), 2.80 (q, *J* = 7.5 Hz, 2H), 2.39 (s, 3H), 2.33 - 2.26 (m, 1H), 2.25 (s, 3H), 2.24 - 2.19 (m, 1H), 1.62 (s, 9H), 1.24 (t, *J* = 7.5 Hz, 3H). ¹³C NMR (151 MHz, CD₃OD) δ 178.15, 154.25, 143.67, 137.80, 137.77, 136.36, 134.87, 131.84, 130.41, 130.20, 128.69, 126.65, 125.63, 125.41, 123.95, 121.12, 75.70, 58.41, 56.64, 54.55, 53.60, 43.65, 28.66, 27.75, 25.19, 20.06, 14.54, 11.39. MS (ESI) (m/z): [M+H]⁺517.30.

### Structure of (S, E)-1-(2-ethyl-4-(1-((3-methyl-4-(1-methyl-1H-pyrazol-4-yl)benzyl)oxy)imino)ethyl)benzyl)pyrrolidine-3-carboxylic acid (Compound 40)

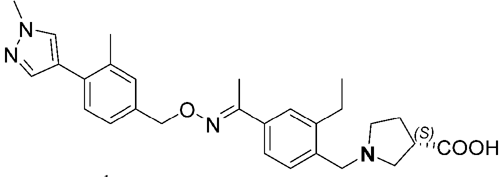

Pale yellow solid, yield 29.5%. ¹H NMR (600 MHz, CD₃OD) δ 7.74 (s, 1H), 7.62 (d, *J* = 1.9 Hz, 1H), 7.61 - 7.60 (m, 1H), 7.56 (dd, *J* = 8.1, 1.9 Hz, 1H), 7.45 (d, *J* = 8.1 Hz, 1H), 7.32 (d, *J* = 7.8 Hz, 1H), 7.28 (d, *J* = 1.8 Hz, 1H), 7.22 (dd, *J* = 7.9, 1.8 Hz, 1H), 5.18 (s, 2H), 4.41 - 4.32 (m, 2H), 3.93 (s, 3H), 3.49 (dd, *J* = 11.2, 5.8 Hz, 1H), 3.35 (dd, *J* = 11.2, 7.0 Hz, 2H), 3.26 (dt, *J* = 10.8, 7.3 Hz, 1H), 3.12 - 3.02 (m, 1H), 2.80 (q, *J* = 7.5 Hz, 2H), 2.38 (s, 3H), 2.30 (ddd, *J* = 15.7, 8.4, 6.2 Hz, 1H), 2.25 (s, 3H), 2.24 - 2.18 (m, 1H), 1.24 (t, *J* = 7.5 Hz, 3H). ¹³C NMR (151 MHz, CD₃OD) δ 177.95, 154.26, 143.69, 138.10, 137.85, 136.50, 134.86, 131.45, 130.44, 130.23, 129.63, 128.60, 126.66, 125.64, 123.96, 121.92, 75.66, 56.60, 54.49, 53.58, 43.61, 37.44, 27.73, 25.22, 20.08, 14.53, 11.38. MS (ESI) (m/z): [M+H]⁺ 475.30.

### Structure of (S, E)-1-(2-ethyl-4-(1-(((3-methyl-4-(thiophen-3-yl)benzyl)oxy)imino)ethyl)benzyl)pyrrolidine-3-carboxylic acid (Compound 41)

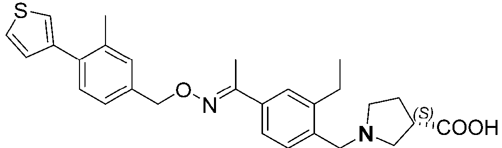

Pale yellow solid, yield 9.5%. ¹H NMR (600 MHz, CD₃OD) δ 7.63 (d, *J* = 1.9 Hz, 1H), 7.56 (dd, *J* = 8.1, 1.9 Hz, 1H), 7.48 - 7.46 (m, 1H), 7.43 (dd, *J* = 4.9, 3.0 Hz, 1H), 7.29 - 7.27 (m, 1H), 7.27 (dd, *J* = 3.0, 1.3 Hz, 1H), 7.26 - 7.22 (m, 2H), 7.13 (dd, *J* = 5.0, 1.3 Hz, 1H), 5.19 (s, 2H), 4.38 (dd, *J* = 6.1, 3.1 Hz, 2H), 3.53 - 3.49 (m, 1H), 3.36 (dd, *J* = 10.8, 4.3 Hz, 2H), 3.28 (dd, *J* = 10.9, 7.2 Hz, 1H), 3.09 (td, *J* = 8.6, 4.2 Hz, 1H), 2.81 - 2.78 (m, 2H), 2.31 (s, 4H), 2.25 (s, 3H), 2.24 - 2.20 (m, 1H), 1.24 (t, *J* = 7.5 Hz, 3H). ¹³C NMR (151 MHz, CD₃OD) δ 178.04, 154.24, 143.72, 141.88, 137.88, 137.04, 136.10, 135.31, 130.50, 129.99, 129.30, 128.45, 126.68, 125.40, 124.71, 123.99, 122.26, 75.67, 56.42, 54.43, 53.60, 43.54, 27.71, 25.26, 19.58, 14.56, 11.41.

### Structure of (S, E)-1-(2-ethyl-4-(1-(((2-(trifluoromethyl)-[1,1'-biphenyl]-4-yl)methoxy)imino)ethyl)benzyl)pyrrolidine-3-carboxylic acid (Compound 42)

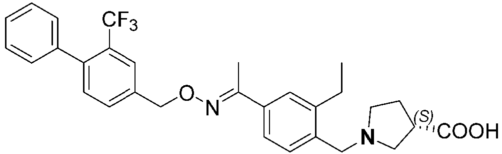

Pale yellow solid, yield 31.8%. ¹H NMR (600 MHz, CD₃OD) δ 7.81 (s, 1H), 7.66 (d, *J* = 7.8 Hz, 1H), 7.63 (d, *J* = 1.9 Hz, 1H), 7.56 (dd, *J* = 8.0, 1.9 Hz, 1H), 7.48 (d, *J* = 8.1 Hz, 1H), 7.38 (dd, *J* = 5.1, 1.9 Hz, 3H), 7.34 (d, *J* = 7.8 Hz, 1H), 7.28 (dd, *J* = 6.7, 2.9 Hz, 2H), 5.32 (s, 2H), 4.41 - 4.32 (m, 2H), 3.49 (dd, *J* = 11.2, 5.8 Hz, 1H), 3.34 (t, *J* = 8.1 Hz, 2H), 3.29 - 3.23 (m, 1H), 3.08 (ddd, *J* = 14.4, 8.7, 5.9 Hz, 1H), 2.81 (q, *J* = 7.5 Hz, 2H), 2.34 - 2.29 (m, 1H), 2.29 (s, 3H), 2.23 (tt, *J* = 13.5, 6.2 Hz, 1H), 1.24 (t, *J* = 7.5 Hz, 3H). ¹³C NMR (151 MHz, CD₃OD) δ 179.63, 154.93, 143.74, 140.81, 139.60, 138.23, 137.55, 132.04, 130.96, 130.48, 128.64, 128.62, 127.45, 127.36, 126.66, 125.35, 125.31, 123.97, 74.69, 56.58, 54.49, 53.60, 43.68, 27.75, 25.23, 14.52, 11.41. MS (ESI) (m/z): [M+H]⁺ 525.25.

### Structure of (S, E)-1-(2-ethyl-4-(1-((3-methyl-4-(1-methyl-1H-pyrrol-3-yl)benzyl)oxy)imino)ethyl)benzyl)pyrrolidine-3-carboxylic acid (Compound 43)

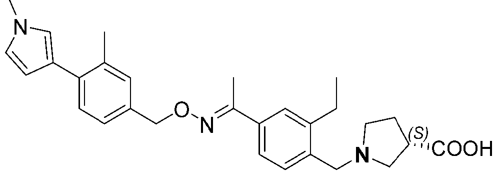

Pale yellow solid, yield 21.5%. ¹H NMR (600 MHz, CD₃OD) δ 7.61 (s, 1H), 7.54 (d, *J* = 7.8 Hz, 1H), 7.44 (d, *J* = 8.1 Hz, 1H), 7.29 (d, *J* = 8.0 Hz, 1H), 7.20 (s, 1H), 7.15 (d, *J* = 7.8 Hz, 1H), 6.74 (s, 1H), 6.64 (s, 1H), 6.21 (s, 1H), 5.15 (s, 2H), 4.35 (t, *J* = 10.7 Hz, 2H), 3.66 (s, 3H), 3.48 (dd, *J* = 11.3, 5.7 Hz, 1H), 3.34 (s, 2H), 3.24 (d, *J* = 9.9 Hz, 1H), 3.07 (d, *J* = 8.1 Hz, 1H), 2.79 (q, *J* = 7.5 Hz, 2H), 2.39 (s, 3H), 2.30 - 2.24 (m, 1H), 2.23 (s, 3H), 2.21 (s, 1H), 1.23 (t, *J* = 7.6 Hz, 3H). ¹³C NMR (151 MHz, CD₃OD) δ 178.09, 154.09, 143.65, 137.89, 135.78, 134.80, 134.36, 130.45, 130.22, 130.11, 128.42, 126.64, 125.44, 123.94, 123.77, 121.28, 120.43, 108.50, 75.92, 56.53, 54.45, 53.57, 43.63, 34.81, 27.73, 25.22, 20.45, 14.53, 11.40. MS (ESI) (m/z): [M+H]⁺ 474.25.

### Structure of (S, E)-1-(2-ethyl-4-(1-((3-methyl-4-(1-methyl-1H-imidazol-5-yl)benzyl)oxy)imino)ethyl)benzyl)pyrrolidine-3-carboxylic acid (Compound 44)

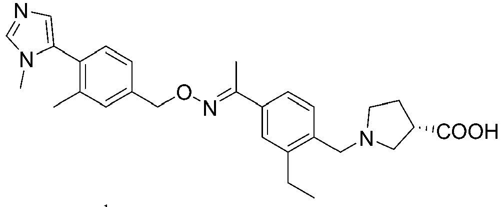

Pale yellow solid, yield 27.1%. ¹H NMR (600 MHz, CD₃OD) δ7.72 (s, 1H), 7.62 (s, 1H), 7.56 (d, *J* = 7.9 Hz, 1H), 7.46 (d, *J* = 8.1 Hz, 1H), 7.38 (s, 1H), 7.32 (d, *J* = 7.8 Hz, 1H), 7.21 (d, *J* = 7.8 Hz, 1H), 6.88 (s, 1H), 5.24 (s, 2H), 4.34 (d, *J* = 4.2 Hz, 2H), 3.45 (s, 4H), 3.34 (s, 2H), 3.26 (s, 1H), 3.07 (q, *J* = 6.0 Hz, 1H), 2.81 (q, *J* = 7.6 Hz, 2H), 2.27 (s, 3H), 2.23 (t, *J* = 6.7 Hz, 2H), 2.18 (s, 3H), 1.24 (t, *J* = 7.5 Hz, 3H). ¹³C NMR (151 MHz, CD₃OD) δ 178.17, 154.52, 143.68, 139.34, 138.14, 137.90, 137.62, 132.16, 130.91, 130.41, 129.62, 128.07, 126.63, 126.33, 125.24, 123.94, 75.41, 56.69, 54.56, 53.60, 43.72, 30.75, 27.78, 25.23, 18.73, 14.54, 11.42. MS (ESI) (m/z): [M+H]⁺ 475.25.

### Structure of (S, E)-1-(2-ethyl-4-(1-(((3,5-dimethylisoxazol-4-yl)-3-methylbenzyl)oxy)imino)ethyl)benzyl)pyrrolidine-3-carboxylic acid (Compound 45)

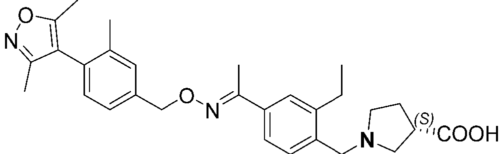

Pale yellow solid, yield 20.7%. ¹H NMR (600 MHz, CD₃OD) δ 7.64 (s, 1H), 7.57 (d, *J* = 8.1 Hz, 1H), 7.48 (d, *J* = 8.1 Hz, 1H), 7.39 (s, 1H), 7.31 (d, *J* = 7.8 Hz, 1H), 7.12 (dd, *J* = 7.8, 4.1 Hz, 1H), 5.23 (s, 2H), 4.40 (d, *J* = 13.6 Hz, 2H), 3.50 (dd, *J* = 11.3, 5.8 Hz, 1H), 3.36 (q, *J* = 10.0, 9.1 Hz, 2H), 3.30 - 3.25 (m, 1H), 3.12 - 3.05 (m, 1H), 2.82 (q, *J* = 7.5 Hz, 2H), 2.31 (dd, *J* = 14.6, 6.8 Hz, 1H), 2.27 (s, 3H), 2.26 - 2.23 (m, 1H), 2.21 (s, 3H), 2.14 (s, 3H), 2.05 (s, 3H), 1.25 (t, *J* = 7.5 Hz, 3H). ¹³C NMR (151 MHz, CD₃OD) δ 178.08, 165.70, 159.25, 154.41, 143.72, 138.64, 137.45, 130.52, 130.46, 129.72, 128.36, 126.66, 125.58, 123.98, 115.90, 75.51, 56.54, 54.47, 53.59, 43.60, 27.73, 25.24, 18.47, 14.55, 11.43, 9.83, 8.99. MS (ESI) (m/z): [M+H]⁺ 490.30.

### Structure of (S, E)-1-(2-ethyl-4-(1-(((3-methylbenzyl)oxy)imino)ethyl)benzyl)pyrrolidine-3-carboxylic acid (Compound 46)

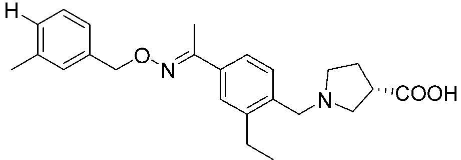

Pale yellow solid, yield 50.1%. ¹H NMR (600 MHz, CD₃OD) δ 7.62 (d, *J* = 1.9 Hz, 1H), 7.55 (dd, *J* = 8.1, 1.9 Hz, 1H), 7.47 (d, *J* = 8.1 Hz, 1H), 7.25 - 7.19 (m, 2H), 7.17 (d, *J* = 7.5 Hz, 1H), 7.10 (d, *J* = 7.5 Hz, 1H), 5.16 (s, 2H), 4.42 - 4.32 (m, 2H), 3.50 (dd, *J* = 11.3, 5.9 Hz, 1H), 3.38 - 3.33 (m, 2H), 3.28 (dd, *J* = 10.9, 7.5 Hz, 1H), 3.09 (ddd, *J* = 14.6, 8.5, 5.8 Hz, 1H), 2.81 (q, *J* = 7.5 Hz, 2H), 2.33 (s, 3H), 2.32 - 2.28 (m, 1H), 2.24 (s, 4H), 1.24 (t, *J* = 7.5 Hz, 3H). ¹³C NMR (151 MHz, CD₃OD) δ 178.42, 154.23, 143.69, 137.94, 137.83, 137.66, 130.47, 128.47, 128.09, 127.88, 126.65, 124.91, 123.96, 75.94, 56.52, 54.52, 53.64, 43.66, 27.75, 25.25, 20.06, 14.55, 11.40. MS (ESI) (m/z): [M+H]⁺ 395.25.

### Structure of (S, E)-1-(2-ethyl-4-(1-(((2',4'-difluoro-2-methyl-[1,1'-biphenyl]-4-yl)methoxy)imino)ethyl)benzyl)pyrrolidine-3-carboxylic acid (Compound 47)

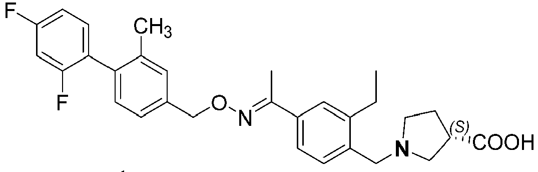

Pale yellow solid, yield 19.0%. ¹H NMR (600 MHz, CD₃OD) δ 7.63 (s, 1H), 7.56 (d, *J* = 7.9 Hz, 1H), 7.47 (d, *J* = 8.1 Hz, 1H), 7.33 (s, 1H), 7.28 (d, *J* = 8.0 Hz, 1H), 7.25 (d, *J* = 7.5 Hz, 1H), 7.14 (d, *J* = 7.9 Hz, 1H), 7.01 (t, *J* = 8.8 Hz, 2H), 5.22 (s, 2H), 4.41 - 4.28 (m, 2H), 3.48 (dd, *J* = 11.1, 5.7 Hz, 1H), 3.37 - 3.32 (m, 2H), 3.25 (q, *J* = 8.0 Hz, 1H), 3.07 (t, *J* = 7.8 Hz, 1H), 2.80 (q, *J* = 7.5 Hz, 2H), 2.34 - 2.28 (m, 1H), 2.26 (s, 3H), 2.22 (d, *J* = 14.8 Hz, 1H), 2.15 (s, 3H), 1.24 (t, *J* = 7.5 Hz, 3H). ¹³C NMR (151 MHz, CD₃OD) δ 177.31, 162.53, 160.90, 159.67, 158.03, 153.59, 142.87, 137.32, 136.91, 135.57, 133.46, 131.48, 131.44, 129.64, 129.00, 128.62, 125.84, 124.47, 123.14, 110.15, 109.98, 102.59, 102.42, 102.24, 74.75, 55.85, 53.67, 52.76, 42.92, 26.96, 24.42, 17.83, 13.73, 10.62. MS (ESI) (m/z): [M+H]⁺ 507.25.

### Structure of (S, E)-1-(2-ethyl-4-(1-(((3-methyl-4-(pyrrolidin-1-yl)benzyl)oxy)imino)ethyl)benzyl)pyrrolidine-3-carboxylic acid (Compound 48)

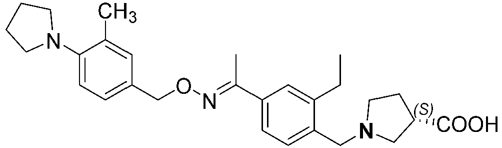

Pale yellow solid, yield 17.5%. ¹H NMR (600 MHz, CD₃OD) δ 7.61 (d, *J* = 1.9 Hz, 1H), 7.54 (dd, *J* = 8.0, 1.9 Hz, 1H), 7.46 (d, *J* = 8.1 Hz, 1H), 7.14 (d, *J* = 2.1 Hz, 1H), 7.11 (dd, *J* = 8.2, 2.2 Hz, 1H), 6.87 (d, *J* = 8.2 Hz, 1H), 5.08 (s, 2H), 4.36 (d, *J* = 8.0 Hz, 2H), 3.50 (dd, *J* = 11.2, 5.7 Hz, 1H), 3.38 - 3.32 (m, 2H), 3.26 (dt, *J* = 10.9, 7.3 Hz, 1H), 3.15 - 3.10 (m, 4H), 3.07 (td, *J* = 8.8, 4.4 Hz, 1H), 2.80 (q, *J* = 7.5 Hz, 2H), 2.33 - 2.30 (m, 1H), 2.29 (s, 3H), 2.26 - 2.22 (m, 1H), 2.20 (s, 3H), 1.94 - 1.88 (m, 4H), 1.24 (t, *J* = 7.5 Hz, 3H). ¹³C NMR (151 MHz, CD₃OD) δ 178.01, 153.87, 149.09, 143.66, 137.93, 131.74, 130.47, 129.79, 128.56, 126.61, 126.49, 123.93, 115.53, 75.99, 56.52, 54.41, 53.55, 50.82, 43.67, 27.75, 25.24, 24.30, 19.25, 14.57, 11.40. MS (ESI) (m/z): [M+H]⁺ 464.30.

### Structure of (S, E)-1-(2-ethyl-4-(1-(((3-methyl-4-(piperidin-1-yl)benzyl)oxy)imino)ethyl)benzyl)pyrrolidine-3-carboxylic acid (Compound 49)

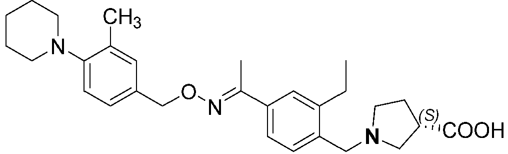

Pale yellow solid, yield 19.4%. ¹H NMR (500 MHz, CD₃OD) δ 7.62 (d, *J* = 1.9 Hz, 1H), 7.55 (dd, *J* = 8.1, 1.9 Hz, 1H), 7.47 (d, *J* = 8.1 Hz, 1H), 7.20 (d, *J* = 2.1 Hz, 1H), 7.17 (dd, *J* = 8.1, 2.1 Hz, 1H), 6.99 (d, *J* = 8.1 Hz, 1H), 5.11 (s, 2H), 4.39 - 4.29 (m, 2H), 3.48 (dd, *J* = 11.1, 5.8 Hz, 1H), 3.37 (s, 1H), 3.35 - 3.31 (m, 1H), 3.29 - 3.22 (m, 1H), 3.12 - 3.04 (m, 1H), 2.86 - 2.77 (m, 6H), 2.32 (dd, *J* = 13.2, 7.4 Hz, 1H), 2.29 (s, 3H), 2.25 (d, *J* = 12.9 Hz, 1H), 2.23 (s, 3H), 1.72 (p, *J* = 5.7 Hz, 4H), 1.59 (q, *J* = 5.8, 5.4 Hz, 2H), 1.25 (t, *J* = 7.5 Hz, 3H). ¹³C NMR (126 MHz, CD₃OD) δ 178.42, 154.03, 152.53, 143.62, 137.78, 132.13, 132.04, 130.84, 130.42, 126.59, 126.44, 123.91, 118.45, 75.89, 56.59, 54.54, 53.60, 53.20, 43.76, 27.78, 26.29, 25.24, 24.08, 16.69, 14.55, 11.42. MS (ESI) (m/z): [M+H]⁺ 478.30.

### Structure of (S, E)-1-(2-ethyl-4-(1-(((3-methyl-4-morpholinylbenzyl)oxy)imino)ethyl)benzyl)pyrrolidine-3-carboxylic acid (Compound 50)

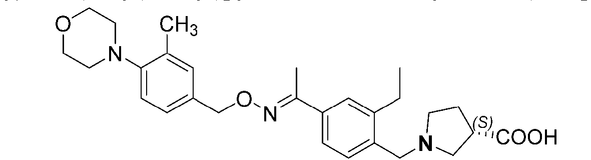

Pale yellow solid, yield 20.7%. ¹H NMR (500 MHz, CD₃OD) δ 7.64 (d, *J* = 1.8 Hz, 1H), 7.57 (dd, *J* = 8.1, 1.9 Hz, 1H), 7.48 (d, *J* = 8.1 Hz, 1H), 7.26 - 7.18 (m, 2H), 7.04 (dt, *J* = 8.1, 1.9 Hz, 1H), 5.13 (s, 2H), 4.43 - 4.33 (m, 2H), 3.88 - 3.77 (m, 4H), 3.55 - 3.47 (m, 1H), 3.37 (dd, *J* = 9.5, 6.3 Hz, 2H), 3.32 - 3.26 (m, 1H), 3.12 (tt, *J* = 8.7, 6.0 Hz, 1H), 2.88 (dq, *J* = 4.8, 2.6 Hz, 4H), 2.82 (q, *J* = 7.6 Hz, 2H), 2.39 - 2.29 (m, 4H), 2.28 - 2.20 (m, 4H), 1.29 - 1.23 (m, 3H). ¹³C NMR (126 MHz, CD₃OD) δ 178.88, 154.10, 151.00, 143.69, 137.88, 132.82, 132.22, 130.99, 130.47, 130.17, 126.64, 126.57, 123.96, 118.46, 75.76, 67.06, 56.48, 54.55, 53.67, 52.11, 43.64, 27.74, 25.25, 16.72, 14.56, 11.41. MS (ESI) (m/z): [M+H]⁺ 480.30.

### Structure of (S, E)-1-(2-ethyl-4-(1-(((2', 3', 4'-trifluoro-[1,1'-biphenyl]-4-yl)methoxy)imino)ethyl)benzyl)pyrrolidine-3-carboxylic acid (Compound 51)

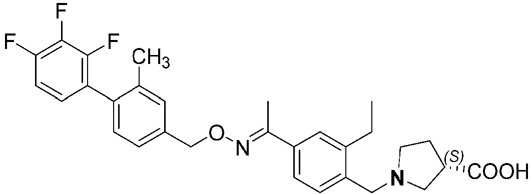

Pale yellow solid, yield 20.1%. ¹H NMR (600 MHz, CD₃OD) δ 7.63 (s, 1H), 7.56 (d, *J* = 8.0 Hz, 1H), 7.48 (d, *J* = 8.2 Hz, 1H), 7.34 (s, 1H), 7.30 (d, *J* = 7.9 Hz, 1H), 7.16 (d, *J* = 7.5 Hz, 2H), 7.03 (q, *J* = 6.3 Hz, 1H), 5.22 (s, 2H), 4.38 (t, *J* = 10.0 Hz, 2H), 3.50 (s, 1H), 3.36 (d, *J* = 8.6 Hz, 2H), 3.28 (d, *J* = 10.5 Hz, 1H), 3.09 (s, 1H), 2.80 (q, *J* = 7.5 Hz, 2H), 2.26 (s, 5H), 2.16 (s, 3H), 1.24 (t, *J* = 7.5 Hz, 3H). ¹³C NMR (151 MHz, CD₃OD) δ 154.43, 143.70, 138.70, 137.73, 136.31, 133.12, 133.11, 130.48, 130.25, 129.75, 129.50, 126.65, 125.36, 125.14, 125.12, 125.06, 123.96, 111.81, 111.76, 111.76, 75.44, 56.57, 56.51, 56.48, 54.74, 54.43, 53.59, 53.01, 43.63, 43.61, 43.56, 43.45, 43.30, 43.11, 27.74, 25.24, 18.57, 18.55, 14.53, 11.42. MS (ESI) (m/z): [M+H]⁺ 525.20.

### The structure of (S, E)-1-(2-ethyl-4-(1-(((2',4',5'-trifluoro-2-methyl-[1,1'-biphenyl]-4-yl)methoxy)imino)ethyl)benzyl)pyrrolidine-3-carboxylic acid (Compound 52)

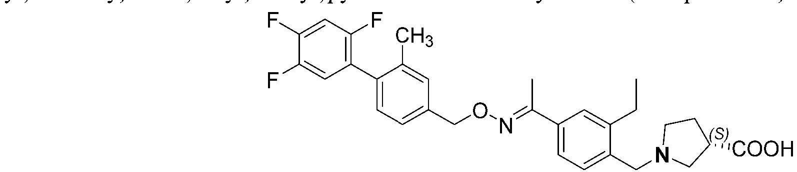

Pale yellow solid, yield 17.9%. ¹H NMR (600 MHz, CD₃OD) δ 7.62 (s, 1H), 7.55 (d, *J* = 8.0 Hz, 1H), 7.51 (d, *J* = 8.3 Hz, 1H), 7.33 (s, 1H), 7.28 (d, *J* = 7.6 Hz, 1H), 7.22 (d, *J* = 6.7 Hz, 1H), 7.14 (d, *J* = 7.3 Hz, 2H), 5.21 (s, 2H), 4.38 (t, *J* = 8.9 Hz, 2H), 3.49 (s, 1H), 3.35 (s, 2H), 3.29 (s, 1H), 3.10 (s, 1H), 2.81 (d, *J* = 7.5 Hz, 2H), 2.30 (d, *J* = 7.4 Hz, 1H), 2.25 (s, 3H), 2.23 (s, 1H), 2.16 (s, 3H), 1.23 (t, *J* = 7.5 Hz, 3H). ¹³C NMR (151 MHz, CD₃OD) δ 155.58, 154.43, 153.96, 150.21, 148.51, 147.31, 145.70, 143.71, 138.63, 137.70, 136.34, 133.18, 130.53, 130.33, 129.70, 129.49, 126.64, 125.34, 123.95, 118.93, 118.76, 105.40, 105.26, 105.20, 105.06, 75.46, 56.40, 54.49, 53.66, 43.71, 27.79, 25.30, 18.59, 18.57, 14.56, 11.43. MS (ESI) (m/z): [M+H]⁺ 525.25.

### Structure of (S, E)-1-(2-ethyl-4-(1-(((4-(6-fluoropyridin-3-yl)-3-methylbenzyl)oxy)imino)ethyl)benzyl)pyrrolidine-3-carboxylic acid (Compound 53)

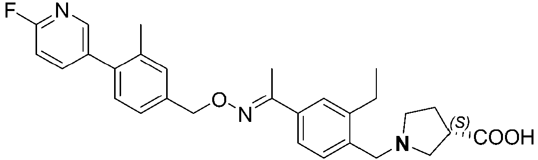

Pale yellow solid, yield 25.7%. ¹H NMR (500 MHz, CD₃OD) δ 8.12 - 8.08 (m, 1H), 7.88 (ddd, *J* = 8.5, 7.8, 2.6 Hz, 1H), 7.62 (d, *J* = 1.9 Hz, 1H), 7.55 (dd, *J* = 8.0, 1.9 Hz, 1H), 7.47 (d, *J* = 8.1 Hz, 1H), 7.35 (d, *J* = 1.7 Hz, 1H), 7.31 (dd, *J* = 7.8, 1.8 Hz, 1H), 7.19 (d, *J* = 7.8 Hz, 1H), 7.11 (ddd, *J* = 8.5, 2.6, 0.7 Hz, 1H), 5.22 (s, 2H), 4.39 - 4.31 (m, 2H), 3.48 (dd, *J* = 11.2, 5.7 Hz, 1H), 3.36 (s, 1H), 3.33 (d, *J* = 2.6 Hz, 1H), 3.29 - 3.21 (m, 1H), 3.08 (tt, *J* = 8.5, 5.8 Hz, 1H), 2.80 (q, *J* = 7.5 Hz, 2H), 2.34 - 2.27 (m, 1H), 2.25 (s, 3H), 2.24 (s, 3H), 2.21 (dd, *J* = 13.5, 6.4 Hz, 1H), 1.23 (t, *J* = 7.6 Hz, 3H). ¹³C NMR (126 MHz, CD₃OD) δ 179.54, 165.00, 163.11, 155.80, 148.23, 148.12, 145.08, 143.92, 143.86, 139.83, 139.04, 137.56, 136.94, 136.90, 136.84, 131.85, 131.43, 130.95, 128.01, 127.07, 125.32, 110.23, 109.93, 76.82, 57.91, 55.84, 54.97, 45.08, 29.14, 26.63, 20.46, 15.94, 12.80. MS (ESI) (m/z): [M+H]⁺ 490.25.

### Structure of (S, E)-1-(2-ethyl-4-(1-(((2,4'-difluoro-[1,1'-biphenyl]-4-yl)methoxy)imino)ethyl)benzyl)pyrrolidine-3-carboxylic acid (Compound 54)

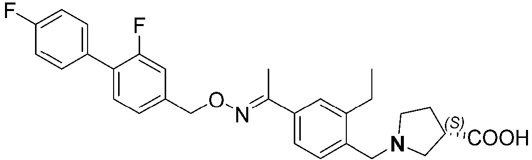

Pale yellow solid, yield 28.1%. ¹H NMR (600 MHz, CD₃OD) δ 7.62 (s, 1H), 7.56 (s, 2H), 7.52 (dd, *J* = 7.3, 5.3 Hz, 2H), 7.42 (t, *J* = 8.0 Hz, 1H), 7.27 (d, *J* = 7.9 Hz, 1H), 7.21 (d, *J* = 11.6 Hz, 1H), 7.15 (t, *J* = 8.8 Hz, 2H), 5.24 (s, 2H), 4.46 - 4.38 (m, 2H), 3.54 (dd, *J* = 11.3, 6.2 Hz, 1H), 3.44 - 3.37 (m, 2H), 3.36 (s, 1H), 3.16 - 3.10 (m, 1H), 2.82 (q, *J* = 7.5 Hz, 2H), 2.34 (dd, *J* = 14.3, 7.3 Hz, 1H), 2.28 (s, 3H), 2.27 - 2.23 (m, 1H), 1.23 (t, *J* = 7.5 Hz, 3H).¹³C NMR (151 MHz, CD₃OD) δ 179.36, 163.26, 161.63, 160.25, 158.62, 154.74, 143.80, 140.37, 137.67, 131.67, 130.67, 130.55, 130.53, 130.49, 130.47, 130.32, 130.29, 130.06 127.05, 126.67, 123.98, 123.75, 123.72, 115.11, 114.95, 114.81, 74.67, 56.19, 54.41, 53.74, 43.59, 27.72, 25.37, 14.56, 11.43. MS (ESI) (m/z): [M+H]⁺ 493.25.

### Structure of (S, E)-1-(2-ethyl-4-(1-((4-(1-methyl-1H-pyrazol-4-yl)benzyl)oxy)imino)ethyl)benzyl)pyrrolidine-3-carboxylic acid (Compound 55)

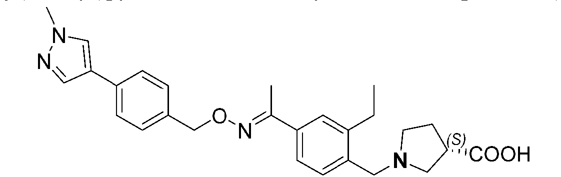

Pale yellow solid, yield 34.3%. ¹H NMR (500 MHz, CD₃OD) δ 7.94 - 7.88 (m, 1H), 7.79 (dd, *J* = 2.7, 1.2 Hz, 1H), 7.62 (q, *J* = 1.8 Hz, 1H), 7.56 (dt, *J* = 8.1, 2.2 Hz, 1H), 7.52 (dt, *J* = 8.3, 2.0 Hz, 2H), 7.45 (d, *J* = 8.1 Hz, 1H), 7.39 (dd, *J* = 8.2, 1.9 Hz, 2H), 5.20 (s, 2H), 4.43 - 4.33 (m, 2H), 3.92 - 3.87 (m, 3H), 3.52 (dd, *J* = 11.3, 5.7 Hz, 1H), 3.37 (dt, *J* = 10.6, 7.1 Hz, 2H), 3.28 (d, *J* = 9.4 Hz, 1H), 3.14 - 3.05 (m, 1H), 2.79 (dtt, *J* = 7.6, 3.9, 2.0 Hz, 2H), 2.31 (dt, *J* = 15.7, 7.4 Hz, 1H), 2.26 - 2.24 (m, 3H), 2.22 (dd, *J* = 13.7, 6.9 Hz, 1H), 1.25 - 1.22 (m, 3H). ¹³C NMR (126 MHz, CD₃OD) δ 177.66, 154.30, 143.73, 137.91, 136.13, 135.97, 132.02, 130.48, 129.94, 128.49, 127.75, 126.69, 124.90, 123.99, 122.83, 75.65, 56.45, 54.38, 53.56, 43.44, 37.53, 27.67, 25.22, 14.54, 11.39. MS (ESI) (m/z): [M+H]⁺ 461.30.

### Structure of (S, E)-1-(2-ethyl-4-(1-((4-(thiophen-3-yl)benzyl)oxy)imino)ethyl)benzyl)pyrrolidine-3-carboxylic acid (Compound 56)

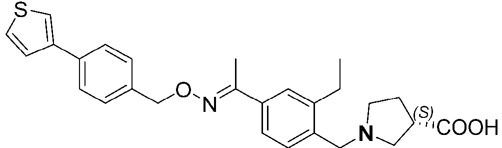

Pale yellow solid, yield 19.7%. ¹H NMR (500 MHz, CD₃OD) δ 7.64 (dd, *J* = 8.5, 2.0 Hz, 3H), 7.61 (dd, *J* = 2.8, 1.5 Hz, 1H), 7.58 (dd, *J* = 8.1, 1.9 Hz, 1H), 7.48 - 7.40 (m, 5H), 5.23 (s, 2H), 4.40 (d, *J* = 2.5 Hz, 2H), 3.53 (dd, *J* = 11.3, 5.8 Hz, 1H), 3.37 (dd, *J* = 12.5, 5.6 Hz, 2H), 3.27 (d, *J* = 10.5 Hz, 1H), 3.10 (ddd, *J* = 11.8, 8.6, 5.7 Hz, 1H), 2.81 (q, *J* = 7.5 Hz, 2H), 2.32 (dq, *J* = 15.8, 7.9 Hz, 1H), 2.27 (s, 3H), 2.22 (dd, *J* = 13.4, 6.7 Hz, 1H), 1.25 (t, *J* = 7.5 Hz, 3H). ¹³C NMR (126 MHz, CD₃OD) δ 154.35, 143.75, 141.83, 137.95, 136.87, 135.39, 130.47, 128.38, 126.72, 125.90, 125.85, 125.71, 124.02, 119.91, 75.59, 56.53, 54.43, 53.55, 43.44, 27.68, 25.21, 14.53, 11.39. MS (ESI) (m/z): [M+H]⁺ 463.20

### Structure of (S, E)-1-(2-ethyl-4-(1-((2-fluoro-4-(thiophen-3-yl)benzyl)oxy)imino)ethyl)benzyl)pyrrolidine-3-carboxylic acid (Compound 57)

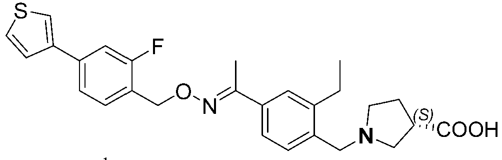

Pale yellow solid, yield 27.7%. ¹H NMR (500 MHz, CD₃OD) δ 7.67 (dd, *J* = 2.9, 1.4 Hz, 1H), 7.63 (d, *J* = 2.0 Hz, 1H), 7.56 (dd, *J* = 8.1, 1.9 Hz, 1H), 7.51 - 7.45 (m, 4H), 7.44 (dd, *J* = 5.1, 1.4 Hz, 1H), 7.40 (dd, *J* = 11.4, 1.6 Hz, 1H), 5.28 (d, *J* = 1.1 Hz, 2H), 4.43 - 4.34 (m, 2H), 3.53 (dd, *J* = 11.3, 5.6 Hz, 1H), 3.43 - 3.34 (m, 2H), 3.28 (dt, *J* = 11.0, 7.4 Hz, 1H), 3.15 - 3.05 (m, 1H), 2.80 (q, *J* = 7.5 Hz, 2H), 2.37 - 2.29 (m, 1H), 2.28 - 2.19 (m, 4H), 1.24 (t, *J* = 7.5 Hz, 3H). ¹³C NMR (126 MHz, CD₃OD) δ 177.72, 162.31, 160.35, 154.57, 143.71, 140.56, 137.91, 137.84, 137.72, 131.06, 130.50, 130.02, 126.66, 126.26, 125.60, 123.97, 123.49, 123.37, 121.53, 121.51, 121.01, 112.54, 112.35, 69.22, 69.19, 56.44, 54.29, 53.52, 43.54, 27.69, 25.22, 14.51, 11.30. MS (ESI) (m/z): [M+H]⁺ 481.20.

### Structure of (S, E)-1-(2-ethyl-4-(1-((2-fluoro-4-(1-methyl-1H-pyrazol-4-yl)benzyl)oxy)imino)ethyl)benzyl)pyrrolidine-3-carboxylic acid (Compound 58)

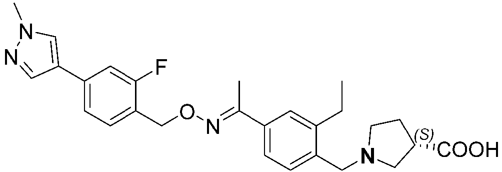

Pale yellow solid, yield 31.6%. ¹H NMR (500 MHz, CD₃OD) δ 8.00 - 7.93 (m, 1H), 7.82 (d, *J* = 0.8 Hz, 1H), 7.63 (d, *J* = 2.0 Hz, 1H), 7.56 (dd, *J* = 8.1, 1.9 Hz, 1H), 7.50 - 7.41 (m, 2H), 7.34 (dd, *J* = 7.8, 1.7 Hz, 1H), 7.30 (dd, *J* = 11.3, 1.7 Hz, 1H), 5.26 (s, 2H), 4.44 - 4.34 (m, 2H), 3.91 (s, 3H), 3.53 (dd, *J* = 11.2, 5.7 Hz, 1H), 3.43 - 3.34 (m, 2H), 3.28 (dt, *J* = 11.0, 7.4 Hz, 1H), 3.10 (tt, *J* = 8.7, 5.7 Hz, 1H), 2.81 (q, *J* = 7.6 Hz, 2H), 2.37 - 2.29 (m, 1H), 2.25 (s, 4H), 1.25 (t, *J* = 7.6 Hz, 3H). ¹³C NMR (126 MHz, CD₃OD) δ 177.73, 162.41, 160.45, 154.51, 143.71, 137.73, 136.16, 134.81, 134.74, 131.22, 131.18, 130.49, 130.06, 128.17, 126.65, 123.95, 122.64, 122.52, 121.79, 121.77, 120.55, 120.52, 111.53, 111.35, 69.24, 69.22, 56.48, 54.31, 53.53, 43.56, 37.61, 27.69, 25.21, 14.50, 11.28 MS (ESI) (m/z): [M+H]⁺ 479.30.

### Structure of (S, E)-1-(2-ethyl-4-(1-(((2'-fluoro-2-methyl-[1,1'-biphenyl]-4-yl)methoxy)imino)ethyl)benzyl)pyrrolidine-3-carboxylic acid (Compound 59)

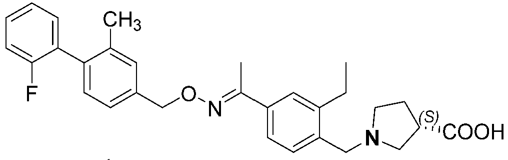

Pale yellow solid, yield 33.5%. ¹H NMR (500 MHz, CD₃OD) δ 7.63 (s, 1H), 7.56 (d, *J* = 8.0 Hz, 1H), 7.49 (d, *J* = 8.1 Hz, 1H), 7.38 (td, *J* = 8.9, 8.2, 3.6 Hz, 1H), 7.32 (s, 1H), 7.28 (d, *J* = 7.7 Hz, 1H), 7.22 (d, *J* = 4.6 Hz, 2H), 7.15 (t, *J* = 8.0 Hz, 2H), 5.22 (s, 2H), 4.38 - 4.31 (m, 2H), 3.48 (dd, *J* = 11.3, 6.1 Hz, 1H), 3.35 (s, 2H), 3.29 - 3.24 (m, 1H), 3.09 (ddd, *J* = 14.7, 8.6, 6.1 Hz, 1H), 2.81 (q, *J* = 7.5 Hz, 2H), 2.31 (dd, *J* = 14.5, 7.0 Hz, 1H), 2.27 (s, 3H), 2.23 (dd, *J* = 13.4, 6.8 Hz, 1H), 2.16 (s, 3H), 1.24 (t, *J* = 7.5 Hz, 3H). ¹³C NMR (126 MHz, CD₃OD) δ 179.07, 160.43, 158.81, 154.38, 143.68, 137.85, 137.75, 136.26, 135.28, 131.31, 131.28, 130.47, 129.67, 129.35, 129.09, 129.04, 128.96, 126.66, 125.19, 123.95, 123.92, 123.89, 115.08, 114.93, 75.63, 56.46, 54.60, 53.70, 43.65, 27.75, 25.28, 18.68, 14.54, 11.44. MS (ESI) (m/z): [M+H]⁺ 489.25.

### Structure of (S, E)-1-(2-ethyl-4-(1-(((4-cyclohexyl-3-methylbenzyl)oxy)imino)ethyl)benzyl)pyrrolidine-3-carboxylic acid (Compound 60)

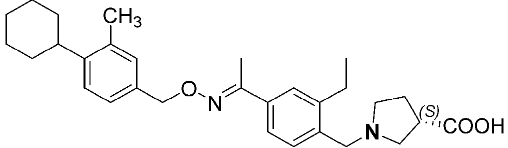

Pale yellow solid, yield 34.5%. ¹H NMR (500 MHz, CD₃OD) δ 7.62 (d, *J* = 2.0 Hz, 1H), 7.55 (dd, *J* = 8.1, 1.9 Hz, 1H), 7.48 (d, *J* = 8.1 Hz, 1H), 7.20 - 7.13 (m, 3H), 5.13 (s, 2H), 4.42 - 4.30 (m, 2H), 3.50 (dd, *J* = 11.2, 5.8 Hz, 1H), 3.36 (dd, *J* = 11.7, 7.9 Hz, 2H), 3.27 (dt, *J* = 10.9, 7.3 Hz, 1H), 3.09 (ddd, *J* = 14.6, 8.9, 5.8 Hz, 1H), 2.81 (q, *J* = 7.5 Hz, 2H), 2.74 (ddd, *J* = 11.5, 9.7, 3.2 Hz, 1H), 2.32 (s, 4H), 2.23 (s, 4H), 1.91 - 1.82 (m, 2H), 1.81 - 1.73 (m, 3H), 1.52 - 1.39 (m, 4H), 1.33 - 1.28 (m, 1H), 1.25 (t, *J* = 7.6 Hz, 3H). ¹³C NMR (126 MHz, CD₃OD) δ 178.14, 154.05, 145.25, 143.65, 137.82, 134.85, 134.63, 130.45, 130.24, 129.88, 126.62, 125.87, 124.97, 123.93, 75.90, 56.52, 54.43, 53.57, 43.69, 39.82, 33.49, 27.76, 26.83, 26.02, 25.24, 18.11, 14.56, 11.42. MS (ESI) (m/z): [M+H]⁺ 477.30.

### Structure of (S, E)-1-(2-ethyl-4-(1-((2-methyl-2', 3', 4' ,5'-tetrahydro-[1,1'-biphenyl]-4-yl)methoxy)imino)ethyl)benzyl)pyrrolidine-3-carboxylic acid (Compound 61)

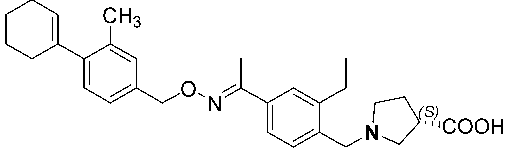

Pale yellow solid, yield 31.2%. ¹H NMR (500 MHz, CD₃OD) δ 7.60 (d, *J* = 2.0 Hz, 1H), 7.52 (dd, *J* = 8.1, 1.9 Hz, 1H), 7.46 (d, *J* = 8.1 Hz, 1H), 7.15 (d, *J* = 1.7 Hz, 1H), 7.11 (dd, *J* = 7.8, 1.8 Hz, 1H), 6.97 (d, *J* = 7.7 Hz, 1H), 5.47 (td, *J* = 3.8, 2.0 Hz, 1H), 5.11 (s, 2H), 4.39 - 4.28 (m, 2H), 3.47 (dd, *J* = 11.1, 5.7 Hz, 1H), 3.37 - 3.31 (m, 2H), 3.24 (dt, *J* = 10.9, 7.3 Hz, 1H), 3.06 (tt, *J* = 8.9, 5.8 Hz, 1H), 2.78 (q, *J* = 7.5 Hz, 2H), 2.33 - 2.21 (m, 5H), 2.20 (s, 3H), 2.16 - 2.10 (m, 4H), 1.73 (tdd, *J* = 8.2, 4.9, 2.6 Hz, 2H), 1.67 (qq, *J* = 5.8, 2.6 Hz, 2H), 1.22 (t, *J* = 7.5 Hz, 3H). ¹³C NMR (126 MHz, CD₃OD) δ 178.11, 154.10, 144.02, 143.65, 138.75, 137.74, 135.99, 134.52, 130.49, 130.29, 129.59, 127.88, 126.60, 125.31, 125.22, 123.93, 75.85, 56.47, 54.35, 53.53, 43.72, 29.82, 27.78, 25.26, 24.97, 22.81, 21.92, 18.61, 14.59, 11.45. MS (ESI) (m/z): [M+H]⁺ 475.30

### Structure of (S, E)-1-(2-ethyl-4-(1-(((4-cyclopentyl-3-methylbenzyl)oxy)imino)ethyl)benzyl)pyrrolidine-3-carboxylic acid (Compound 62)

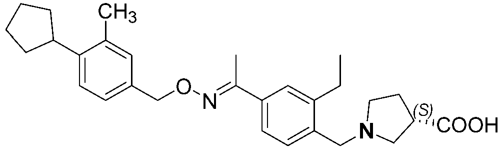

Pale yellow solid, yield 33.5%. ¹H NMR (500 MHz, CD₃OD) δ 7.61 (d, *J* = 1.9 Hz, 1H), 7.54 (dd, *J* = 8.0, 1.9 Hz, 1H), 7.47 (d, *J* = 8.1 Hz, 1H), 7.19 (d, *J* = 7.8 Hz, 1H), 7.16 (d, *J* = 1.9 Hz, 1H), 7.13 (d, *J* = 1.8 Hz, 1H), 5.11 (s, 2H), 4.45 - 4.30 (m, 2H), 3.52 (dd, *J* = 11.3, 5.8 Hz, 1H), 3.41 - 3.32 (m, 2H), 3.28 (dd, *J* = 11.0, 7.4 Hz, 1H), 3.19 (tt, *J* = 9.2, 7.5 Hz, 1H), 3.09 (ddd, *J* = 14.6, 8.8, 5.8 Hz, 1H), 2.79 (q, *J* = 7.6 Hz, 2H), 2.31 (s, 4H), 2.21 (s, 4H), 1.99 (ddddd, *J* = 12.2, 10.9, 6.7, 2.8, 1.4 Hz, 2H), 1.85 - 1.76 (m, 2H), 1.72 - 1.64 (m, 2H), 1.58 - 1.47 (m, 2H), 1.23 (t, *J* = 7.5 Hz, 3H). ¹³C NMR (126 MHz, CD₃OD) δ 178.02, 154.03, 143.86, 143.70, 137.92, 135.45, 134.86, 130.52, 129.89, 129.76, 126.65, 125.83, 124.80, 123.98, 75.90, 56.35, 54.35, 53.58, 43.56, 41.31, 33.26, 27.72, 25.27, 25.10, 18.61, 14.59, 11.43. MS (ESI) (m/z): [M+H]⁺ 463.30

### Structure of (S, E)-1-(2-ethyl-4-(1-((4-(cyclopent-1-en-1-yl)-3-methylbenzyl)oxy)imino)ethyl)benzyl)pyrrolidine-3-carboxylic acid (Compound 63)

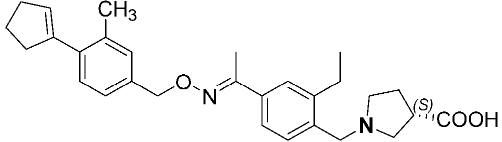

Pale yellow solid, yield 30.4%. ¹H NMR (500 MHz, CD₃OD) δ 7.60 (d, *J* = 1.9 Hz, 1H), 7.53 (dd, *J* = 8.1, 1.9 Hz, 1H), 7.46 (d, *J* = 8.1 Hz, 1H), 7.18 (d, *J* = 1.7 Hz, 1H), 7.16 - 7.10 (m, 2H), 5.73 (p, *J* = 2.2 Hz, 1H), 5.13 (s, 2H), 4.39 - 4.26 (m, 2H), 3.48 (dd, *J* = 11.2, 5.7 Hz, 1H), 3.37 - 3.32 (m, 1H), 3.29 (s, 1H), 3.26 - 3.20 (m, 1H), 3.06 (ddd, *J* = 14.6, 8.8, 5.8 Hz, 1H), 2.78 (q, *J* = 7.5 Hz, 2H), 2.67 - 2.58 (m, 2H), 2.51 (tq, *J* = 7.2, 2.4 Hz, 2H), 2.31 (s, 3H), 2.30 - 2.19 (m, 5H), 1.97 (p, *J* = 7.5 Hz, 2H), 1.22 (t, *J* = 7.5 Hz, 3H). ¹³C NMR (126 MHz, CD₃OD) δ 178.11, 154.18, 143.66, 143.14, 137.75, 137.50, 136.20, 135.06, 130.47, 130.29, 130.01, 128.84, 127.61, 126.61, 125.17, 123.93, 75.74, 56.50, 54.38, 53.54, 43.71, 36.25, 33.04, 27.76, 25.24, 23.30, 20.04, 14.57, 11.43. MS (ESI) (m/z): [M+H]⁺ 461.30.

### Structure of (S, E)-1-(2-ethyl-4-(1-((2,2'-difluoro-[1,1'-biphenyl]-4-yl)methoxy)imino)ethyl)benzyl)pyrrolidine-3-carboxylic acid (Compound 64)

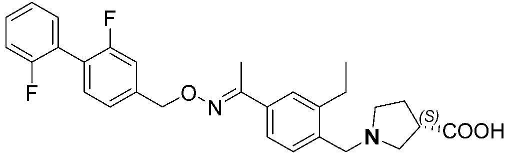

Pale yellow solid, yield 28.9 %. ¹H NMR (500 MHz, CD₃OD) δ 7.63 (d, *J* = 1.9 Hz, 1H), 7.57 (dd, *J* = 8.1, 2.0 Hz, 1H), 7.47 (d, *J* = 8.1 Hz, 1H), 7.44 - 7.40 (m, 1H), 7.39 (d, *J* = 2.5 Hz, 1H), 7.38 (d, *J* = 2.6 Hz, 1H), 7.31 (dd, *J* = 7.9, 1.6 Hz, 1H), 7.28 - 7.25 (m, 1H), 7.24 (d, *J* = 1.7 Hz, 1H), 7.19 (ddd, *J* = 9.6, 8.2, 1.2 Hz, 1H), 5.28 (s, 2H), 4.31 (d, *J* = 3.9 Hz, 2H), 3.44 (dd, *J* = 10.6, 5.7 Hz, 1H), 3.29 (d, *J* = 9.8 Hz, 2H), 3.23 (s, 1H), 3.07 (ddd, *J* = 14.5, 8.6, 6.0 Hz, 1H), 2.81 (q, *J* = 7.5 Hz, 2H), 2.31 (d, *J* = 0.8 Hz, 3H), 2.29 - 2.25 (m, 1H), 2.23 (dd, *J* = 13.6, 7.0 Hz, 1H), 1.27 - 1.23 (m, 3H). ¹³C NMR (126 MHz, CD₃OD) δ 160.79, 160.67, 158.83, 154.90, 143.63, 141.17, 141.11, 137.38, 131.32, 131.22, 130.37, 129.77, 129.71, 126.62, 123.99, 123.96, 123.90, 123.32, 123.30, 123.15, 122.70, 122.57, 115.27, 115.10, 114.64, 114.46, 74.66, 56.68, 54.68, 53.65, 43.80, 27.79, 25.22, 14.50, 11.45. MS (ESI) (m/z): [M+H]⁺ 493.20.

### Structure of (S, E)-1-(2-ethyl-4-(1-((2-fluoro-4-(6-fluoropyridin-3-yl)benzyl)oxy)imino)ethyl)benzyl)pyrrolidine-3-carboxylic acid (Compound 65)

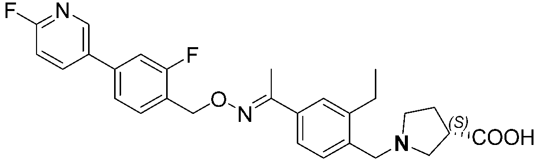

Pale yellow solid, yield 20.3%. ¹H NMR (500 MHz, CD₃OD) δ 8.49 - 8.43 (m, 1H), 8.23 - 8.16 (m, 1H), 7.64 (q, *J* = 1.8 Hz, 1H), 7.61 (dt, *J* = 7.8, 1.8 Hz, 1H), 7.58 (dq, *J* = 9.9, 1.9 Hz, 1H), 7.49 (s, 1H), 7.47 (d, *J* = 4.2 Hz, 1H), 7.44 (ddd, *J* = 10.5, 3.9, 2.0 Hz, 1H), 7.16 (dq, *J* = 8.5, 2.4 Hz, 1H), 5.34 (d, *J* = 2.2 Hz, 2H), 4.43 - 4.34 (m, 2H), 3.52 (dd, *J* = 11.2, 5.8 Hz, 1H), 3.41 - 3.37 (m, 1H), 3.36 - 3.34 (m, 1H), 3.28 (dd, *J* = 10.9, 7.3 Hz, 1H), 3.10 (tt, *J* = 8.9, 5.8 Hz, 1H), 2.85 - 2.78 (m, 2H), 2.37 - 2.29 (m, 1H), 2.27 (q, *J* = 2.6, 2.2 Hz, 3H), 2.23 (dd, *J* = 13.5, 6.7 Hz, 1H), 1.25 (tt, *J* = 7.6, 1.9 Hz, 3H). ¹³C NMR (126 MHz, CD₃OD) δ 162.39, 154.77, 145.28, 145.16, 143.72, 140.36, 140.29, 138.32, 137.59, 131.37, 131.33, 130.46, 130.34, 126.66, 125.13, 125.01, 123.96, 122.37, 113.51, 113.32, 109.52, 109.22, 69.01, 56.52, 54.43, 53.58, 43.59, 27.71, 25.21, 14.50, 11.28. MS (ESI) (m/z): [M+H]⁺ 494.20

### Structure of (S, E)-1-(2-ethyl-4-(1-(((6'-fluoro-[2,3'-bipyridin]-5-yl)methoxy)imino)ethyl)benzyl)pyrrolidine-3-carboxylic acid (Compound 66)

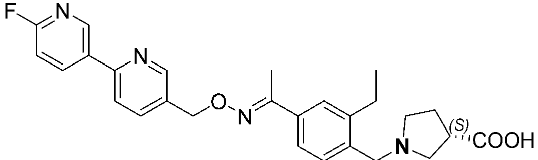

Pale yellow solid, yield 17.2%. ¹H NMR (500 MHz, CD₃OD) δ 8.84 - 8.80 (m, 1H), 8.72 (dd, *J* = 2.3, 0.9 Hz, 1H), 8.54 (ddd, *J* = 8.6, 7.6, 2.6 Hz, 1H), 7.98 (dd, *J* = 8.1, 2.2 Hz, 1H), 7.92 (dd, *J* = 8.1, 0.9 Hz, 1H), 7.64 (d, *J* = 2.0 Hz, 1H), 7.58 (dd, *J* = 8.1, 1.9 Hz, 1H), 7.49 (d, *J* = 8.1 Hz, 1H), 7.19 (ddd, *J* = 8.6, 2.6, 0.6 Hz, 1H), 5.33 (s, 2H), 4.45 - 4.35 (m, 2H), 3.53 (dd, *J* = 11.2, 5.7 Hz, 1H), 3.42 - 3.37 (m, 1H), 3.37 - 3.34 (m, 1H), 3.29 (dd, *J* = 11.0, 7.3 Hz, 1H), 3.11 (ddt, *J* = 11.2, 8.4, 5.9 Hz, 1H), 2.82 (q, *J* = 7.5 Hz, 2H), 2.34 (dt, *J* = 15.1, 7.7 Hz, 1H), 2.29 (s, 3H), 2.28 - 2.21 (m, 1H), 1.25 (t, *J* = 7.5 Hz, 3H). ¹³C NMR (126 MHz, CD₃OD) δ 177.73, 164.91, 162.99, 155.08, 152.89, 149.35, 145.86, 145.74, 143.78, 140.31, 140.25, 137.56, 137.50, 133.65, 132.98, 132.94, 130.50, 130.31, 126.69, 123.99, 120.45, 109.42, 109.12, 72.79, 56.49, 54.38, 53.57, 43.52, 27.69, 25.22, 14.53, 11.38. MS (ESI) (m/z): [M+H]⁺ 477.20.

### Structure of (S, E)-1-(2-ethyl-4-(1-(((4-(5-fluoropyrazin-2-yl)-3-methylbenzyl)oxy)imino)ethyl)benzyl)pyrrolidine-3-carboxylic acid (Compound 67)

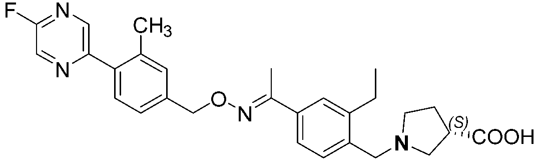

Pale yellow solid, yield 16.3%. ¹H NMR (500 MHz, CD₃OD) δ 8.59 (dd, *J* = 8.3, 1.4 Hz, 1H), 8.38 (d, *J* = 1.6 Hz, 1H), 7.66 (d, *J* = 1.9 Hz, 1H), 7.60 (dd, *J* = 8.1, 1.9 Hz, 1H), 7.51 (d, *J* = 8.1 Hz, 1H), 7.43 (d, *J* = 7.7 Hz, 1H), 7.40 (s, 1H), 7.38 - 7.35 (m, 1H), 5.27 (s, 2H), 4.49 - 4.41 (m, 2H), 3.58 (dd, *J* = 11.4, 6.0 Hz, 1H), 3.45 (d, *J* = 8.2 Hz, 1H), 3.41 (d, *J* = 7.7 Hz, 1H), 3.39 - 3.34 (m, 1H), 3.20 - 3.12 (m, 1H), 2.83 (q, *J* = 7.5 Hz, 2H), 2.38 (s, 3H), 2.35 (dd, *J* = 9.1, 6.9 Hz, 1H), 2.30 (s, 3H), 2.28 - 2.24 (m, 1H), 1.29 - 1.25 (m, 3H). ¹³C NMR (126 MHz, CD₃OD) δ 179.74, 154.46, 152.91, 143.82, 141.30, 141.23, 139.46, 137.93, 136.38, 135.09, 131.95, 131.65, 130.58, 130.32, 129.70, 129.58, 126.73, 125.50, 124.04, 75.35, 56.22, 54.40, 53.64, 43.26, 27.61, 25.28, 19.04, 14.57, 11.40. MS (ESI) (m/z): [M+H]⁺ 491.25.

### Structure of (S, E)-1-(2-ethyl-4-(1-(((4-(6-fluoropyridin-3-yl)benzyl)oxy)imino)ethyl)benzyl)pyrrolidine-3-carboxylic acid (Compound 68)

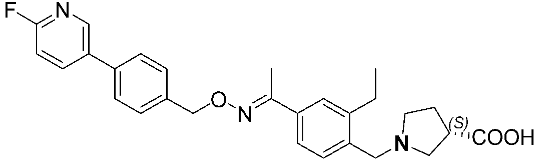

Pale yellow solid, yield 27.6%. ¹H NMR (500 MHz, CD₃OD) δ 8.41 (dt, *J* = 2.7, 0.8 Hz, 1H), 8.16 (ddd, *J* = 8.5, 7.6, 2.6 Hz, 1H), 7.63 (d, *J* = 1.9 Hz, 1H), 7.62 (d, *J* = 1.8 Hz, 1H), 7.61 (d, *J* = 2.0 Hz, 1H), 7.57 (dd, *J* = 8.1, 1.9 Hz, 1H), 7.54 (d, *J* = 1.8 Hz, 1H), 7.52 (d, *J* = 1.8 Hz, 1H), 7.48 (d, *J* = 8.1 Hz, 1H), 7.13 (ddd, *J* = 8.5, 2.6, 0.7 Hz, 1H), 5.28 (s, 2H), 4.43 - 4.34 (m, 2H), 3.52 (dd, *J* = 11.2, 5.7 Hz, 1H), 3.37 (ddd, *J* = 11.0, 8.0, 3.1 Hz, 2H), 3.29 (dt, *J* = 11.0, 7.3 Hz, 1H), 3.10 (tt, *J* = 8.5, 5.7 Hz, 1H), 2.81 (q, *J* = 7.5 Hz, 2H), 2.38 - 2.30 (m, 1H), 2.28 (s, 3H), 2.24 (dt, *J* = 13.6, 6.8 Hz, 1H), 1.24 (t, *J* = 7.5 Hz, 3H). ¹³C NMR (126 MHz, CD₃OD) δ 177.94, 163.99, 162.09, 154.52, 145.03, 144.92, 143.73, 140.27, 140.21, 138.46, 137.72, 135.77, 134.78, 134.74, 130.48, 130.19, 128.57, 126.66, 126.64, 123.97, 109.39, 109.09, 75.30, 56.45, 54.36, 53.56, 43.56, 27.71, 25.24, 14.55, 11.42.MS (ESI) (m/z): [M+H]⁺ 476.20.

### Structure of (S, E)-1-(2-ethyl-4-(1-(((3-fluoro-4-(6-fluoropyridin-3-yl)benzyl)oxy)imino)ethyl)benzyl)pyrrolidine-3-carboxylic acid (Compound 69)

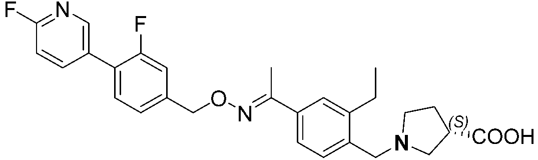

Pale yellow solid, yield 29.2%. ¹H NMR (500 MHz, CD₃OD) δ 8.39 (s, 1H), 8.14 (d, *J* = 8.5 Hz, 1H), 7.64 (d, *J* = 4.0 Hz, 1H), 7.60 - 7.55 (m, 1H), 7.53 (dd, *J* = 8.0, 2.3 Hz, 1H), 7.47 (dd, *J* = 8.2, 2.1 Hz, 1H), 7.38 - 7.33 (m, 1H), 7.32 (d, *J* = 11.2 Hz, 1H), 7.19 (dd, *J* = 8.9, 2.9 Hz, 1H), 5.29 (d, *J* = 4.3 Hz, 2H), 4.41 - 4.30 (m, 2H), 3.48 (dd, *J* = 10.8, 5.4 Hz, 1H), 3.37 (s, 1H), 3.26 (q, *J* = 9.3, 8.4 Hz, 2H), 3.09 (tt, *J* = 8.5, 5.9 Hz, 1H), 2.83 (q, *J* = 5.8, 4.4 Hz, 2H), 2.34 - 2.22 (m, 5H), 1.27 - 1.22 (m, 3H). ¹³C NMR (126 MHz, CD₃OD) δ 178.47, 163.95, 162.05, 160.52, 158.55, 154.93, 146.85, 146.82, 146.74, 146.70, 143.71, 142.17, 142.11, 141.75, 141.69, 137.46, 130.72, 130.44, 130.22, 130.19, 129.71, 126.64, 123.99, 123.97, 123.94, 123.35, 123.24, 115.19, 115.01, 109.24, 108.94, 74.50, 56.56, 54.53, 53.63, 43.69, 27.74, 25.23, 14.52, 11.43.. MS (ESI) (m/z): [M+H]⁺ 494.20

### Structure of (S, E)-1-(2-ethyl-4-(1-(((4-(pyrimidin-5-yl)benzyl)oxy)imino)ethyl)benzyl)pyrrolidine-3-carboxylic acid (Compound 70)

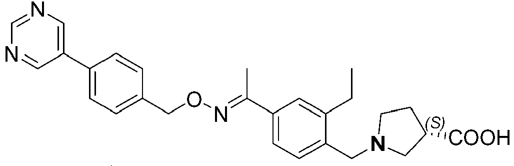

Pale yellow solid, yield 19.8%. ¹H NMR (500 MHz, CD₃OD) δ 9.17 - 9.12 (m, 1H), 9.11 - 9.05 (m, 2H), 7.75 (dd, *J* = 9.5, 3.5 Hz, 2H), 7.65 - 7.55 (m, 4H), 7.47 (d, *J* = 8.1 Hz, 1H), 5.32 (d, *J* = 4.0 Hz, 2H), 4.33 (s, 2H), 3.43 (dd, *J* = 10.8, 6.1 Hz, 1H), 3.31 - 3.20 (m, 3H), 3.08 (q, *J* = 7.5, 7.0 Hz, 1H), 2.88 - 2.74 (m, 2H), 2.31 - 2.29 (m, 3H), 2.28 - 2.19 (m, 2H), 1.25 (ddd, *J* = 9.6, 4.3, 2.7 Hz, 3H). ¹³C NMR (126 MHz, CD₃OD) δ 178.39, 156.47, 154.70, 154.64, 143.64, 139.57, 137.42, 134.33, 133.16, 130.98, 130.38, 128.74, 126.71, 126.58, 123.88, 75.16, 56.68, 54.58, 53.59, 43.81, 27.79, 25.22, 14.53, 11.43. MS (ESI) (m/z): [M+H]⁺ 459.20

### Structure of (S, E)-1-(2-ethyl-4-(1-(((3-fluoro-4-(pyrimidin-5-yl)benzyl)oxy)imino)ethyl)benzyl)pyrrolidine-3-carboxylic acid (Compound 71)

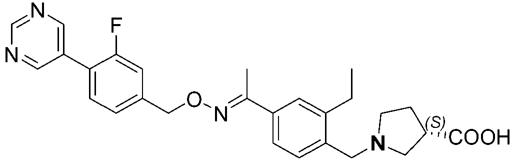

Pale yellow solid, yield 30.0%. ¹H NMR (600 MHz, CD₃OD) δ 9.39 - 9.24 (m, 1H), 9.14 (d, *J* = 8.3 Hz, 2H), 7.84 - 7.46 (m, 6H), 5.45 (d, *J* = 7.7 Hz, 2H), 4.58 - 4.42 (m, 2H), 3.67 - 3.57 (m, 1H), 3.53 (s, 1H), 3.45 (s, 1H), 3.39 (d, *J* = 9.6 Hz, 1H), 3.28 - 3.19 (m, 1H), 2.96 (q, *J* = 7.5 Hz, 2H), 2.49 - 2.44 (m, 2H), 2.42 (d, *J* = 2.3 Hz, 1H), 2.37 (q, *J* = 6.8 Hz, 1H), 1.40 - 1.37 (m, 3H). ¹³C NMR (126 MHz, CD₃OD) δ 178.30, 160.69, 158.72, 156.78, 156.20, 156.16, 155.06, 143.69, 142.91, 142.85, 137.34, 130.42, 130.09, 130.07, 129.97, 129.95, 126.61, 124.22, 124.20, 123.92, 120.82, 120.71, 115.26, 115.07, 74.40, 56.66, 54.53, 53.59, 43.77, 27.77, 25.22, 14.52, 11.43. MS (ESI) (m/z): [M+H]⁺ 477.20

### Structure of (S, E)-1-(2-ethyl-4-(1-(((3-methyl-4-(thiophen-2-yl)benzyl)oxy)imino)ethyl)benzyl)pyrrolidine-3-carboxylic acid (Compound 72)

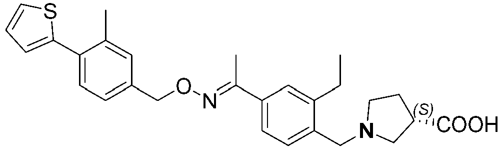

Pale yellow solid, yield 18.3%. ¹H NMR (500 MHz, CD₃OD) δ 7.62 (s, 1H), 7.54 (d, *J* = 7.8 Hz, 1H), 7.47 (d, *J* = 8.0 Hz, 1H), 7.39 (d, *J* = 4.6 Hz, 1H), 7.38 - 7.28 (m, 2H), 7.23 (d, *J* = 7.8 Hz, 1H), 7.14 - 7.01 (m, 2H), 5.19 (s, 2H), 4.34 (d, *J* = 6.8 Hz, 2H), 3.48 (dd, *J* = 11.2, 5.8 Hz, 1H), 3.35 (s, 1H), 3.31 (s, 1H), 3.25 (q, *J* = 10.5, 8.7 Hz, 1H), 3.13 - 3.02 (m, 1H), 2.79 (q, *J* = 7.3 Hz, 2H), 2.37 (s, 3H), 2.24 (q, *J* = 6.5 Hz, 5H), 1.23 (t, *J* = 7.5 Hz, 3H). ¹³C NMR (126 MHz, CD₃OD) δ 178.28, 154.41, 143.66, 142.51, 137.70, 135.65, 133.55, 130.47, 130.21, 129.96, 126.81, 126.62, 126.22, 125.44, 124.87, 123.95, 75.48, 56.50, 54.42, 53.56, 43.76, 27.79, 25.25, 20.01, 14.58, 11.49. MS (ESI) (m/z): [M+H]⁺ 477.20

### Structure of (S, E)-1-(2-ethyl-4-(1-((4-(thiophen-2-yl)benzyl)oxy)imino)ethyl)benzyl)pyrrolidine-3-carboxylic acid (Compound 73)

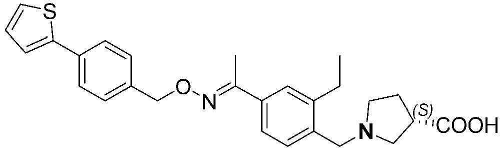

Pale yellow solid, yield 21.2%. ¹H NMR (500 MHz, CD₃OD) δ 7.64 - 7.59 (m, 3H), 7.56 (dd, *J* = 8.1, 2.0 Hz, 1H), 7.47 (d, *J* = 8.1 Hz, 1H), 7.43 - 7.39 (m, 2H), 7.36 (ddd, *J* = 8.2, 4.4, 1.2 Hz, 2H), 7.08 (dd, *J* = 5.1, 3.6 Hz, 1H), 5.22 (s, 2H), 4.40 - 4.30 (m, 2H), 3.49 (dd, *J* = 11.2, 5.8 Hz, 1H), 3.35 (d, *J* = 7.7 Hz, 1H), 3.32 (s, 1H), 3.26 (dt, *J* = 10.9, 7.3 Hz, 1H), 3.12 - 3.04 (m, 1H), 2.80 (q, *J* = 7.5 Hz, 2H), 2.34 - 2.18 (m, 5H), 1.24 (t, *J* = 7.5 Hz, 3H). ¹³C NMR (126 MHz, CD₃OD) δ 178.13, 154.46, 143.72, 143.69, 137.71, 137.42, 133.91, 130.48, 130.27, 128.47, 127.75, 126.64, 125.28, 124.49, 123.95, 122.92, 75.43, 56.49, 54.41, 53.56, 43.70, 27.76, 25.25, 14.55, 11.46. MS (ESI) (m/z): [M+H]⁺ 463.20

### Structure of (S, E)-1-(2-ethyl-4-(1-((4-(isothiazol-4-yl)benzyl)oxy)imino)ethyl)benzyl)pyrrolidine-3-carboxylic acid (Compound 74)

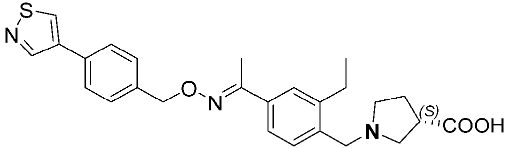

Pale yellow solid, yield 11.6%. ¹H NMR (500 MHz, CD₃OD) δ 9.07 (s, 1H), 8.86 (s, 1H), 7.72 - 7.68 (m, 2H), 7.63 (d, *J* = 1.9 Hz, 1H), 7.57 (dd, *J* = 8.1, 1.9 Hz, 1H), 7.52 (d, *J* = 8.1 Hz, 1H), 7.50 - 7.46 (m, 2H), 5.26 (s, 2H), 4.46 - 4.37 (m, 2H), 3.52 (dd, *J* = 11.3, 6.0 Hz, 1H), 3.44 - 3.39 (m, 1H), 3.39 (d, *J* = 4.0 Hz, 1H), 3.35 (d, *J* = 6.9 Hz, 1H), 3.12 (ddd, *J* = 14.4, 8.7, 5.9 Hz, 1H), 2.82 (q, *J* = 7.5 Hz, 2H), 2.37 - 2.22 (m, 5H), 1.24 (t, *J* = 7.5 Hz, 3H). ¹³C NMR (126 MHz, CD₃OD) δ 178.11, 155.89, 154.49, 143.75, 143.06, 139.51, 138.10, 137.76, 131.87, 130.56, 130.04, 128.57, 126.66, 126.46, 123.98, 75.38, 56.36, 54.37, 53.59, 43.65, 27.77, 25.29, 14.57, 11.44. MS (ESI) (m/z): [M+H]⁺ 464.20

### Structure of (S, E)-1-(2-ethyl-4-(1-((4-(isothiazol-5-yl)benzyl)oxy)imino)ethyl)benzyl)pyrrolidine-3-carboxylic acid (Compound 75)

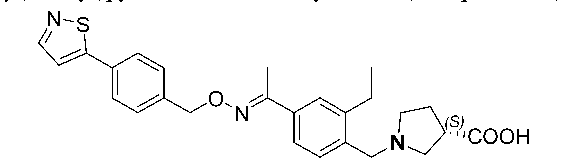

Pale yellow solid, yield 14.9%. ¹H NMR (500 MHz, CD₃OD) δ 8.48 (d, *J* = 1.8 Hz, 1H), 7.72 - 7.66 (m, 2H), 7.63 (d, *J* = 1.9 Hz, 1H), 7.60 (d, *J* = 1.8 Hz, 1H), 7.57 (dd, *J* = 8.0, 1.9 Hz, 1H), 7.52 (s, 2H), 7.51 (s, 1H), 5.27 (s, 2H), 4.46 - 4.36 (m, 2H), 3.52 (dd, *J* = 11.3, 5.9 Hz, 1H), 3.43 - 3.38 (m, 1H), 3.36 (dd, *J* = 12.2, 5.6 Hz, 2H), 3.12 (dq, *J* = 11.3, 4.4, 2.9 Hz, 1H), 2.82 (q, *J* = 7.5 Hz, 2H), 2.38 - 2.23 (m, 5H), 1.24 (t, *J* = 7.5 Hz, 3H). ¹³C NMR (126 MHz, CD₃OD) δ 177.97, 167.11, 158.52, 154.68, 143.76, 140.25, 137.69, 130.55, 130.11, 129.81, 128.63, 126.67, 126.35, 123.99, 119.97, 75.14, 56.38, 54.37, 53.58, 43.64, 27.77, 25.28, 14.56, 11.44. MS (ESI) (m/z): [M+H]⁺ 464.20

### Structure of (S, E)-1-(2-ethyl-4-(1-(((3-methyl-4-(pyrimidin-4-yl)benzyl)oxy)imino)ethyl)benzyl)pyrrolidine-3-carboxylic acid (Compound 76)

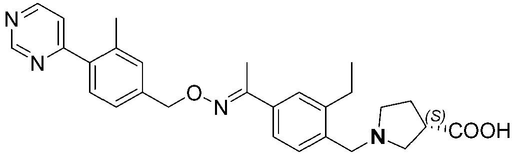

Pale yellow solid, yield 19.9%. ¹H NMR (500 MHz, CD₃OD) δ 9.21 (d, *J* = 1.4 Hz, 1H), 8.83 (d, *J* = 5.3 Hz, 1H), 7.65 (dt, *J* = 3.4, 1.5 Hz, 2H), 7.59 (dd, *J* = 8.1, 1.9 Hz, 1H), 7.49 (dd, *J* = 7.8, 6.3 Hz, 2H), 7.39 (d, *J* = 8.6 Hz, 2H), 5.28 (s, 2H), 4.49 - 4.39 (m, 2H), 3.56 (dd, *J* = 11.3, 5.6 Hz, 1H), 3.45 - 3.34 (m, 2H), 3.30 (t, *J* = 5.6 Hz, 1H), 3.13 (p, *J* = 7.2 Hz, 1H), 2.83 (q, *J* = 7.5 Hz, 2H), 2.43 (s, 3H), 2.41 - 2.32 (m, 1H), 2.30 (s, 3H), 2.29 - 2.23 (m, 1H), 1.26 (t, *J* = 7.5 Hz, 3H). ¹³C NMR (126 MHz, CD₃OD) δ 167.30, 157.63, 156.90, 154.54, 143.78, 140.16, 137.83, 136.66, 136.11, 130.53, 130.43, 129.94, 129.44, 126.71, 125.53, 124.02, 121.62, 75.26, 56.40, 54.34, 53.54, 29.38, 27.65, 25.24, 19.11, 14.57, 11.41. MS (ESI) (m/z): [M+H]⁺ 473.20

### Structure of (S, E)-1-(2-ethyl-4-(1-(((4-(5-fluoropyrimidin-2-yl)-3-methylbenzyl)oxy)imino)ethyl)benzyl)pyrrolidine-3-carboxylic acid (Compound 77)

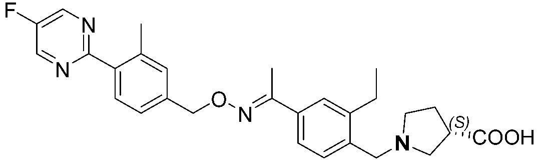

Pale yellow solid, yield 15.4%. ¹H NMR (500 MHz, CD₃OD) δ 8.82 (d, *J* = 1.9 Hz, 2H), 7.76 (d, *J* = 7.7 Hz, 1H), 7.64 (d, *J* = 2.0 Hz, 1H), 7.57 (dd, *J* = 8.1, 2.0 Hz, 1H), 7.48 (d, *J* = 8.1 Hz, 1H), 7.35 (d, *J* = 8.9 Hz, 2H), 5.26 (s, 2H), 4.44 - 4.34 (m, 2H), 3.52 (dd, *J* = 11.3, 5.4 Hz, 1H), 3.41 - 3.33 (m, 2H), 3.32 - 3.25 (m, 1H), 3.10 (s, 1H), 2.82 (q, *J* = 7.6 Hz, 2H), 2.51 (s, 3H), 2.29 (d, *J* = 1.4 Hz, 5H), 1.25 (td, *J* = 7.5, 1.7 Hz, 3H). ¹³C NMR (126 MHz, CD₃OD) δ 163.34, 157.57, 155.48, 154.51, 144.66, 144.50, 143.73, 139.67, 137.77, 137.07, 136.48, 130.50, 130.46, 130.20, 130.14, 126.67, 125.12, 123.98, 75.35, 56.58, 54.40, 53.55, 29.35, 27.76, 25.23, 19.92, 14.55, 11.43. MS (ESI) (m/z): [M+H]⁺ 491.20

### Structure of (S, E)-1-(2-ethyl-4-(1-(((3-methyl-4-(pyrimidin-2-yl)benzyl)oxy)imino)ethyl)benzyl)pyrrolidine-3-carboxylic acid (Compound 78)

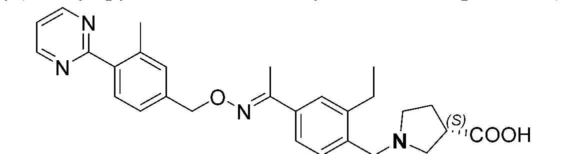

Pale yellow solid, yield 21.4%. ¹H NMR (500 MHz, CD₃OD) δ 8.89 (d, *J* = 4.9 Hz, 2H), 7.69 (d, *J* = 7.9 Hz, 1H), 7.65 (d, *J* = 1.9 Hz, 1H), 7.59 (dd, *J* = 8.0, 1.9 Hz, 1H), 7.52 (d, *J* = 8.1 Hz, 1H), 7.44 (t, *J* = 5.0 Hz, 1H), 7.37 (d, *J* = 7.2 Hz, 2H), 5.27 (s, 2H), 4.47 - 4.39 (m, 2H), 3.56 (dd, *J* = 11.3, 5.9 Hz, 1H), 3.42 (dt, *J* = 11.2, 7.4 Hz, 2H), 3.35 (d, *J* = 7.0 Hz, 1H), 3.15 (td, *J* = 8.6, 4.1 Hz, 1H), 2.83 (q, *J* = 7.5 Hz, 2H), 2.48 (s, 3H), 2.35 (dd, *J* = 14.4, 7.4 Hz, 1H), 2.30 (s, 4H), 1.26 (t, *J* = 7.5 Hz, 3H). ¹³C NMR (126 MHz, CD₃OD) δ 167.16, 157.00, 154.46, 143.78, 139.72, 137.86, 137.42, 136.88, 130.58, 130.37, 129.95, 129.87, 126.70, 125.17, 124.01, 119.06, 75.37, 56.32, 54.38, 53.60, 43.49, 27.71, 25.29, 19.54, 14.57, 11.41. MS (ESI) (m/z): [M+H]⁺ 473.20

### Structure of (S, E)-1-(2-ethyl-4-(1-(((2-fluoro-4-(2-fluoropyrimidin-5-yl)benzyl)oxy)imino)ethyl)benzyl)pyrrolidine-3-carboxylic acid (Compound 79)

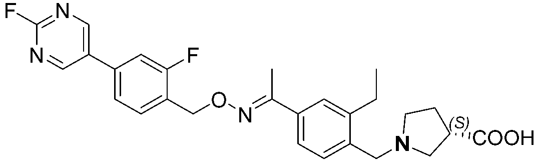

Pale yellow solid, yield 14.3%. ¹H NMR (500 MHz, CD₃OD) δ 8.98 (d, *J* = 1.4 Hz, 2H), 7.67 - 7.62 (m, 2H), 7.57 (dd, *J* = 8.0, 1.9 Hz, 1H), 7.52 (t, *J* = 8.6 Hz, 3H), 5.34 (s, 2H), 4.47 - 4.37 (m, 2H), 3.54 (dd, *J* = 11.4, 5.8 Hz, 1H), 3.43 - 3.36 (m, 2H), 3.35 (s, 1H), 3.18 - 3.09 (m, 1H), 2.83 (q, *J* = 7.5 Hz, 2H), 2.39 - 2.29 (m, 1H), 2.27 (s, 4H), 1.25 (t, *J* = 7.5 Hz, 3H). ¹³C NMR (126 MHz, CD₃OD) δ 163.34, 162.29, 161.60, 160.32, 159.08, 158.99, 154.85, 143.77, 137.60, 131.55, 131.52, 130.54, 130.13, 126.66, 126.05, 125.93, 123.98, 122.45, 113.63, 113.45, 68.93, 56.36, 54.37, 53.60, 43.53, 27.71, 25.25, 14.52, 11.28. MS (ESI) (m/z): [M+H]⁺ 495.20

### Structure of (S, E)-1-(2-ethyl-4-(1-(((4-(2-fluoropyrimidin-5-yl)-3-methylbenzyl)oxy)imino)ethyl)benzyl)pyrrolidine-3-carboxylic acid (Compound 80)

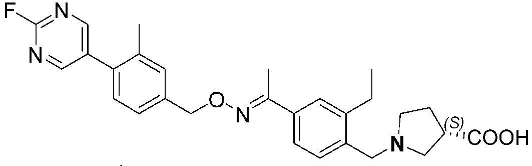

Pale yellow solid, yield 22.2%. ¹H NMR (500 MHz, CD₃OD) δ 8.69 (d, *J* = 1.6 Hz, 2H), 7.64 (d, *J* = 1.9 Hz, 1H), 7.57 (dd, *J* = 8.1, 1.9 Hz, 1H), 7.49 (d, *J* = 8.1 Hz, 1H), 7.41 (s, 1H), 7.38 (dd, *J* = 7.8, 1.8 Hz, 1H), 7.27 (d, *J* = 7.8 Hz, 1H), 5.25 (s, 2H), 4.42 - 4.34 (m, 2H), 3.51 (dd, *J* = 11.1, 5.7 Hz, 1H), 3.35 (t, *J* = 3.5 Hz, 2H), 3.31 - 3.24 (m, 1H), 3.10 (td, *J* = 8.7, 8.0, 4.3 Hz, 1H), 2.82 (q, *J* = 7.5 Hz, 2H), 2.30 (s, 4H), 2.28 (s, 4H), 1.25 (t, *J* = 7.5 Hz, 3H). ¹³C NMR (126 MHz, CD₃OD) δ 177.99, 162.77, 161.05, 160.69, 160.59, 157.18, 154.51, 143.71, 139.35, 137.62, 135.80, 133.62, 133.58, 132.50, 130.47, 130.44, 130.16, 129.72, 126.62, 125.87, 123.94, 75.29, 56.58, 54.39, 53.55, 43.69, 27.75, 25.24, 18.95, 14.56, 11.40. MS (ESI) (m/z): [M+H]⁺ 491.20

### Structure of (E)-1-(2-ethyl-4-(1-(((3-methyl-4-(pyrimidin-5-yl)benzyl)oxy)imino)ethyl)benzyl)azetidine-3-carboxylic acid (Compound 81)

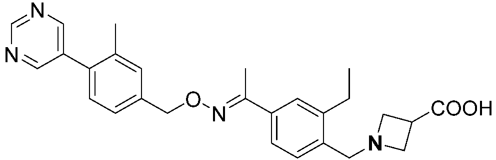

Pale yellow solid, yield 35.6%. ¹H NMR (500 MHz, CD₃OD) δ 9.15 (s, 1H), 8.85 - 8.75 (m, 2H), 7.63 (s, 1H), 7.56 (d, *J* = 8.1 Hz, 1H), 7.45 - 7.35 (m, 3H), 7.26 (t, *J* = 6.5 Hz, 1H), 5.25 (d, *J* = 3.0 Hz, 2H), 4.40 (s, 2H), 4.16 (p, *J* = 9.5 Hz, 4H), 3.41 (p, *J* = 8.3 Hz, 1H), 2.80 (q, *J* = 7.5 Hz, 2H), 2.30 (s, 3H), 2.27 (s, 3H), 1.27 - 1.22 (m, 3H). ¹³C NMR (126 MHz, CD₃OD) δ 176.27, 156.56, 156.36, 154.50, 143.54, 139.32, 137.57, 135.63, 133.39, 130.18, 129.64, 129.54, 126.62, 125.90, 123.97, 75.30, 57.16, 54.94, 34.92, 25.19, 18.97, 14.46, 11.41. MS (ESI) (m/z): [M+H]⁺ 459.20

### Structure of (E)-1-(2-ethyl-4-(1-(((3-methyl-4-(pyrimidin-5-yl)benzyl)oxy)imino)ethyl)benzyl)piperidine-4-carboxylic acid (Compound 82)

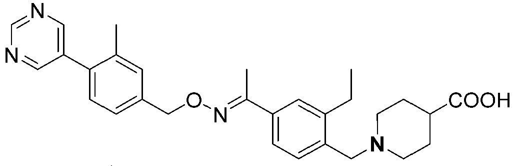

Pale yellow solid, yield 30.3%. ¹H NMR (500 MHz, CD₃OD) δ 9.15 (s, 1H), 8.80 (s, 2H), 7.63 (d, *J* = 1.9 Hz, 1H), 7.56 (dd, *J* = 8.1, 1.9 Hz, 1H), 7.47 (d, *J* = 8.1 Hz, 1H), 7.42 (s, 1H), 7.39 (dd, *J* = 7.8, 1.7 Hz, 1H), 7.27 (d, *J* = 7.8 Hz, 1H), 5.26 (s, 2H), 4.13 (s, 2H), 3.30 - 3.27 (m, 1H), 2.90 - 2.78 (m, 4H), 2.46 - 2.32 (m, 2H), 2.31 (s, 3H), 2.29 (s, 3H), 2.05 (dd, *J* = 14.6, 4.0 Hz, 2H), 1.90 (q, *J* = 12.8, 12.2 Hz, 2H), 1.25 (t, *J* = 7.5 Hz, 3H). ¹³C NMR (126 MHz, CD₃OD) δ179.59 (d, *J* = 2.2 Hz), 156.57, 156.35, 154.63, 144.37, 139.34, 137.46, 135.63, 135.60, 133.38, 131.20, 130.18, 129.63, 126.58, 125.90, 123.73, 75.28, 57.31, 52.14, 40.79, 26.66, 25.39, 18.97, 14.65, 11.45. MS (ESI) (m/z): [M+H]⁺ 487.30

### Structure of (S, E)-1-(2-ethyl-4-(1-(((3-methyl-4-(4-methylpyrimidin-5-yl)benzyl)oxy)imino)ethyl)benzyl)pyrrolidine-3-carboxylic acid (Compound 83)

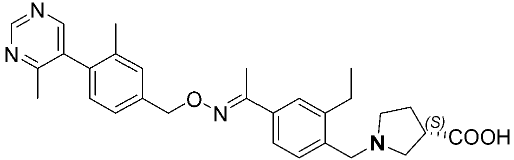

Pale yellow solid, yield 42.5%. ¹H NMR (500 MHz, Methanol-*d*₄) δ 9.03 (s, 1H), 8.48 (s, 1H), 7.66 (d, *J* = 1.9 Hz, 1H), 7.59 (dd, *J* = 8.1, 1.9 Hz, 1H), 7.51 (d, *J* = 8.1 Hz, 1H), 7.43 (d, *J* = 1.7 Hz, 1H), 7.38 (dd, *J* = 7.8, 1.7 Hz, 1H), 7.17 (d, *J* = 7.8 Hz, 1H), 5.28 (s, 2H), 4.48 - 4.36 (m, 2H), 3.53 (d, *J* = 7.7 Hz, 1H), 3.43 - 3.37 (m, 2H), 3.31 (s, 1H), 3.13 (s, 1H), 2.84 (q, *J* = 7.5 Hz, 2H), 2.33 (s, 4H), 2.30 (s, 4H), 2.11 (s, 3H), 1.27 (t, *J* = 7.5 Hz, 3H). ¹³C NMR (126 MHz, Methanol-*d*₄) δ 165.58, 156.48, 156.26, 154.49, 143.76, 139.12, 137.77, 135.87, 134.85, 134.16, 130.53, 130.18, 129.81, 129.17, 126.68, 125.69, 124.00, 75.40, 56.58, 54.41, 53.58, 48.46, 27.77, 25.25, 20.95, 18.56, 14.57, 11.43. MS (ESI) (m/z): [M+H]⁺ 487.30

### Structure of (S, E)-1-(2-ethyl-4-(1-(((3-methyl-4-(pyridazin-4-yl)benzyl)oxy)imino)ethyl)benzyl)pyrrolidine-3-carboxylic acid (Compound 84)

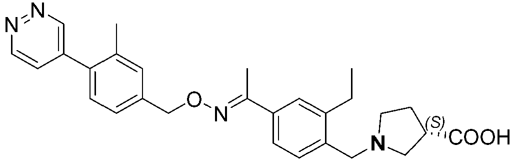

Pale yellow solid, yield 27.3%. ¹H NMR (500 MHz, CD₃OD) δ 9.27 - 9.23 (m, 2H), 7.77 (dd, *J* = 5.3, 2.4 Hz, 1H), 7.65 (d, *J* = 1.9 Hz, 1H), 7.58 (dd, *J* = 8.1, 1.9 Hz, 1H), 7.48 (d, *J* = 8.0 Hz, 1H), 7.45 (s, 1H), 7.42 (dd, *J* = 7.8, 1.7 Hz, 1H), 7.34 (d, *J* = 7.8 Hz, 1H), 5.28 (s, 2H), 4.36 (d, *J* = 2.5 Hz, 2H), 3.47 (dd, *J* = 11.1, 6.0 Hz, 1H), 3.37 - 3.34 (m, 1H), 3.31 (d, *J* = 7.1 Hz, 1H), 3.27 (q, *J* = 9.5, 8.4 Hz, 1H), 3.09 (ddd, *J* = 12.3, 8.4, 5.8 Hz, 1H), 2.83 (q, *J* = 7.5 Hz, 2H), 2.36 (s, 3H), 2.33 - 2.22 (m, 5H), 1.27 (t, *J* = 7.5 Hz, 3H). ¹³C NMR (151 MHz, CD₃OD) δ 178.13, 154.62, 151.80, 150.95, 143.69, 141.24, 139.97, 137.58, 135.47, 134.18, 130.41, 130.34, 129.36, 127.39, 126.63, 125.97, 123.94, 75.20, 56.73, 54.58, 53.60, 43.75, 27.78, 25.22, 18.89, 14.54, 11.39. MS (ESI) (m/z): [M+H]⁺ 473.30

### Structure of (S, E)-1-(2-ethyl-4-(1-(((3-fluoro-4-(thiophen-3-yl)benzyl)oxy)imino)ethyl)benzyl)pyrrolidine-3-carboxylic acid (Compound 85)

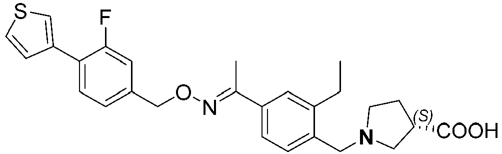

Pale yellow solid, yield 26.5%. ¹H NMR (500 MHz, CD₃OD) δ 7.66 (dt, *J* = 2.8, 1.4 Hz, 1H), 7.64 - 7.59 (m, 2H), 7.55 (dd, *J* = 8.1, 1.9 Hz, 1H), 7.49 - 7.45 (m, 2H), 7.44 (dt, *J* = 5.1, 1.4 Hz, 1H), 7.26 - 7.18 (m, 2H), 5.23 (s, 2H), 4.39 - 4.30 (m, 2H), 3.49 (dd, *J* = 11.1, 5.8 Hz, 1H), 3.35 (d, *J* = 9.5 Hz, 1H), 3.32 - 3.30 (m, 1H), 3.25 (ddd, *J* = 14.6, 7.7, 3.8 Hz, 1H), 3.08 (ddd, *J* = 14.6, 8.7, 5.8 Hz, 1H), 2.79 (q, *J* = 7.6 Hz, 2H), 2.33 - 2.20 (m, 5H), 1.23 (t, *J* = 7.6 Hz, 3H). ¹³C NMR (126 MHz, CD₃OD) δ 178.15, 160.47, 158.51, 154.74, 143.70, 139.64, 139.58, 137.55, 135.32, 135.31, 130.48, 130.44, 129.15, 129.11, 127.08, 127.05, 126.63, 125.21, 123.94, 123.69, 123.67, 123.14, 123.08, 122.77, 122.67, 115.25, 115.06, 74.68, 56.49, 54.38, 53.55, 43.69, 27.75, 25.23, 14.53, 11.45. MS (ESI) (m/z): [M+H]⁺ 481.20

### Structure of (S, E)-1-(2-ethyl-4-(1-(((2,6-difluoro-4-(6-fluoropyridin-3-yl)benzyl)oxy)imino)ethyl)benzyl)pyrrolidine-3-carboxylic acid (Compound 86)

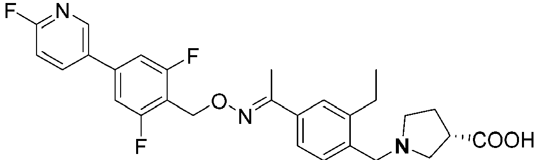

Pale yellow solid, yield 26.9%. ¹H NMR (500 MHz, CD₃OD) δ 8.49 (d, *J* = 2.6 Hz, 1H), 8.22 (ddd, *J* = 8.5, 7.5, 2.7 Hz, 1H), 7.64 (d, *J* = 1.9 Hz, 1H), 7.56 (dd, *J* = 8.1, 1.9 Hz, 1H), 7.47 (d, *J* = 8.1 Hz, 1H), 7.35 (d, *J* = 8.3 Hz, 2H), 7.19 - 7.15 (m, 1H), 5.33 (s, 2H), 4.42 - 4.34 (m, 2H), 3.51 (dd, *J* = 11.2, 5.8 Hz, 1H), 3.39 - 3.34 (m, 2H), 3.32 - 3.25 (m, 1H), 3.10 (ddd, *J* = 14.4, 8.5, 5.8 Hz, 1H), 2.81 (q, *J* = 7.6 Hz, 2H), 2.36 - 2.24 (m, 2H), 2.20 (s, 3H), 1.26 (t, *J* = 7.3 Hz, 3H). ¹³C NMR (126 MHz, CD₃OD) δ 172.97, 165.98, 164.82, 164.08, 162.83, 156.01, 146.90, 146.78, 145.01, 141.80, 141.73, 140.95, 138.87, 131.81, 127.93, 125.25, 114.64, 111.05, 111.03, 110.84, 110.74, 64.06, 57.88, 55.90, 55.04, 45.00, 29.11, 26.57, 20.85, 15.78, 12.33. MS (ESI) (m/z): [M+H]⁺ 512.20

### Structure of (S, E)-1-(2-ethyl-4-(1-(((2-fluoro-4-(5-fluoropyrazin-2-yl)benzyl)oxy)imino)ethyl)benzyl)pyrrolidine-3-carboxylic acid (Compound 87)

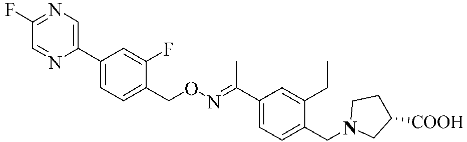

White solid, yield 78.9%. ¹H NMR (500 MHz, Methanol-*d*₄) δ 8.77 (s, 1H), 8.56 (s, 1H), 7.90 - 7.79 (m, 2H), 7.65 - 7.59 (m, 2H), 7.56 (s, 1H), 7.48 (d, *J* = 8.1 Hz, 1H), 5.34 (s, 2H), 4.44 - 4.34 (m, 2H), 3.55 - 3.46 (m, 1H), 3.36 (d, *J* = 7.7 Hz, 1H), 3.28 (dd, *J* = 10.9, 7.3 Hz, 1H), 3.13 - 3.05 (m, 1H), 2.81 (q, *J* = 8.6 Hz, 2H), 2.33 (dt, *J* = 15.2, 7.9 Hz, 1H), 2.28 (s, 3H), 2.23 (dd, *J* = 13.5, 6.8 Hz, 1H), 2.03 (s, 1H), 1.28 - 1.21 (m, 3H). ¹³C NMR (126 MHz, Methanol-*d*₄) δ 154.84, 143.72, 138.45, 138.38, 137.57, 132.58, 132.27, 131.05, 130.46, 130.30, 126.64, 123.95, 121.93, 113.21, 113.02, 69.00, 56.53, 54.37, 53.56, 43.58, 27.71, 25.21, 11.29. MS(m/z): [M+H]⁺ 495.20

### Structure of (S, E)-1-(2-ethyl-4-(1-(((3-fluoro-4-(5-fluoropyrazin-2-yl)benzyl)oxy)imino)ethyl)benzyl)pyrrolidine-3-carboxylic acid (Compound 88)

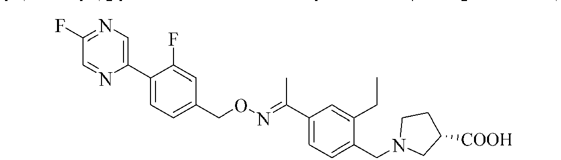

Yellow solid, yield 75.0%. ¹H NMR (500 MHz, Methanol-*d*₄) δ 8.67 (s, 1H), 8.62 (d, *J* = 8.3 Hz, 1H), 7.63 (s, 1H), 7.56 (d, *J* = 7.4 Hz, 1H), 7.49 (d, *J* = 8.1 Hz, 1H), 7.38 (d, *J* = 8.0 Hz, 1H), 7.32 (d, *J* = 12.1 Hz, 1H), 5.30 (s, 2H), 4.37 (d, *J* = 11.1 Hz, 2H), 3.29 (t, *J* = 6.7 Hz, 1H), 3.09 (t, *J* = 7.6 Hz, 1H), 2.82 (q, *J* = 7.5 Hz, 2H), 2.31 (s, 3H), 2.26 - 2.17 (m, 1H), 2.03 (s, 1H), 1.95 (s, 1H), 1.24 (t, *J* = 7.5 Hz, 3H).¹³C NMR (126 MHz, Methanol-*d*₄) δ 155.04, 143.70, 143.17, 141.41, 141.34, 141.24, 137.37, 132.95, 132.65, 130.83, 130.54, 130.52, 130.46, 126.61, 123.93, 123.82, 115.16, 114.98, 74.39, 56.65, 54.53, 53.57, 43.85, 25.24, 14.52, 11.44. MS(m/z): [M+H]⁺ 495.20

### Structure of (S, E)-1-(2-ethyl-4-(1-((4-(5-fluoropyrazin-2-yl)benzyl)oxy)imino)ethyl)benzyl)pyrrolidine-3-carboxylic acid (Compound 89)

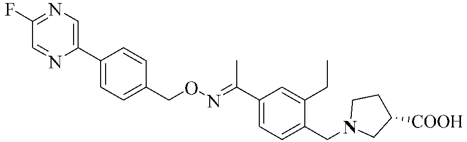

White solid, yield 76.6%. ¹H NMR (500 MHz, Methanol-*d*₄) δ 8.79 - 8.69 (m, 1H), 8.58 - 8.51 (m, 1H), 8.03 (dd, *J* = 16.8, 5.3 Hz, 2H), 7.63 (d, *J* = 7.0 Hz, 1H), 7.58 - 7.54 (m, 3H), 7.47 (d, *J* = 8.1 Hz, 1H), 5.30 (s, 2H), 4.41 - 4.30 (m, 2H), 3.50 (dd, *J* = 10.7, 5.5 Hz, 1H), 3.37 (s, 1H), 3.28 - 3.20 (m, 1H), 3.09 (dd, *J* = 10.2, 4.2 Hz, 1H), 2.85 - 2.78 (m, 2H), 2.36 - 2.31 (m,1H), 2.30 (s, 3H), 2.24 (q, *J* = 6.9, 6.4 Hz, 1H), 1.28 - 1.20 (m, 3H).¹³C NMR (126 MHz, Methanol-*d*₄) δ 154.62, 143.69, 140.08, 138.24, 138.17, 132.34, 132.04, 130.43, 128.28, 126.65, 126.44, 123.94, 75.24, 56.57, 54.45, 53.58, 43.66, 27.73, 25.22, 14.53, 11.42. MS(m/z): [M+H]⁺ 477.20

### Structure of (E)-1-(2-ethyl-4-(1-(((3-methyl-4-(5-fluoropyrazin-2-yl)-benzyl)oxy)imino)ethyl)benzyl)piperidine-4-carboxylic acid (Compound 90)

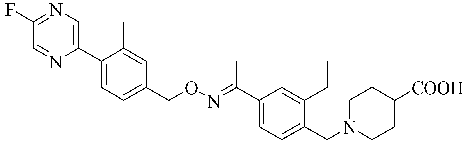

White solid, yield 28.8%. ¹H NMR (500 MHz, Methanol-*d*₄) δ 8.58 (d, *J* = 8.2 Hz, 1H), 8.37 (s, 1H), 7.60 (s, 1H), 7.52 (d, *J* = 10.0 Hz, 1H), 7.41 (dd, *J* = 18.7, 7.9 Hz, 4H), 5.26 (s, 2H), 3.96 (s, 2H), 3.18 (d, *J* = 11.6 Hz, 2H), 2.80 (q, *J* = 7.6 Hz, 2H), 2.66 (d, *J* = 11.5 Hz, 2H), 2.38 (s, 3H), 2.33 (d, *J* = 15.1 Hz, 1H), 2.28 (s, 3H), 2.02 - 1.93 (m, 2H), 1.84 (d, *J* = 12.0 Hz, 2H), 1.24 (t, *J* = 7.6 Hz, 3H). ¹³C NMR (126 MHz, Methanol-*d*₄) δ 158.27, 154.80, 144.11, 141.30, 139.50, 136.91, 136.35, 131.93, 131.63, 130.86, 130.33, 129.57, 126.42, 125.50, 123.52, 75.26, 57.89, 52.46, 27.35, 25.33, 19.05, 14.60, 11.48. MS(m/z): [M+H]⁺ 505.20

### Structure of (E)-1-(2-ethyl-4-(1-(((3-methyl-4-(5-fluoropyrazin-2-yl)-benzyl)oxy)imino)ethyl)benzyl)azetidine-3-carboxylic acid (Compound 91)

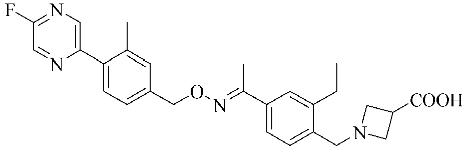

White solid, yield 62.5%. ¹H NMR (500 MHz, Methanol-*d*₄) δ 8.58 (d, *J* = 8.3 Hz, 1H), 8.37 (s, 1H), 7.63 (s, 1H), 7.56 (d, *J* = 8.0 Hz, 1H), 7.40 (dd, *J* = 19.3, 7.9 Hz, 4H), 5.26 (s, 2H), 4.40 (s, 2H), 4.16 (d, *J* = 8.3 Hz, 4H), 2.79 (q, *J* = 7.5 Hz, 3H), 2.37 (s, 7H), 2.28 (s, 3H), 1.25 (t, *J* = 7.5 Hz, 3H). ¹³C NMR (126 MHz, Methanol-*d*₄) δ 176.26, 160.27, 158.28, 154.47, 152.86, 143.53, 141.29, 141.22, 139.45, 137.66, 136.37, 131.94, 131.64, 130.32, 129.57, 129.51, 126.66, 125.50, 123.98, 75.33, 57.18, 54.93, 34.86, 25.16, 19.04, 14.45, 11.39. MS(m/z): [M+H]⁺ 477.20

### Structure of (E)-1-(2-ethyl-4-(1-(((3-methyl-4-(5-fluoropyrazin-2-yl)-benzyl)oxy)imino)ethyl)benzyl)piperidine-3-carboxylic acid (Compound 92)

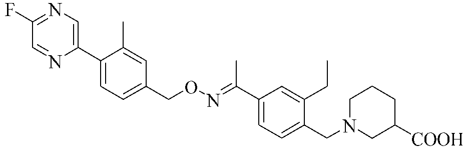

White solid, yield 83.5%. ¹H NMR (500 MHz, Methanol-*d*₄) δ 8.58 (d, *J* = 7.0 Hz, 1H), 8.37 (s, 1H), 7.65 (s, 1H), 7.57 (d, *J* = 8.0 Hz, 1H), 7.48 (d, *J* = 8.1 Hz, 2H), 7.45 - 7.35 (m, 6H), 5.26 (s, 5H), 4.23 (d, *J* = 3.6 Hz, 5H), 3.37 (s, 4H), 3.12 (d, *J* = 10.1 Hz, 5H), 2.82 (dq, *J* = 14.9, 7.3 Hz, 3H), 2.65 (s, 2H), 2.38 (s, 7H), 2.29 (s, 6H), 1.85 (d, *J* = 48.6 Hz, 11H), 1.27 (t, *J* = 7.5 Hz, 6H).¹³C NMR (126 MHz, Methanol-*d*₄) δ 160.27, 158.28, 154.54, 152.92, 144.11, 141.30, 141.22, 139.48, 137.76, 136.37, 135.06, 131.94, 131.64, 130.98, 130.33, 129.58, 126.60, 125.51, 123.93, 75.33, 54.53, 52.79, 48.46, 25.20, 19.04, 14.45, 11.41. MS(m/z): [M+H]⁺ 505.20

### Structure of (S, E)-1-(2-ethyl-4-(1-(((2-fluoro-4-(5-fluoropyrazin-2-yl)-3-methylbenzyl)oxy)imino)ethyl)benzyl)pyrrolidine-3-carboxylic acid (Compound 93)

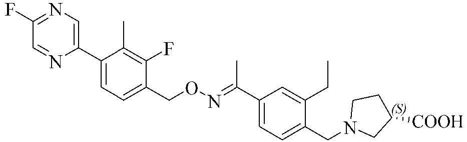

White solid, yield 70.1 %. ¹H NMR (500 MHz, Methanol-*d*₄) δ 8.59 (d, *J* = 8.2 Hz, 1H), 8.37 (s, 1H), 7.63 (s, 1H), 7.56 (d, *J* = 8.0 Hz, 1H), 7.49 (d, *J* = 8.1 Hz, 1H), 7.42 (t, *J* = 7.5 Hz, 1H), 7.25 (d, *J* = 7.9 Hz, 1H), 5.33 (s, 2H), 4.44 - 4.33 (m, 2H), 3.52 (dd, *J* = 11.2, 5.6 Hz, 1H), 3.41 - 3.35 (m, 2H), 3.28 (dt, *J* = 10.8, 7.3 Hz, 1H), 3.10 (dq, *J* = 8.7, 4.3, 2.9 Hz, 1H), 2.81 (q, *J* = 7.5 Hz, 2H), 2.27 (s, 8H), 1.24 (t, *J* = 7.4 Hz, 3H). ¹³C NMR (126 MHz, Methanol-*d*₄) δ 177.94, 160.40, 160.38, 158.43, 158.40, 154.73, 151.66, 143.71, 141.43, 141.35, 137.54, 132.19, 131.88, 130.49, 130.34, 127.45, 127.41, 126.63, 125.98, 125.85, 124.93, 124.90, 123.94, 123.68, 123.54, 69.41, 69.38, 56.46, 54.32, 53.54, 43.62, 27.73, 25.23, 14.53, 11.32, 10.54, 10.49. MS(m/z): [M+H]⁺ 509.19

### Structure of (S, E)-1-(2-ethyl-4-(1-(((2-fluoro-4-(5-fluoropyrazin-2-yl)-5-methylbenzyl)oxy)imino)ethyl)benzyl)pyrrolidine-3-carboxylic acid (Compound 94)

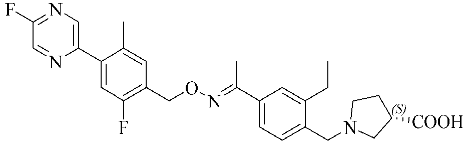

White solid, yield 71.5 %. ¹H NMR (500 MHz, Methanol-*d*₄) δ 8.60 (dd, *J* = 8.2, 1.4 Hz, 1H), 8.41 (t, *J* = 1.5 Hz, 1H), 7.65 (d, *J* = 1.9 Hz, 1H), 7.58 (dd, *J* = 8.1, 1.9 Hz, 1H), 7.48 (d, *J* = 8.1 Hz, 1H), 7.45 (d, *J* = 7.2 Hz, 1H), 7.25 (d, *J* = 10.3 Hz, 1H), 5.31 (s, 2H), 4.43 - 4.35 (m, 2H), 3.52 (dd, *J* = 11.2, 5.8 Hz, 1H), 3.39 - 3.35 (m, 2H), 3.29 (dd, *J* = 12.9, 5.5 Hz, 1H), 3.11 (ddd, *J* = 14.4, 8.7, 5.8 Hz, 1H), 2.82 (q, *J* = 7.5 Hz, 2H), 2.35 (s, 3H), 2.33 - 2.30 (m, 1H), 2.27 (s, 3H), 2.24 (dd, *J* = 13.7, 6.5 Hz, 1H), 1.25 (d, *J* = 7.5 Hz, 3H). ¹³C NMR (126 MHz, Methanol-*d*₄) δ 178.22, 160.41, 160.09, 158.41, 158.14, 154.72, 151.50, 143.70, 141.35, 141.27, 137.60, 136.95, 133.09, 133.05, 132.26, 132.08, 131.96, 130.46, 130.37, 126.65, 125.86, 125.74, 123.96, 116.17, 115.99, 69.06, 56.50, 54.48, 53.62, 43.57, 27.71, 25.21, 18.32, 14.50, 11.27. MS(m/z): [M+H]⁺ 509.20

### Structure of (R, E)-1-(2-ethyl-4-(1-(((4-(5-fluoropyrazin-2-yl)-3-methylbenzyl)oxy)imino)ethyl)benzyl)pyrrolidine-3-carboxylic acid (Compound 95)

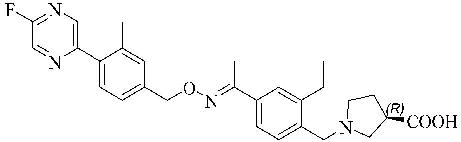

Pale yellow solid, yield 21.4%. ¹H NMR (500 MHz, Methanol-*d*₄) δ 8.57 (dd, *J* = 8.3, 1.3 Hz, 1H), 8.36 (t, *J* = 1.5 Hz, 1H), 7.64 (d, *J* = 1.9 Hz, 1H), 7.57 (dd, *J* = 8.1, 1.9 Hz, 1H), 7.49 (d, *J* = 8.1 Hz, 1H), 7.41 (d, *J* = 7.8 Hz, 1H), 7.39 - 7.34 (m, 2H), 5.26 (s, 2H), 4.44 - 4.34 (m, 2H), 3.52 (dd, *J* = 11.2, 5.7 Hz, 1H), 3.37 (td, *J* = 9.1, 8.2, 3.3 Hz, 2H), 3.28 (dt, *J* = 11.0, 7.4 Hz, 1H), 3.10 (ddd, *J* = 14.5, 8.8, 5.7 Hz, 1H), 2.82 (q, *J* = 7.5 Hz, 2H), 2.37 (s, 3H), 2.32 (td, *J* = 7.9, 3.6 Hz, 1H), 2.28 (s, 3H), 2.27 - 2.22 (m, 1H), 1.25 (t, *J* = 7.5 Hz, 3H). ¹³C NMR (126 MHz, Methanol-*d*₄) δ 177.86, 160.26, 158.26, 154.49, 152.89, 152.85, 143.73, 141.28, 141.21, 139.46, 137.71, 136.37, 135.07, 135.06, 131.94, 131.64, 130.50, 130.33, 130.21, 129.58, 126.65, 125.50, 123.96, 75.34, 56.48, 54.34, 53.54, 43.60, 27.72, 25.24, 19.06, 14.55, 11.42. MS (ESI) (m/z): [M+H]⁺ 491.25.

### Example 2. Relationship between S1PR4 and NDUFA10 mutations and muscular dystrophy diseases

Muscular dystrophy is a progressive muscle disease caused by genetic mutations, characterized by chronic oxidative stress and inflammation. Clinically, it manifests as progressive muscle weakness, affecting the limbs, throat, and facial muscles to varying degrees. It can be divided into congenital muscular dystrophy and late-onset muscular dystrophy based on the onset time. Generally, congenital muscular dystrophy shows obvious muscle weakness symptoms at birth or in the first few months after birth. While, late-onset muscular dystrophy presents with muscle weakness symptoms only after the patient has achieved independent walking.

Currently, the main treatment strategies for muscular dystrophy are gene therapy and antiinflammatory therapy. Gene therapy uses small molecules or antisense oligonucleotides to inhibit nonsense mutations that cause abnormal termination of transcription or induce skipping of specified exons, leading to the restoration of the reading frame and the production of dystrophin. Alternatively, adeno-associated viruses are used as vectors to introduce a normal wild-type gene to express dystrophin. However, the limitations of gene therapy are the immune response to the vector and the limited packaging size of the vector. In addition, gene therapy is only effective for patients with specific pathogenic gene variants and cannot be applied to all patients with muscular dystrophy. Inflammation is a secondary result caused by gene variants. The advantage of inhibiting inflammation is that it can be applied to all subtypes of muscular dystrophy, regardless of the mutated gene. Glucocorticoids have been used to reduce inflammation levels in the treatment of muscular dystrophy, however, the effects of glucocorticoids are limited and side effects are significant. Therefore, we need to find new inflammation targets.

In this example, the inventors discovered through whole-genome sequencing of muscular dystrophy patients that all patients had mutations in NDUFA10 and S1PR4. By using mouse models with NDUFA10 mutations and S1PR4 mutations, it was found that NDUFA10 mutations lead to mitochondrial dysfunction, S1PR4 mutations lead to increased inflammation levels, and double-mutant mice obtained by crossing S1PR4 mutant mice with NDUFA10 mutant mice exhibited muscular dystrophy phenotypes. Administration of S1PR4 agonists can improve the phenotype of muscular dystrophy, while reducing muscle inflammation and fibrosis, indicating that S1PR4 and NDUFA10 play important roles in the pathogenesis of muscular dystrophy and provide new inflammation-related targets for the treatment of muscular dystrophy.

The inventors discovered that males in a family began to exhibit a muscular dystrophy phenotype starting from the age of 50, and this phenotype worsened with increasing age. Through whole-genome sequencing, it was found that all patients with muscular dystrophy had mutations in both NDUFA10 and S1PR4. The study revealed that NDUFA10 and S1PR4 can link oxidative stress and chronic inflammatory responses in muscular dystrophy. In the NDUFA10 mutant mouse model, it was found that NDUFA10 mutation can lead to mitochondrial dysfunction, with no gender selectivity, as both male and female mice exhibited this phenotype. In the S1PR4 mutant mouse model, it was found that S1PR4 mutation can lead to elevated inflammation levels, also with no gender selectivity, as both male and female mice showed this phenotype. When mice had both NDUFA10 and S1PR4 mutations, male mice developed a muscular dystrophy phenotype, which, compared with normal mice, included reduced grip strength in the limbs, elevated serum creatine kinase levels, and the presence of muscle fibrosis and inflammation, among other symptoms. The results showed that mice with a single mutation in either of the two genes did not develop muscular dystrophy, while mice with double mutations did. The use of S1PR4 agonists can improve muscle performance and the muscular dystrophy phenotype in mice.

### I. Experimental Background

Muscular dystrophy (MD) is a group of genetic disorders characterized by progressive muscle wasting associated with chronic oxidative stress and inflammation. It is characterized by progressive muscle weakness and atrophy, and can be inherited in an autosomal recessive, autosomal dominant, or X-linked manner.

The inventors conducted genetic screening on patients from a muscular dystrophy family and found mutations in the S1PR4 gene and NDUFA10 gene in the patients. It was hypothesized that mutations in S1PR4 and NDUFA10 may cause muscular dystrophy. Therefore, the inventors constructed a S1PR4 mutant mouse model and a NDUFA10 mutant mouse model, and hybridized the two mouse strains to obtain S1PR4/NDUFA10 double-mutant mice, to verify whether these two mutations lead to muscular dystrophy.

### II. Study on the Association Between NDUFA10 and S1PR4 Mutations and Myopathy

The inventors discovered that both males and females in a family began to exhibit a muscular dystrophy phenotype starting at the age of 50, which worsened with age, showing strong characteristics of a familial genetic disease. Whole-genome sequencing was then performed on this family, and bioinformatics analysis of the sequencing results identified 23 gene mutations shared by all patients but absent in their healthy spouses (partial table shown below). Only columns W and X need to be considered in the table: column W lists the mutated gene names, and column X shows the corresponding mutation sites. Following this are the test results for each sample, with four data columns per sample. In the first data column (marked green), 0|1 indicates a heterozygous mutation, 1|1 indicates a homozygous mutation, and 0|0 indicates no mutation.

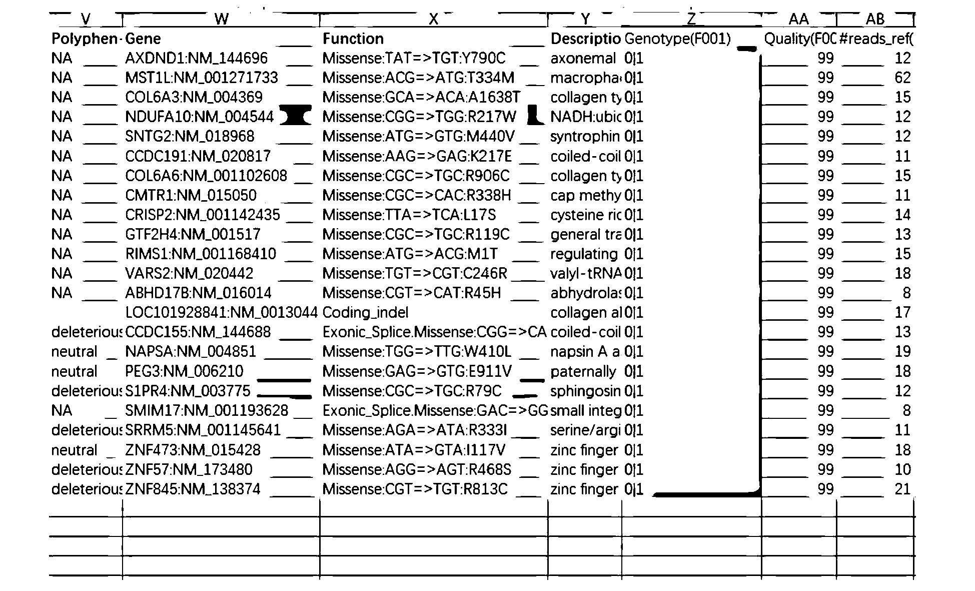

Through extensive research on muscular dystrophy and the functions of 23 mutated genes, two genes, NDUFA10 and S1PR4, have attracted attention. Among them, the former is a subunit of mitochondrial respiratory chain complex I, which may be related to energy production in the mitochondrion. Since muscle cells have high energy demands, mutations may affect muscle cell function by reducing energy production efficiency in the mitochondrion. The latter is associated with immune function and may be the main cause of changes in muscle tissue characteristics in patients with muscular dystrophy. Therefore, the inventors first investigated NDUFA10 and S1PR4 mutations as potential pathogenic gene mutations for muscular dystrophy.

Figure 1 shows the results of target-disease association studies: Figure 1 shows the identification of mutations S1PR4 R79C and NDUFA10 R217W in late-onset muscular dystrophy spectrum disorders. (a) Pedigree chart of a four-generation Chinese family. Patients are marked with filled black symbols, individuals with mild symptoms with black squares, males with squares and females with circles; the first and second generations are affected. Symbols below each member indicate status carrying mutations S1PR4 R79C (green) and NDUFA10 R217W (purple), respectively. (b) S1PR4 R79C is located within intracellular loop 1 (ICL1). (c) Sanger sequencing validation of SIPR4 R79C. (d) NDUFA10 R217W is located within the deoxynucleoside kinase domain (dNK). (e) Sanger sequencing validation of NDUFA10 R217W.

In the figure, the fully blacked-out individuals are patients with confirmed diagnoses, all of whom carry both a heterozygous mutation in S1PR4 and a heterozygous mutation in NDUFA10. Other offspring have inherited one mutation, two mutations, or no mutations at all, consistent with Mendelian inheritance patterns. Since the disease manifests only at a certain age, it is not yet possible to determine or confirm whether the next generation will develop the condition. However, some individuals report mild muscular symptoms; these are marked with 1/4 black shading by the inventor for reference.

Whole-genome sequencing conducted on families with muscular dystrophy revealed mutations in both NDUFA10 and S1PR4 in all affected individuals. In a mouse model with NDUFA10 mutation, NDUFA10 deficiency was found to cause mitochondrial dysfunction with no sex selectivity, as this phenotype was present in both males and females. In a mouse model with S1PR4 mutation, S1PR4 mutation was found to lead to elevated inflammation with no sex selectivity, as this phenotype was also present in both males and females. The results showed that neither single-gene mutant mouse developed muscular dystrophy. Double-mutant mice obtained by crossing S1PR4 mutant mice with NDUFAFA10 mutant mice exhibited a muscular dystrophy phenotype. Compared with normal mice, they showed reduced limb grip strength, elevated serum creatine kinase, and muscle fibrosis and inflammation. Therefore, the double-mutant mice developed muscular dystrophy.

### III. Experimental Procedure

### 3.1 Limb Grip Strength Detection

The mouse was placed horizontally on the grip test board. The mouse's tail was held and pulled gently in the opposite direction without using excessive force. The tail was gradually pulled until the mouse released the grip force sensor. The maximum value recorded by the grip force sensor is the grip strength. The test was repeated for 3 times and the average value was taken.

### 3.2 Detection of Serum Creatine Kinase Content

After the completion of administration, blood was collected via the orbital sinus, allowed to stand for 30 minutes, centrifuged at 3000 rpm at 4°C for 15 minutes, and serum was extracted, stored frozen at -20°C, and detected in accordance with the kit instructions.

### 3.3 Pathological Examination of Muscle Tissue Sections

After the experiment, the tibialis anterior, extensor digitorum longus, and diaphragm of the mice were harvested, fixed, sectioned, and subjected to HE staining and Sirius Red staining to observe the degree of muscle fibrosis.

### IV. Experimental Results

### 4.1 S1PR4 mutation does not cause muscular dystrophy

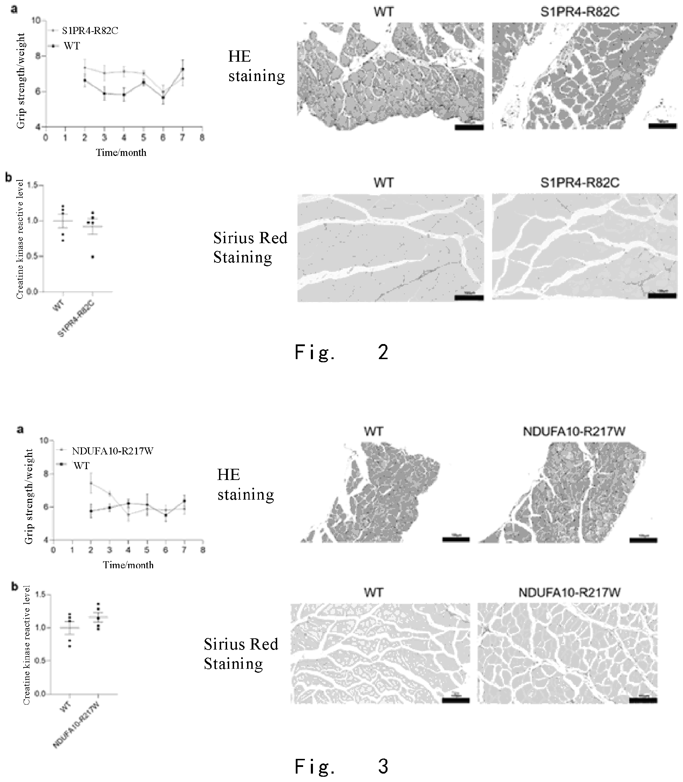

Mice with S1PR4 mutation showed no difference in paw grip strength compared with wild-type mice (Figure 2a). Serum creatine kinase levels in S1PR4 mutant mice showed no significant difference compared with WT mice (Figure 2b). No muscular fibrosis was observed in the muscles of S1PR4 mutant mice (HE staining and Sirius Red staining). These results indicate that S1PR4 mutation does not cause muscular dystrophy.

### 4.2 NDUFA10 mutation does not cause muscular dystrophy

Mice with NDUFA10 mutation showed no difference in paw grip strength compared with wild-type mice (Figure 3a). Serum creatine kinase levels in NDUFA10 mutant mice showed no significant difference compared with WT mice (Figure 3b). No muscle fibrosis was observed in the muscles of NDUFA10 mutant mice (HE staining and Sirius Red staining). These results indicate that NDUFA10 mutation does not cause muscular dystrophy.

### 4.3 S1PR4/NDUFA10 mutations cause muscular dystrophy

Mice with S1PR4/NDUFA10 mutations exhibited a significant decrease in paw grip strength compared with normal mice starting at 5 months of age (Figure 4a). Serum creatine kinase levels were significantly elevated in S1PR4/NDUFA10 mutant mice (Figure 4b). Muscle of S1PR4/NDUFA10 mutant mice showed nucleated fiber and collagen deposition, indicating muscular fibrosis (HE staining and Sirius Red staining), which supports a diagnosis of muscular dystrophy.

Based on all the above experimental results, double-mutant mice develop muscular dystrophy after reaching 5 months of age, with significantly reduced grip strength, markedly elevated creatine kinase levels, and muscle fibrosis. Since mutation of S1PR4 or NDUFA10 alone does not cause muscular dystrophy, whereas simultaneous mutation of both genes results in the disease, it is concluded that targeting one of these loci can block disease onset. Therefore, the development of targeted drugs against S1PR4 and NDUFA10 for the treatment of muscular dystrophy is of research value.

### Example 3. Discovery of Selective S1PR4 Agonists

Based on the above research findings in mutant mice, that is, any single gene mutation does not result in a muscular dystrophy phenotype, whereas double mutations induce such a phenotype, the inventors propose a therapeutic strategy of targeting and regulating one of the mutant proteins to reverse the muscular dystrophy phenotype. Compared with NDUFA10, S1PR4 has a more mature research background, with a well-defined binding pocket and ease of targeting, and was therefore first selected as a therapeutic target. Against this target, the inventors have developed a series of selective agonists for S1PR4.

### Bioactivity assay of S1PR4 agonist

### I. Experimental Principle

The Tango assay detects ligand-dependent GPCR activity through the interaction between β-arrestin and GPCRs. The TEV separation technology relies on the complementation of two inactive fragments of TEV protease (NTEV, residues 1-118, and CTEV, residues 119-221) to reconstitute functional protease. The NTEV fragment and the tetracycline transactivator (tTA), an artificial transcription factor, are fused at the C-terminus of the GPCR, while the CTEV fragment is fused to human β-arrestin 2. To enhance the stability of the interaction between GPCR and β-arrestin, a short sequence from the C-terminus of the human arginine vasopressin receptor 2 (V2 tail) is inserted between the GPCR and NTEV. The V2 tail contains multiple GRK phosphorylation sites, which strengthen β-arrestin binding upon phosphorylation. The CTEV fragment carries the stabile point mutation S219P and is truncated at residue 221 to remove the autoinhibitory C-terminal tail. Meanwhile, β-arrestin 2 is truncated to lack the entire C-terminal tail (residues 383-410 deleted). This truncation confers robust stimulus-dependent receptor desensitization, thereby improving detection sensitivity compared with wild-type β-arrestin 2. The UAS reporter construct consists of upstream activating sequence (UAS) elements, a minimal CMV promoter, and a tTA-dependent luciferase reporter gene. Therefore, constructing Tango cells requires the co-transfection of the above three plasmids into host cells. Upon GPCR activation and β-arrestin recruitment, the TEV enzyme at the β-arrestin terminus cleaves tTA from the GPCR C-terminus. The released tTA initiates tTA-dependent luciferase transcription, and compound activity is ultimately determined by measuring luminescence intensity.

### II. Experimental Procedure

1) Tango cell line from was digested from the culture dish respectively. After cell counting, the cells were plated at a density of 10,000 cells per well with 700 µL/well into a white opaque 96-well plate (add 60 µL D'Hanks to the surrounding wells), and incubated overnight (20 h) in a 5% CO₂ incubator at 37°C. The cell density will reach approximately 90% the next day.
2) After 20 h, 30 µL of serially diluted compound solution was added to each well. The control group was supplemented with 30 µL F12K complete medium. Each group had three replicate wells.
3) Samples were collected after 20 h of drug treatment. The cells were taken from the incubator and subjected to luciferase assay using the Bright Glo^{™} Luciferase Assay System: 5 µL of reagent per well was added, gently shaken in darkness at room temperature for 10 min, then detected.
4) The plate was read on a microplate reader, the Relative Light Unit (RLU) was measured, the luminescence values were recorded, the data was processed with GraphPad Prism software, and the EC₅₀ of the compound was calculated.

### III. Test Results:

(All compounds were tested at a final concentration of 45 µM, with 9 serial dilutions at 5-fold intervals. 1 µM BAF-312 was used as the positive control for S1PR1, and 25 µM BAF-312 was used as the positive control for S1PR4.)

| No. of compound | Structural formula | Tango-hS1P1-CHO | Tango-hS1P4-CHO | Tango-hS1P4-R-79C-CHO |
|---|---|---|---|---|
| | | EC₅₀(nM) | EC₅₀(nM) | EC₅₀(nM) |
| BAF312 | 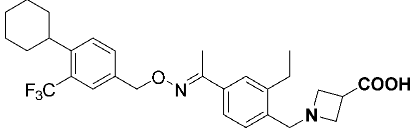 | 1.04+0.10 | 155.44±2 1.75 | 157.15+7.90 |
| 1 | 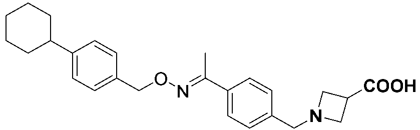 | 698.90+ 157.79 | 2969.00+ 471.63 | 1908.00+79 8.15 |
| 2 | 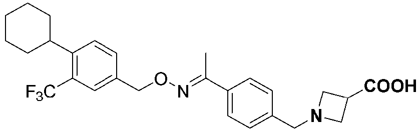 | 4.48+0.26 | 324.45±2 4.55 | 236.94+54. 45 |
| 3 | 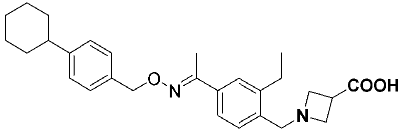 | 578.00+ 102.33 | 2157.00+ 771.50 | 1338.00+27 8.75 |
| 4 | 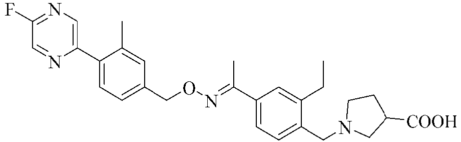 | **162.2±2 3.44** | **10.59±1.19** | |
| 5 | 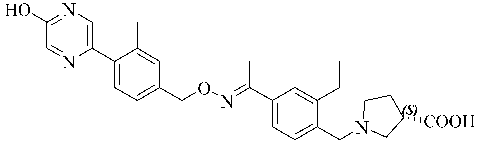 | **133.2±2 3.44** | **4.22±1.19** | |
| 6 | 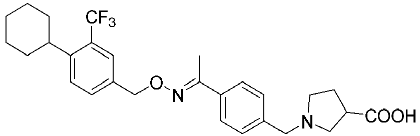 | 21.77±3. 02 | 318.40+8 1.40 | 291.50±95. 50 |
| 7 | 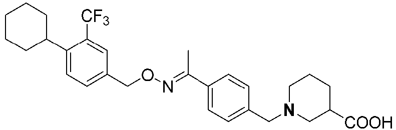 | 508.40+ 86.94 | 2504.00+ 518.88 | 3822.00±13 20.00 |
| 8 | 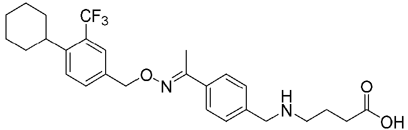 | 51.64±0. 38 | 2399.00+ 157.50 | 728.70+64. 50 |
| 9 | 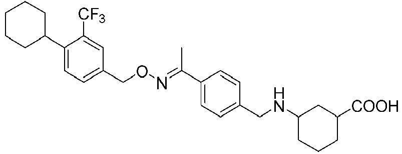 | 455.50± 36.20 | 2352.00± 239.04 | 1426.00±37 3.84 |
| 10 | 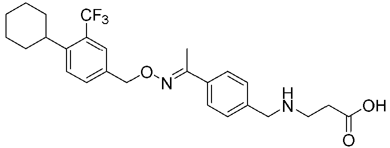 | 10.30±0. 38 | 261.60± 37.30 | 159.60±4.90 |
| 11 | 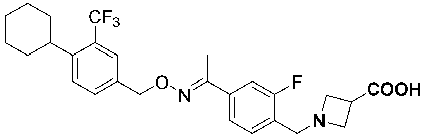 | 5.63±0.15 | 573.50± 106.45 | 254.80±10. 90 |
| 12 | 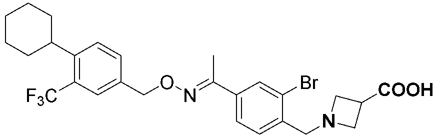 | 17.73±3. 25 | 2251.00± 701.50 | 1678.00±45 5.00 |
| 13 | 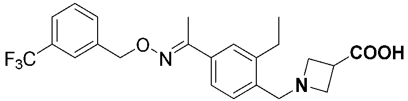 | 5099.00 ±1511.48 | 3238.00± 1129.35 | 3111.00±25 8.91 |
| 14 | 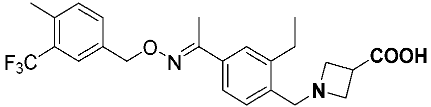 | 5991.00 ±830.82 | 3665.00± 387.61 | 7543.00±90 1.31 |
| 15 | 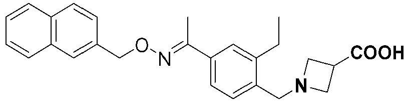 | 806.60± 241.09 | 560.10± 184.62 | 560.50±134 .72 |
| 16 | 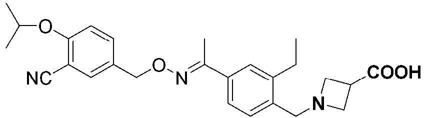 | 23.17± 10.86 | 3315.00± 863.14 | 1270.00±45 6.82 |
| 17 | 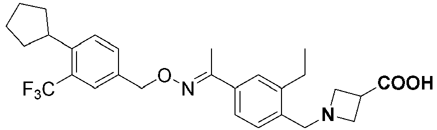 | 2.21±0.58 | 389.50±1 95.42 | 95.57±42.08 |
| 18 | 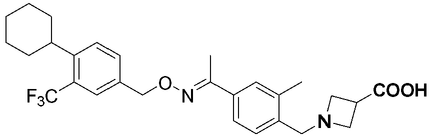 | 2.95±0.69 | 266.40± 62.77 | 63.06±21.76 |
| 19 | 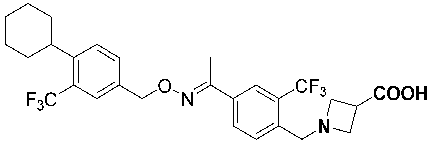 | 8.63±0.39 | 563.90±184.36 | 121.40±30.39 |
| 20 | 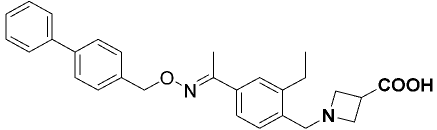 | 781.80± 24.90 | 234.60±5 4.29 | 273.10±54. 21 |
| 21 | 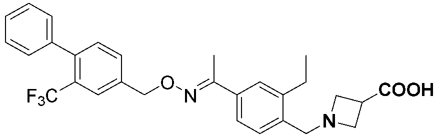 | 11.96±0. 03 | 192.20±6 4.93 | 70.78±27.0 7 |
| 22 | 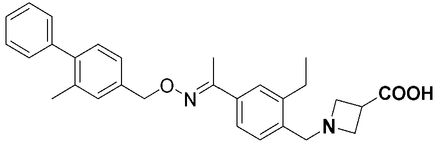 | 55.47±7. 82 | 114.50±83 .50 | 27.03±6.93 |
| 23 | 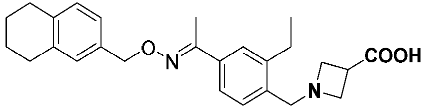 | 560.50± 24.80 | 569.50±1 57.62 | 585.90±80. 57 |
| 24 | 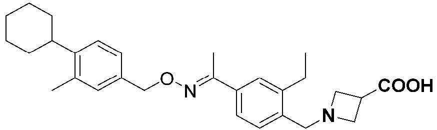 | 11.64±2. 84 | 257.10±5 7.40 | 219.20±80. 40 |
| 25 | 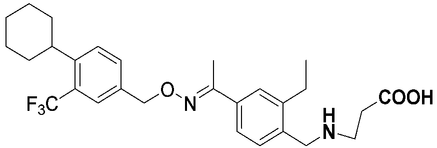 | 1.06±0.2 8 | 269.60±9 9.60 | 157.10±55. 19 |
| 26 | 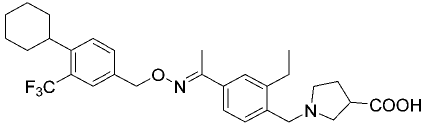 | 0.31±0.0 2 | 146.90±3 0.79 | 57.40±17.4 2 |
| 27 | 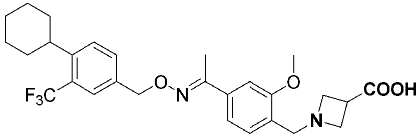 | 55.50±3. 43 | 1697.00±+ 588.45 | 1130.00±74 .84 |
| 28 | 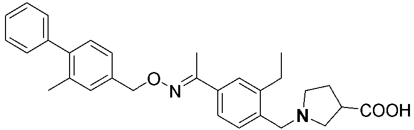 | 99.07±1 6.20 | 23.56±7.4 3 | 57.19±16.7 8 |
| 29 | 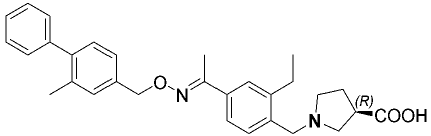 | 169.20± 32 | 54.41±0.7 2 | 87.17±4.08 |
| 30 | 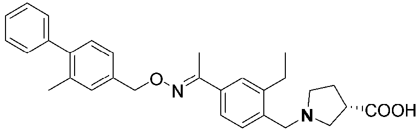 | 87.64±3. 35 | 9.09±0.67 | 14.97±3.78 |
| 31 | 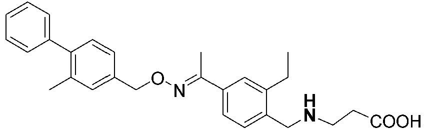 | 128.30± 10.00 | 77.07±3.0 4 | 73.10±4.91 |
| 32 | 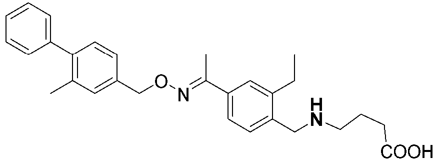 | 88.02±3. 66 | 116.00±2 0.29 | 110.50±6.85 |
| 33 | 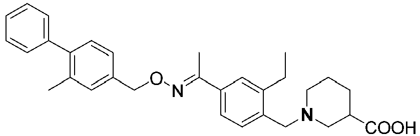 | 454.20± 84.20 | 127.10±9. 85 | 175.40±36. 50 |
| 34 | 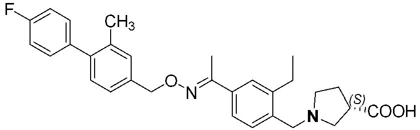 | 86.87 | 2.405 | |
| 35 | 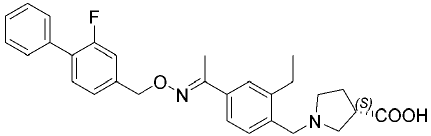 | 103.6 | 3.791 | |
| 36 | 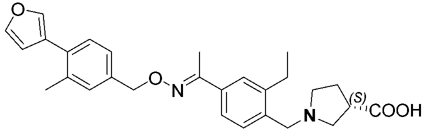 | 103.3 | 1.667 | |
| 37 | 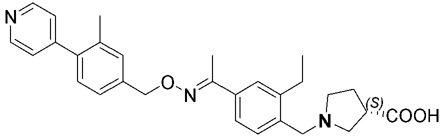 | 643.8 | 15.59 | |
| 38 | 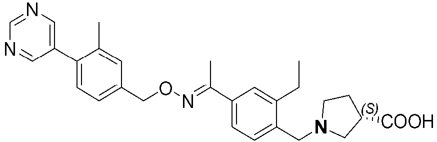 | 2974±21 6.74 | 13.99±2.3 9 | |
| 39 | 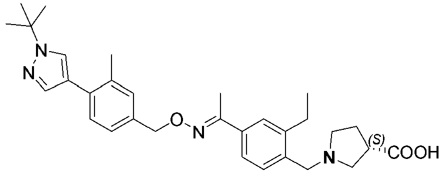 | > 10000 | 311.40±4 9.28 | |
| 40 | 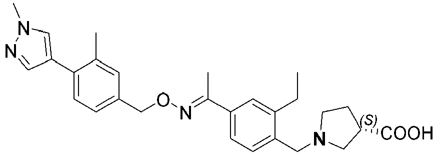 | > 10000 | 73.63±12. 88 | |
| 41 | 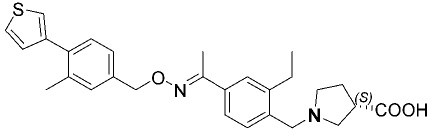 | 51.55±1. 82 | 2.91±0.01 | |
| 42 | 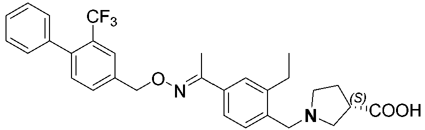 | 3.825±0. 24 | 20.53±0.9 8 | |
| 43 | 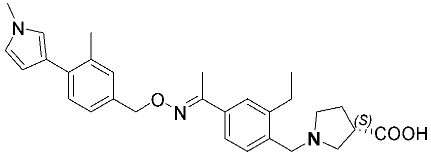 | 2092±22 3.54 | 55.33±8.3 2 | |
| 44 | 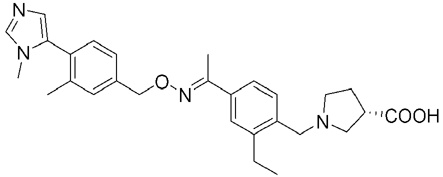 | >10000 | 878.5±45. 87 | |
| 45 | 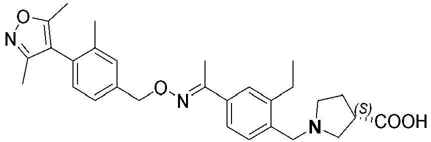 | > 10000 | 407.40±7 4.54 | |
| 46 | 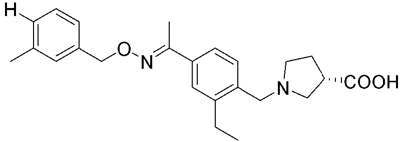 | 5606±42 0.18 | 487.50±1 8.97 | |
| 47 | 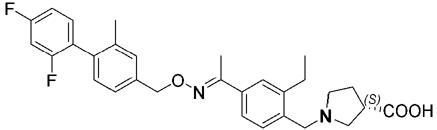 | 131.7±4. 40 | 4.67±0.71 | |
| 48 | 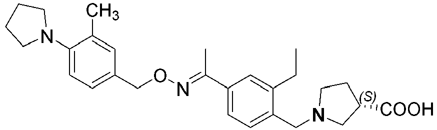 | **276.4±2 6.98** | **210.8±10 0.8** | |
| 49 | 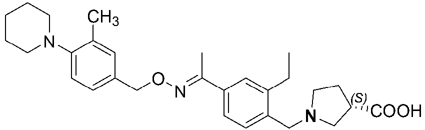 | **66.07±8. 58** | **181.3±10 0.8** | |
| 50 | 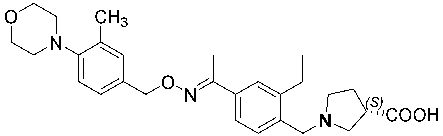 | **3365±39 7.2** | **101.9±97. 13** | |
| 51 | 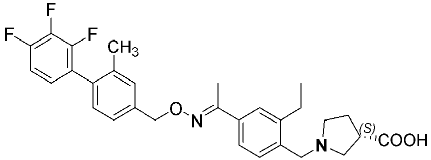 | **195±27. 65** | **118±46.2 1** | |
| 52 | 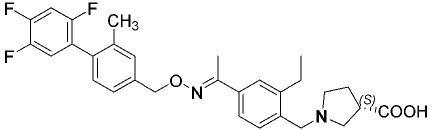 | **218.8±6 6.2** | **26.31±3.2 6** | |
| 53 | 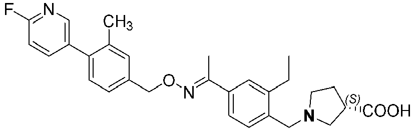 | **238.9±9 7.56** | **7.02±0.59** | HYY20 |
| 54 | 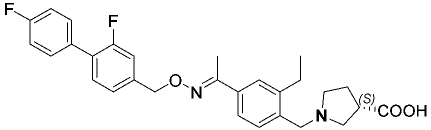 | **765.3±8 1.76** | **13.53±8.5 8** | |
| 55 | 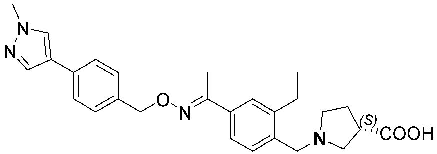 | **>10000** | **268.7** | |
| 56 | 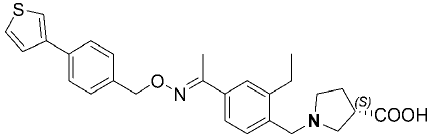 | **686.1±4 9.07** | **5.05±1.84** | |
| 57 | 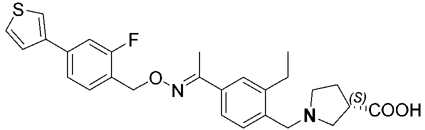 | **664.1±1 19** | **6.52±0.07 3** | |
| 58 | 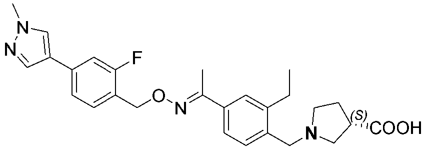 | **510.1±2 50.2** | **>10000** | .1 |
| 59 | 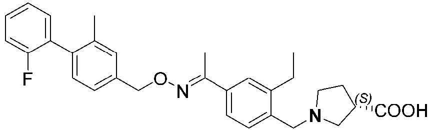 | **86.08±2 0.7** | **44.25±23. 21** | |
| 60 | 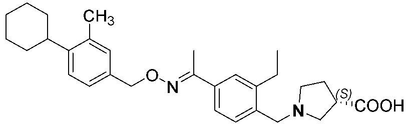 | **3.50±1.6 4** | **71.69** | |
| 61 | 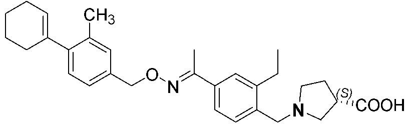 | **4.75±1.8 8** | **3.33±2.5** | |
| 62 | 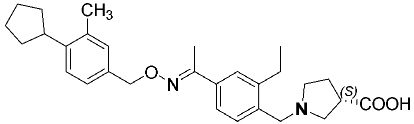 | **1.13±0.3 818** | **3.31±0.24** | |
| 63 | 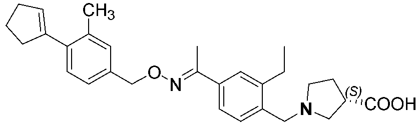 | **21.89±1. 03** | **5.86±0.27** | |
| 64 | 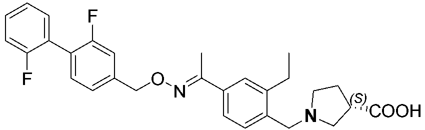 | **239.1±1. 63** | **7.77±1.02** | |
| 65 | 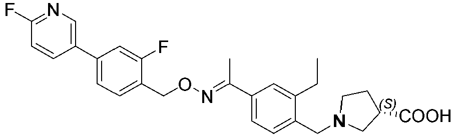 | **>10000** | **10.51±0.2 8** | |
| 66 | 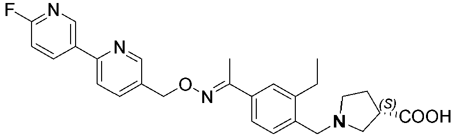 | **>10000** | **421.9±10. 61** | |
| 67 | 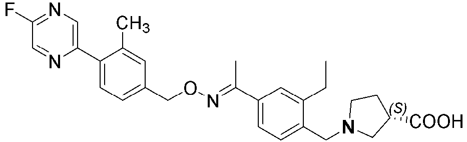 | **194.8±1 6.05** | **1.80±0.43** | |
| 68 | 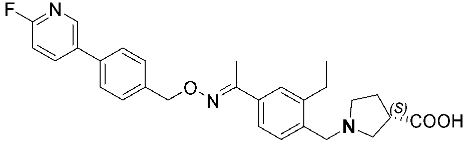 | **7536±94 1.2** | **33.69±12. 04** | |
| 69 | 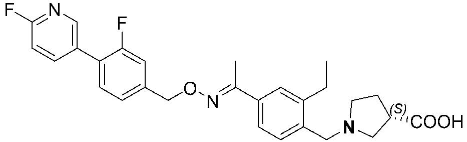 | **4981±52 5.4** | **8.28±0.38** | |
| 70 | 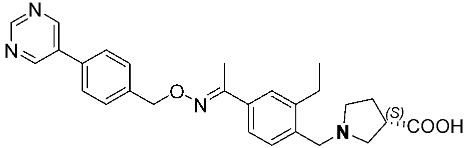 | **>10000** | **36.17±6.58** | |
| 71 | 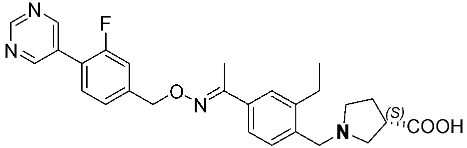 | **>10000** | **34.06±6.0 8** | |
| 72 | 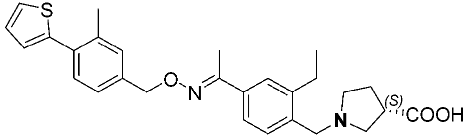 | **112.1±0. 85** | **1.51±0.64** | |
| 73 | 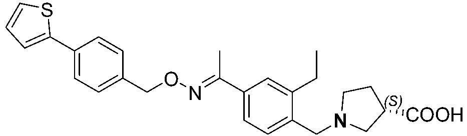 | **1113±55 .86** | **10.68±0.11** | |
| 74 | 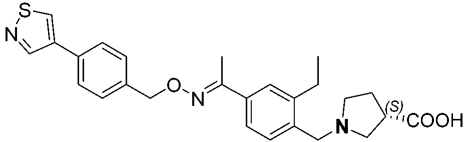 | **> 10000** | **13.13±0.02** | |
| 75 | 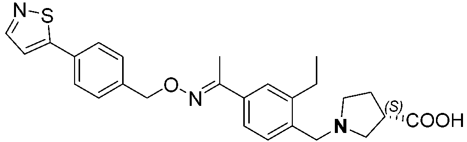 | **> 10000** | **34.17±0.22** | |
| 76 | 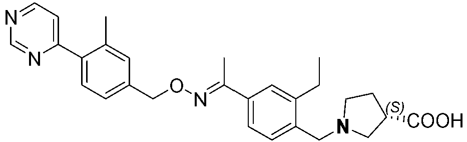 | **549.1±7 7.78** | **4.39±0.91** | |
| 77 | 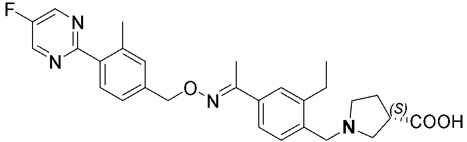 | **270.9±8. 77** | **5.93±4.12** | |
| 78 | 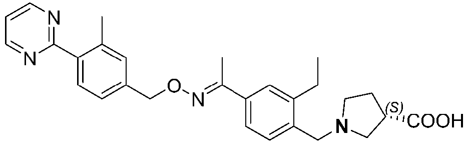 | **1888±12 7.3** | **215.8±64. 91** | |
| 79 | 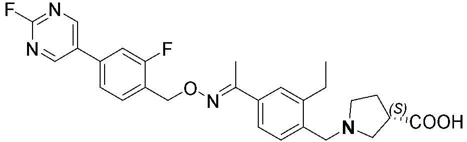 | **>10000** | **444.7±88. 81** | |
| 80 | 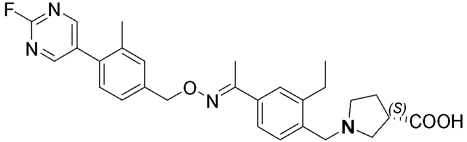 | **2082±40 .31** | **18.05±0.525** | |
| 81 | 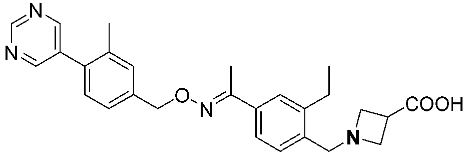 | **2254±23 6.9** | **102.7±9.1** | |
| 82 | 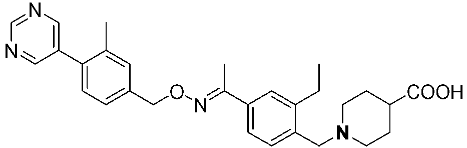 | **> 10000** | **42.7±2.28** | |
| 83 | 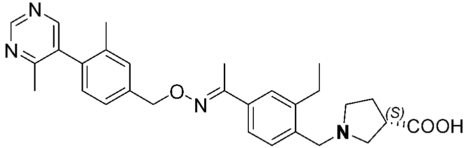 | **> 10000** | **40.95±0.22** | |
| 84 | 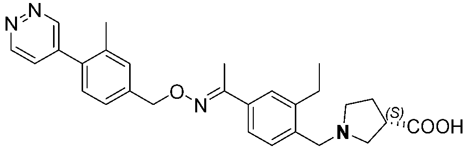 | **> 10000** | **254.1±72. 34** | |
| 85 | 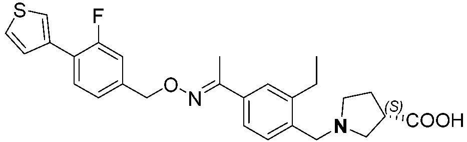 | **173.1±9. 69** | **8.52±0.21** | |
| 86 | 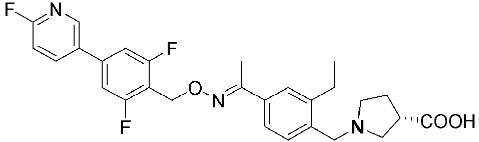 | **> 10000** | **42.22±10. 71** | |
| 87 | 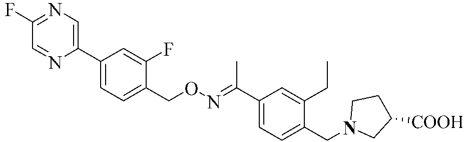 | **> 10000** | **17.4±0.39** | |
| 88 | 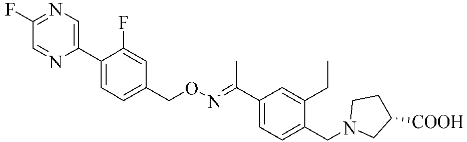 | **3207±111.7** | **27.38±1.12** | |
| 89 | 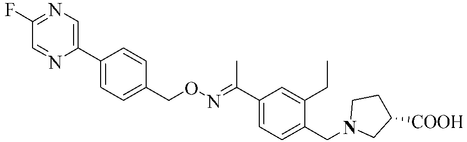 | **5250±234.8** | **135.2±17.75** | |
| 90 | 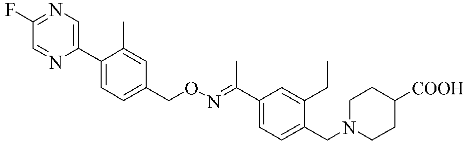 | **5950±72.83** | **> 10000** | |
| 91 | 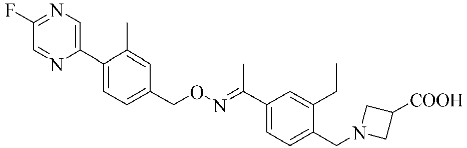 | **54.45±0.20** | **> 10000** | |
| 92 | 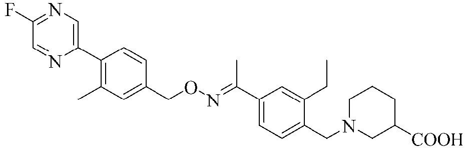 | **> 10000** | **> 10000** | |
| 93 | 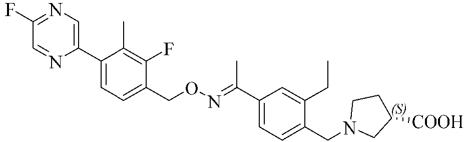 | **347.9±40.44** | **12.03±1.00** | |
| 94 | 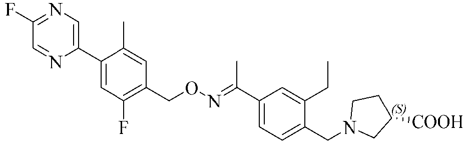 | **613.9±50.25** | **13.94±1.48** | |
| 95 | 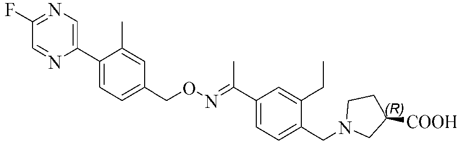 | **125.0±14.50** | **75.38±5.72** | |

Although some S1PR4 regulators have been reported, most of these compounds have unclear mechanisms, lack detailed structure-activity relationship analysis, and have no *in vivo* pharmacokinetic parameters available, making it impossible to evaluate their *in vivo* efficacy. Starting from BAF312, an S1PR1/5 agonist, as the lead compound, the inventors modified the polar head and hydrophobic moiety of BAF312 based on a β-arrestin recruitment assay system for S1PR1 and S1PR4, synthesized and evaluated a series of compounds. Through comprehensive structure-activity relationship studies on BAF312 at S1PR1/4, the inventors found that replacing the cyclohexyl group of BAF312 with a benzene ring and the trifluoromethyl group with a methyl group to yield Compound 22 significantly reduced S1PR1 activity while retaining S1PR4 activity. Subsequently, the inventors modified the polar head region of Compound 22 and discovered that the introduction of chiral pyrrolidine carboxylic acid further enhanced the compound's activity on S1PR4, leading to the identification of the selective S1PR4 agonist Compound 30. Test results showed that Compound 30 exhibited a β-arrestin recruitment activity on S1PR4 with an EC₅₀ value of 9.09 nM. Further selectivity profiling across S1PR1-5 revealed that, compared with the endogenous ligand S1P, Compound 30 showed no agonist activity on S1PR2/3/5 and approximately 10-fold selectivity over S1PR1, further confirming it as a selective S1PR4 agonist with markedly higher agonistic activity on S1PR4 than S1P. Meanwhile, Compound 67 displayed superior S1PR4 selectivity and activity relative to Compound 30, with an S1PR4 activity of 1.80 nM and more than 100-fold selectivity over S1PR1.

### Selectivity of Compounds 30 and 67 on S1PRs

| Receptor | EC₅₀/nM (Emax/%) **30** | EC₅₀/nM (Emax/%) **67** | EC₅₀/nM S1P |
|---|---|---|---|
| S1PR1 | 87.64±3.35 (101.66)*^{a}* | 194.8± 16.05 nM *^{a}* | 8.52±1.06*^{a}* |
| S1PR2 | >10000 (-6.90)*^{b}* | >10000 nM *^{a}* | 2.95±1.37*^{b}* |
| S1PR3 | >10000 (2.25)*^{c}* | >10000 nM *^{a}* | 20.60±3.37*^{c}* |
| S1PR4 | 9.09±0.67 (103.51)*^{a}* | 1.80±0.43 nM *^{a}* | 100.00±21.85*^{a}* |
| S1PR5 | >10000 (19.51)*^{b}* | 3740±221.3 nM *^{a}* | 2.00±1.90*^{b}* |

*^{a}* β-arrestin recruitment activity based on Tango-S1PR1-CHO-K1 and Tango-S1PR4-CHO-K1 cells, expressed as mean EC₅₀ values ± SD. *^{b}* FLIPR assays were performed using S1PR2-CHO and S1PR5-CHO cell lines, expressed as mean EC₅₀ values ± SD. *^{c}* β-arrestin recruitment activity based on PathHunter CHO-K1-S1PR3 cells, expressed as mean EC₅₀ values ± SD. Data are the mean of at least two independent determinations, reported as mean ± SD (standard deviation).

### Example 4. Pharmacokinetic Property Testing of Compounds

In the structure-activity relationship discussion, the inventors obtained key compound 22. Based on compound 22, the polar head of the compound was modified as chiral pyrrolidine carboxylic acid, yielding selective S1PR4 agonists 30 and 67. We conducted pharmacokinetic property analyses on compounds 22, 30, and 67, respectively. It was found that compound 22 retained a cyclobutanoic acid polar head similar to BAF312, but exhibited dual activity on S1PR1 and S1PR4, with a bioavailability of only 23.5%, resulting in poor druggability. In contrast, the newly discovered compounds 30 and 67 not only showed excellent S1PR4 agonist activity but also outstanding oral absorption properties, with significantly improved *in vivo* exposure. Their oral bioavailabilities reached 119.7% and 76.21%, respectively, demonstrating greater druggable potential.

### Pharmacokinetic parameters of Compound 22 ^{a}

| Parameter | Compound **22** | |
|---|---|---|
| | IV (1 mg/kg) | PO (10 mg/kg) |
| Tmax (h) | 0.08±0 | 0.83±0.29 |
| T_{1/2} (h) | 0.42±0.03 | 2.16±0.96 |
| Cmax (ng/mL) | 995.32±53.20 | 544.44±221.09 |
| AUC₀₋ₜ (h*ng/mL) | 475.87±43.64 | 1118.59±399.59 |
| CL_obs (L/h/kg) | 2.08±0.19 | 8.90±3.86 |
| Vss_obs (L/kg) | 0.88±0.07 | 27.73±16.85 |
| *F* (%) | -- | 23.5 |

### Pharmacokinetic parameters of Compound 30 ^{a}

| Parameter | Compound **30** | |
|---|---|---|
| | IV (1 mg/kg) | PO (10 mg/kg) |
| Tmax (h) | 0.08±0 | 2.00±0 |
| T_{1/2} (h) | 2.15±0.06 | 2.78±0.12 |
| Cmax (ng/mL) | 437.70±82.58 | 1031.52±91.90 |
| AUC₀₋ₜ (h*ng/mL) | 823.51±284.02 | 9860.13±794.15 |
| CL_obs (L/h/kg) | 1.21±0.44 | 1.02±0.08 |
| Vss_obs (L/kg) | 3.59±1.16 | 4.08±0.32 |
| *F* (%) | -- | 119.7 |

| | | |
|---|---|---|
| *^{a}* SD rats weighing 200-220 g (male, 3 rats per group) were used for the study. | | |

### Pharmacokinetic parameters of Compound 67 ^{a}

| Parameter | Compound 67 | |
|---|---|---|
| | IV (1 mg/kg) | PO (10 mg/kg) |
| Tmax (h) | 0.08±0.00 | 1.83±1.89 |
| T_{1/2} (h) | 1.18±0.01 | 2.17±0.11 |
| Cmax (ng/mL) | 958.08±45.87 | 986.13±277.10 |
| AUC₀₋ₜ (h*ng/mL) | 836.22±26.98 | 6372.51±1509.83 |
| CL_obs (L/h/kg) | 1.19±0.04 | 1.55±0.27 |
| Vss_obs (L/kg) | 1.52±0.02 | 4.88±1.17 |
| *F* (%) | -- | 76.21 |

| | | |
|---|---|---|
| *^{a}* SD rats weighing 200-220 g (male, 3 rats per group) were used for the study. | | |

### Example 5. Internalization Function Test

S1P can induce receptor internalization and inhibit the egress of lymphocytes from secondary lymphoid tissues, thereby reducing inflammatory responses in body tissues, which is the key biological effect underlying the therapeutic activity for diseases of S1P-targeted drugs. Therefore, the recovery degree of S1PR4 after internalization caused by Compound 30 was evaluated, and the recovery effect following receptor internalization caused by Compound 30 was assessed in cells with high S1PR4 expression. S1PR4-293T cells were treated with 1 µM S1P, BAF312, and Compound 30 at 37 °C for 1.5 hours, and the expression of S1PR4 receptor on the cell surface was detected immediately, or at 5 hours or 20 hours after treatment. As shown in Figure 5, BAF312, an agonist of S1PR1 and S1PR5, cannot induce S1PR4 internalization due to its lack of agonistic activity on S1PR4. S1P can induce S1PR4 internalization, with receptor expression recovering after 20 hours. The selective S1PR4 agonist Compound 30 exhibits sustained internalization of S1PR4 for up to 20 hours, demonstrating that Compound 30 is a potent and selective S1PR4 agonist. The recovery effect of S1PR4 after internalization induced by Compound 30 is shown in Figure 5.

### Example 6. Use of an S1PR4 agonist to improve muscle performance and muscular dystrophy phenotype in S1PR4-R82C/NDUFA10-R217W double-mutant mice (Compound 30)

Based on the above research on the relationship between targets and myopathy, as well as the discovery of selective S1PR4 regulators, the inventors evaluated the *in vivo* efficacy of Compound 30 in a muscular dystrophy disease model caused by the S1PR4-R82C/NDUFA10-R217W double mutation.

The experimental results show that administration of the S1PR4 agonist can improve the phenotype of muscular dystrophy, while reducing muscle inflammation and fibrosis.

This study demonstrates that mutations in S1PR4 and NDUFA10 play important roles in the pathogenesis of muscular dystrophy, and provides new inflammation-related targets and therapeutic drug options for treating muscular dystrophy.

### Experimental Animals and Grouping

Experimental Animals: A total of 34 S1PR4-R82C/NDUFA10-R217W mice were divided into 6 groups as shown below.

| Group No | Grouping | No. of animal | Route of administration | Frequency of administration |
|---|---|---|---|---|
| 1 | Model | 6 | intragastric administration | Once daily |
| 2 | Prednisolone 1 mg/kg | 5 | intragastric administration | Once daily |
| 3 | BAF312 1 mg/kg | 5 | intragastric administration | Once daily |
| 4 | 30 0.3 mg/kg | 6 | intragastric administration | Once daily |
| 5 | 30 1 mg/kg | 6 | intragastric administration | Once daily |
| 6 | 30 3 mg/kg | 6 | intragastric administration | Once daily |

### 1. Main reagents and drugs

Positive control prednisolone (1 mg/kg), BAF312(1 mg/kg), compound 30 (0.3/1/3 mg/kg)

### 2 Experiment method

Intragastric administration was initiated on Day 0, and body weight was measured daily during the experiment to adjust the drug dosage. Grip strength was measured at the beginning and end of the experiment to observe motor changes in mice. Changes in the limb grip strength, serum creatine kinase content, and muscle fibrosis/inflammation in mice were detected as indicators for evaluating drug efficacy.

### 2.1 Tested items after completion of treatment

### 2.1.1 Limb grip strength test

The mouse was placed horizontally on the grip test board. The mouse's tail was held and pulled gently in the opposite direction without using excessive force. The tail was gradually pulled until the mouse released the grip force sensor. The maximum value recorded by the grip force sensor is the grip strength. The test was repeated for 3 times and the average value was taken.

### 2.1.2 Detection of Serum Creatine Kinase Content

After the completion of administration, blood was collected via the orbital sinus, allowed to stand for 30 minutes, centrifuged at 3000 rpm at 4°C for 15 minutes, and serum was extracted, stored frozen at -20°C, and detected in accordance with the kit instructions.

### 2.1.3 Pathological Examination of Muscle Tissue Sections

After the experiment, the tibialis anterior, extensor digitorum longus, and diaphragm of the mice were harvested, fixed, sectioned, and subjected to HE staining and Sirius Red staining to observe the degree of muscle fibrosis. The slice was subjected to immunofluorescence staining with F4/80 to observe the level of muscle inflammation.

Changes in body weight of double-mutant mice after administration are shown in Figure 6:
After administration, there was no difference in the body weight gain trend for double-mutant mice in the BAF312 1 mg/kg group and the compound 30 0.3, 1, 3 mg/kg groups as compared with the model group. The body weight gain trend in the prednisolone 1 mg/kg group was relatively slow compared with the other groups.

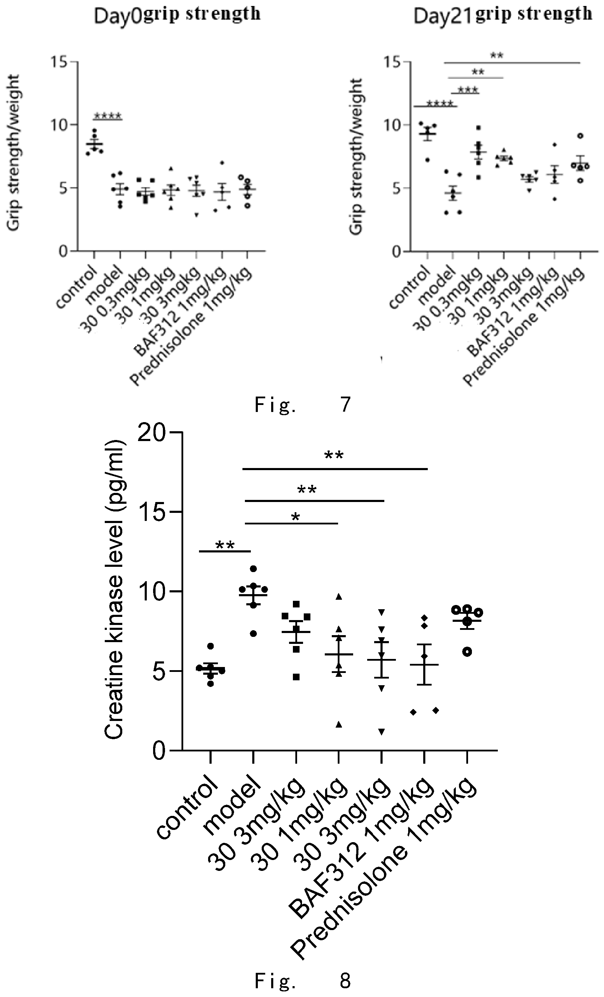

Changes in grip strength of double-mutant mice after administration are shown in Figure 7:
After the administration in double-mutant mice, grip strength was measured at the start and end of treatment. The ratio of grip strength to body weight was calculated to represent changes in mouse grip strength, thereby eliminating the influence of body weight. At the beginning of treatment, the grip strength of double-mutant mice was significantly lower than that of wild-type mice. After 21 days of treatment, the grip strength was significantly increased in the 0.3 mg/kg and 1 mg/kg Compound 30 groups as well as the 1 mg/kg prednisolone group, demonstrating favorable therapeutic effects.

Changes in creatine kinase of double-mutant mice are shown in Figure 8:
The creatine kinase level in the serum of double-mutant mice was significantly higher than that in healthy mice. After treatment, Compound 30 at 1 mg/kg and 4 mg/kg, as well as BAF312 at 1 mg/kg, significantly reduced the creatine kinase level, whereas the prednisolone 1 mg/kg group showed no such lowering effect.

Currently, the diagnostic criterion for muscular dystrophy is muscle biopsy, in which the improvement of muscular dystrophy is ultimately determined by observing muscle fibrosis and inflammation levels. The degree of fibrosis can be assessed by counting the number of centrally nucleated fibers in muscle tissue using HE staining. In normal muscle fibers, the intercellular space is regular, nuclei are peripherally located, and there is no inflammatory cell infiltration. Fibrotic muscle exhibits hypertrophy and atrophy, resulting in variable muscle fiber size, rounded contours of hypertrophic fibers, internal migration of myonuclei, and extensive inflammatory cell infiltration around damaged cells. Regenerating muscle fibers with centrally located nuclei are almost entirely absent in undamaged muscle and can therefore serve as a marker of previous necrosis and regeneration cycles.

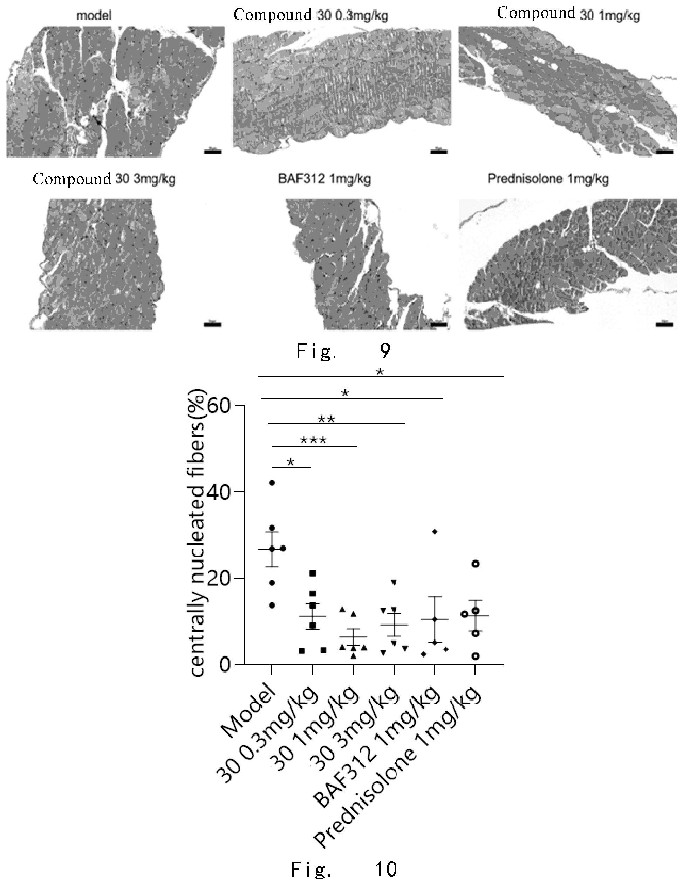

Representative images of the centrally nucleated fibers in double-mutant mice are shown in Figure 9, and the statistics of centrally nucleated fibers are shown in Figure 10.

The degree of muscle fibrosis can be determined by counting the number of centrally nucleated fibers in the muscle. Statistical results showed that the model group exhibited the highest percentage of centrally nucleated fibers. After treatment, all treatment groups significantly reduced the number of centrally nucleated fibers. Compound 30 at 1 mg/kg produced the greatest reduction in centrally nucleated fibers, which was stronger than that of BAF312 1 mg/kg and prednisolone 1 mg/kg, indicating that the therapeutic effect of Compound 30 at 1 mg/kg was superior to BAF312 1 mg/kg and prednisolone 1 mg/kg. Moreover, the reduction magnitude of Compound 30 at 1 mg/kg was higher than that at 3 mg/kg with no significant difference in therapeutic effects, demonstrating that Compound 30 can achieve therapeutic efficacy at 1 mg/kg.

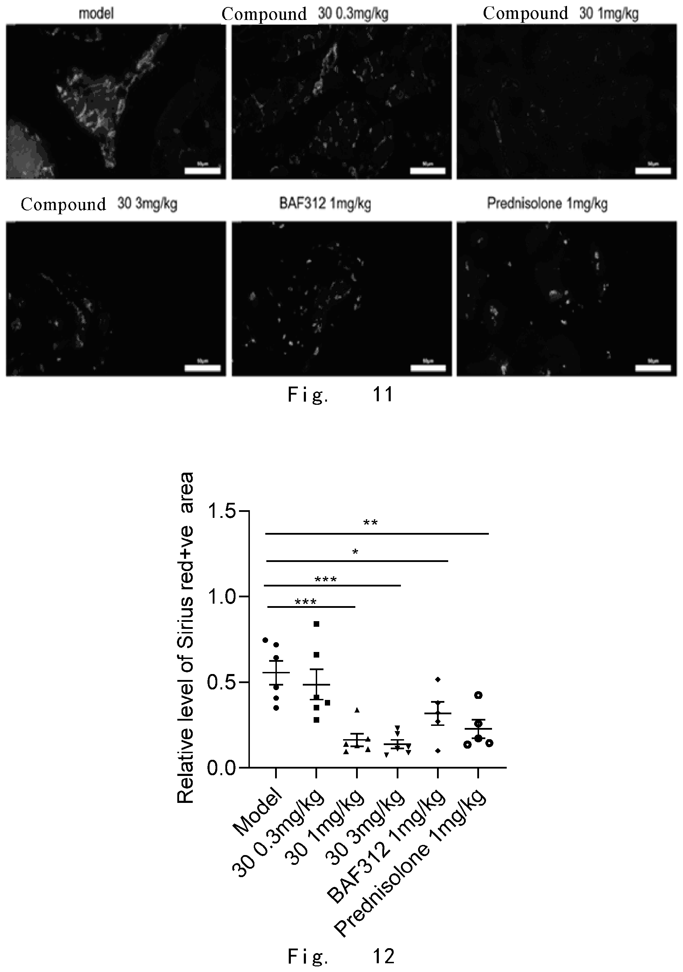

Representative images of collagen area in double-mutant mice are shown in Figure 11; and the statistics of collagen area are shown in Figure 12.

Another indicator for assessing the degree of fibrosis is collagen area. Compared with healthy individuals, patients with muscular dystrophy exhibit collagen deposition, resulting in a significantly increased proportion of collagen area in muscle. Experimental statistical results showed that the model group exhibited the highest collagen area ratio. After administration, the groups treated with Compound 30 at 1 mg/kg, 3 mg/kg, BAF312 at 1 mg/kg, and prednisolone at 1 mg/kg significantly reduced collagen deposition.

Based on a comprehensive analysis of the number of centrally nucleated fibers and the proportion of collagen deposition, Compound 30 1 mg/kg, Compound 30 3 mg/kg, BAF312 1 mg/kg, and Prednisolone 1 mg/kg groups significantly ameliorated fibrosis in double-mutant mice. The therapeutic effect of Compound 30 at 1 mg/kg was stronger than that of Prednisolone at 1 mg/kg, indicating that Compound 30 at 1 mg/kg is sufficient to achieve a therapeutic effect.

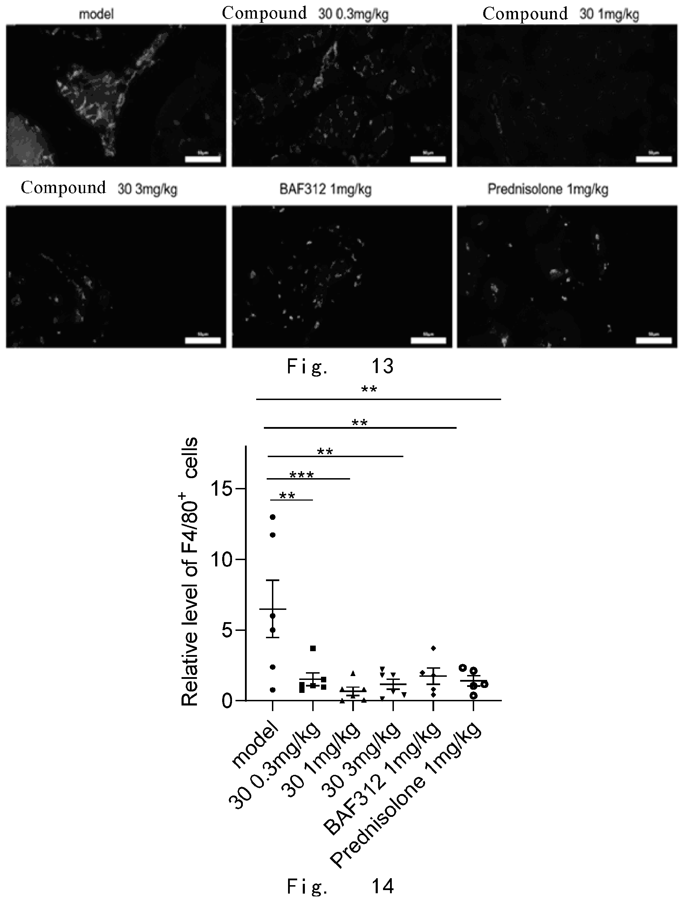

Representative immunofluorescence images of macrophages in double-mutant mice are shown in Figure 13; and statistical graphs of macrophage immunofluorescence are shown in Figure 14.

One of the typical pathological features of muscular dystrophy is muscle inflammation. After the treatment in double-mutant mice, all drug-treated groups exhibited significantly reduced macrophage content and alleviated muscle inflammation, with the 1 mg/kg Compound 30 group showing the greatest reduction.

Based on all the above results, Compound 30 at 1 mg/kg can significantly improve the pathological features of double-mutant mice, including reducing creatine kinase levels, decreasing fibrosis, and alleviating muscle inflammation, thereby exerting a therapeutic effect. Its therapeutic efficacy is stronger than that of the positive drug prednisolone at 1 mg/kg, indicating that Compound 30 is expected to become a new compound for treating muscular dystrophy.

### Example 7. In Vivo Pharmacodynamic Test of Duchenne Muscular Dystrophy (Compound 30)

To verify the application potential of Compound 30 in other myopathy models, we further selected the Duchenne muscular dystrophy model for *in vivo* efficacy evaluation. The experimental protocol and results are as follows:

### 1. Experimental materials

### Experimental Animals and Grouping

Experimental Animals: A total of 35 Duchenne muscular dystrophy model mice were divided into 6 groups as shown below.

| Group No. | Grouping | No. of animal | Route of administration | Frequency of administration |
|---|---|---|---|---|
| 1 | Model | 6 | intragastric administration | Once daily |
| 2 | Prednisolone 1 mg/kg | 5 | intragastric administration | Once daily |
| 3 | BAF312 1 mg/kg | 6 | intragastric administration | Once daily |
| 4 | **30** 0.3 mg/kg | 6 | intragastric administration | Once daily |
| 5 | **30** 1 mg/kg | 6 | intragastric administration | Once daily |
| 6 | **30** 3 mg/kg | 6 | intragastric administration | Once daily |

### Main Reagents and Drugs

Positive control prednisone (1 mg/kg): Duchenne muscular dystrophy is the most prevalent subtype of muscular dystrophy and also the most extensively studied and reported among all subtypes. Currently approved treatments for Duchenne muscular dystrophy include antiinflammatory glucocorticoids and gene therapy.

### 2. Experimental Methods

Intragastric administration was initiated from Day 0. Body weight was measured daily during the experiment to adjust the drug dosage. Grip strength tests were performed every 2 days to observe changes in the mice's grip strength until the experiment concluded on Day 21. Changes in the limb grip strength, serum creatine kinase content, and muscle fibrosis/inflammation in the mice were detected as indicators for evaluating drug efficacy.

### 2.1 Tested items After Treatment

### 2.1.1 Limb Grip Strength Test

The mouse was placed horizontally on the grip test board. The mouse's tail was held and pulled gently in the opposite direction without using excessive force. The tail was gradually pulled until the mouse released the grip force sensor. The maximum value recorded by the grip force sensor is the grip strength. The test was repeated for 3 times and the average value was taken.

### 2.1.2 Detection of Serum Creatine Kinase Content

After the completion of administration, blood was collected via the orbital sinus, allowed to stand for 30 minutes, centrifuged at 3000 rpm at 4°C for 15 minutes, and serum was extracted, stored frozen at -20°C, and detected in accordance with the kit instructions.

### 2.1.3 Pathological Examination of Muscle Tissue Sections

After the experiment, the tibialis anterior, extensor digitorum longus, and diaphragm of the mice were harvested, fixed, sectioned, and subjected to HE staining and Sirius Red staining to observe the degree of muscle fibrosis. The slice was subjected to immunofluorescence staining with F4/80 to observe the level of muscle inflammation.

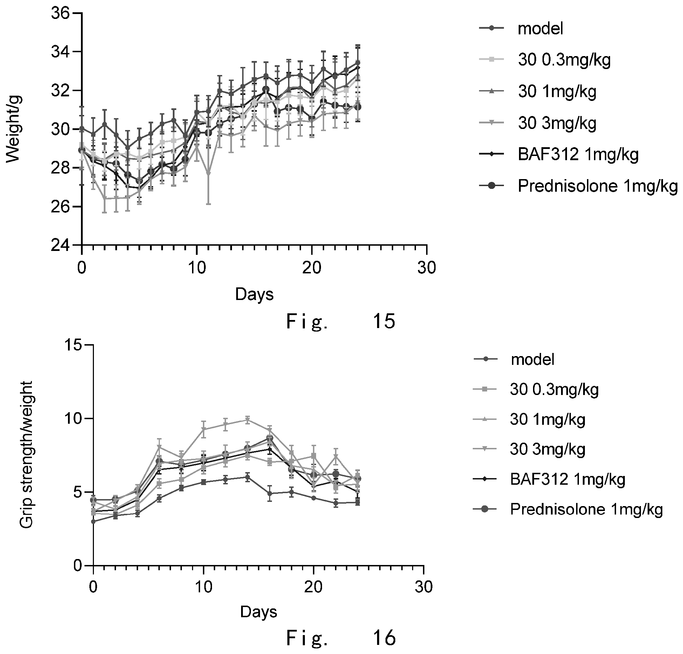

Changes in the body weight of Duchenne muscular dystrophy model mice after administration are shown in Figure 15.

After administration, there was no difference in the body weight growth trend in the BAF312 1 mg/kg group, prednisolone group, and Compound 30 0.3 and 1 mg/kg groups for Duchenne muscular dystrophy model mice, compared with the model group. The body weight growth trend in the Compound 30 3 mg/kg group was relatively slow compared with the other groups.

The changes in grip strength of the Duchenne muscular dystrophy model mice after administration are shown in Figure 16.

After the administration in a mouse model of Duchenne muscular dystrophy, grip strength was measured every 2 days. The ratio of grip strength to body weight was calculated to represent changes in mouse grip strength, thereby eliminating the influence of body weight. In the first 14 days, grip strength increased in all groups, with the increasing rate in the treatment groups being higher than that in the model group. In particular, the increase in grip strength for Compound 30 at 3 mg/kg group was the most significant, demonstrating a favorable therapeutic effect. However, after 14 days, all groups began to exhibit a decline in grip strength. Analysis suggested that this might be due to excessively frequent grip strength measurements, as the mice had become accustomed to the test and no longer exhibited vigorous grasping resistance.

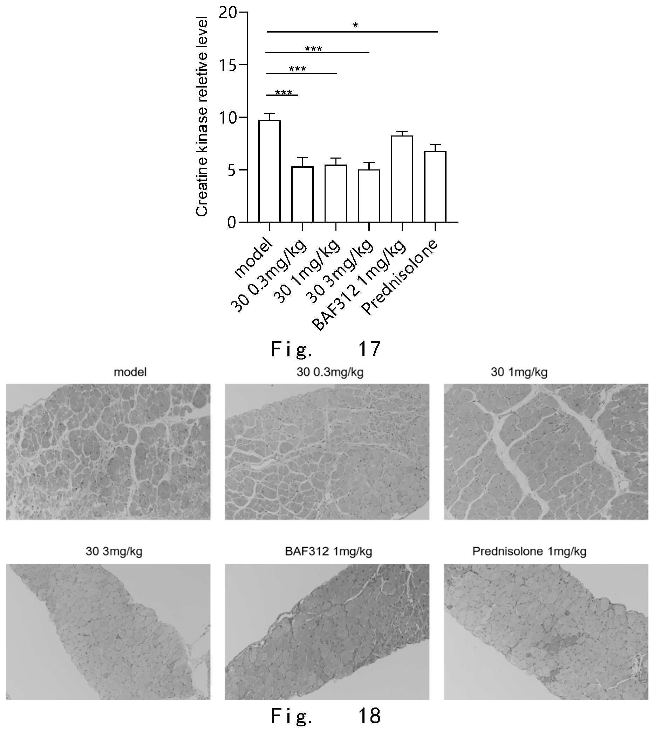

Changes in creatine kinase in a mouse model of Duchenne muscular dystrophy are shown in Figure 17.

The creatine kinase level in the serum of Duchenne muscular dystrophy model mice was significantly higher than that in healthy mice. After treatment, Compound 30 at 0.3 mg/kg, 1 mg/kg, 3 mg/kg and prednisolone at 1 mg/kg significantly reduced creatine kinase levels, while the BAF312 1 mg/kg group showed no such effect. Moreover, the reduction in the 30 1 mg/kg group was greater than that in the prednisolone 1 mg/kg group, indicating that the therapeutic effect of 30 at 1 mg/kg was superior to prednisolone.

Currently, the gold criterion for diagnosing muscular dystrophy is muscle biopsy, in which the improvement of muscular dystrophy is ultimately determined by observing muscle fibrosis and inflammation levels. The degree of fibrosis can be assessed by counting the number of centrally nucleated fibers in muscle tissue using HE staining. In normal muscle fibers, the intercellular space is regular, nuclei are peripherally located, and there is no inflammatory cell infiltration. Fibrotic muscle exhibits hypertrophy and atrophy, resulting in variable muscle fiber size, rounded contours of hypertrophic fibers, internal migration of myonuclei, and extensive inflammatory cell infiltration around damaged cells. Regenerating muscle fibers with centrally located nuclei are almost entirely absent in undamaged muscle and can therefore serve as a marker of previous necrosis and regeneration cycles.

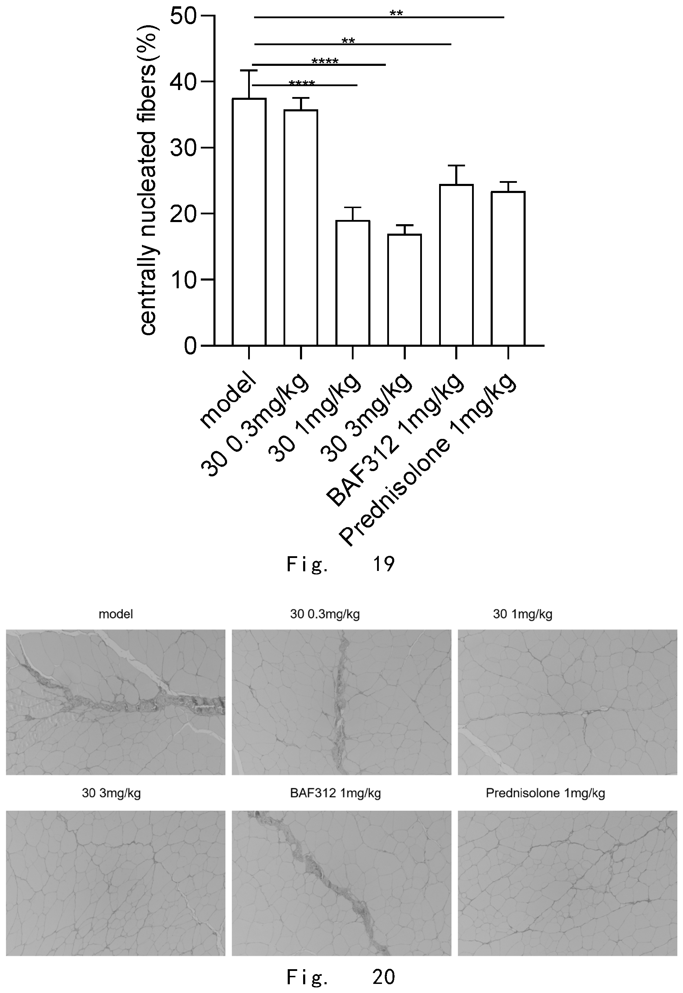

The centrally nucleated fibers of the Duchenne muscular dystrophy model mice are shown in Figure 18; the statistics of nucleated fibers are presented in Figure 19.

The degree of muscle fibrosis can be determined by counting the number of centrally nucleated fibers in muscle. The statistical results showed that the model group exhibited the highest percentage of centrally nucleated fibers. After the treatment, all the Compound 30 1 mg/kg, Compound 30 3 mg/kg, BAF312 1 mg/kg, and Prednisolone 1 mg/kg groups significantly reduced the number of centrally nucleated fibers, while the Compound 30 0.3 mg/kg group showed no significant difference compared with the model group. The Compound 30 1 mg/kg and Compound 30 3 mg/kg groups exhibited the greatest reduction in centrally nucleated fibers, which was stronger than that in the BAF312 1 mg/kg and Prednisolone 1 mg/kg groups, indicating that the therapeutic effects of Compound 30 1 mg/kg and Compound 30 3 mg/kg were superior to those of BAF312 1 mg/kg and Prednisolone 1 mg/kg. Moreover, there was no difference in the therapeutic effects between Compound 30 1 mg/kg and Compound 30 3 mg/kg, suggesting that Compound 30 at 1 mg/kg is sufficient to achieve the therapeutic effect.

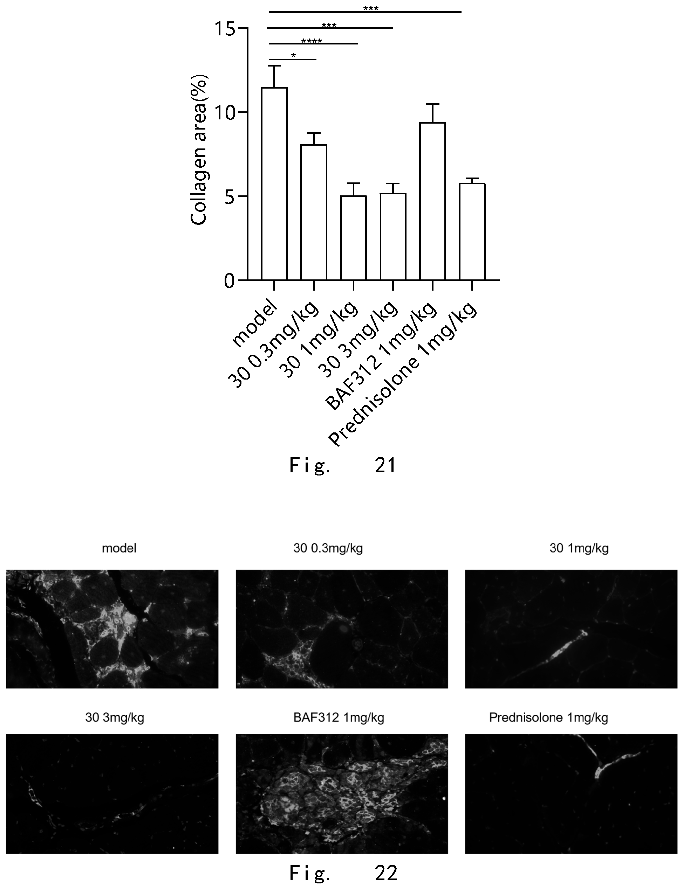

The collagen area of the Duchenne muscular dystrophy model mice is shown in Figure 20; the collagen area statistics are shown in Figure 21.

Another indicator for assessing the fibrosis degree is collagen area. Compared with healthy individuals, patients with muscular dystrophy exhibit collagen deposition, resulting in a significantly increased proportion of collagen area in muscle. Experimental statistical results showed that the model group had the highest collagen area ratio. After the treatment, the Compound 30 at 0.3 mg/kg, 1 mg/kg, 3 mg/kg and prednisolone at 1 mg/kg groups significantly reduced collagen deposition. The BAF312 1 mg/kg group showed no difference compared with the model group, indicating that it could not reduce collagen deposition. Compound 30 at 1 mg/kg and 3 mg/kg exerted a stronger effect on reducing collagen deposition than prednisolone at 1 mg/kg, and there was no difference in the effect between Compound 30 1 mg/kg and 3 mg/kg.

By combining the number of centrally nucleated fibers and the proportion of collagen deposition, it can be seen that the Compound 30 1 mg/kg, 3 mg/kg and Prednisolone 1 mg/kg groups significantly ameliorated the fibrosis degree in Duchenne muscular dystrophy mice, while the BAF312 1 mg/kg group showed a poor improvement effect on muscle fibrosis. The therapeutic effects of Compound 30 at 1 mg/kg and 3 mg/kg were stronger than that of Prednisolone 1 mg/kg, and there was no difference between Compound 30 1 mg/kg and 3 mg/kg, indicating that Compound 30 at 1 mg/kg is sufficient to achieve a therapeutic effect.

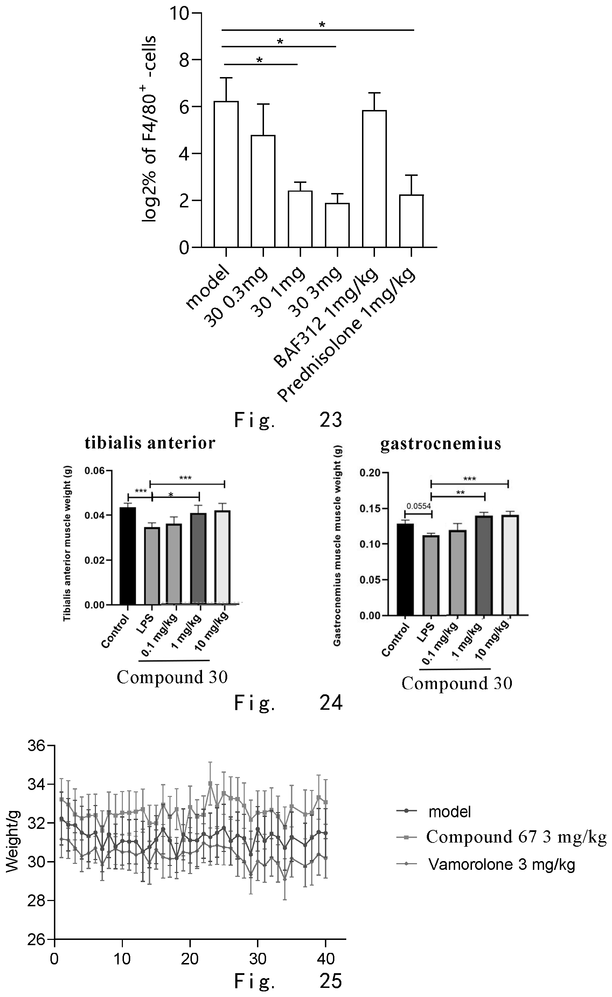

Representative immunofluorescence images of macrophages in a Duchenne muscular dystrophy mouse model are shown in Figure 22; statistical graphs of macrophage immunofluorescence are shown in Figure 23.

One of the typical pathological features of Duchenne muscular dystrophy is muscle inflammation. Glucocorticoids, the first-line drugs for treating Duchenne muscular dystrophy, exert therapeutic effects by reducing muscle inflammation. After the administration, the Compound 30 1 mg/kg, 3 mg/kg and prednisolone 1 g/kg groups significantly reduced macrophage content and alleviated muscle inflammation in Duchenne muscular dystrophy model mice. The BAF312 1 mg/kg group showed no improvement in muscle inflammation.

Based on all the above results, Compound 30 at 1 mg/kg and 3 mg/kg, as well as prednisolone at 1 mg/kg, can significantly improve the pathological characteristics of Duchenne muscular dystrophy model mice, including reducing creatine kinase levels, decreasing fibrosis, and alleviating muscle inflammation, thereby exerting therapeutic effects. Moreover, the therapeutic effects of Compound 30 at 1 mg/kg and 3 mg/kg are superior to those of the positive drug prednisolone at 1 mg/kg, indicating that Compound 30 is expected to become a novel compound for treating Duchenne muscular dystrophy, which further demonstrates the research value of selective S1PR4 agonists in the treatment of myopathy.

### Example 8. Pharmacodynamic Evaluation of Compound 30 on Sepsis-Induced Myopathy

Sepsis-induced myopathy (SIM) is defined as a rapidly progressive muscle disorder that can affect the electrophysiological and morphological features of muscles, simultaneously involving both respiratory and limb muscles. Approximately 40% of patients with severe sepsis develop intensive care unit-acquired weakness (ICUAW), characterized by loss of muscle mass, reduction in muscle fiber size, and decreased muscle strength, ultimately leading to persistent physical impairment in patients.

Animal models of sepsis can be established by injecting exogenous toxins into animals. In this study, a sepsis model was established via intraperitoneal injection of lipopolysaccharide to observe the effects of compounds on the occurrence and development of acquired weakness. This is a widely used method for modeling sepsis. After injection of lipopolysaccharide, mice showed increased severity of sepsis, decreased forelimb grip strength, reduced amplitude and prolonged latency of compound muscle action potential (CMAP), as well as decreased muscle fiber diameter and cross-sectional area, indicating successful establishment of a mouse model of sepsis-induced acquired weakness.

### Experimental method

A skeletal muscle atrophy model induced by lipopolysaccharide can simulate clinical muscle atrophy caused by sepsis.

Mice were given a single intraperitoneal injection of LPS (1 mg/kg), followed by 14 consecutive days of intraperitoneal injection of either vehicle (1% DMSO dissolved in physiological saline) or Compound 30. Body weight was measured daily. After 14 days of LPS treatment, the mice were anesthetized, and the gastrocnemius and tibialis anterior muscles were harvested.

### Experimental materials

### 2.1 Experimental Animals and Grouping

| Group No. | Grouping | No. of animal | Route of administration | Frequency of administration |
|---|---|---|---|---|
| 1 | Control group | 8 | No administration | No administration |
| 2 | Model group | 8 | No administration | No administration |
| 3 | LPS + Compound **30** (0.1mg/kg) | 8 | intraperitoneal injection | Once daily |
| 4 | LPS + Compound **30** (1mg/kg) | 8 | intraperitoneal injection | Once daily |
| 5 | LPS + Compound **30** (10mg/kg) | 8 | intraperitoneal injection | Once daily |

### 2.2 Main reagents and drugs

### LPS, Compound 30;

### 3. Experimental results

A single intraperitoneal injection of LPS (1 mg/kg) was administered. 14 days after injection, the survival rate of the control group was 100% (8 survived/8 total), that of the LPS group was 50% (4 survived/8 total), that of the high-dose Compound 30 group was 87.5% (7 survived/8 total), that of the medium-dose Compound 30 group was 50% (4 survived/8 total), and that of the low-dose Compound 30 group was 75%.

The effects of Compound 30 on the tibialis anterior and gastrocnemius muscles in a LPS-induced sepsis model are shown in Figure 24.

### Results:

After 14 days of LPS treatment, the mice were anesthetized, and the gastrocnemius and tibialis anterior muscles were harvested. Compared with the control group, the gastrocnemius and tibialis anterior muscles in the model group showed atrophy, and medium and high doses of Compound 30 can significantly inhibit the atrophy of the tibialis anterior muscle.

### Example 9. Pharmacodynamic Evaluation of In Vivo Efficacy of Duchenne Muscular Dystrophy (Compound 67) in mdx Mice

### 1. Experimental materials

### 1.1 Experimental Animals and Grouping

Experimental animals: A total of 18 Duchenne muscular dystrophy model mice were divided into 3 groups with 6 mice in each group.

| Group No. | Grouping | No. of animal | Route of administrat ion | Frequency of administration |
|---|---|---|---|---|
| 1 | Model | 6 | intragastric administrat ion | Once daily |
| 2 | Compound **67** 3 mg/kg | 6 | intragastric administrat ion | Once daily |
| 3 | Vamorolone 20 mg/kg | 6 | intragastric administrat ion | Once daily |

### 1.2 Main reagents and drugs

Positive control drug Vamorolone (20 mg/kg): Duchenne muscular dystrophy is the most prevalent subtype of muscular dystrophy and also the most extensively studied and reported. Recently, Vamorolone, a steroid drug approved by the U.S. FDA for marketing, is indicated for the treatment of Duchenne muscular dystrophy, while reducing the occurrence of side effects during treatment.

### Main Instruments and Equipment

### Grip Strength Meter

### Shanghai Xinruan Information Technology Co., Ltd.

### 2. Experimental method

Intragastric administration was initiated on Day 0. Body weight was measured daily during the period to adjust the drug dosage. Grip strength and rotarod tests were performed on Day 20 and Day 42. Changes in the limb grip strength, exercise duration, and muscle fibrosis/inflammation in mice were detected as indicators for evaluating drug efficacy.

### 2.1 Test items after treatment

### 2.1.1 Limb grip strength test

The mouse was placed horizontally on the grip test board. The mouse's tail was held and pulled gently in the opposite direction without using excessive force. The tail was gradually pulled until the mouse released the grip force sensor. The maximum value recorded by the grip force sensor is the grip strength. The test was repeated for 3 times and the average value was taken.

### 2.1.2 Rotarod test

The rotarod test was conducted on Day 0, Day 20, and Day 42 respectively. The test was performed at an accelerating speed (0-16 rpm), reaching the maximum speed after 300 seconds, with a maximum test duration of 600 seconds. The time until the animal fell off was recorded⁷.

### 2.1.3 Pathological examination of muscle tissue sections

After the experiment, the tibialis anterior, extensor digitorum longus, and diaphragm of the mice were harvested, fixed, sectioned, and subjected to HE staining and Sirius Red staining to observe the degree of muscle fibrosis.

### Experimental results:

After treatment in Duchenne muscular dystrophy model mice, there was no significant difference in body weight changes among each treatment group compared with the model group (Figure 25).

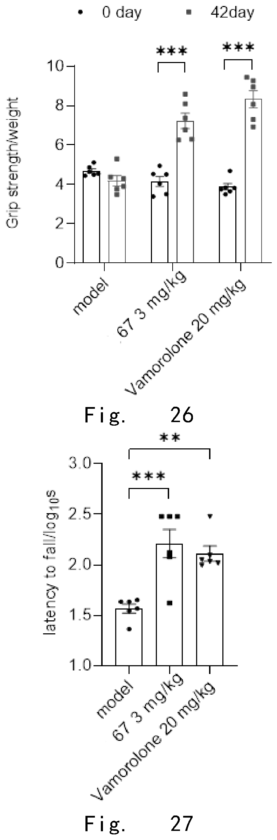

After 42 days of treatment, compared with the model group, the grip strength of mice in each administration group increased significantly. Compound 67 at 3 mg/kg and Vamorolone at 20 mg/kg resulted in a marked increase in grip strength, demonstrating favorable therapeutic effects (Figure 26).

The rotarod duration can be used to evaluate the exercise endurance of animals. On Day 42 after administration, compared with the model group, the rotarod time of each administration group increased to some extent. Grip strength was significantly elevated in the 3 mg/kg Compound 67 group and the 20 mg/kg Vamorolone group, indicating favorable therapeutic effects. Moreover, the increase in grip strength was more pronounced in the 3 mg/kg Compound 67 group than in the 20 mg/kg Vamorolone group (Figure 27).

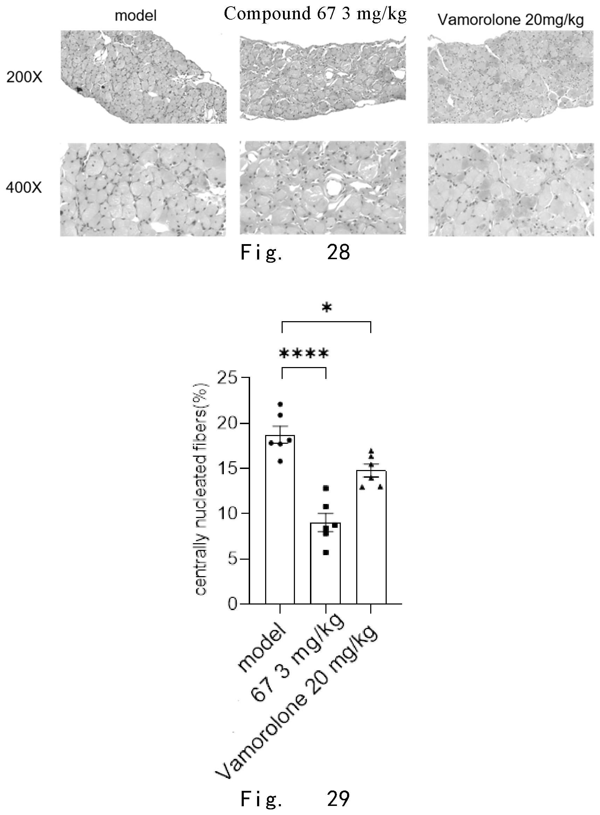

Currently, the gold criterion for diagnosing muscular dystrophy is muscle biopsy, in which the improvement of muscular dystrophy is ultimately determined by observing muscle fibrosis and inflammation levels. The degree of fibrosis can be assessed by counting the number of centrally nucleated fibers in muscle tissue using HE staining. In normal muscle fibers, the intercellular space is regular, nuclei are peripherally located, and there is no inflammatory cell infiltration. Fibrotic muscle exhibits hypertrophy and atrophy, resulting in variable muscle fiber size, rounded contours of hypertrophic fibers, internal migration of myonuclei, and extensive inflammatory cell infiltration around damaged cells. Regenerating muscle fibers with centrally located nuclei are almost entirely absent in undamaged muscle and can therefore serve as a marker of previous necrosis and regeneration cycles. The centrally nucleated fibers after administration of Compound 67 and Vamorolone are shown in Figure 28. The statistics of nucleated fibers after administration of Compound 67 and Vamorolone are shown in Figure 29.

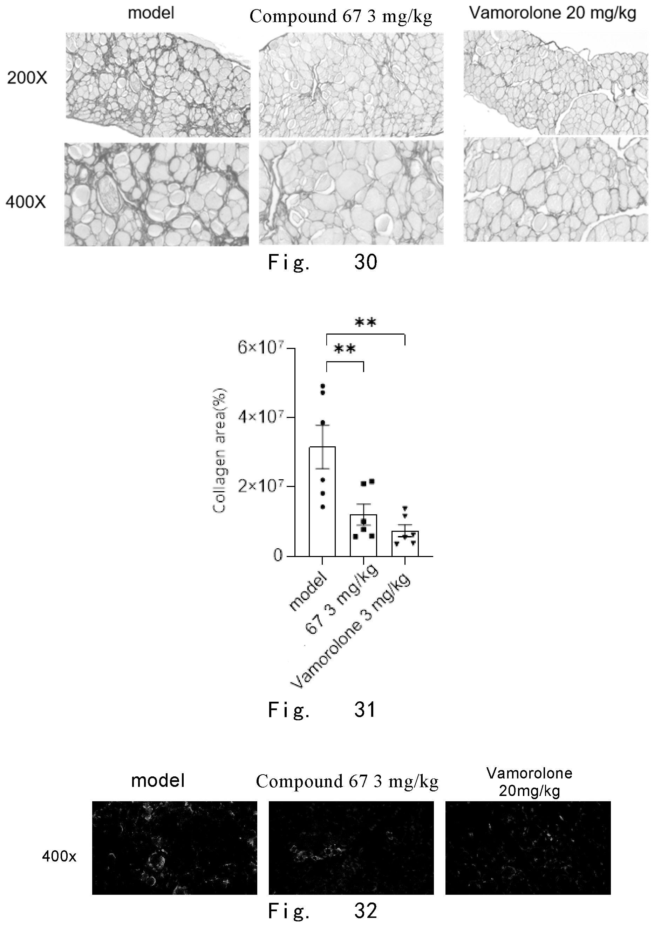

The degree of muscle fibrosis can be determined by counting the number of centrally nucleated fibers in the muscle. Statistical results showed that the model group exhibited the highest percentage of centrally nucleated fibers. After treatment, Compound 67 at 3 mg/kg and Vamorolone at 20 mg/kg significantly reduced the number of centrally nucleated fibers. The Compound 67 3 mg/kg group exhibited the greatest reduction in centrally nucleated fibers, surpassing both the BAF312 1 mg/kg group and the Vamorolone 20 mg/kg group, indicating that the therapeutic effect of Compound 67 at 3 mg/kg was superior to that of Vamorolone at 20 mg/kg. The collagen area following administration of Compound 67 and Vamorolone is shown in Figure 30.

Another indicator for assessing the fibrosis degree is collagen area. Compared with healthy subjects, patients with muscular dystrophy exhibit collagen deposition, resulting in a significantly increased proportion of collagen area in muscle. Experimental statistical results showed that the model group exhibited the highest collagen area ratio. After administration, Compound 67 at 3 mg/kg and Vamorolone at 20 mg/kg groups significantly reduced collagen deposition. The effect of Compound 67 at 3 mg/kg on reducing collagen deposition was comparable to that of Vamorolone at 20 mg/kg. Combining the number of centrally nucleated fibers and the proportion of collagen deposition, it can be concluded that treatment with Compound 67 at 3 mg/kg and Vamorolone at 20 mg/kg significantly ameliorated fibrosis in mice with Duchenne muscular dystrophy. The collagen area statistics following the administration of Compound 67 and Vamorolone are shown in Figure 31.

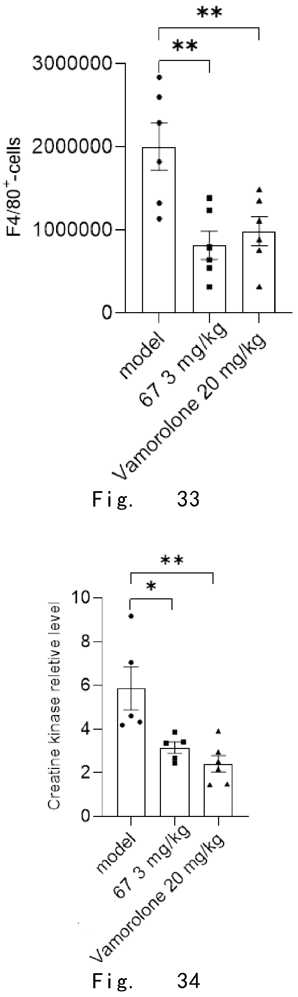

One of the typical pathological features of Duchenne muscular dystrophy is muscle inflammation. Glucocorticoids, the first-line drugs for treating Duchenne muscular dystrophy, exert therapeutic effects by reducing muscle inflammation. After administration in Duchenne muscular dystrophy model mice, Compound 67 3 mg/kg and Vamorolone 20 mg/kg groups significantly reduced macrophage levels and alleviated muscle inflammation. Furthermore, there was no significant difference in the reduction of F4/80 between the Compound 67 3 mg/kg and Vamorolone 20 mg/kg groups, indicating comparable effects in improving muscle inflammation. The levels of F4/80 macrophage marker (muscle inflammation) after the administration of Compound 67 and Vamorolone are shown in Figure 32. The statistical analysis of F4/80 macrophage marker levels (muscle inflammation) after the administration of Compound 67 and Vamorolone is shown in Figure 33.

The serum creatine kinase level in the Duchenne muscular dystrophy model mice was significantly higher than that in healthy mice. After treatment, the Vamorolone 20 mg/kg group and Compound 67 3 mg/kg group significantly reduced the creatine kinase level. Moreover, the creatine kinase-lowering effect in the Compound 67 3 mg/kg group was stronger than that in the Vamorolone 20 mg/kg group, indicating that the therapeutic effect in the Compound 67 3 mg/kg group was superior to that in the Vamorolone 20 mg/kg group. The statistics of serum creatine kinase after the administration of Compound 67 and Vamorolone are shown in Figure 34.

Based on all the above results, Compound 67 at 3 mg/kg and Vamorolone at 20 mg/kg can significantly improve the symptoms and pathological characteristics in Duchenne muscular dystrophy model mice, including reducing fibrosis levels, alleviating muscle inflammation, and lowering serum creatine kinase content, thereby achieving therapeutic effects.

### Discussion:

Through extensive and in-depth research, the inventors first confirmed that S1PR4 and NDUFA10 play important roles in the pathogenesis of muscular dystrophy, providing relevant new targets for treating muscular dystrophy. Furthermore, a series of small-molecule S1P receptor regulators not previously reported in the literature were designed and synthesized. Cell-level activity assays were performed on the obtained compounds, yielding a number of compounds capable of regulating S1PR4 and the S1PR4 mutant protein. In addition, *in vivo* pharmacokinetic parameters were determined for the most potent S1PR4 agonist, which was found to possess favorable oral absorption properties and certain development potential. In internalization function assays, Compounds 30 and 67 exhibited sustained internalization capacity, which is unattainable by non-selective agonists. In the Duchenne muscular dystrophy mouse model and the S1PR4-R82C/NDUFA10-R217W muscular dystrophy mouse model, Compounds 30 and 67 demonstrated significantly superior *in vivo* efficacy compared to the positive control prednisone and BAF312, further validating the significant value of targeting S1PR4 in the treatment of muscular dystrophic diseases, as well as the remarkable ameliorative and therapeutic effects of Compounds 30 and 67 against diseases caused by S1PR4 and S1PR4 mutations. Meanwhile, in the sepsis model induced by LPS treatment, medium and high doses of Compounds 30 and 67 significantly inhibited tibialis anterior muscle atrophy.

The above work lays the foundation for treating muscular dystrophy diseases mediated by the S1PR4 receptor and other myopathies.

All documents mentioned in the present invention are incorporated herein by reference as if each document were individually incorporated by reference. Furthermore, it should be understood that after reading the above teachings of the present invention, a skilled person may make various alterations or modifications to the present invention, and such equivalent forms shall also fall within the scope defined by the appended claims of the present application.

## Claims

1. A compound of formula I, or an optical isomer or a pharmaceutically acceptable salt thereof: 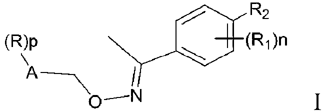
A is selected from: a substituted or unsubstituted C₅-C₈ aromatic ring (such as phenyl) or C₈-C₁₄ fused aromatic ring (such as naphthyl), substituted or unsubstituted five-membered or six-membered heterocyclic groups containing 1-3 heteroatoms selected from N or O;
R is independently selected from the group consisting of a hydrogen, halogen, cyano, substituted or unsubstituted C₁-C₁₀ alkyl (for example, a methyl, trifluoromethyl, trifluoroethyl), substituted or unsubstituted C₃-C₈ cycloalkyl or cycloalkenyl, substituted or unsubstituted C₁-C₅ alkoxy, substituted or unsubstituted C₅-C₈ aryl (for example, phenyl), substituted or unsubstituted five-membered or six-membered heterocyclic group or heteroaryl containing 1-2 heteroatoms selected from N, O or S, substituted or unsubstituted C₁-C₁₀ alkyl formyl, substituted or unsubstituted C₅-C₈ aryl formyl; p is an integer from 1 to 3;
R¹ is selected from the group consisting of a hydrogen, halogen, cyano, substituted or unsubstituted C₁-C₅ alkoxy, substituted or unsubstituted C₁-C₁₀ alkyl, substituted or unsubstituted C₃-C₈ cycloalkyl, substituted or unsubstituted C₂-C₆ alkenyl, substituted or unsubstituted C₃-C₆ cycloalkenyl, substituted or unsubstituted C₃-C₈ lactone group, substituted or unsubstituted C₁-C₁₀ amide group, substituted or unsubstituted C₅-C₈ aryl, substituted or unsubstituted five-membered or six-membered heterocyclic group containing 1-2 heteroatoms selected from N, O or S, substituted or unsubstituted C₅-C₈ aromatic heterocyclic group;
n is an integer selected from 1-3;
R² is selected from the following group: 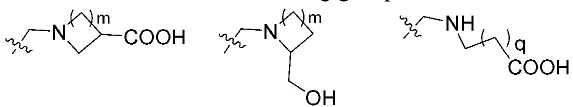
m is an integer selected from 1-4;
q is an integer selected from 1-2.

2. The compound of claim 1, wherein the compound is represented by the following formula II: 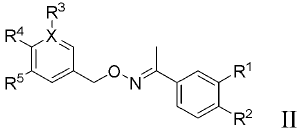 wherein,
R¹ is selected from the group consisting of a hydrogen, halogen (preferably F, Cl or Br), cyano, substituted or unsubstituted C₁-C₅ alkoxy (preferably methoxy), substituted or unsubstituted C₁-C₁₀ alkyl (preferably a substituted or unsubstituted C₁-C₆ alkyl, such as a methyl, ethyl, trifluoromethyl), substituted or unsubstituted C₃-C₈ cycloalkyl, substituted or unsubstituted C₂-C₆ alkenyl, substituted or unsubstituted C₃-C₆ cycloalkenyl, substituted or unsubstituted C₅-C₈ aryl, substituted or unsubstituted five-membered or six-membered heterocyclic group containing 1-2 heteroatoms selected from N, O or S, and substituted or unsubstituted C₅-C₈ aromatic heterocyclic group;
R² is selected from the following group: 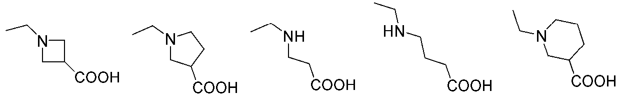
X is selected from C or N;
R³ is not present or is a substituent selected from the following group: a hydrogen, cyano, halogen, substituted or unsubstituted C₁-C₁₀ alkyl (for example, a methyl, trifluoromethyl, trifluoroethyl), substituted or unsubstituted C₃-C₈ cycloalkyl, cyano, substituted or unsubstituted C₁-C₅ alkoxy, substituted or unsubstituted C₁-C₁₀ alkyl formyl, substituted or unsubstituted C₅-C₈ aryl formyl, substituted or unsubstituted C₅-C₈ aryl, substituted or unsubstituted five-membered or six-membered heterocyclic group containing 1-2 heteroatoms selected from N, O or S;
R⁵ is selected from the group consisting of a hydrogen, cyano, halogen, substituted or unsubstituted C₁-C₁₀ alkyl (for example, a methyl, trifluoromethyl, trifluoroethyl), substituted or unsubstituted C₃-C₈ cycloalkyl, cyano, substituted or unsubstituted C₁-C₅ alkoxy, substituted or unsubstituted C₁-C₁₀ alkyl formyl, substituted or unsubstituted C₅-C₈ aryl formyl, substituted or unsubstituted C₅-C₈ aryl, substituted or unsubstituted five-membered or six-membered heterocyclic group containing 1-2 heteroatoms selected from N, O or S;
R⁴ is selected from the group consisting of a hydrogen, halogen, substituted or unsubstituted C₁-C₁₀ alkyl, substituted or unsubstituted C₃-C₈ cycloalkyl or cycloalkenyl, substituted or unsubstituted C₅-C₈ aryl, substituted or unsubstituted five-membered or six-membered heterocyclic group or heteroaryl groups containing 1-2 heteroatoms selected from N, O or S, substituted or unsubstituted C₁-C₁₀ alkyl formyl, and substituted or unsubstituted aryl formyl.

3. The compound of claim 1, wherein the compound is represented by the following formula III: 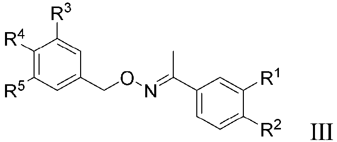 wherein,
R¹ is selected from the following group: a hydrogen, halogen (preferably F, Cl, or Br), cyano, substituted or unsubstituted C₁-C₅ alkoxy (preferably a methoxy), substituted or unsubstituted C₁-C₁₀ alkyl (preferably a substituted or unsubstituted C₁-C₆ alkyl, such as a methyl, ethyl, trifluoromethyl), substituted or unsubstituted C₃-C₈ cycloalkyl, substituted or unsubstituted C₂-C₆ alkenyl, substituted or unsubstituted C₃-C₆ cycloalkenyl, substituted or unsubstituted C₅-C₈ aryl, substituted or unsubstituted five-membered or six-membered heterocyclic group containing 1-2 heteroatoms selected from N, O, or S, substituted or unsubstituted C₅-C₈ aromatic heterocyclic group;
R² is selected from the following group: 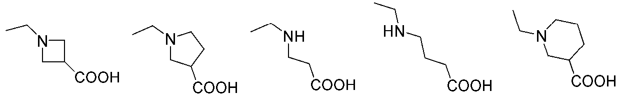
R³ is selected from the group consisting of a hydrogen, cyano, halogen, substituted or unsubstituted C₁-C₁₀ alkyl (for example, a methyl, trifluoromethyl, trifluoroethyl), substituted or unsubstituted C₃-C₈ cycloalkyl, cyano, substituted or unsubstituted C₁-C₅ alkoxy, substituted or unsubstituted C₁-C₁₀ alkyl formyl, substituted or unsubstituted C₅-C₈ aryl formyl, substituted or unsubstituted C₅-C₈ aryl, substituted or unsubstituted five-membered or six-membered heterocyclic group containing 1-2 heteroatoms selected from N, O or S;
R⁵ is selected from the following group: a hydrogen, cyano, halogen, substituted or unsubstituted C₁-C₁₀ alkyl (for example, a methyl, trifluoromethyl, trifluoroethyl), substituted or unsubstituted C₃-C₈ cycloalkyl, cyano, substituted or unsubstituted C₁-C₅ alkoxy, substituted or unsubstituted C₁-C₁₀ alkyl formyl, substituted or unsubstituted C₅-C₈ aryl formyl, substituted or unsubstituted C₅-C₈ aryl, substituted or unsubstituted five-membered or six-membered heterocyclic group containing 1-2 heteroatoms selected from N, O or S;
R⁴ is selected from the group consisting of a hydrogen, halogen, substituted or unsubstituted C₁-C₁₀ alkyl, substituted or unsubstituted C₃-C₈ cycloalkyl or cycloalkenyl, substituted or unsubstituted C₅-C₈ aryl, substituted or unsubstituted five-membered or six-membered heterocyclic group or heteroaryl containing 1-2 heteroatoms selected from N, O or S, substituted or unsubstituted C₁-C₁₀ alkyl formyl, and substituted or unsubstituted aryl formyl.

4. A compound selected from the following group, or an optical isomer or a pharmaceutically acceptable salt thereof:

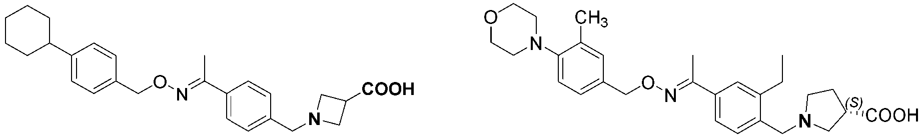

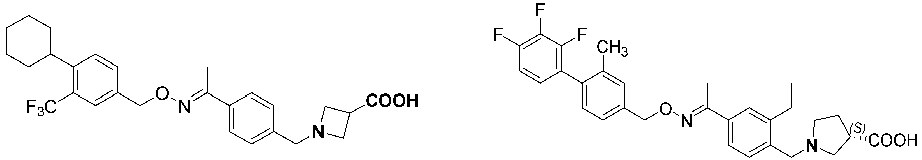

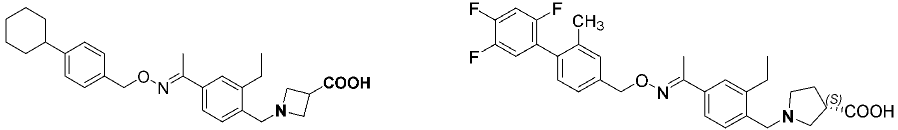

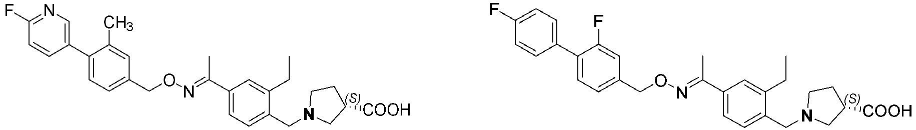

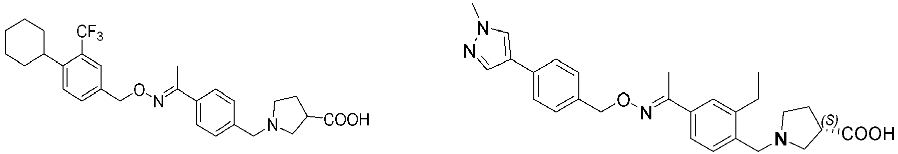

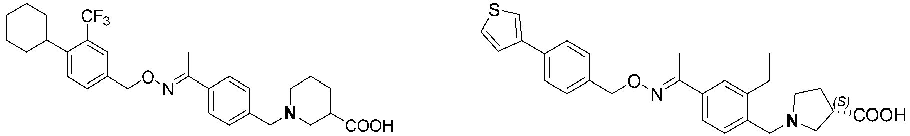

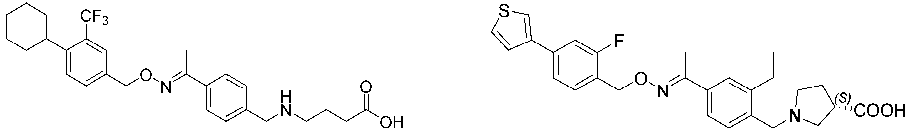

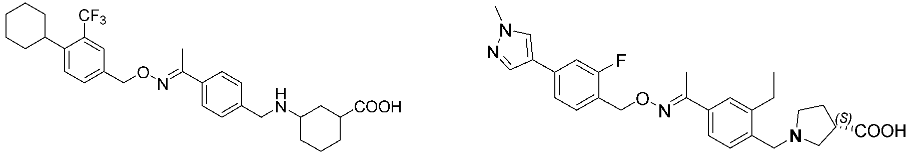

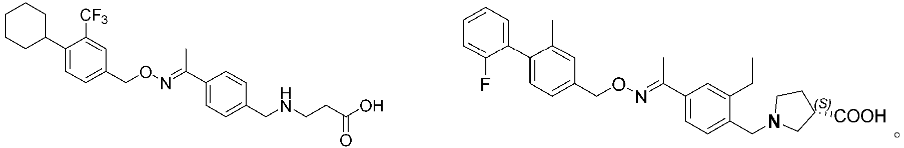

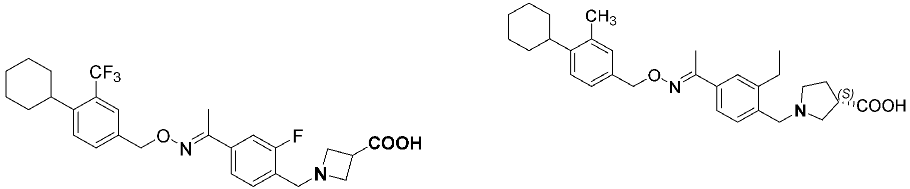

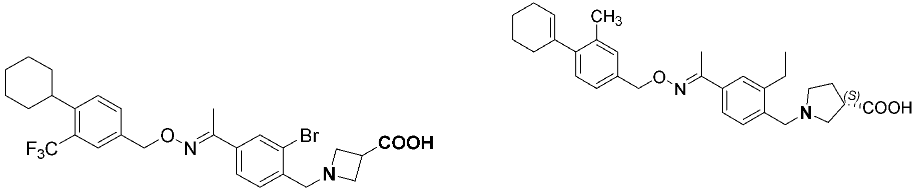

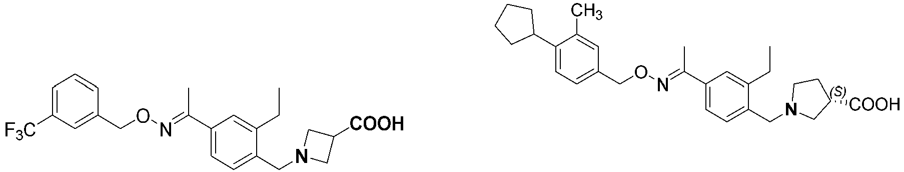

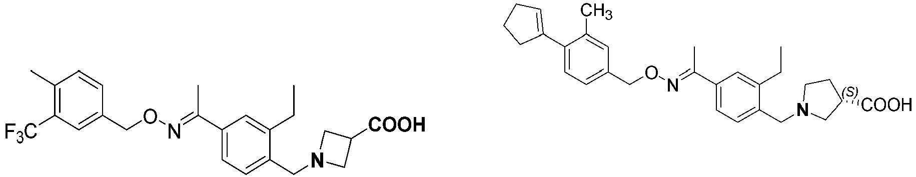

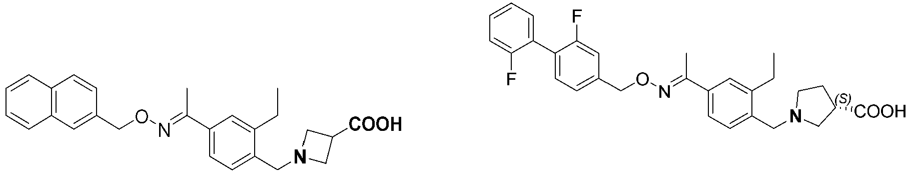

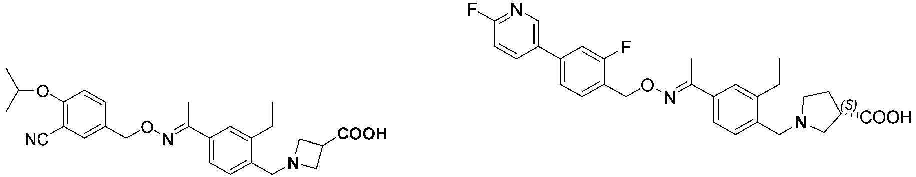

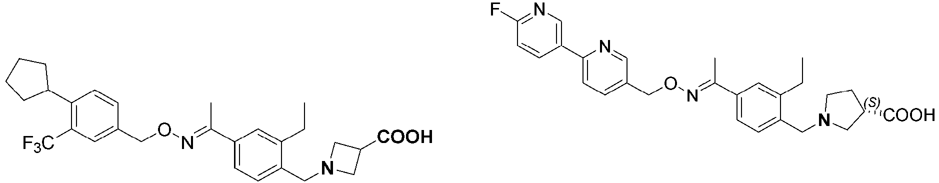

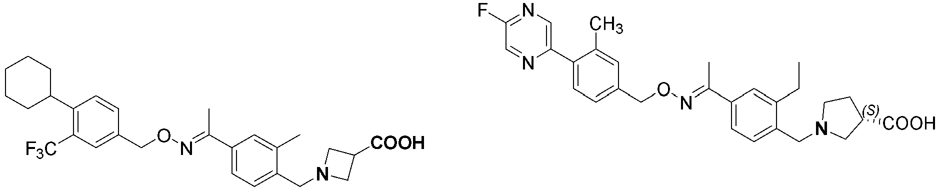

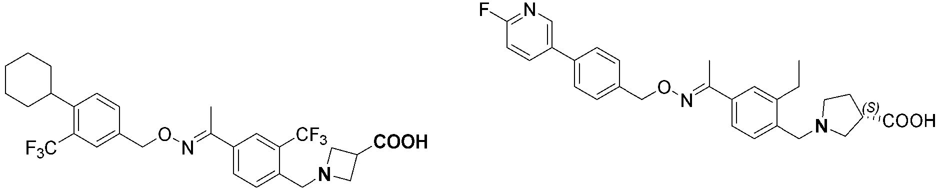

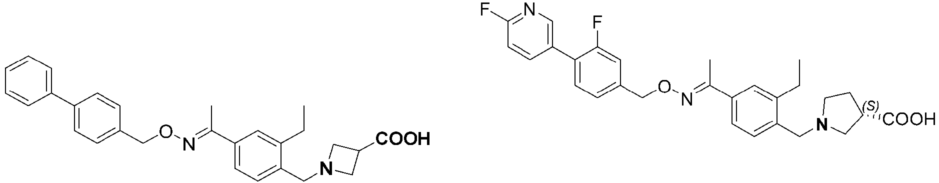

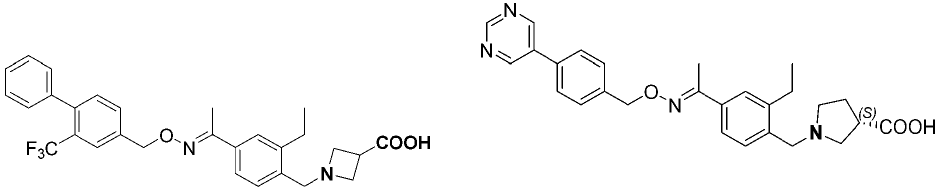

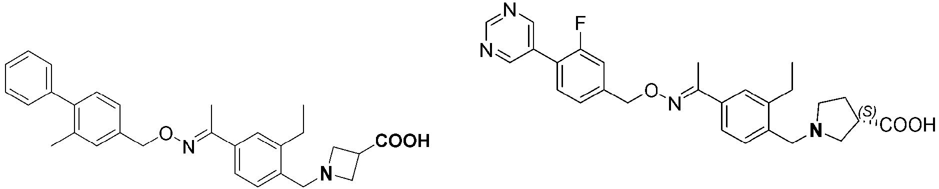

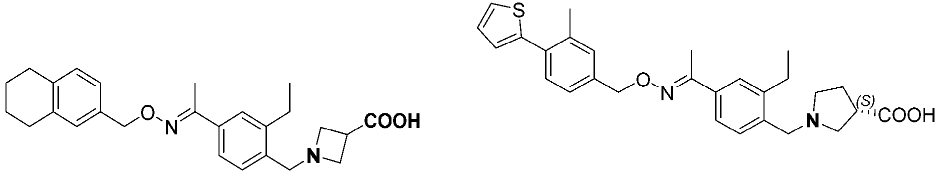

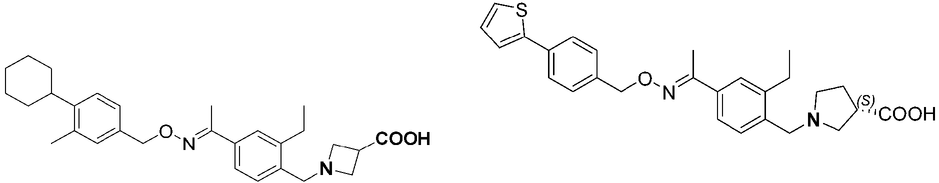

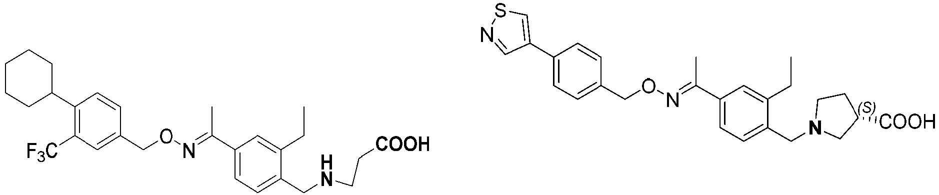

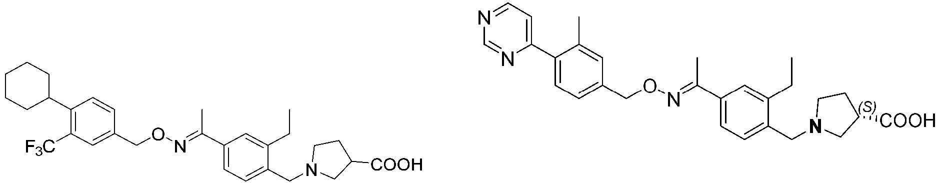

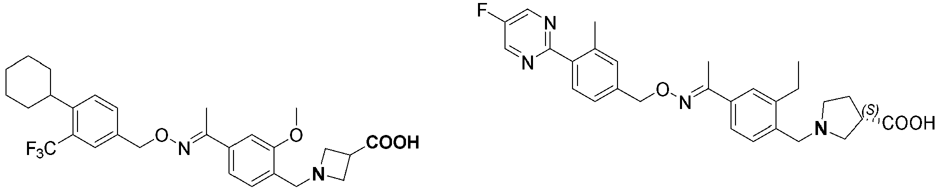

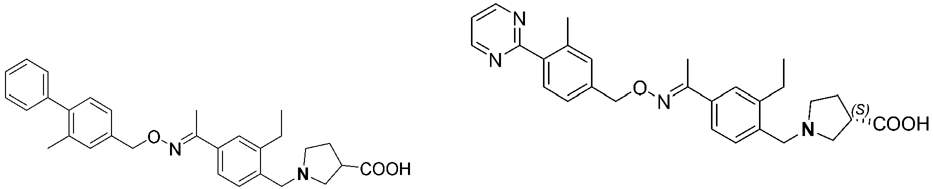

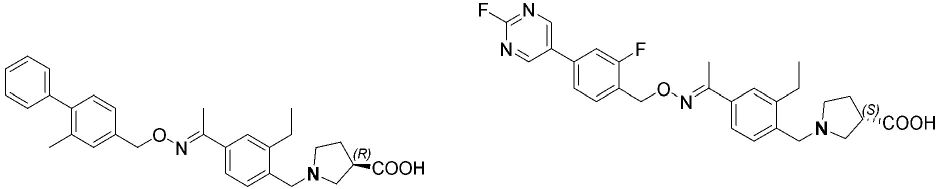

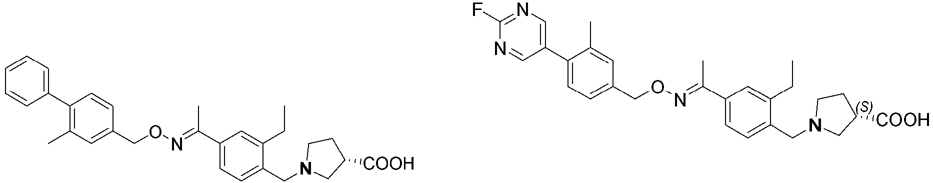

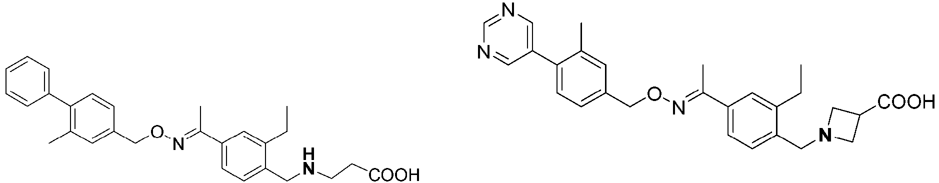

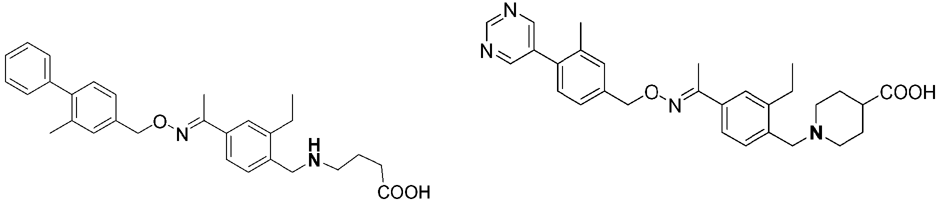

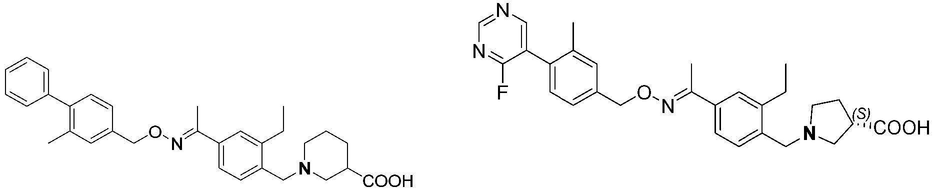

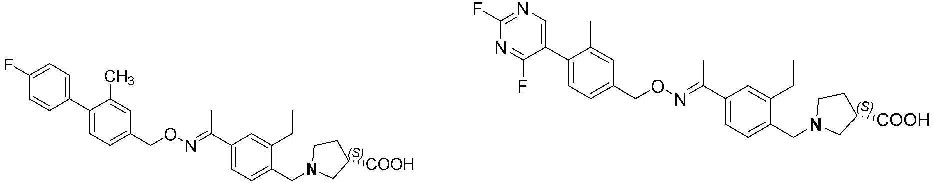

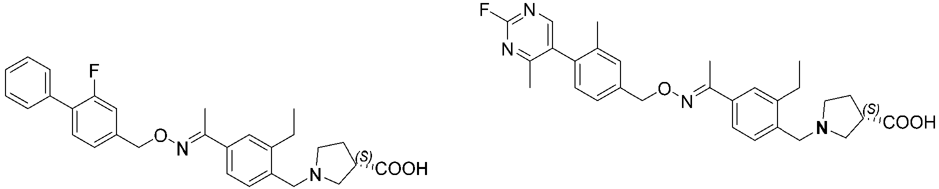

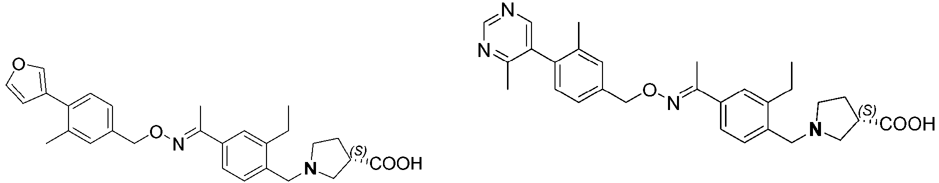

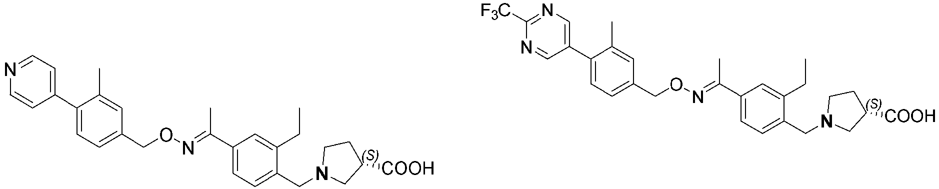

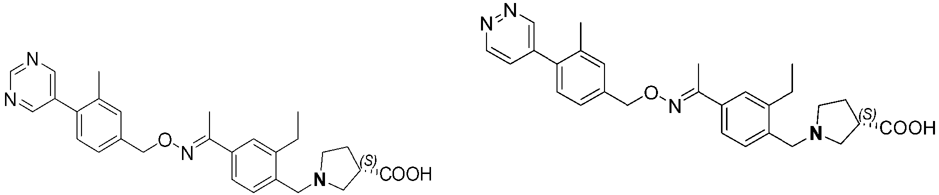

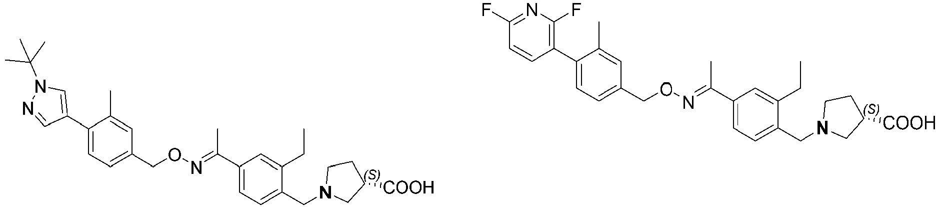

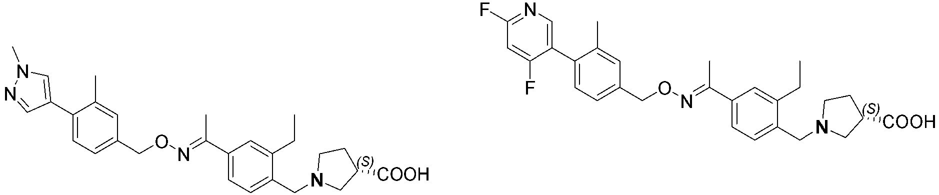

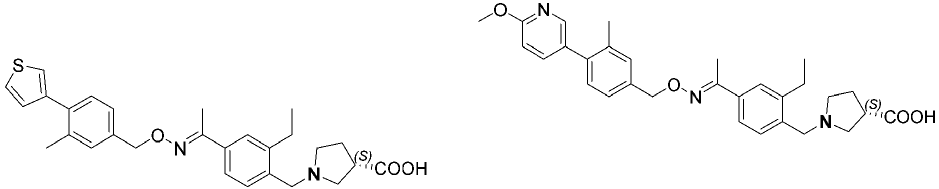

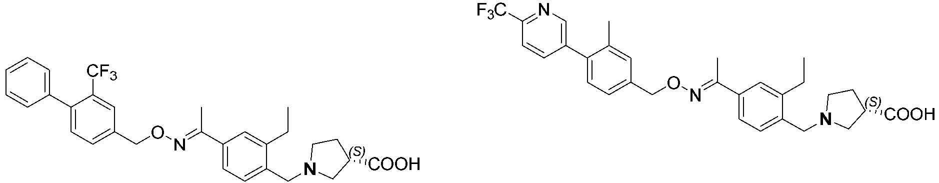

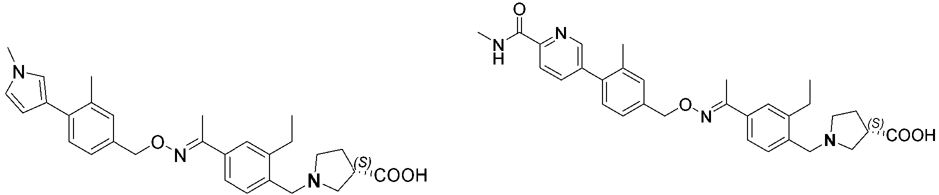

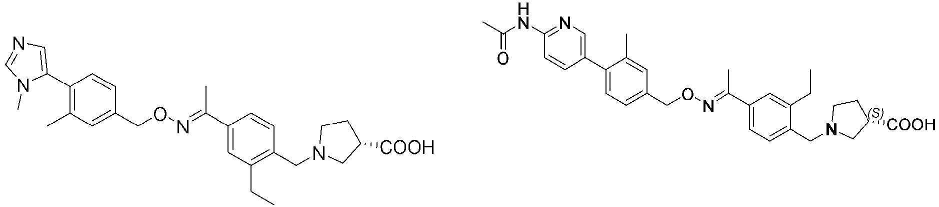

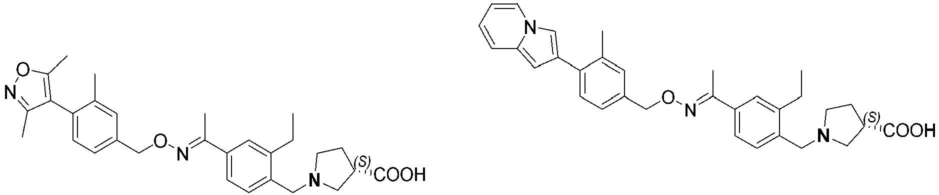

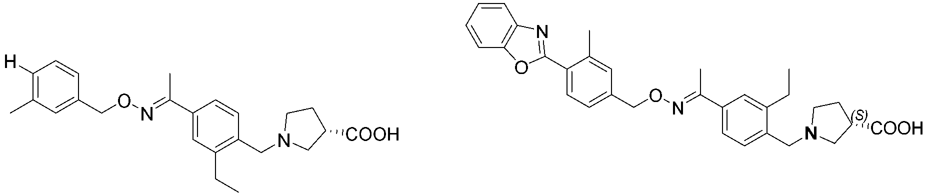

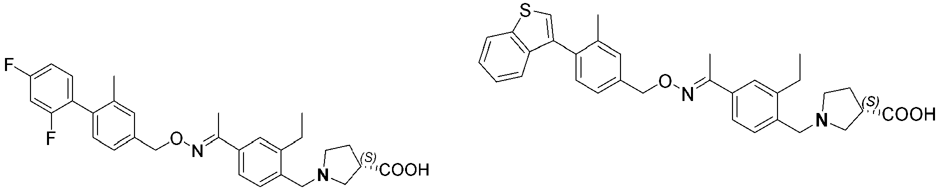

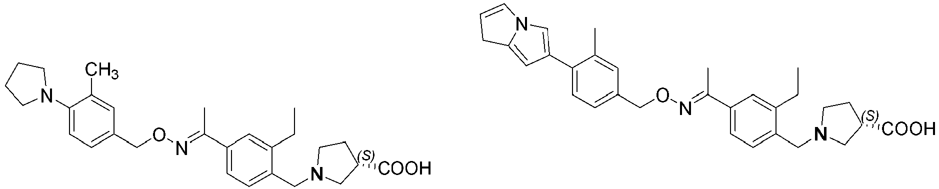

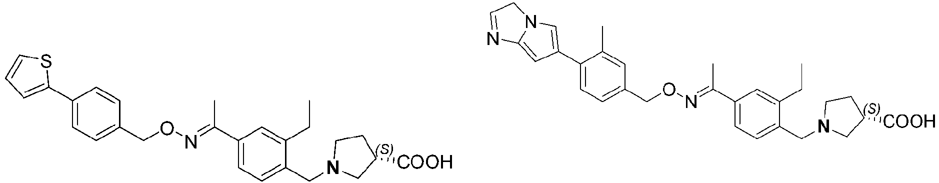

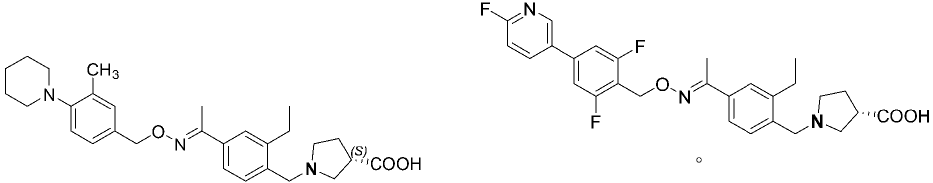

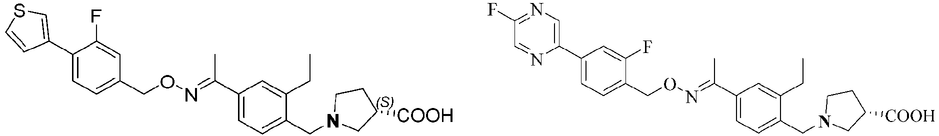

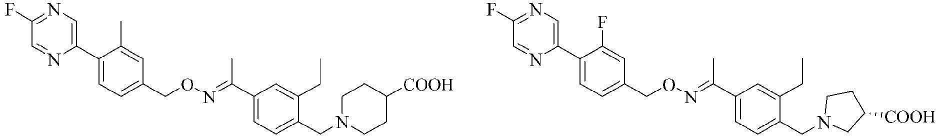

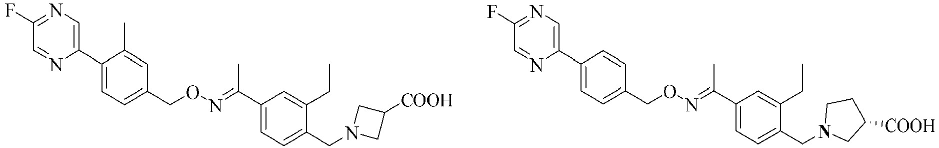

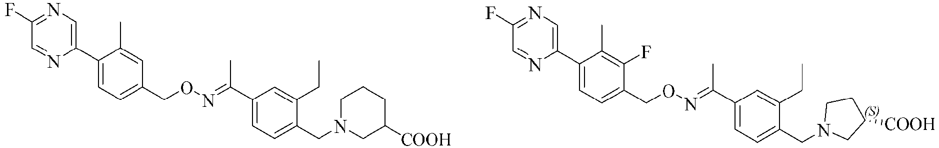

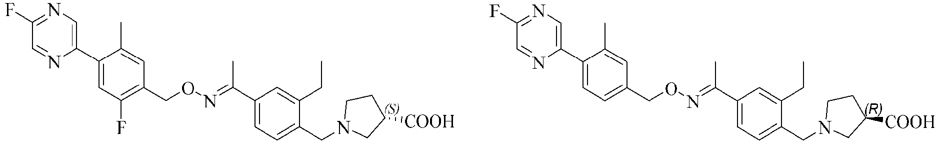

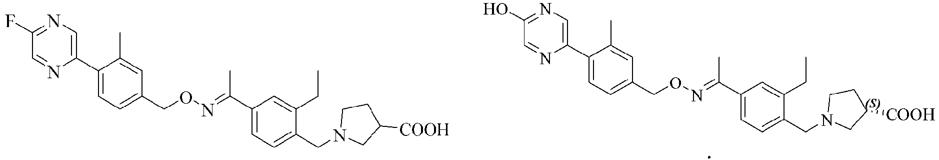

5. The compound of claim 3, wherein in Formula III,
R¹ is selected from the group consisting of: a hydrogen, halogen (preferably F, Cl, or Br), cyano, substituted or unsubstituted C₁-C₅ alkoxy (preferably a methoxy), substituted or unsubstituted C₁-C₆ alkyl (preferably a methyl, ethyl, propyl);
R² is: 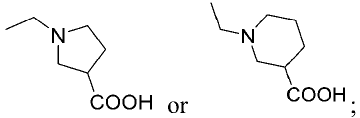
R³ and R⁵ are independently selected from the group consisting of a hydrogen, substituted or unsubstituted C₁-C₆ alkyl (preferably a methyl, ethyl or propyl), and halogen (preferably F);
R⁴ is selected from the group consisting of a substituted or unsubstituted C₅-C₈ aryl (preferably a phenyl or F-substituted phenyl), substituted or unsubstituted five-membered or six-membered heterocyclic group or heteroaryl containing 1-2 heteroatoms selected from N, O or S.

6. A compound selected from the following group, or an optical isomer or a pharmaceutically acceptable salt thereof:

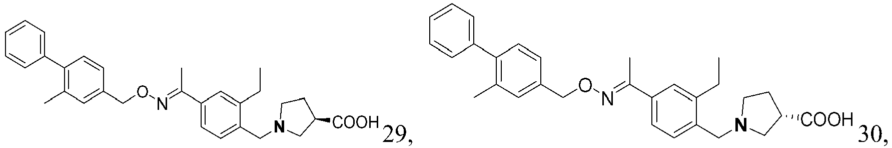

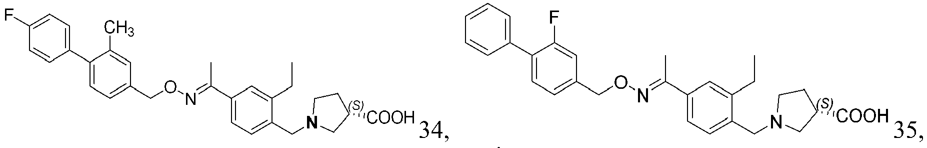

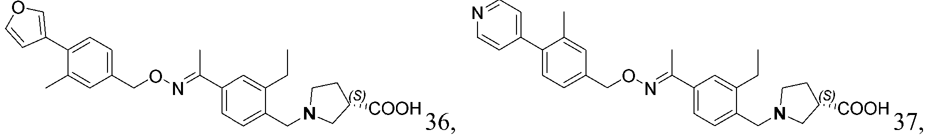

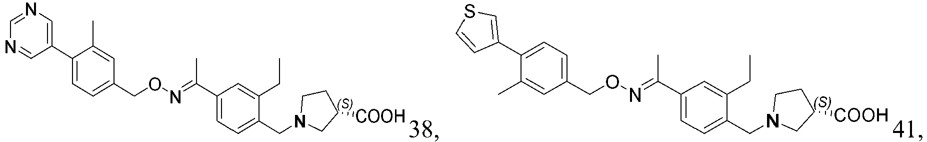

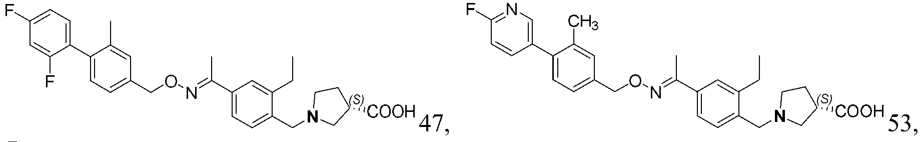

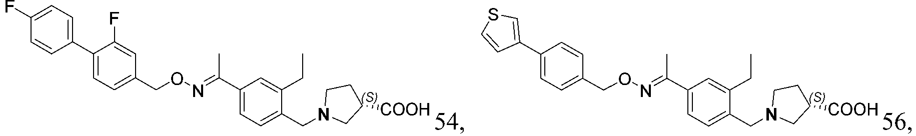

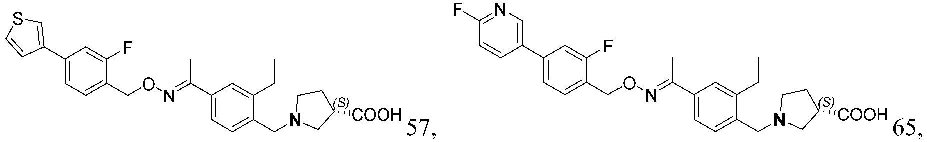

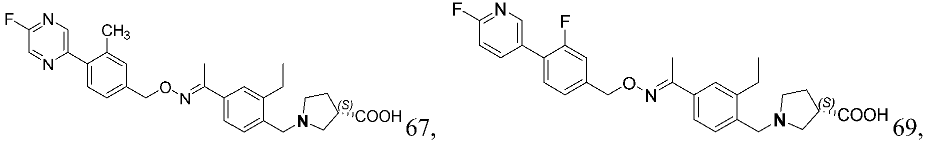

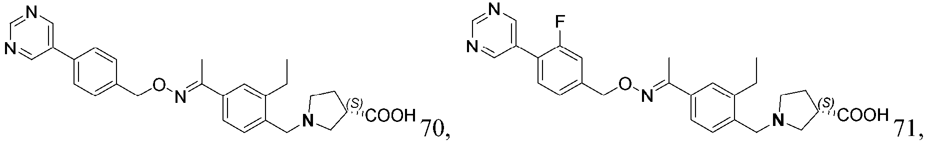

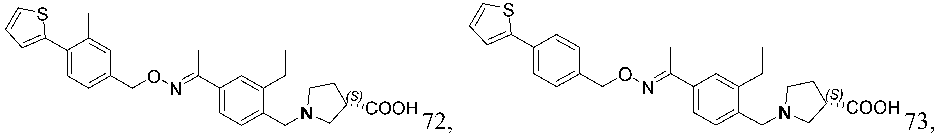

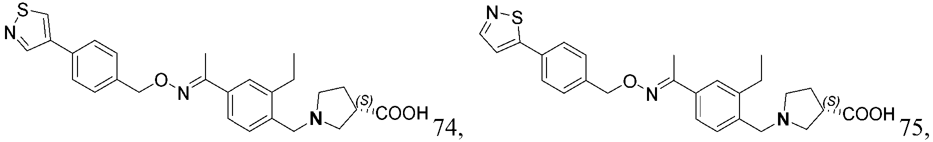

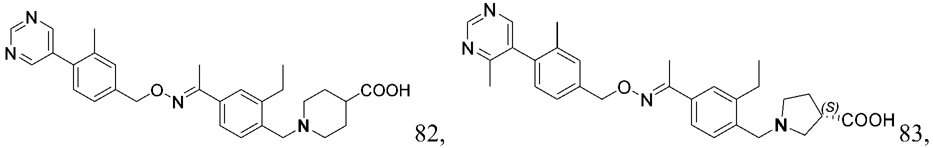

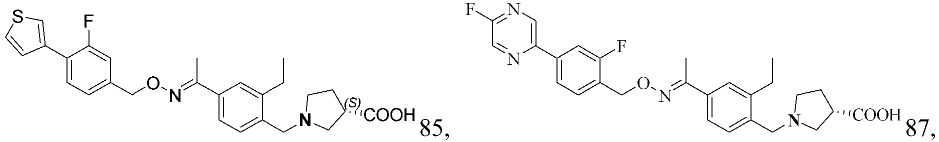

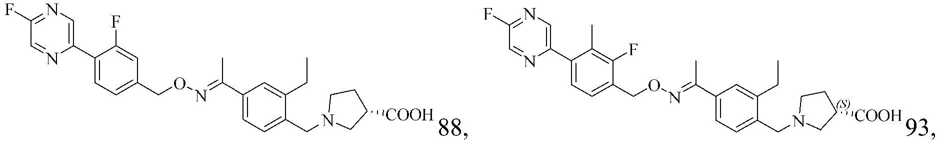

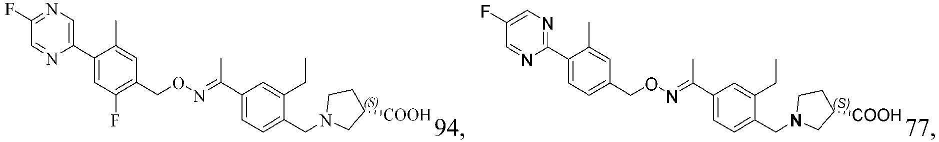

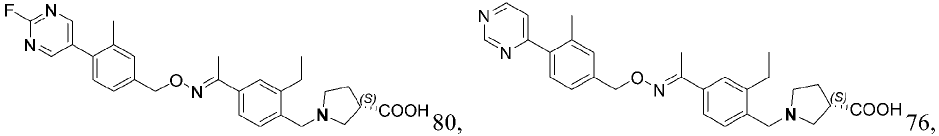

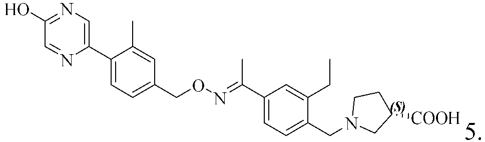

7. The compound of claim 3, wherein in Formula III,
R¹ is selected from the group consisting of: a cyano, substituted or unsubstituted C₁-C₅ alkoxy (preferably a methoxy), substituted or unsubstituted C₁-C₆ alkyl (preferably a methyl, ethyl, propyl);
R² is: 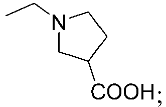
R³ and R⁵ are independently selected from the group consisting of a hydrogen, substituted or unsubstituted C₁-C₃ alkyl (preferably a methyl, ethyl or propyl), and halogen (preferably F);
R⁴ is selected from the group consisting of a substituted or unsubstituted C₅-C₈ aryl (preferably a phenyl or F-substituted phenyl), and substituted or unsubstituted five-membered or six-membered heteroaryl containing 1-2 heteroatoms selected from N, O or S.

8. A compound selected from the following group, or an optical isomer or a pharmaceutically acceptable salt thereof:

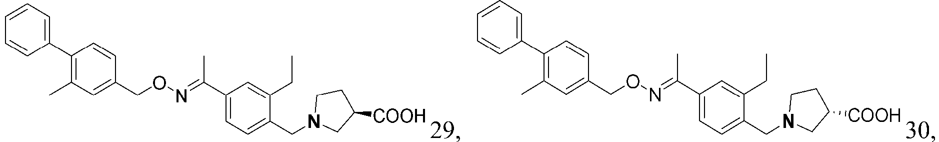

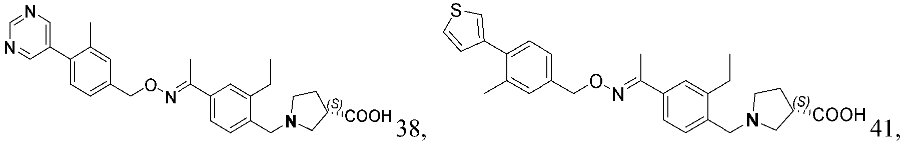

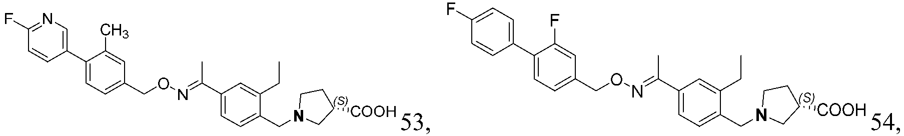

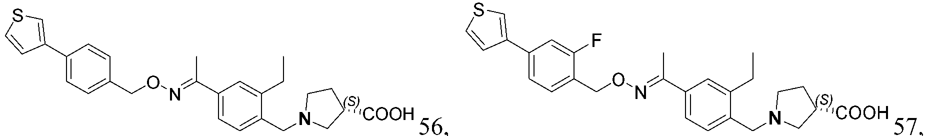

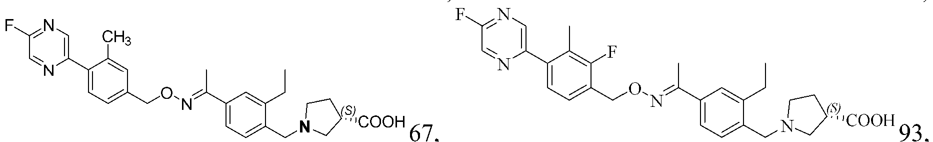

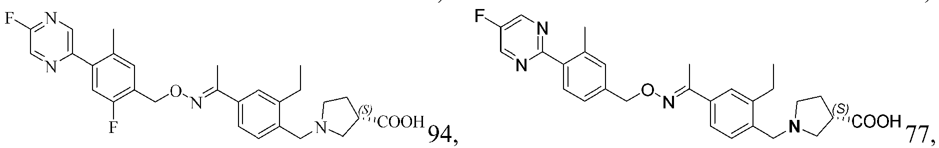

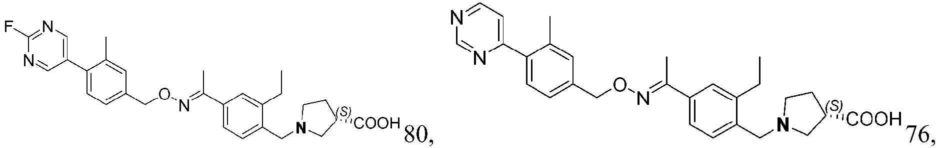

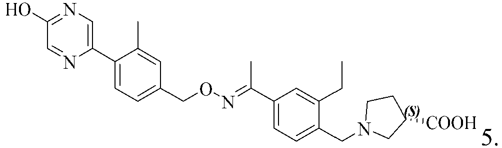

9. A pharmaceutical composition, comprising the compound according to any one of claims 1-8, or an optical isomer or a pharmaceutically acceptable salt thereof, as well as a pharmaceutically acceptable carrier or excipient.

10. Use of the compound according to any one of claims 1-8, or an optical isomer thereof or a pharmaceutically acceptable salt thereof, in the preparation of an S1PR regulator. In a preferred embodiment, the S1PR regulator is a selective agonist of S1PR4 or a selective agonist of S1PR4 mutant.

11. A method for regulating S1PR in a subject, comprising the step of administering an effective amount of the compound according to any one of claims 1-8, or an optical isomer thereof, or a pharmaceutically acceptable salt thereof, or the pharmaceutical composition according to claim 9 to a subject in need thereof.

12. The compound according to any one of claims 1-8, or an optical isomer, pharmaceutically acceptable salt thereof, or the pharmaceutical composition according to claim 9, for treating or preventing diseases mediated by S1PR4.

13. A medicament comprising the compound according to any one of claims 1-8, or an optical isomer thereof, a pharmaceutically acceptable salt thereof, or the pharmaceutical composition according to claim 9, for treating or preventing diseases mediated by S1PR4.
